# EUROPÄISCHE PATENTSCHRIFT

(11) **EP 1 286 673 B1**
(45) Veröffentlichungstag und Bekanntmachung des Hinweises auf die Patenterteilung: **25.05.2005**
(21) Anmeldenummer: 01945258.0
(22) Anmeldetag: 06.06.2001
(51) Int. Cl.: A61K 31/55, A61K 31/5513, C07K 5/023, C07D 277/08, C07D 417/12, C07D 333/38, C07D 263/48, C07D 409/12, C07D 413/12, C07D 307/68, A61P 9/00, A61P 31/00, A61P 35/00, A61P 19/00, A61P 29/00, A61P 17/00

(54) **LIGANDEN VON INTEGRINREZEPTOREN**
LIGANDS OF INTEGRIN RECEPTORS
LIGANDS DE RECEPTEURS D'INTEGRINE

(30) Priorität: 06.06.2000 DE 10027514
(43) Veröffentlichungstag der Anmeldung: 05.03.2003
(73) Patentinhaber: Basf Aktiengesellschaft, 67061 Ludwigshafen (DE)
(72) Erfinder: GENESTE, Hervé, 67141 Neuhofen (DE); KLING, Andreas, 68239 Mannheim (DE); LANGE, Udo, 68163 Mannheim (DE); SEITZ, Werner, 68723 Plankstadt (DE); GRAEF, Claudia, Isabella, 68161 Mannheim (DE); SUBKOWSKI, Thomas, 68526 Ladenburg (DE); HORNBERGER, Wilfried, 67434 Neustadt (DE); LAUTERBACH, Arnulf, 67067 Ludwigshafen (DE)
(74) Vertreter: Grünecker, Kinkeldey, Stockmair & Schwanhäusser Anwaltssozietät
(86) Internationale Anmeldenummer: PCT/EP2001/006397
(87) Internationale Veröffentlichungsnummer: WO 2001/093840

(56) Entgegenhaltungen:
- WO-A-96/26190
- WO-A-98/14192
- WO-A-99/05107
- WO-A-99/06049
- US-A- 5 606 054

## Beschreibung

Die Erfindung betrifft die Verwendung von cyclischen Verbindungen als Liganden von Integrinrezeptoren, insbesondere als Liganden des α_{V}β₃-Integrinrezeptors, die neuen Verbindungen selbst, deren Verwendung, sowie Arzneimittelzubereitungen, enthaltend diese Verbindungen.

Integrine sind Zelloberflächen-Glycoproteinrezeptoren, die Wechselwirkungen zwischen gleichartigen und unterschiedlichen Zellen sowie zwischen Zellen und extrazellulären Matrixproteinen vermitteln. Sie sind an physiologischen Prozessen, wie z.B. Embryogenese, Hämostase, Wundheilung, Immunantwort und Bildung/Aufrechterhaltung der Gewebearchitektur beteiligt.

Störungen in der Genexpression von Zelladhäsionsmolekülen sowie Funktionsstörungen der Rezeptoren können zur Pathogenese vieler Erkrankungen, wie beispielsweise Tumore, thromboembolische Ereignisse, kardiovaskuläre Erkrankungen, Lungenkrankheiten, Erkrankungen des ZNS, der Niere, des Gastrointestinaltraktes oder Entzündungen beitragen.

Integrine sind Heterodimere aus jeweils einer α- und einer β-Transmembran-Untereinheit, die nicht-kovalent verbunden sind. Bisher wurden 16 verschiedene α- und 8 verschiedene β-Untereinheiten und 22 verschiedene Kombinationen identifiziert.

Integrin αᵥβ₃, auch Vitronectinrezeptor genannt, vermittelt die Adhäsion an eine Vielzahl von Liganden - Plasmaproteine, extrazelluläre Matrixproteine, Zelloberflächenproteine -, von denen der Großteil die Aminosäuresequenz RGD enthält (Cell, 1986, 44, 517-518; Science 1987, 238, 491-497), wie beispielsweise Vitronectin, Fibrinogen, Fibronectin, von Willebrand Faktor, Thrombospondin, Osteopontin, Laminin, Collagen, Thrombin, Tenascin, MMP-2, bone-sialo-Protein II, verschiedene virale, pilzliche, parasitäre und bakterielle Proteine, natürliche Integrin-Antagonisten wie Disintegrine, Neurotoxine - Mambin - und Blutegelproteine - Decorsin, Ornatin - sowie einige nicht-RGD-Liganden, wie beispielsweise Cyr-61 und PECAM-1 (L. Piali, J. Cell Biol. 1995, 130, 451-460; Buckley, J. Cell Science 1996, 109, 437-445, J. Biol. Chem. 1998, 273, 3090-3096).

Mehrere Integrinrezeptoren zeigen Kreuzreaktivität mit Liganden, die das RGD-Motiv enthalten. So erkennt Integrin α_{IIb}β₃, auch Plättchen-Fibrinogen-Rezeptor genannt, Fibronectin, Vitronectin, Thrombospondin, von Willebrand Faktor und Fibrinogen.

Integrin αᵥβ₃ ist u.a. exprimiert auf Endothelzellen, Blutplättchen, Monocyten/Makrophagen, Glattmuskelzellen, einigen B-Zellen, Fibroblasten, Osteoclasten und verschiedenen Tumorzellen, wie beispielsweise Melanome, Glioblastome, Lungen-, Brust-, Prostata- und Blasenkarzinome, Osteosarkome oder Neuroblastome.

Eine erhöhte Expression beobachtet man unter verschiedenen pathologischen Bedingungen, wie beispielsweise im prothrombotischen Zustand, bei Gefäßverletzung, Tumorwachstum oder -metastasierung oder Reperfusion und auf aktivierten Zellen, insbesondere auf Endothelzellen, Glattmuskelzellen oder Makrophagen.

Eine Beteiligung von Integrin αᵥβ₃ ist unter anderem bei folgenden Krankheitsbildern nachgewiesen:
Kardiovaskuläre Erkrankungen wie Atherosklerose, Restenose nach Gefäßverletzung, und Angioplastie (Neointimabildung, Glattmuskelzellmigration und Proliferation) (J. Vasc. Surg. 1994, 19, 125-134; Circulation 1994, 90, 2203-2206),
akutes Nierenversagen (Kidney Int. 1994, 46, 1050-1058; Proc. Natl. Acad. Sci. 1993, 90, 5700-5704; Kidney Int. 1995, 48, 1375-1385),
Angiogenese-assoziierte Mikroangiopathien wie beispielsweise diabetische Retinopathie oder rheumatische Arthritis (Ann. Rev. Physiol 1987, 49, 453-464; Int. Ophthalmol. 1987, 11, 41-50; Cell 1994, 79, 1157-1164; J. Biol. Chem. 1992, 267, 10931-10934),
arterielle Thrombose,
Schlaganfall (Phase II Studien mit ReoPro, Centocor Inc., 8th annual European Stroke Meeting),
Krebserkrankungen, wie beispielsweise bei der Tumormetastasierung oder beim Tumorwachstum (tumorinduzierte Angiogenese) (Cell 1991, 64, 327-336; Nature 1989, 339, 58-61; Science 1995, 270, 1500-1502),
Osteoporose (Knochenresorption nach Proliferation, Chemotaxis und Adhäsion von Osteoclasten an Knochenmatrix) (FASEB J. 1993, 7, 1475-1482; Exp. Cell Res. 1991, 195, 368-375, Cell 1991, 64, 327-336),
Bluthochdruck (Am. J. Physiol. 1998, 275, H1449 - H1454),
Psoriasis (Am. J. Pathol. 1995, 147, 1661-1667),
Hyperparathyroismus,
Paget'sche Erkrankung (J. Clin. Endocrinol. Metab. 1996, 81, 1810 - 1820),
maligne Hypercalcemie (Cancer Res. 1998, 58, 1930 - 1935),
metastatische osteolytische Läsionen (Am. J. Pathol. 1997, 150, 1383 - 1393),
Pathogen-Protein (z.B. HIV-1 tat) induzierte Prozesse (z.B. Angiogenese, Kaposi's Sarkom) (Blood 1999, 94, 663 - 672)
Entzündung (J. Allergy Clin. Immunol. 1998, 102, 376 - 381),
Herzinsuffizienz, CHF, sowie bei
anti-viraler, anti-parasitärer, anti-pilzliche oder anti-bakterieller Therapie und Prophylaxe (Adhäsion und Internalisierung) (J. Infect. Dis. 1999, 180, 156 - 166; J. Virology 1995, 69, 2664 - 2666; Cell 1993, 73, 309 - 319).

Aufgrund seiner Schlüsselrolle sind pharmazeutische Zubereitungen, die niedermolekulare Integrin αᵥβ₃ Liganden enthalten, u.a. in den genannten Indikationen von hohem therapeutischen bzw. diagnostischen Nutzen.

Vorteilhafte αᵥβ₃-Integrinrezeptorliganden binden an den Integrin αᵥβ₃ Rezeptor mit einer erhöhten Affinität.

Besonders vorteilhafte αᵥβ₃-Integrinrezeptorliganden weisen gegenüber dem Integrin αᵥβ₃ zusätzlich eine erhöhte Selektivität auf und sind bezüglich des Integrins α_{IIb}β₃ mindestens um den Faktor 10 weniger wirksam, bevorzugt mindestens um den Faktor 100.

Für eine Vielzahl von Verbindungen, wie anti-αᵥβ₃ monoklonale Antikörper, Peptide, die die RGD-Bindungssequenz enthalten, natürliche, RGD-enthaltenden Proteine (z.B. Disintegrine) und niedermolekulare Verbindungen ist eine Integrin αᵥβ₃ antagonistische Wirkung gezeigt und ein positiver in vivo Effekt nachgewiesen worden (FEBS Letts 1991, 291, 50-54; J. Biol. Chem. 1990, 265, 12267-12271; J. Biol. Chem. 1994, 269, 20233-20238; J. Cell Biol 1993, 51, 206-218; J. Biol. Chem. 1987, 262, 17703-17711; Bioorg. Med. Chem. 1998, 6, 1185-1208).

Antagonisten des α_{V}β₃-Integrinrezeptors auf Basis eines bicyclischen Strukturelements sind in WO 9906049, WO 9905107, WO 9814192, WO 9724124, WO 9724122 und WO 9626190 beschrieben.

EP 540 334 und WO 9308174 beschreiben bicyclische Antagonisten des α_{IIb}β₃-Integrinrezeptors.

WO 9407488 A1 beschreibt Verbindungen mit bicyclischem Molekülgerüst, die die Freigabe des Wachstumshormons beschleunigen.

Ferner sind Vasopressin-Antagonisten mit bicyclischem Molekülgerüst in den Schriften EP 620216, WO 9534540, WO 9408582, WO 9802432, WO 9420473, JP 09221476 A1, JP 11060488 A1, WO 9404525, JP 04321669 A1, WO 9722591, sowie in Matsuhisa et al., Chem. Pharm. Bull. 1999, 47, 3, 329-339 beschrieben.

Der Erfindung lag die Aufgabe zugrunde, neue Integrinrezeptorliganden mit vorteilhaften Eigenschaften zur Verfügung zu stellen.

Die Erfindung betrifft daher Arzneimittel enthaltend Verbindungen der Formel I

B-G-L I

als Liganden von Integrinrezeptoren,
wobei B, G und L folgende Bedeutung haben:
L ein Strukturelement der Formel I_{L}

   -U-T I_{L}
wobei
- T: eine Gruppe COOH, ein zu COOH hydrolisierbarer Rest oder ein zu COOH bioisosterer Rest und
- -U-: -(X_{L})ₐ-(CR_{L}¹R_{L}²)_{b}-, -CR_{L}¹=CR_{L}²-, Ethinylen oder =CR_{L}¹⁻ bedeuten, wobei
a 0 oder 1,
b 0, 1 oder 2
X_{L} CR_{L}³R_{L}⁴, NR_{L}⁵, Sauerstoff oder Schwefel,
R_{L}¹, R_{L}², R_{L}³, R_{L}⁴ unabhängig voneinander Wasserstoff, -T, -OH, -NR_{L}⁶R_{L}⁷, -CO-NH₂, einen Halogenrest, einen verzweigten oder unverzweigten, gegebenenfalls substituierten C₁-C₆-Alkyl-, C₂-C₆-Alkenyl-, C₂-C₆-Alkinyl-, C₃-C₇-Cycloalkyl-, -CO-NH(C₁-C₆-Alkyl), -CO-N(C₁-C₆-Alkyl)₂ oder C₁-C₄-Alkoxyrest, einen gegebenenfalls substituierten Rest C₁-C₂-Alkylen-T, C₂-Alkenylen-T oder C₂-Alkinylen-T, einen gegebenenfalls substituierten Aryl- oder Arylalkylrest oder jeweils unabhängig voneinander zwei Reste R_{L}¹ und R_{L}² oder R_{L}³ und R_{L}⁴ oder gegebenenfalls R_{L}¹ und R_{L}³ zusammen einen, gegebenenfalls substituierten 3 bis 7 gliedrigen gesättigten oder ungesättigten Carbocyclus oder Heterocyclus, der bis zu drei verschiedene oder gleiche Heteroatome O, N, S enthalten kann,
R_{L}⁵, R_{L}⁶, R_{L}⁷ unabhängig voneinander Wasserstoff, einen verzweigten oder unverzweigten, gegebenenfalls substituierten C₁-C₆-Alkyl-, C₃-C₇-Cycloalkyl-, CO-O-C₁-C₆-Alkyl-, SO₂-C₁-C₆-Alkyl- oder CO-C₁-C₆-Alkylrest oder einen, gegebenenfalls substituierten CO-O-Alkylen-Aryl-, SO₂-Aryl-, CO-Aryl-, SO₂-Alkylen-Aryl- oder CO-Alkylen-Arylrest,
bedeuten,
- G: ein Strukturelement der Formel I_{G} 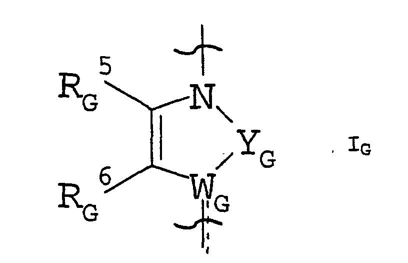
wobei
das Strukturelement B über den Ringstickstoff und das Strukturelement L über W_{G} an das Strukturelement G gebunden ist,
- Y_{G}: CO, CS, C=NR_{G}² oder CR_{G}³R_{G}⁴,
- R_{G}²: Wasserstoff, eine Hydroxy-Gruppe, einen verzweigten oder unverzweigten, gegebenenfalls substituierten C₁-C₆-Alkyl-, C₁-C₄-Alkoxy-, C₃-C₇-Cycloalkyl- oder -O-C₃-C₇-Cycloalkylrest oder einen gegebenenfalls substituierten Aryl-, -O-Aryl, Arylalkyl- oder -O-Alkylen-Arylrest,
- R_{G}³, R_{G}⁴: unabhängig voneinander Wasserstoff oder einen verzweigten oder unverzweigten, gegebenenfalls substituierten C₁-C₆-Alkyl-, C₂-C₆-Alkenyl-, C₂-C₆-Alkinyl- oder C₁-C₄-Alkoxyrest oder beide Reste R_{G}³ und R_{G}⁴ zusammen ein cyclisches Acetal -O-CH₂-CH₂-O- oder -O-CH₂-O- oder beide Reste R_{G}³ und R_{G}⁴ zusammen einen, gegebenenfalls substituierten C₃-C₇-Cycloalkylrest,
- R_{G}⁵ und R_{G}⁶: unabhängig voneinander Wasserstoff, eine Hydroxygruppe, einen verzweigten oder unverzweigten, gegebenenfalls substituierten C₁-C₆-Alkyl- oder C₁-C₄-Alkoxyrest, einen gegebenfalls substituierten Aryl- oder Arylalkylrest oder beide Reste R_{G}⁵ und R_{G}⁶ zusammen einen, gegebenenfalls substituierten, anelierten, ungesättigten oder aromatischen 3- bis 10-gliedrigen Carbocyclus oder Heterocyclus, der bis zu drei verschiedene oder gleiche Heteroatome O, N, S enthalten kann,
- W_{G}: ein Strukturelement ausgewählt aus der Gruppe der Strukturelemente der Formeln I_{WG}¹ bis I_{WG}⁴, 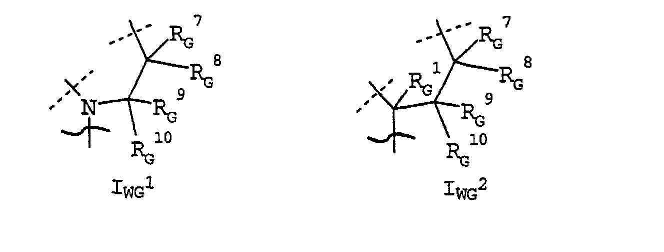 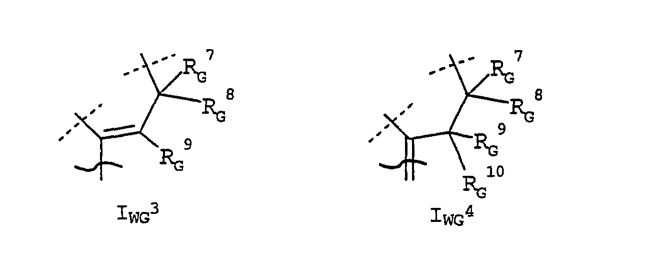
- R_{G}¹: Wasserstoff, Halogen, eine Hydroxy-Gruppe oder einen verzweigten oder unverzweigten, gegebenenfalls substituierten C₁-C₆-Alkyl- oder C₁-C₄-Alkoxyrest,
- R_{G}⁷, R_{G}⁸, R_{G}⁹, R_{G}¹⁰: unabhängig voneinander Wasserstoff, eine Hydroxygruppe, -CN, Halogen, einen verzweigten oder unverzweigten, gegebenenfalls substituierten C₁-C₆-Alkyl-, C₂-C₆-Alkenyl-, C₂-C₆-Alkinyl-, C₁-C₄-Alkylen-C₃-C₇-Cycloalkyl-, C₁-C₄-Alkylen-C₃-C₇-Heterocycloalkyl- oder C₁-C₄-Alkylen-C₃-C₇-Heterocycloalkenylrest, einen verzweigten oder unverzweigten, gegebenenfalls substituierten Rest C₁-C₄-Alkylen-OR_{G}¹¹, C₁-C₄-Alkylen-CO-OR_{G}¹¹, C₁-C₄-Alkylen-O-CO-R_{G}¹¹, C₁-C₄-Alkylen-CO-R_{G}¹¹, C₁-C₄-Alkylen-SO₂-NR_{G}¹²R_{G}¹³, C₁-C₄-Alkylen-CO-NR_{G}¹²R_{G}¹³, C₁-C₄-Alkylen-O-CO-NR_{G}¹²R_{G}¹³, C₁-C₄-Alkylen-NR_{G}¹²R_{G}¹³ oder C₁-C₄-Alkylen-SR_{G}¹¹, C₁-C₄-Alkylen-SO-R_{G}¹¹, einen Rest -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G}¹²R_{G}¹³, -SO₂-NR_{G}¹²R_{G}¹³, -CO-NR_{G}¹²R_{G}¹³, -NR_{G}¹²R_{G}¹³ oder CO-R_{G}¹¹, einen gegebenenfalls substituierten C₃-C₇-Cycloalkyl-, C₃-C₇-Heterocycloalkyl-, C₃-C₇-Heterocycloalkenyl-, Aryl-, Hetaryl-, Arylalkyl- oder Hetarylalkylrest oder jeweils unabhängig voneinander zwei Reste R_{G}⁷ und R_{G}⁹ oder R_{G}⁸ und R_{G}¹⁰ oder R_{G}⁷ und R_{G}⁸ oder R_{G}⁹ und R_{G}¹⁰ zusammen einen, gegebenenfalls substituierten, gesättigten oder ungesättigten, nicht aromatischen, 3 bis 7 gliedrigen Carbocyclus oder Heterocyclus der bis zu 3 Heteroatome ausgewählt aus der Gruppe O, N, S und bis zu zwei Doppelbindungen enthalten kann,
- R_{G}¹¹: Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest,
- R_{G}¹², R_{G}¹³: unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NRG¹¹RG¹¹* oder -CO-R_{G}¹¹ und
- R_{G}¹¹*: einen von R_{G}¹¹ unabhängigen Rest R_{G}¹¹
bedeuten,
- B: ein Strukturelement, enthaltend mindestens ein Atom das unter physiologischen Bedingungen als Wasserstoff-Akzeptor Wasserstoffbrücken ausbilden kann, wobei mindestens ein Wasserstoff-Akzeptor-Atom entlang des kürzestmöglichen Weges entlang des Strukturelementgerüstes einen Abstand von 5 bis 14 Atombindungen zu Strukturelement G aufweist,
sowie die physiologisch verträglichen Salze, Prodrugs und die enantiomerenreinen oder diastereomerenreinen und tautomeren Formen.

In Strukturelement L wird unter T eine Gruppe COOH, ein zu COOH hydrolisierbarer Rest oder ein zu COOH bioisosterer Rest verstanden.

Unter einem zu COOH hydrolisierbaren Rest wird ein Rest verstanden, der nach Hydrolyse in eine Gruppe COOH übergeht.

Beispielhaft sei für einen zu COOH hydrolisierbaren Rest T die Gruppe 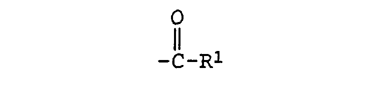 erwähnt, in der R¹ die folgende Bedeutung hat:
a) OM, wobei M ein Metallkation, wie ein Alkalimetallkation, wie Lithium, Natrium, Kalium, das Äquivalent eines Erdalkalimetallkations, wie Calcium, Magnesium und Barium oder ein umweltverträgliches organisches Ammoniumion wie beispielsweise primäres, sekundäres, tertiäres oder quartäres C₁-C₄-Alkylammonium oder Ammoniumion sein kann, wie beispielsweise ONa, OK oder OLi,
b) ein verzweigter oder unverzweigter, gegebenenfalls mit Halogen substituierter C₁-C₈-Alkoxyrest, wie beispielsweise Methoxy, Ethoxy, Propoxy, 1-Methylethoxy, Butoxy, 1-Methylpropoxy, 2-Methylpropoxy, 1,1-Dimethylethoxy, insbesondere Methoxy, Ethoxy, 1-Methylethoxy, Pentoxy, Hexoxy, Heptoxy, Octoxy, Difluormethoxy, Trifluormethoxy, Chlordifluormethoxy, 1-Fluorethoxy, 2-Fluorethoxy, 2,2-Difluorethoxy, 1,1,2,2-Tetrafluorethoxy, 2,2,2-Trifluorethoxy, 2-Chlor-1,1,2-trifluorethoxy oder Pentafluorethoxy
c) ein verzweigter oder unverzweigter, gegebenenfalls mit Halogen substituierten C₁-C₄-Alkylthiorest wie Methylthio, Ethylthio, Propylthio, 1-Methylethylthio, Butylthio, 1-Methylpropylthio, 2-Methylpropylthio oder 1,1-Dimethylethylthiorest
d) ein gegebenenfalls substituierter -O-Alkylen-Arylrest, wie beispielsweise -O-Benzyl
e) R¹ ferner ein Rest -(O)ₘN(R¹⁸)(R¹⁹),
   in dem m für 0 oder 1 steht und R¹⁸ und R¹⁹, die gleich oder unterschiedlich sein können, die folgende Bedeutung haben:
   Wasserstoff,
   einen verzweigten oder unverzweigten, gegebenenfalls substituierten
      C₁-C₆-Alkylrest, wie beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl oder 1-Ethyl-2-methylpropyl oder die entsprechenden substituierten Reste, vorzugsweise Methyl, Ethyl, Propyl, Butyl oder i-Butyl,
      C₂-C₆-Alkenylrest, wie beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl oder 3-Methyl-2-pentenyl oder die entsprechenden substituierten Reste,
      C₂-C₆-Alkinylrest, wie beispielsweise Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise 2-Propinyl, 2-Butinyl, 1-Methyl-2-propinyl oder 1-Methyl-2-butinyl oder die entsprechenden substituierten Reste,
      C₃-C₈-Cycloalkyl, wie beispielsweise Cyclopropyl, Cyclobutyl, Cyclopentyl, Cyclohexyl und Cycloheptyl, Cyclooctyl oder die entsprechenden substituierten Reste,
      oder einen Phenylrest, gegebenenfalls ein- oder mehrfach, beispielsweise ein- bis dreifach substituiert durch Halogen, Nitro, Cyano, C₁-C₄-Alkyl, C₁-C₄-Halogenalkyl, C₁-C₄-Alkoxy, C₁-C₄-Halogenalkoxy oder C₁-C₄-Alkylthio wie beispielsweise 2-Fluorphenyl, 3-Chlorphenyl, 4-Bromphenyl, 2-Methylphenyl, 3-Nitrophenyl, 4-Cyanophenyl, 2-Trifluormethylphenyl, 3-Methoxyphenyl, 4-Trifluorethoxyphenyl, 2-Methylthiophenyl, 2,4-Dichlorphenyl, 2-Methoxy-3-methylphenyl, 2,4-Dimethoxyphenyl, 2-Nitro-5-cyanophenyl, 2,6-Difluorphenyl,
      oder R¹⁸ und R¹⁹ bilden gemeinsam eine zu einem Cyclus geschlossene, gegebenenfalls substituierte, z.B. durch C₁-C₄-Alkyl substituierte C₄-C₇-Alkylenkette, die ein Heteroatom, ausgewählt aus der Gruppe Sauerstoff, Schwefel oder Stickstoff, enthalten kann wie beispielsweise -(CH₂)₄-, -(CH₂)₅-, -(CH₂)₆-, -(CH₂)₇-, -(CH₂)₂-O-(CH₂)₂-, -CH₂-S-(CH₂)₃-, -(CH₂)₂-O-(CH₂)₃-, -NH-(CH₂)₃-, -CH₂-NH-(CH₂)₂-, -CH₂-CH=CH-CH₂-, -CH=CH-(CH₂)₃-, -CO-(CH₂)₂-CO- oder -CO-(CH₂)₃-CO-.

Unter einem zu COOH bioisosteren Rest werden Reste verstanden, die in Wirkstoffen die Funktion einer Gruppe COOH durch äquivalente Bindungsdonor/Akzeptorfähigkeiten oder durch äquivalente Ladungsverteilung ersetzen können.

Beispielhaft seien als zu -COOH bioisostere Reste die Reste, wie in "The Practice of Medicinal Chemistry, Editor: C.G. Wermuth, Academic Press 1996, Seite 125 und 216 beschrieben genannt, insbesondere die Reste -P=O(OH)₂, -SO₃H, Tetrazol oder Acylsulfonamide.

Bevorzugte Reste T sind -COOH, -CO-O-C₁-C₈-Alkyl oder -CO-O-Benzyl.

Der Rest -U- in Strukturelement L stellt einen Spacer, ausgewählt aus der Gruppe -(X_{L})ₐ-(CR_{L}¹R_{L}²)_{b}-, -CR_{L}¹=CR_{L}²-, Ethinylen oder =CR_{L}¹- dar. Im Fall des Restes =CR_{L}¹- ist das Strukturelement L mit dem Strukturelement G über eine Doppelbindung verknüpft.

X_{L} bedeutet einen Rest CR_{L}³R_{L}⁴, NR_{L}⁵, Sauerstoff oder Schwefel.

Bevorzugte Reste -U- sind die Reste -CR_{L}¹=CR_{L}²-, Ethinylen oder -(X_{L})ₐ-(CR_{L}¹R_{L}²)_{b}-, wobei X_{L} vorzugsweise CR_{L}³R_{L}⁴ (a = 0 oder 1) oder Sauerstoff (a = 1) bedeutet.

Besonders bevorzugte Reste -U- sind die Reste -(X_{L})ₐ-(CR_{L}¹R_{L}²)_{b}-, wobei X_{L} vorzugsweise CR_{L}³R_{L}⁴ (a = 0 oder 1) oder Sauerstoff (a = 1) bedeutet.

Unter einem Halogenrest wird unter R_{L}¹, R_{L}², R_{L}³ oder R_{L}⁴ in Strukturelement L beispielsweise F, Cl, Br oder I, vorzugsweise F verstanden.

Unter einem verzweigten oder unverzweigten C₁-C₆-Alkylrest werden unter R_{L}¹, R_{L}², R_{L}³ oder R_{L}⁴ in Strukturelement L beispielsweise Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl oder 1-Ethyl-2-methylpropyl, vorzugsweise verzweigte oder unverzweigte C₁-C₄-Alkylreste wie beispielsweise Methyl, Ethyl, Propyl, 1-Methylethyl, Butyl, 1-Methylpropyl, 2-Methylpropyl oder 1,1-Dimethylethyl, besonders bevorzugt Methyl verstanden.

Unter einem verzweigten oder unverzweigten C₂-C₆-Alkenylrest werden unter R_{L}¹, R_{L}², R_{L}³ oder R_{L}⁴ in Strukturelement L beispielsweise Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, insbesondere 2-Propenyl, 2-Butenyl, 3-Methyl-2-butenyl oder 3-Methyl-2-pentenyl verstanden.

Unter einem verzweigten oder unverzweigten C₂-C₆-Alkinylrest werden unter R_{L}¹, R_{L}², R_{L}³ oder R_{L}⁴ in Strukturelement L beispielsweise Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, vorzugsweise Ethinyl, 2-Propinyl, 2-Butinyl, 1-Methyl-2-propinyl oder 1-Methyl-2-butinyl verstanden.

Unter einem verzweigten oder unverzweigten C₃-C₇-Cycloalkylrest werden unter R_{L}¹, R_{L}², R_{L}³ oder R_{L}⁴ in Strukturelement L beispielsweise Cyclopropyl, Cyclobutyl, Cyclopentyl, Cyclohexyl oder Cycloheptyl verstanden.

Unter einem verzweigten oder unverzweigten C₁-C₄-Alkoxyrest werden unter R_{L}¹, R_{L}², R_{L}³ oder R_{L}⁴ in Strukturelement L beispielsweise Methoxy, Ethoxy, Propoxy, 1-Methylethoxy, Butoxy, 1-Methylpropoxy, 2-Methylpropoxy oder 1,1-Dimethylethoxy verstanden.

Die Reste -CO-NH(C₁-C₆-Alkyl), -CO-N(C₁-C₆-Alkyl)₂ stellen sekundäre bzw. tertiäre Amide dar und setzten sich aus der Amidbindung und den entsprechenden C₁-C₆-Alkylresten wie vorstehend für R_{L}¹, R_{L}², R_{L}³ oder R_{L}⁴ beschrieben zusammen.

Die Reste R_{L}¹, R_{L}², R_{L}³ oder R_{L}⁴ können weiterhin einen Rest
C₁-C₂-Alkylen-T, wie beispielsweise Methylen-T oder Ethylen-T, C₂-Alkenylen-T, wie beispielsweise Ethenylen-T oder C₂-Alkinylen-T, wie beispielsweise Ethinylen-T,
einen Arylrest, wie beispielsweise Phenyl, 1-Naphthyl oder 2-Naphthyl oder
einen Arylalkylrest, wie beispielsweise Benzyl oder Ethylenphenyl (Homobenzyl)
darstellen, wobei die Reste gegebenenfalls substituiert sein können.

Ferner können jeweils unabhängig voneinander zwei Reste R_{L}¹ und R_{L}² oder R_{L}³ und R_{L}⁴ oder gegebenenfalls R_{L}¹ und R_{L}³ zusammen einen, gegebenenfalls substituierten 3 bis 7 gliedrigen gesättigten oder ungesättigten Carbocyclus oder Heterocyclus, der bis zu drei verschiedene oder gleiche Heteroatome O, N, S enthalten kann, darstellen.

Alle Reste für R_{L}¹, R_{L}², R_{L}³ oder R_{L}⁴ können gegebenenfalls substituiert sein. Für die Reste R_{L}¹, R_{L}², R_{L}³ oder R_{L}⁴ und alle weiteren, nachstehenden substituierten Reste der Beschreibung kommen, wenn die Substituenten nicht näher spezifiziert sind, unabhängig voneinander bis zu 5 Substituenten, beispielsweise ausgewählt aus der folgenden Gruppe in Frage:
-NO₂, -NH₂, -OH, -CN, -COOH, -O-CH₂-COOH, Halogen, einen verzweigten oder unverzweigten, gegebenenfalls substituierten C₁-C₄-Alkyl-, wie beispielsweise Methyl, CF₃, C₂F₅ oder CH₂F, -CO-O-C₁-C₄-Alkyl-, C₃-C₆-Cycloalkyl-, C₁-C₄-Alkoxy-, C₁-C₄-Thioalkyl-, -NH-CO-O-C1-C4-Alkyl, -O-CH₂-COO-C₁-C₄-Alkyl, -NH-CO-C₁-C₄-Alkyl, -CO-NH-C₁-C₄-Alkyl, -NH-SO₂-C₁-C₄-Alkyl, -SO₂-NH-C₁-C₄-Alkyl, -N(C₁-C₄-Alkyl)₂, -NH-C₁-C₄-Alkyl-, oder -SO₂-C₁-C₄-Alkylrest, wie beispielsweise -SO₂-CF₃, einen gegebenfalls substituierten -NH-CO-Aryl-, -CO-NH-Aryl-, -NH-CO-O-Aryl-, -NH-CO-O-Alkylen-Aryl-, -NH-SO₂-Aryl-, -SO₂-NH-Aryl-, -CO-NH-Benzyl-, -NH-SO₂-Benzyl- oder -SO₂-NH-Benzylrest, einen gegebenenfalls substituierten Rest -SO₂-NR²R³ oder -CO-NR²R³ wobei die Reste R² und R³ unabhängig voneinander die Bedeutung wie nachstehend R_{L}⁵ haben können oder beide Reste R² und R³ zusammen einen 3 bis 6 gliedrigen, gegebenenfalls substituierten, gesättigten, ungesättigten oder aromatischen Heterocyclus, der zusätzlich zum Ringstickstoff bis zu drei weitere verschiedene oder gleiche Heteroatome O, N, S enthalten kann, und gegebenenfalls zwei an diesem Heterocyclus substituierte Reste zusammen einen anelierten, gesättigten, ungesättigten oder aromatischen Carbocyclus oder Heterocyclus, der bis zu drei verschiedene oder gleiche Heteroatome O, N, S enthalten kann darstellen und der Cyclus gegebenenfalls substituiert oder an diesem Cyclus ein weiterer, gegebenenfalls substituierter Cyclus ankondensiert sein kann.

Wenn nicht näher spezifiziert, können bei allen endständig gebundenen, substituierten Hetarylresten der Beschreibung zwei Substituenten einen anelierten 5- bis 7 gliedrigen, ungesättigten oder aromatischen Carbocyclus bilden.

Bevorzugte Reste R_{L}¹, R_{L}², R_{L}³ oder R_{L}⁴ sind unabhängig voneinander Wasserstoff, Halogen, ein verzweigter oder unverzweigter, gegebenenfalls substituierter C₁-C₄-Alkyl-, C₁-C₄-Alkoxy-, oder C₃-C₇-Cycloalkylrest oder der Rest -NR_{L}⁶R_{L}⁷.

Besonders bevorzugte Reste R_{L}¹, R_{L}², R_{L}³ oder R_{L}⁴ sind unabhängig voneinander Wasserstoff, Fluor oder ein verzweigter oder unverzweigter, gegebenenfalls substituierter C₁-C₄-Alkylrest, vorzugsweise Methyl.

Die Reste R_{L}⁵, R_{L}⁶, R_{L}⁷ in Strukturelement L bedeuten unabhängig voneinander Wasserstoff, einen verzweigten oder unverzweigten, gegebenenfalls substituierten
C₁-C₆-Alkylrest, beispielsweise wie vorstehend für R_{L}¹ beschrieben,
C₃-C₇-Cycloalkylrest, beispielsweise wie vorstehend für R_{L}¹ beschrieben,
CO-O-C₁-C₆-Alkyl-, SO₂-C₁-C₆-Alkyl- oder CO-C₁-C₆-Alkylrest, der sich aus der Gruppe CO-O, SO₂ oder CO und beispielsweise aus den vorstehend für R_{L}¹ beschriebenen C₁-C₆-Alkylresten zusammensetzt,
oder einen, gegebenenfalls substituierten CO-O-Alkylen-Aryl-, SO₂-Aryl-, SO₂-Alkylen-Aryl- oder CO-Alkylen-Arylrest, der sich aus der Gruppe CO-O, SO₂, oder CO und beispielsweise aus den vorstehend für R_{L}¹ beschriebenen Aryl- oder Arylalkylresten zusammensetzt.

Bevorzugte Reste für R_{L}⁶ in Strukturelement L sind Wasserstoff, ein verzweigter oder unverzweigter, gegebenenfalls substituierter C₁-C₄-Alkyl-, CO-O-C₁-C₄-Alkyl-, CO-C₁-C₄-Alkyl- oder SO₂-C₁-C₄-Alkylrest oder ein gegebenenfalls substituierter CO-O-Benzyl-, SO₂-Aryl-, SO₂-Alkylen-Aryl- oder CO-Arylrest.

Bevorzugte Reste für R_{L}⁷ in Strukturelement L sind Wasserstoff oder ein verzweigter oder unverzweigter, gegebenenfalls substituierter C₁-C₄-Alkylrest.

Bevorzugte Strukturelemente L setzen sich aus den bevorzugten Resten des Strukturelementes zusammen.

Besonders bevorzugte Strukturelemente L setzen sich aus den besonders bevorzugten Resten des Strukturelementes zusammen.

G stellt ein Strukturelement der Formel I_{G} dar, 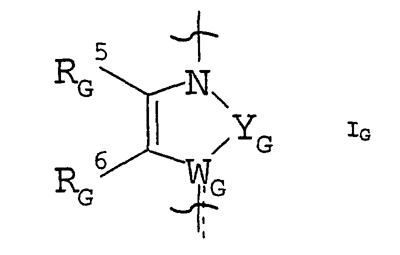 wobei das Strukturelement B über den Ringstickstoff und das Strukturelement L über W_{G} an das Strukturelement G, gegebenenfalls über eine Doppelbindung gebunden ist.

Y_{G} in Strukturelement G bedeutet CO, CS, C=NR_{G}² oder CR_{G}³R_{G}⁴, vorzugsweise CO, C=NR_{G}² oder CR_{G}³R_{G}⁴, besonders bevorzugt CO oder CR_{G}³R_{G}⁴.

R_{G}² in Strukturelement G bedeutet Wasserstoff, eine Hydroxy-Gruppe, einen verzweigten oder unverzweigten, gegebenenfalls substituierten C₁-C₆-Alkyl-, C₁-C₄-Alkoxy- oder C₃-C₇-Cycloalkylrest, beispielsweise wie jeweils vorstehend für R_{L}¹ beschrieben,
einen gegebenenfalls substituierten -O-C₃-C₇-Cycloalkylrest, der sich aus einer Ethergruppe und beispielsweise aus dem vorstehend für R_{L}¹ beschriebenen C₃-C₇-Cycloalkylrest zusammensetzt,
einen gegebenenfalls substituierten Aryl- oder Arylalkylrest, beispielsweise wie jeweils vorstehend für R_{L}¹ beschrieben oder
einen gegebenenfalls substituierten -O-Aryl oder -O-Alkylen-Arylrest, der sich aus einer Gruppe -O- und beispielsweise aus den vorstehend für R_{L}¹ beschriebenen Aryl- bzw. Arylalkylresten zusammensetzt.

Unter verzweigten oder unverzweigten, gegebenenfalls substituierten C₁-C₆-Alkyl-, C₂-C₆-Alkenyl-, C₂-C₆-Alkinyl- oder C₁-C₄-Alkoxyresten werden für R_{G}³ oder R_{G}⁴ in Strukturelement G unabhängig voneinander, beispielsweise die entsprechenden jeweils vorstehend für R_{L}¹ beschriebenen Reste verstanden.

Ferner können beide Reste R_{G}³ und R_{G}⁴ zusammen ein cyclisches Acetal, wie beispielsweise -O-CH₂-CH₂-O- oder -O-CH₂-O- bilden.

Weiterhin können beide Reste R_{G}³ und R_{G}⁴ zusammen einen gegebenenfalls substituierten C₃-C₇-Cycloalkylrest bilden.

Bevorzugte Reste für R_{G}³ oder R_{G}⁴ sind unabhängig voneinander Wasserstoff, C₁-C₄-Alkyl oder C₁-C₄-Alkoxy.

Unter verzweigten oder unverzweigten, gegebenenfalls substituierten C₁-C₆-Alkyl- oder C₁-C₄-Alkoxyresten und gegebenfalls substituierten Aryl- oder Arylalkylresten werden für R_{G}⁵ und R_{G}⁶ in Strukturelement G unabhängig voneinander beispielsweise die entsprechenden jeweils vorstehend für R_{L}¹ beschriebenen Reste verstanden.

Ferner können beide Reste R_{G}⁵ und R_{G}⁶ zusammen einen, gegebenenfalls substituierten, anelierten, ungesättigten oder aromatischen 3- bis 10-gliedrigen Carbocyclus oder Heterocyclus, der bis zu drei verschiedene oder gleiche Heteroatome O, N, S enthalten kann, bilden.

Bevorzugte Reste für R_{G}⁵ und R_{G}⁶ sind unabhängig voneinander Wasserstoff oder gegebenfalls substituierte Arylreste, vorzugsweise Phenyl oder Arylalkylreste, vorzugsweise Benzyl sowie jeweils beide Reste R_{G}⁵ und R_{G}⁶ zusammen ein, gegebenenfalls substituierter, anelierter, ungesättigter oder aromatischer 3- bis 10-gliedriger Carbocyclus oder Heterocyclus, der bis zu drei verschiedene oder gleiche Heteroatome O, N, S enthalten kann.

Bei besonders bevorzugten Resten für R_{G}⁵ und R_{G}⁶ bilden beide Reste R_{G}⁵ und R_{G}⁶ zusammen einen, gegebenenfalls substituierten, anelierten, ungesättigten oder aromatischen 3- bis 6-gliedrigen Carbocyclus oder Heterocyclus, beispielsweise ausgewählt aus einer der folgenden zweifach gebundenen Strukturformeln: 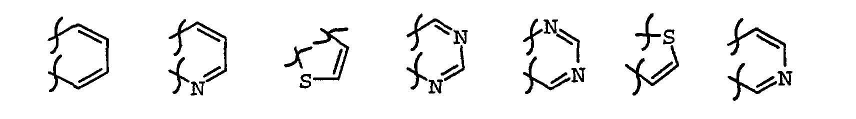 insbesondere ausgewählt aus einer der folgenden, zweifach gebundenen Strukturformeln:

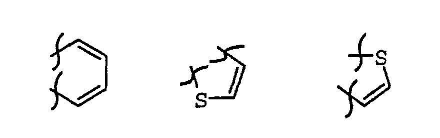

Als Substituenten dieser anelierten, ungesättigten oder aromatischen 3- bis 10-gliedrigen Carbocyclen oder Heterocyclen die R_{G}⁵ und R_{G}⁶ zusammen bilden können, kommen insbesondere Substituenten wie vorstehend allgemein beschrieben in Frage.

Besonders bevorzugte Substituenten dieser anelierten, ungesättigten oder aromatischen 3- bis 10-gliedrigen Carbocyclen oder Heterocyclen die R_{G}⁵ und R_{G}⁶ zusammen bilden können, sind unabhängig voneinander bis zu vier Substituenten ausgewählt aus der folgenden Gruppe:

Hydroxy, -CN, F oder Cl oder ein verzweigten oder unverzweigten, gegebenenfalls substituierten C₁-C₄-Alkoxy- oder C₁-C₄-Alkylrest, wie beispielsweise Methoxy, Methyl, CF₃, C₂F₅ oder CH₂F.

W_{G} stellt ein Strukturelement ausgewählt aus der Gruppe der Strukturelemente der Formeln I_{WG}¹ bis I_{WG}⁴ dar, wobei die gestrichelten Linien die Atombindungen innerhalb des Strukturelements G schneiden und das mit R_{G}⁷ und R_{G}⁸ substituierte Kohlenstoffatom an Y_{G} gebunden ist.

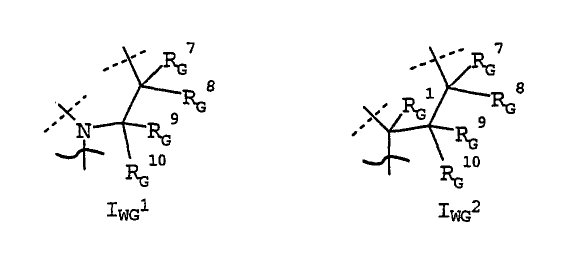

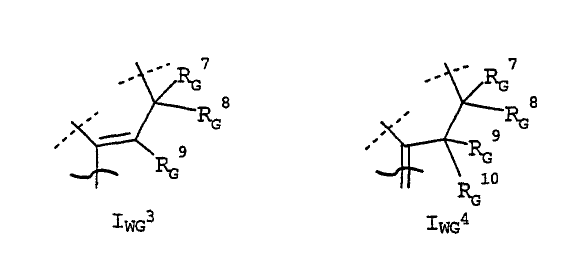

In einer bevorzugten Ausführungsform stellt W_{G} ein Strukturelement ausgewählt aus der Gruppe der Strukturelemente der Formeln I_{WG}² und I_{WG}³, insbesondere das Strukturelement der Formel I_{WG}² dar.

R_{G}¹ in Strukturelement W_{G} bedeutet Wasserstoff, Halogen, wie beispielsweise, Cl, F, Br oder I, eine Hydroxy-Gruppe oder einen verzweigten oder unverzweigten, gegebenenfalls substituierten C₁-C₆-Alkyl-, vorzugsweise C₁-C₄-Alkyl-, oder C₁-C₄-Alkoxyrest beispielsweise wie jeweils vorstehend für R_{L}¹ beschrieben.

Besonders bevorzugte Reste für R_{G}¹ sind Wasserstoff, Methoxy oder Hydroxy.

R_{G}⁷, R_{G}⁸, R_{G}⁹ und R_{G}¹⁰ in Strukturelement G bedeuten unabhängig voneinander Wasserstoff, eine Hydroxygruppe, CN, Halogen, wie beispielsweise F, Cl, Br, I, einen verzweigten oder unverzweigten, gegebenenfalls substituierten
C₁-C₆-Alkylrest, wie beispielsweise gegebenenfalls substituiertes Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 1,2-Dimethylpropyl, 1,1-Dimethylpropyl, 2,2-Dimethylpropyl, 1-Ethylpropyl, Hexyl, 1-Methylpentyl, 1,2-Dimethylbutyl, 1,3-Dimethylbutyl, 2,3-Dimethylbutyl, 1,1-Dimethylbutyl, 2,2 -Dimethylbutyl, 3,3-Dimethylbutyl, 1,1,2-Trimethylpropyl, 1,2,2-Trimethylpropyl, 1-Ethylbutyl, 2-Ethylbutyl oder 1-Ethyl-2-methylpropyl,
C₂-C₆-Alkenylrest, wie beispielsweise gegebenenfalls substituiertes Vinyl, 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 3-Methyl-3-pentenyl, 4-Methyl-3-pentenyl, 1-Methyl-4-pentenyl, 2-Methyl-4-pentenyl, 3-Methyl-4-entenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl oder 1-Ethyl-2-methyl-2-propenyl,
C₂-C₆-Alkinylrest, wie beispielsweise gegebenenfalls substituiertes Ethinyl, 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl oder 1-Ethyl-1-methyl-2-propinyl,
einen gegebenenfalls substituierten
C₃-C₇-Cycloalkylrest, wie beispielsweise gegebenenfalls substituiertes Cyclopropyl, Cyclobutyl, Cyclopentyl, Cyclohexyl oder Cycloheptyl,
C₃-C₇-Heterocycloalkylrest, wie beispielsweise gegebenenfalls substituiertes Aziridinyl, Diaziridinyl, Oxiranyl, Oxaziridinyl, Oxetanyl, Thiiranyl, Thietanyl, Pyrrolidinyl, Piperazinyl, Morpholinyl, Piperidinyl, Tetrahydrofuranyl, Tetrahydropyranyl, 1,4-Dioxanyl, Hexahydroazepinyl, Oxepanyl, 1,2-Oxathiolanyl oder Oxazolidinyl,
C₃-C₇-Heterocycloalkenylrest, wie beispielsweise gegebenenfalls substituiertes Azirinyl, Diazirinyl, Thiirenyl, Thietyl, Pyrrolinyle, Oxazolinyle, Azepinyl, Oxepinyl, α-Pyranyl, β-Pyranyl, γ-Pyranyl, Dihydropyranyle, 2,5-Dihydro-pyrrolinyl oder 4,5-Dihydrooxazolyl,
einen verzweigten oder unverzweigten, gegebenenfalls substituierten C₁-C₄-Alkylen-C₃-C₇-Cycloalkylrest, der sich beispielsweise aus verzweigten oder unverzweigten C₁-C₄-Alkylenresten wie beispielsweise Methylen, Ethylen, Propylen, n-Butylen, iso-Butylen oder t-Butylen und beispielsweise den vorstehend erwähnten C₃-C₇-Cycloalkylresten zusammensetzt,
einen verzweigten oder unverzweigten gegebenenfalls substituierten C₁-C₄-Alkylen-C₃-C₇-Heterocycloalkyl- oder C₁-C₄-Alkylen-C₃-C₇-Heterocycloalkenylrest, die sich aus gegebenenfalls substituierten C₁-C₄-Alkylen-Resten, wie beispielsweise Methylen, Ethylen, Propylen, n-Butylen, iso-Butylen oder t-Butylen und beispielsweise den vorstehend erwähnten C₃-C₇-Heterocycloalkyl- oder C₃-C₇-Heterocycloalkenylresten zusammensetzen, wobei die Reste bevorzugt sind, die im cyclischen Teil ein oder zwei Heteroatome ausgewählt aus der Gruppe N, O oder S und bis zu zwei Doppelbindungen enthalten,
einen verzweigten oder unverzweigten, gegebenenfalls substituierten Rest C₁-C₄-Alkylen-O-R_{G}¹¹, C₁-C₄-Alkylen-CO-OR_{G}¹¹, C₁-C₄-Alkylen-O-CO-R_{G}¹¹, C₁-C₄-Alkylen-CO-R_{G}¹¹, C₁-C₄-Alkylen-SO₂-NR_{G}¹²R_{G}¹³, C₁-C₄-Alkylen-CO-NR_{G}¹²R_{G}¹³, C₁-C₄-Alkylen-O-CO-NR_{G}¹²R_{G}¹³, C₁-C₄-Alkylen-NR_{G}¹²R_{G}¹³, C₁-C₄-Alkylen-SR_{G}¹¹ oder C₁-C₄-Alkylen-SO-R_{G}¹¹, die sich aus verzweigten oder unverzweigten, gegebenenfalls substituierten C₁-C₄-Alkylen-Resten, wie beispielsweise Methylen, Ethylen, Propylen, n-Butylen, Iso-Butylen oder t-Butylen, den enstprechenden Gruppen -O-, -CO-, -S-, -N und den nachstehend beschriebenen, endständigen Resten R_{G}¹¹, R_{G}¹² und R_{G}¹³ zusammensetzen,
einen gegebenenfalls substituierten
Arylrest, vorzugsweise gegebenenfalls substituiertes Phenyl, 1-Naphthyl oder 2-Naphthyl,
Arylalkylrest, vorzugsweise gegebenenfalls substituiertes Benzyl oder Ethylenphenyl (Homobenzyl),
Hetarylrest, vorzugsweise gegebenenfalls substituiertes 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 3-Isoxazolyl, 4-Isoxazolyl, 5-Isoxazolyl, Thiadiazolyl, Oxadiazolyl oder Triazinyl oder deren anellierten Derivate wie beispielsweise Indazolyl, Indolyl, Benzothiophenyl, Benzofuranyl, Indolinyl, Benzimidazolyl, Benzthiazolyl, Benzoxazolyl, Chinolinyl oder Isochinolinyl,
Hetarylalkylrest, vorzugsweise gegebenenfalls substituiertes -CH₂-2-Pyridyl, -CH₂-3-Pyridyl, -CH₂-4-Pyridyl, -CH₂-2-Thienyl, -CH₂-3-Thienyl, -CH₂-2-Thiazolyl, -CH₂-4-Thiazolyl, CH₂-5-Thiazolyl, -CH₂-CH₂-2-Pyridyl, -CH₂-CH₂-3-Pyridyl, -CH₂-CH₂-4-Pyridyl, -CH₂-CH₂-2-Thienyl, -CH₂-CH₂-3-Thienyl, -CH₂-CH₂-2-Thiazolyl, -CH₂-CH₂-4-Thiazolyl oder -CH₂-CH₂-5-Thiazolyl oder
einen Rest -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G}¹²R_{G}¹³, -SO₂-NR_{G}¹²R_{G}¹³, -CO-NR_{G}¹²R_{G}¹³, -NR_{G}¹²R_{G}¹³, CO-R_{G}¹¹.

Ferner könnenjeweils unabhängig voneinander zwei Reste R_{G}⁷ und R_{G}⁹ oder R_{G}⁸ und R_{G}¹⁰ oder R_{G}⁷ und R_{G}⁸ oder R_{G}⁹ und R_{G}¹⁰ zusammen einen, gegebenenfalls substituierten, gesättigten oder ungesättigten, nicht aromatischen, 3 bis 7 gliedrigen Carbocyclus oder Heterocyclus der bis zu 3 Heteroatome ausgewählt aus der Gruppe O, N, S und bis zu zwei Doppelbindungen enthalten kann, bilden.

Bevorzugte Reste für R_{G}⁷, R_{G}⁸, R_{G}⁹ und R_{G}¹⁰ im Strukturelement G sind unabhängig voneinander Wasserstoff, eine Hydroxygruppe, -CN, Halogen, ein verzweigter oder unverzweigter, gegebenenfalls substituierter C₁-C₆-Alkyl-, C₂-C₆-Alkenyl-, C₂-C₆-Alkinyl-, C₁-C₄-Alkylen-C₃-C₇-Cycloalkyl-, C₁-C₄-Alkylen-C₃-C₇-Heterocycloalkyl- oder C₁-C₄-Alkylen-C₃-C₇-Heterocycloalkenylrest, ein verzweigter oder unverzweigter, gegebenenfalls substituierter Rest C₁-C₄-Alkylen-OR_{G}¹¹, C₁-C₄-Alkylen-CO-OR_{G}¹¹, C₁-C₄-Alkylen-O-CO-R_{G}¹¹, C₁-C₄-Alkylen-CO-R_{G}¹¹, C₁-C₄-Alkylen-SO₂-NR_{G}¹²R_{G}¹³, C₁-C₄-Alkylen-CO-NR_{G}¹²R_{G}¹³, C₁-C₄-Alkylen-O-CO-NR_{G}¹²R_{G}¹³, C₁-C₄-Alkylen-NR_{G}¹²R_{G}¹³ oder C₁-C₄-Alkylen-SR_{G}¹¹, C₁-C₄-Alkylen-SO-R_{G}¹¹, ein Rest -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G}¹²R_{G}¹³, -SO₂-NR_{G}¹²R_{G}¹³, -CO-NR_{G}¹²R_{G}¹³, -NR_{G}¹²R_{G}¹³ oder CO-R_{G}¹¹, ein gegebenenfalls substituierter C₃-C₇-Cycloalkyl-, C₃-C₇-Heterocycloalkyl-, C₃-C₇-Heterocycloalkenyl-, Aryl-, Hetaryl-, Arylalkyl- oder Hetarylalkylrest, wie jeweils vorstehend beschrieben.

Besonders bevorzugte Reste für R_{G}⁷, R_{G}⁸, R_{G}⁹ und R_{G}¹⁰ im Strukturelement G sind unabhängig voneinander Wasserstoff, F oder ein verzweigter oder unverzweigter, gegebenenfalls substituierter C₁-C₄-Alkylrest, wie vorstehend beschrieben.

Unter einem verzweigten oder unverzweigten, gegebenenfalls substituierten C₁-C₈-Alkylrest werden für R_{G}¹¹, R_{G}¹² und R_{G}¹³ unabhängig voneinander beispielsweise die vorstehend für R_{G}¹ erwähnten C₁-C₆-Alkylreste verstanden, zuzüglich der Reste Heptyl und Octyl.

Bevorzugte Substituenten der verzweigten oder unverzweigten, gegebenenfalls substituierten C₁-C₈-Alkylreste sind für R_{G}¹¹, R_{G}¹² und R_{G}¹³ unabhängig voneinander die Reste Halogen, Hydroxy, C₁-C₄-Alkoxy, -CN, -COOH und -CO-O-C₁-C₄-Alkyl.

Unter einem verzweigten oder unverzweigten, gegebenenfalls substituierten C₂-C₆-Alkenyl-, C₂-C₆-Alkinyl- oder C₁-C₄-Alkylen-C₃-C₇-Cycloalkylrest, einem gegebenenfalls substituierten C₃-C₇-Cycloalkyl-, Aryl-, Arylalkyl-, Hetaryl- oder Hetarylalkylrest werden für R_{G}¹¹, R_{G}¹² und R_{G}¹³ unabhängig voneinander beispielsweise die entsprechenden, vorstehend für R_{G}¹ erwähnten Reste verstanden.

Bevorzugte, verzweigte oder unverzweigte, gegebenenfalls substituierte -C₁-C₅-Alkylen-C₁-C₄-Alkoxy-Reste für R_{G}¹¹, R_{G}¹² und R_{G}¹³ sind unabhängig voneinander Methoxymethylen, Ethoxymethylen, t-Butoxymethylen, Methoxyethylen oder Ethoxyethylen.

Bevorzugte, verzweigte oder unverzweigte, gegebenenfalls substituierte mono- und bis-Alkylaminoalkylen- oder Acylaminoalkylenreste für R_{G}¹¹, R_{G}¹² und R_{G}¹³ sind unabhängig voneinander verzweigte oder unverzweigte, gegebenenfalls substituierte Reste -C₁-C₄-Alkylen-NH(C₁-C₄-Alkyl), -C₁-C₄-Alkylen-N(C₁-C₄-Alkyl)₂ bzw. -C₁-C₄-Alkylen-NH-CO-C₁-C₄-Alkyl.

Bevorzugte gegebenenfalls substituierte Heterocycloalkyl-, Heterocycloalkenyl-, C₁-C₄-Alkylen-Heterocycloalkyl- oder C₁-C₄-Alkylen-Heterocycloalkenylreste für R_{G}¹¹, R_{G}¹² und R_{G}¹³ sind unabhängig voneinander die vorstehend für R_{G}¹ beschriebenen C₃-C₇-Heterocycloalkyl-, C₃-C₇-Heterocycloalkenyl-, C₁-C₄-Alkylen-C₃-C₇-Heterocycloalkyl- oder C₁-C₄-Alkylen-C₃-C₇-Heterocycloalkenylreste.

Besonders bevorzugte, gegebenenfalls substituierte Heterocycloalkyl-, Heterocycloalkenyl-, C₁-C₄-Alkylen-Heterocycloalkyl- oder C₁-C₄-Alkylen-Heterocycloalkenylreste für R_{G}¹¹, R_{G}¹² und R_{G}¹³ sind unabhängig voneinander die vorstehend für R_{G}¹ beschriebenen C₃-C₇-Heterocycloalkyl-, C₃-C₇-Heterocycloalkenyl-, C₁-C₄-Alkylen-C₃-C₇-Heterocycloalkyl- oder C₁-C₄-Alkylen-C₃-C₇-Heterocycloalkenylreste, wobei im cyclischen Teil ein oder zwei Heteroatome ausgewählt aus der Gruppe N, O oder S und bis zu zwei Doppelbindungen enthalten sind.

Ferner können R_{G}¹² und R_{G}¹³ unabhängig voneinander einen Rest -SO₂-R_{G}¹¹, -CO-O-R_{G}¹¹, -CO-NR_{G}¹¹R_{G}¹¹* oder -CO-R_{G}¹¹ bedeuten, wobei R_{G}¹¹*einen von R_{G}¹¹ unabhängigen Rest R_{G}¹¹ darstellt.

Bevorzugte Strukturelemente G setzen sich aus mindestens einem bevorzugten Rest des Strukturelements G zusammen, während die restlichen Reste breit variabel sind.

Besonders bevorzugte Strukturelemente G setzen sich aus den bevorzugten Resten des Strukturelements G zusammen.

Ganz besonders bevorzugte Strukturelemente G setzen sich aus den besonders bevorzugten Resten des Strukturelements G zusammen.

Unter Strukturelement B wird ein Strukturelement verstanden, enthaltend mindestens ein Atom das unter physiologischen Bedingungen als Wasserstoff-Akzeptor Wasserstoffbrücken ausbilden kann, wobei mindestens ein Wasserstoff-Akzeptor-Atom entlang des kürzestmöglichen Weges entlang des Strukturelementgerüstes einen Abstand von 5 bis 14 Atombindungen zu Strukturelement G aufweist. Die Ausgestaltung des Strukturgerüstes des Strukturelementes B ist weit variabel.

Als Atome, die unter physiologischen Bedingungen als Wasserstoff-Akzeptoren Wasserstoffbrücken ausbilden können, kommen beispielsweise Atome mit Lewisbaseneigenschaften in Frage, wie beispielsweise die Heteroatome Stickstoff, Sauerstoff oder Schwefel.

Unter physiologischen Bedingungen wird ein pH-Wert verstanden, der an dem Ort in einem Organismus herrscht, an dem die Liganden mit den Rezeptoren in Wechselwirkung treten. Im vorliegenden Fall weisen die physiologischen Bedingungen einen pH-Wert von beispielsweise 5 bis 9 auf.

In einer bevorzugten Ausführungsform bedeutet das Strukturelement B ein Strukturelement der Formel I_{B}

A-E- I_{B}

bedeutet, wobei A und E folgende Bedeutung haben:
- A: ein Strukturelement ausgewählt aus der Gruppe:
ein 4- bis 8-gliedriger monocyclischer gesättigter, ungesättigter oder aromatischer Kohlenwasserstoff, der bis zu 4 Heteroatome, ausgewählt aus der Gruppe O, N oder S, enthalten kann, wobei jeweils unabhängig voneinander der gegebenenfalls enthaltene Ring-Stickstoff oder die Kohlenstoffe substituiert sein können,
   mit der Maßgabe daß mindestens ein Heteroatom, ausgewählt aus der Gruppe O, N oder S im Strukturelement A enthalten ist,
   oder
ein 9- bis 14-gliedriger polycyclischer gesättigter, ungesättigter oder aromatischer Kohlenwasserstoff, der bis zu 6 Heteroatome, ausgewählt aus der Gruppe N, O oder S, enthalten kann, wobei jeweils unabhängig voneinander der gegebenenfalls enthaltene Ring-Stickstoff oder die Kohlenstoffe substituiert sein können,
   mit der Maßgabe daß mindestens ein Heteroatom, ausgewählt aus der Gruppe O, N oder S im Strukturelement A enthalten ist,
ein Rest 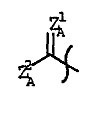 wobei
   Z_{A}¹ Sauerstoff, Schwefel oder gegebenenfalls substituierter Stickstoff und
   Z_{A}² gegebenenfalls substituierten Stickstoff, Sauerstoff oder Schwefel bedeuten,
oder ein Rest 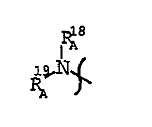 wobei
R_{A}¹⁸, R_{A}¹⁹ unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NR_{G}¹¹R_{G}¹¹* oder -CO-R_{G}¹¹ bedeuten,
und
- E: ein Spacer-Strukturelement, das Strukturelement A mit dem Strukturelement G kovalent verbindet, wobei die Anzahl der Atombindungen entlang des kürzestmöglichen Weges entlang des Strukturelementgerüstes E 5 bis 14 beträgt.

In einer besonders bevorzugten Ausführungsform bedeutet das Strukturelement A ein Strukturelement ausgewählt aus der Gruppe der Strukturelemente der Formeln I_{A}¹ bis I_{A}¹⁸, 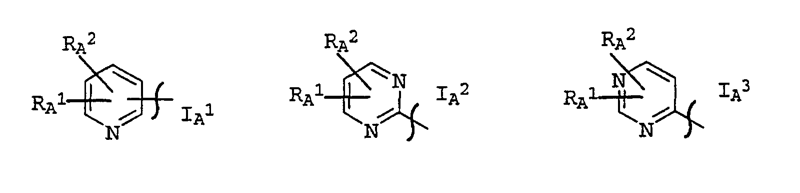 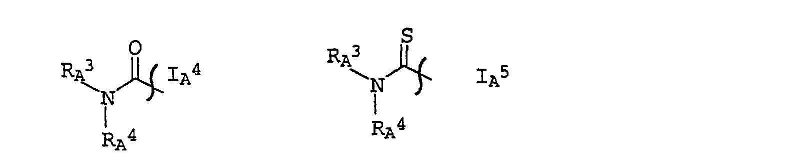 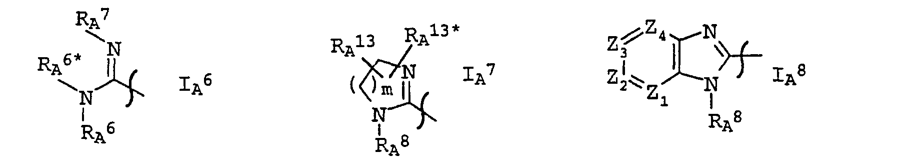 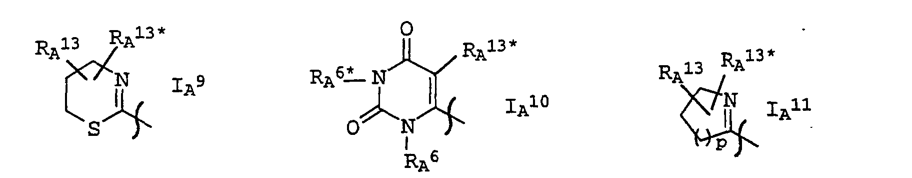 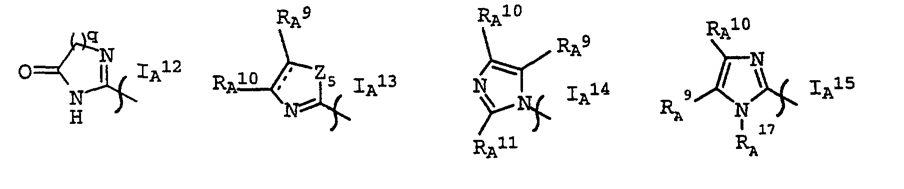 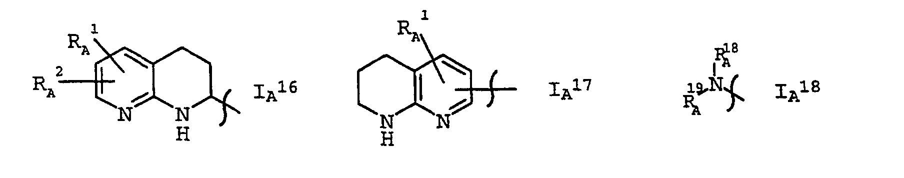 wobei
- m,p,q: unabhängig voneinander 1,2 oder 3,
- R_{A}¹, R_{A}²: unabhängig voneinander Wasserstoff, CN, Halogen, einen verzweigten oder unverzweigten, gegebenenfalls substituierten C₁-C₆-Alkyl- oder CO-C₁-C₆-Alkylrest oder einen gegebenenfalls substituierten Aryl-, Arylalkyl-, Hetaryl-, Hetarylalkyl- oder C₃-C₇-Cycloalkylrest oder einen Rest CO-O-R_{A}¹⁴, O-R_{A}¹⁴, S-R_{A}¹⁴, NR_{A}¹⁵R_{A}¹⁶, CO-NR_{A}¹⁵R_{A}¹⁶ oder SO₂NR_{A}¹⁵R_{A}¹⁶ oder beide Reste R_{A}¹ und R_{A}² zusammen einen anellierten, gegebenenfalls substituierten, 5- oder 6-gliedrigen, ungesättigten oder aromatischen Carbocyclus oder Heterocyclus der bis zu drei Heteroatome, ausgewählt aus der Gruppe 0, N, oder S enthalten kann,
- R_{A}¹³, R_{A}¹³*: unabhängig voneinander Wasserstoff, CN, Halogen, einen verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-NR_{A}¹⁵R_{A}¹⁶,
wobei
R_{A}¹⁴ Wasserstoff, einen verzweigten oder unverzweigten, gegebenenfalls substituierten C₂-C₆-Alkyl-, Alkylen-C₁-C₄-Alkoxy-, C₂-C₆-Alkenyl-, C₂-C₆-Alkinyl- oder C₁-C₆-Alkylen-C₃-C₇-Cycloalkylrest oder einen gegebenfalls substituierten C₃-C₇-Cycloalkyl-, Aryl-, Arylalkyl-, Hetaryl- oder Hetarylalkylrest,
R_{A}¹⁵, R_{A}16, unabhängig voneinander Wasserstoff, einen verzweigten oder unverzweigten, gegebenenfalls substituierten C₁-C₆-Alkyl-, CO-C₁-C₆-Alkyl-, SO₂-C₁-C₆-Alkyl-, COO-C₁-C₆-Alkyl-, CO-NH-C₁-C₆-Alkyl-, Arylalkyl-, COO-Alkylen-Aryl-, SO₂-Alkylen-Aryl-, CO-NH-Alkylen-Aryl-, CO-NH-Alkylen-Hetaryl- oder Hetarylalkylrest oder einen gegebenenfalls substituierten C₃-C₇-Cycloalkyl-, Aryl-, CO-Aryl-, CO-NH-Aryl-, SO₂-Aryl, Hetaryl, CO-NH-Hetaryl-, oder CO-Hetarylrest bedeuten,
- R_{A}³, R_{A}⁴: unabhängig voneinander Wasserstoff, -(CH₂)ₙ-(X_{A})ⱼ-R_{A}¹², oder beide Reste zusammen einen 3 bis 8 gliedrigen, gesättigten, ungesättigten oder aromatischen N-Heterocyclus der zusätzlich zwei weitere, gleiche oder verschiedene Heteroatome O, N, oder S enthalten kann, wobei der Cyclus gegebenenfalls substituiert oder an diesem Cyclus ein weiterer, gegebenenfalls substituierter, gesättigter, ungesättigter oder aromatischer Cyclus ankondensiert sein kann,
wobei
- n: 0, 1, 2 oder 3,
- j: 0 oder 1,
- X_{A}: -CO-, -CO-N(R_{X}¹)-, -N(R_{X}¹)-CO-, -N(R_{X}¹)-CO-N(R_{X}¹*)-, -N(R_{X}¹)-CO-O-, -O-, -S-, -SO₂-, -SO₂-N(R_{X}¹)-, -SO₂-O-, -CO-O-, -O-CO-, -O-CO-N(R_{X}¹)-, -N(R_{X}¹)- oder -N(R_{X}¹)-SO₂-,
- R_{A}¹²: Wasserstoff, einen verzweigten oder unverzweigten, gegebenenfalls substituierten C₁-C₆-Alkylrest, einen gegebenenfalls mit C₁-C₄-Alkyl oder Aryl substituierten C₂-C₆-Alkinyl- oder C₂-C₆-Alkenylrest oder einen mit bis zu drei gleichen oder verschiedenen Resten substituierten, 3-6 gliedrigen, gesättigten oder ungesättigten Heterocyclus, der bis zu drei verschiedene oder gleiche Heteroatome O, N, S enthalten kann, C₃-C₇-Cycloalkyl-, Aryl- oder Heteroarylrest, wobei zwei Reste zusammen einen anellierten, gesättigten, ungesättigten oder aromatischen Carbocyclus oder Heterocyclus, der bis zu drei verschiedene oder gleiche Heteroatome O, N, S enthalten kann, darstellen können und der Cyclus gegebenenfalls substituiert oder an diesem Cyclus ein weiterer, gegebenenfalls substituierter, gesättigter, ungesättigter oder aromatischer Cyclus ankondensiert sein kann, oder der Rest R_{A}¹² bildet zusammen mit R_{X}¹ oder R_{X}¹* einen gesättigten oder ungesättigten C₃-C₇-Heterocyclus, der gegebenenfalls bis zu zwei weitere Heteroatome, ausgewählt aus der Gruppe O, S oder N enthalten kann,
- R_{X}¹, R_{X}¹*: unabhängig voneinander Wasserstoff, einen verzweigten oder unverzweigten, gegebenenfalls substituierten C₁-C₆-Alkyl-, C₁-C₆-Alkoxyalkyl, C₂-C₆-Alkenyl-, C₂-C₁₂-Alkinyl-, CO-C₁-C₆-Alkyl-, CO-O-C₁-C₆-Alkyl- oder SO₂-C₁-C₆-Alkylrest oder einen gegebenenfalls substituierten C₃-C₇-Cycloalkyl-, Aryl, Arylalkyl-, CO-O-Alkylen-Aryl-, CO-Alkylen-Aryl-, CO-Aryl, SO₂-Aryl-, Hetaryl, CO-Hetaryl- oder SO₂-Alkylen-Arylrest,
- R_{A}⁶, R_{A}⁶*: Wasserstoff, einen verzweigten oder unverzweigten, gegebenenfalls substituierten C₁-C₄-Alkyl-, -CO-O-C₁-C₄-Alkyl-, Arylalkyl-, -CO-O-Alkylen-Aryl-, -CO-O-Allyl-, -CO-C₁-C₄-Alkyl-, -CO-Alkylen-Aryl-, C₃-C₇-Cycloalkyl- oder -CO-Allylrest oder in Strukturelement I_{A}⁷ beide Reste R_{A}⁶ und R_{A}⁶* zusammen einen gegebenenfalls substituierten, gesättigten, ungesättigten oder aromatischen Heterocyclus, der zusätzlich zum Ringstickstoff bis zu zwei weitere verschiedene oder gleiche Heteroatome O, N, S enthalten kann,
- R_{A}⁷: Wasserstoff, -OH, -CN, -CONH₂, einen verzweigten oder unverzweigten, gegebenenfalls substituierten C₁-C₄-Alkyl-, C₁-C₄-Alkoxy-, C₃-C₇-Cycloalkyl- oder -O-CO-C₁-C₄-Alkylrest, oder einen gegebenenfalls substituierten Arylalkyl-, -O-Alkylen-Aryl-, -O-CO-Aryl-, -O-CO-Alkylen-Aryl- oder -O-CO-Allylrest, oder beide Reste R_{A}⁶ und R_{A}⁷ zusammen einen gegebenenfalls substituierten, ungesättigten oder aromatischen Heterocyclus, der zusätzlich zum Ringstickstoff bis zu zwei weitere verschiedene oder gleiche Heteroatome O, N, S enthalten kann,
- R_{A}⁸: Wasserstoff, einen verzweigten oder unverzweigten, gegebenenfalls substituierten C₁-C₄-Alkyl-, CO-C₁-C₄-Alkyl-, SO₂-C₁-C₄-Alkyl- oder CO-O-C₁-C₄-Alkylrest oder einen gegebenenfalls substituierten Aryl-, CO-Aryl-, SO₂-Aryl, CO-O-Aryl, CO-Alkylen-Aryl-, SO₂-Alkylen-Aryl-, CO-O-Alkylen-Aryl- oder Alkylen-Arylrest,
- R_{A}⁹, R_{A}¹⁰: unabhängig voneinander Wasserstoff, -CN, Halogen, einen verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-NR_{A}¹⁵R_{A}¹⁶, oder beide Reste R_{A}⁹ und R_{A}¹⁰ zusammen in Strukturelement I_{A}¹⁴ einen 5 bis 7 gliedrigen gesättigten, ungesättigten oder aromatischen Carbocyclus oder Heterocyclus, der bis zu drei verschiedene oder gleiche Heteroatome O, N, S enthalten kann und gegebenenfalls mit bis zu drei gleichen oder verschiedenen Resten substituiert ist,
- R_{A}¹¹: Wasserstoff, -CN, Halogen, einen verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-NR_{A}¹⁵R_{A}¹⁶,
- R_{A}¹⁷: Wasserstoff oder in Strukturelement I_{A}¹⁶ beide Reste R_{A}⁹ und R_{A}¹⁷ zusammen einen 5 bis 7 gliedrigen gesättigten, ungesättigten oder aromatischen Heterocyclus, der zusätzlich zum Ringstickstoff bis zu drei verschiedene oder gleiche Heteroatome O, N, S enthalten kann und gegebenenfalls mit bis zu drei gleichen oder verschiedenen Resten substituiert ist,
- R_{A}¹⁸, R_{A}¹⁹: unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen von R_{G}¹¹ unabhängigen Rest -SO₂-R_{G}¹¹, -CO-OR_{G}¹¹, -CO-NR_{G}¹¹R_{G}¹¹* oder -CO-R_{G}¹¹
- Z¹, Z², Z³, Z⁴: unabhängig voneinander Stickstoff, C-H, C-Halogen oder einen verzweigten oder unverzweigten, gegebenenfalls substituieren C-C₁-C₄-Alkyl- oder C-C₁-C₄-Alkoxyrest,
- Z⁵: NR_{A}⁸, Sauerstoff oder Schwefel
bedeuten.

In einer weiteren ganz besonders bevorzugten Ausführungsform bedeutet das Strukturelement A ein Strukturelement der Formeln I_{A}¹, I_{A}⁴, I_{A}⁷, I_{A}⁸ oder I_{A}⁹.

Unter einem verzweigten oder unverzweigten, gegebenenfalls substituierten C₁-C₆-Alkylrest werden für R_{A}¹ oder R_{A}² unabhängig voneinander beispielsweise die entsprechenden vorstehend für R_{G}¹ beschriebenen Reste, vorzugsweise Methyl oder Trifluormethyl verstanden.

Der verzweigte oder unverzweigte, gegebenenfalls substituierte Rest CO-C₁-C₆-Alkyl setzt sich für R_{A}¹ oder R_{A}² in den Strukturelementen I_{A}¹, I_{A}², I_{A}³ oder I_{A}¹⁷ beispielsweise aus der Gruppe CO und den vorstehenden für R_{A}¹ oder R_{A}² beschrieben, verzweigten oder unverzweigten, gegebenenfalls substituierten C₁-C₆-Alkylresten zusammen.

Unter gegebenenfalls substituierten Hetaryl-, Hetarylalkyl-, Aryl-, Arylalkyl- oder C₃-C₇-Cycloalkylresten werden für R_{A}¹ oder R_{A}² unabhängig voneinander beispielsweise die entsprechenden, vorstehend für R_{G}⁷ beschriebenen, Reste verstanden.

Die gegebenenfalls substituierten Reste CO-O-R_{A}¹⁴, O-R_{A}¹⁴, S-R_{A}¹⁴, NR_{A}¹⁵R_{A}¹⁶, CO-NR_{A}¹⁵R_{A}¹⁶ oder SO₂NR_{A}¹⁵R_{A}¹⁶ setzten sich für R_{A}¹ oder R_{A}² beispielsweise aus den Gruppen CO-O, O, S, N, CO-N bzw. SO₂-N und den nachstehend näher beschriebenen Resten R_{A}¹⁴, R_{A}¹⁵ bzw. R_{A}¹⁶ zusammen.

Ferner können beide Reste R_{A}¹ und R_{A}² zusammen einen anellierten, gegebenenfalls substituierten, 5- oder 6-gliedrigen, ungesättigten oder aromatischen Carbocyclus oder Heterocyclus der bis zu drei Heteroatome, ausgewählt aus der Gruppe O, N, oder S enthalten kann, bilden.

R_{A}¹³ und R_{A}¹³* bedeuten unabhängig voneinander Wasserstoff, CN,
Halogen, wie beispielsweise Fluor, Chlor, Brom oder Iod,
einen verzweigten oder unverzweigten, gegebenenfalls substituierten C₁-C₆-Alkylrest, wie beispielsweise vorstehend für R_{G}¹ beschrieben, vorzugsweise Methyl oder Trifluormethyl oder
einen gegebenenfalls substituierten Aryl-, Arylalkyl-, Hetaryl- oder C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂NR_{A}¹⁵R_{A}¹⁶ oder CO-NR_{A}¹⁵R_{A}¹⁶ wie jeweils vorstehend für R_{A}¹ beschrieben.

Bevorzugte Reste für R_{A}¹³ und R_{A}¹³* sind die Reste Wasserstoff, F, Cl, ein verzweigter oder unverzweigter, gegebenenfalls substituierter C₁-C₆-Alkylrest, gegebenenfalls substituiertes Aryl oder Arylalkyl oder ein Rest CO-O-R_{A}¹⁴, O-R_{A}¹⁴, NR_{A}¹⁵R_{A}¹⁶, SO₂-NR_{A}¹⁵R_{A}¹⁶ oder CO-NR_{A}¹⁵R_{A}¹⁶.

Unter einem verzweigten oder unverzweigten, gegebenenfalls substituierten C₁-C₆-Alkyl-, C₃-C₇-Cycloalkyl-, Alkylen-Cycloalkyl-, Alkylen-C₁-C₄-Alkoxy-, C₂-C₆-Alkenyl- oder C₂-C₆-Alkinylrest werden für R_{A}¹⁴ in Strukturelement A beispielsweise die entsprechenden, vorstehend für R_{G}⁷ beschriebenen Reste verstanden.

Unter gegebenfalls substituierten Aryl-, Arylalkyl-, Hetaryl- oder Alkylhetarylresten werden für R_{A}¹⁴ in Strukturelement A beispielsweise die entsprechenden, vorstehend für RG⁷ beschriebenen Reste verstanden.

Bevorzugte Reste für R_{A}¹⁴ sind Wasserstoff, ein verzweigter oder unverzweigter, gegebenenfalls substituierter C₁-C₆-Alkylrest und gegebenenfalls substituiertes Benzyl.

Unter einem verzweigten oder unverzweigten, gegebenenfalls substituierten C₁-C₆-Alkyl- oder Arylalkylrest oder einem gegebenenfalls substituierten C₃-C₇-Cycloalkyl-, Aryl-, Hetaryl- oder Hetarylalkylrest werden für R_{A}¹⁵ oder R_{A}¹⁶ unabhängig voneinander beispielsweise die entsprechenden, vorstehend für R_{A}¹⁴ beschriebenen Reste verstanden.

Die verzweigten oder unverzweigten, gegebenenfalls substituierten CO-C₁-C₆-Alkyl-, SO₂-C₁-C₆-Alkyl-, COO-C₁-C₆-Alkyl-, CO-NH-C₁-C₆-Alkyl-, COO-Alkylen-Aryl-, CO-NH-Alkylen-Aryl-, CO-NH-Alkylen-Hetaryl- oder SO₂-Alkylen-Arylreste oder die gegebenenfalls substituierten CO-Aryl-, SO₂-Aryl, CO-NH-Aryl-, CO-NH-Hetaryl- oder CO-Hetarylreste setzten sich für R_{A}¹⁵ oder R_{A}¹⁶ beispielsweise aus den entsprechenden Gruppen -CO-, -SO₂-, -CO-O-, -CO-NH- und den entsprechend, vorstehend beschriebenen verzweigten oder unverzweigten, gegebenenfalls substituierten C₁-C₆-Alkyl-, Hetarylalkyl- oder Arylalkylresten oder den entsprechenden gegebenenfalls substituierten Aryl- oder Hetarylresten zusammen.

Unter einem Rest -(CH₂)ₙ-(X_{A})ⱼ-R_{A}¹² wird für R_{A}³ oder R_{A}⁴ unabhängig voneinander ein Rest verstanden, der sich aus den entsprechenden Resten -(CH₂)ₙ-, (X_{A})ⱼ und R_{A}¹² zusammensetzt. Dabei kann n: 0, 1, 2 oder 3 und j: 0 oder 1 bedeuten.

X_{A} stellt einen zweifach gebundenen Rest, ausgewählt aus der Gruppe -CO-, -CO-N(R_{X}¹)-, -N(R_{X}¹)-CO-, -N(R_{X}¹)-CO-N(R_{X}¹*)-, -N(R_{X}¹)-CO-O-, -O-, -S-, -SO₂-, -SO₂-N(R_{X}¹)-, -SO₂-O-, -CO-O-, -O-CO-, -O-CO-N(R_{X}¹)-, -N(R_{X}¹)- oder -N(R_{X}¹)-SO₂- dar.

R_{A}¹² bedeutet Wasserstoff,
einen verzweigten oder unverzweigten, gegebenenfalls substituierten C₁-C₆-Alkylrest, wie vorstehend für R_{G}⁷ beschrieben,
einen gegebenenfalls mit C₁-C₄-Alkyl oder Aryl substituierten C₂-C₆-Alkinyl- oder C₂-C₆-Alkenylrest,
oder einen mit bis zu drei gleichen oder verschiedenen Resten substituierten, 3-6 gliedrigen, gesättigten oder ungesättigten Heterocyclus, der bis zu drei verschiedene oder gleiche Heteroatome O, N, S enthalten kann, wie beispielsweise gegebenenfalls substituiertes 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 2-(1,3,4-Thiadiazolyl), 2-(1,3,4)-Oxadiazolyl, 3-Isoxazolyl, 4-Isoxazolyl, 5-Isoxazolyl, Triazinyl.

Ferner können R_{A}¹² und R_{X}¹ oder R_{X}¹* zusammen einen gesättigten oder ungesättigten C₃-C₇-Heterocyclus bilden, der gegebenenfalls bis zu zwei weitere Heteroatome, ausgewählt aus der Gruppe O, S oder N enthalten kann.

Vorzugsweise bildet der Rest R_{A}¹² zusammen mit dem Rest R_{X}¹ oder R_{X}¹* ein cyclisches Amin als C₃-C₇-Heterocyclus, für den Fall, daß die Reste am gleichen Stickstoffatom gebunden sind, wie beispielsweise N-Pyrrolidinyl, N-Piperidinyl, N-Hexahydroazepinyl, N-Morpholinyl oder N-Piperazinyl, wobei bei Heterocyclen die freie Aminprotonen tragen, wie beispielsweise N-Piperazinyl die freien Aminprotonen durch gängige Aminschutzgruppen, wie beispielsweise Methyl, Benzyl, Boc (tert.-Butoxycarbonyl), Z (Benzyloxycarbonyl), Tosyl, -SO₂-C₁-C₄-Alkyl, -SO₂-Phenyl oder -SO₂-Benzyl ersetzt sein können.

Unter einem verzweigten oder unverzweigten, gegebenenfalls substituierten C₁-C₆-Alkyl-, C₂-C₁₂-Alkinyl-, vorzugsweise C2-C6-Al-kinyl- oder C₂-C₆-Alkenylrest, einen gegebenenfalls substituierten C₃-C₇-Cycloalkyl-, Aryl-, Arylalkyl- oder Hetarylrest werden für R_{X}¹ und R_{X}¹* unabhängig voneinander beispielsweise die entsprechenden, vorstehend für R_{G}⁷ beschriebenen Reste verstanden.

Bevorzugte, verzweigte oder unverzweigte, gegebenenfalls substituierte C₁-C₆-Alkoxyalkyl für R_{X}¹ und R_{X}¹* sind unabhängig voneinander Methoxymethylen, Ethoxymethylen, t-Butoxymethylen, Methoxyethylen oder Ethoxyethylen.

Bevorzugte, verzweigte oder unverzweigte, gegebenenfalls substituierte Reste CO-C₁-C₆-Alkyl, CO-O-C₁-C₆-Alkyl, SO₂-C₁-C₆-Alkyl, CO-O-Alkylen-Aryl, CO-Alkylen-Aryl, CO-Aryl, SO₂-Aryl, CO-Hetaryl oder SO₂-Alkylen-Aryl setzen sich vorzugsweise aus den vorstehend beschriebenen C₁-C₆-Alkyl-, Arylalkyl-, Aryl- oder Hetarylresten und den Resten -CO-, -O-, -SO₂- zusammen.

Bevorzugte Reste für R_{X}¹ und R_{X}¹* sind unabhängig voneinander Wasserstoff, Methyl, Cyclopropyl, Allyl und Propargyl.

R_{A}³ und R_{A}⁴ können ferner zusammen einen 3 bis 8 gliedrigen, gesättigten, ungesättigten oder aromatischen N-Heterocyclus der zusätzlich zwei weitere, gleiche oder verschiedene Heteroatome O, N, oder S enthalten kann, bilden, wobei der Cyclus gegebenenfalls substituiert oder an diesem Cyclus ein weiterer, gegebenenfalls substituierter, gesättigter, ungesättigter oder aromatischer Cyclus ankondensiert sein kann,

R_{A}⁵ bedeutet einen verzweigten oder unverzweigten, gegebenenfalls substituierten C₁-C₆-Alkyl-, Arylalkyl-, C₁-C₄-Alkyl-C₃-C₇-Cycloalkyl- oder C₃-C₇-Cycloalkylrest oder einen gegebenenfalls substituierten Aryl, Hetaryl-, Heterocycloalkyl- oder Heterocycloalkenylrest, wie beispielsweise vorstehend für R_{G}⁷ beschrieben.

R_{A}⁶ und R_{A}⁶* bedeuten unabhängig voneinander Wasserstoff, einen verzweigten oder unverzweigten, gegebenenfalls substituierten
C₁-C₄-Alkylrest, wie beispielsweise gegebenenfalls substituiertes Methyl, Ethyl, Propyl, 1-Methylethyl, Butyl, 1-Methylpropyl, 2-Methylpropyl oder 1,1-Dimethylethyl,
-CO-O-C₁-C₄-Alkyl- oder -CO-C₁-C₄-Alkylrest wie beispielsweise aus der Gruppe -CO-O- bzw. -CO- und den vorstehend beschriebenen C₁-C₄-Alkylresten zusammengesetzt,
Arylalkylrest, wie vorstehend für R_{G}⁷ beschrieben,
-CO-O-Alkylen-Aryl- oder -CO-Alkylen-Arylrest wie beispielsweise aus der Gruppe -CO-O- bzw. -CO- und den vorstehend beschriebenen Arylalkylresten zusammengesetzt,
-CO-O-Allyl- oder -CO-Allylrest,
oder C₃-C₇-Cycloalkylrest, wie beispielsweise vorstehend für R_{G}7 beschrieben.

Ferner können beide Reste R_{A}⁶ und R_{A}⁶* in Strukturelement I_{A}⁷ zusammen einen gegebenenfalls substituierten, gesättigten, ungesättigten oder aromatischen Heterocyclus, der zusätzlich zum Ringstickstoff bis zu zwei weitere verschiedene oder gleiche Heteroatome O, N, S enthalten kann, bilden.

R_{A}⁷ bedeutet Wasserstoff, -OH, -CN, -CONH₂, einen verzweigten oder unverzweigten, gegebenenfalls substituierten C₁-C₄-Alkylrest, beispielsweise wie vorstehend für R_{A}⁶ beschrieben, C₁-C₄-Alkoxy-, Arylalkyl- oder C₃-C₇-Cycloalkylrest, beispielsweise wie vorstehend für R_{L}¹⁴ beschrieben, einen verzweigten oder unverzweigten, gegebenenfalls substituierten -O-CO-C₁-C₄-Alkylrest, der sich aus der Gruppe -O-CO- und beispielsweise aus den vorstehend erwähnten C₁-C₄-Alkylresten zusammensetzt oder einen gegebenenfalls substituierten -O-Alkylen-Aryl-, -O-CO-Aryl-, -O-CO-Alkylen-Aryl- oder -O-CO-Allylrest der sich aus den Gruppen -O- bzw. -O-CO- und beispielsweise aus den entsprechenden vorstehend für R_{G}⁷ beschriebenen Resten zusammensetzt.

Ferner können beide Reste R_{A}⁶ und R_{A}⁷ zusammen einen gegebenenfalls substituierten, ungesättigten oder aromatischen Heterocyclus, der zusätzlich zum Ringstickstoff bis zu zwei weitere verschiedene oder gleiche Heteroatome O, N, S enthalten kann, bilden.

Unter einem verzweigten oder unverzweigten, gegebenenfalls substituierten C₁-C₄-Alkylrest oder einen gegebenenfalls substituierten Aryl-, oder Arylalkylrest werden für R_{A}⁸ in Strukturelement A beispielsweise die entsprechenden, vorstehend für R_{A}¹⁵ beschriebenen Reste verstanden, wobei sich die Reste CO-C₁-C₄-Alkyl, SO₂-C₁-C₄-Alkyl, CO-O-C₁-C₄-Alkyl, CO-Aryl, SO₂-Aryl, CO-O-Aryl, CO-Alkylen-Aryl, SO₂-Alkylen-Aryl oder CO-O-Alkylen-Aryl analog zu den anderen zusammengesetzten Resten aus der Gruppe CO, SO₂ oder COO und beispielsweise aus dem entsprechenden vorstehend für R_{A}¹⁵ beschriebenen C₁-C₄-Alkyl-, Aryl- oder der Arylalkylresten zusammensetzten und diese Reste gegebenenfalls substituiert sein können.

Unter einem verzweigten oder unverzweigten, gegebenenfalls substituierten C₁-C₆-Alkylrest oder einen gegebenenfalls substituierten Aryl-, Arylalkyl-, Hetaryl- oder C₃-C₇-Cycloalkylrest werden jeweils für R_{A}⁹ oder R_{A}¹⁰ unabhängig voneinander beispielsweise die entsprechenden, vorstehend für R_{A}¹⁴ beschriebenen Reste verstanden, vorzugsweise Methyl oder Trifluormethyl.

Unter einem Rest CO-O-R_{A}¹⁴, O-R_{A}¹⁴, S-R_{A}¹⁴, SO₂-NR_{A}¹⁵R_{A}¹⁶, NR_{A}¹⁵R_{A}¹⁶ oder CO-NR_{A}¹⁵R_{A}¹⁶ werden jeweils für R_{A}⁹ oder R_{A}¹⁰ unabhängig voneinander beispielsweise die entsprechenden, vorstehend für R_{A}¹³ beschriebenen Reste verstanden.

Ferner können beide Reste R_{A}⁹ und R_{A}¹⁰ zusammen in Strukturelement I_{A}¹⁴ einen 5 bis 7 gliedrigen gesättigten, ungesättigten oder aromatischen Carbocyclus oder Heterocyclus, der bis zu drei verschiedene oder gleiche Heteroatome O, N, S enthalten kann und gegebenenfalls mit bis zu drei gleichen oder verschiedenen Resten substituiert ist, bilden.

Unter Substituenten werden in diesem Fall insbesondere Halogen, CN, ein verzweigter oder unverzweigter, gegebenenfalls substituierter C₁-C₄-Alkylrest, wie beispielsweise Methyl oder Trifluormethyl oder die Reste O-R_{A}¹⁴, S-R_{A}¹⁴, NR_{A}¹⁵R_{A}¹⁶, CO-NR_{A}¹⁵R_{A}¹⁶ oder - ((R_{A}⁸)HN)C=N-R_{A}⁷ verstanden.

Unter einem verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-N_{RA}¹⁵R_{A}¹⁶ werden für R_{A}¹¹ beispielsweise die entsprechenden, vorstehend für R_{A}⁹ beschriebenen Reste verstanden.

Ferner können in Strukturelement I_{A}¹⁶ beide Reste R_{A}⁹ und R_{A}¹⁷ zusammen einen 5 bis 7 gliedrigen gesättigten, ungesättigten oder aromatischen Heterocyclus, der zusätzlich zum Ringstickstoff bis zu drei verschiedene oder gleiche Heteroatome O, N, S enthalten kann und gegebenenfalls mit bis zu drei gleichen oder verschiedenen Resten substituiert ist, bilden.

Unter einem verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NR_{G}¹¹R_{G}¹¹* oder -CO-R_{G}¹¹ werden für R_{A}¹⁸ und R_{A}¹⁹ unabhängig voneinander beispielsweise die vorstehend für R_{G}¹² beschriebenen Reste, vorzugsweise Wasserstoff oder einen verzweigten oder unverzweigten, gegebenenfalls substituierten C₁-C₈-Alkylrest verstanden.

Z¹, Z², Z³, Z⁴ bedeuten unabhängig voneinander Stickstoff, C-H, C-Halogen, wie beispielsweise C-F, C-Cl, C-Br oder C-I oder einen verzweigten oder unverzweigten, gegebenenfalls substituieren C-C₁-C₄-Alkylrest, der sich aus einem Kohlenstoffrest und beispielsweise einem vorstehend für R_{A}⁶ beschriebenen C₁-C₄-Alkylrest zusammensetzt oder einen verzweigten oder unverzweigten, gegebenenfalls substituieren C-C₁-C₄-Alkoxyrest, der sich aus einem Kohlenstoffrest und beispielsweise einem vorstehend für R_{A}⁷ beschriebenen C₁-C₄-Alkoxyrest zusammensetzt.

Z⁵ bedeutet Sauerstoff, Schwefel oder einen Rest NR_{A}⁸.

Bevorzugte Strukturelemente A setzen sich aus mindestens einem bevorzugten Rest der zum Strukturelement A gehörenden Reste zusammen, während die restlichen Reste breit variabel sind.

Besonders bevorzugte Strukturelemente A setzen sich aus den bevorzugten Resten des Strukturelements A zusammen.

In einer bevorzugten Ausführungsform wird unter dem Spacerstrukturelement E ein Strukturelement verstanden, daß aus einem verzweigten oder unverzweigten, gegebenenfalls substituierten und Heteroatome enthaltenden aliphatischen C₂-C₃₀-Kohlenwasserstoffrest und/oder aus einem 4- bis 20 gliedrigen, gegebenenfalls substituierten und Heteroatome enthaltenden, aliphatischen oder aromatischen mono- oder polycyclischen Kohlenwasserstoffrest besteht.

In einer weiter bevorzugten Ausführungsform wird das Spacer-Strukturelement E aus zwei bis vier Teilstrukturelementen, ausgewählt aus der Gruppe E¹ und E² zusammensetzt, wobei die Reihenfolge der Verknüpfung der Teilstrukturelemente beliebig ist und E¹ und E² folgende Bedeutung haben:
- E¹: ein Teilstrukturelement der Formel I_{E1}

- (Y_{E})ₖ₁-(CR_{E}¹R_{E}²)_{c}- (Q_{E})ₖ₂-(CR_{E}³R_{E}⁴)_{d-} I_{E1}

und
- E²: ein Teilstrukturelement der Formel I_{E2}

-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2} ,

wobei
c, d, f, g, h unabhängig voneinander 0, 1 oder 2,
k1, k2, k3, k4, k5, k6 unabhängig voneinander 0 oder 1,
X_{E}, Q_{E} unabhängig voneinander einen gegebenenfalls substituierten 4 bis 11-gliedrigen mono- oder polycyclischen, aliphatischen oder aromatischen Kohlenwasserstoff, der bis zu 6 Doppelbindungen und bis zu 6 gleiche oder verschiedene Heteroatome, ausgewählt aus der Gruppe N, O oder S enthalten kann, wobei die Ringkohlenstoffe und/oder die Ringstickstoffe gegebenenfalls substituiert sein können,
Y_{E}, Z_{E} unabhängig voneinander CO, CO-NR_{E}¹², NR_{E}¹²-CO, Schwefel, SO, SO₂, SO₂-NR_{E}¹², NR_{E}¹²-SO₂, CS, CS-NR_{E}¹², NR_{E}¹²-CS, CS-O, O-CS, CO-O, O-CO, Sauerstoff, Ethinylen, CR_{E}¹³-O-CR_{E}¹⁴, C(=CR_{E}¹³R_{E}¹⁴), CR_{E}¹³=CR_{E}¹⁴, -CR_{E}¹³(OR_{E}¹⁵)-CHR_{E}¹⁴- oder -CHRE¹³-CR_{E}¹⁴(OR_{E}¹⁵)-,
R_{E}¹, R_{E}², R_{E}³, R_{E}⁴, R_{E}⁵, R_{E}⁶, R_{E}⁷, R_{E}⁸, R_{E}⁹, R_{E}¹⁰ unabhängig voneinander Wasserstoff, Halogen, eine Hydroxygruppe, einen verzweigten oder unverzweigten, gegebenenfalls substituierten C₁-C₆-Alkyl-, C₂-C₆-Alkenyl-, C₂-C₆-Alkinyl- oder Alkylen-Cycloalkylrest, einen Rest -(CH₂)ₓ-(W_{E})_{z}-R_{E}¹⁷, einen gegebenenfalls substituierten C₃-C₇-Cycloalkyl-, Aryl-, Arylalkyl-, Hetaryl- oder Hetarylalkylrest oder unabhängig voneinander jeweils zwei Reste R_{E}¹ und R_{E}² oder R_{E}³ und R_{E}⁴ oder R_{E}⁵ und R_{E}⁶ oder R_{E}⁷ und R_{E}⁸ oder R_{E}⁹ und R_{E}¹⁰ zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann,
x 0, 1, 2, 3 oder 4,
z 0 oder 1,
W_{E} -CO-, -CO-N(R_{w}²)-, -N(R_{w}²)-CO-, -N(R_{w}²)-CO-N(R_{w}²*)-, -N(R_{w}²)-CO-O-, -O-, -S-, -SO₂-, -SO₂-N(R_{w}²)-, -SO₂-O-, -CO-O-, -O-CO-, -O-CO-N(R_{w}²⁾-, -N(R_{w}²)- oder -N(R_{w}²)-SO₂-,
R_{w}², R_{w}²* unabhängig voneinander Wasserstoff, einen verzweigten oder unverzweigten, gegebenenfalls substituierten C₁-C₆-Alkyl-, C₂-C₆-Alkenyl-, C₂-C₈-Alkinyl-, CO-C₁-C₆-Alkyl-, CO-O-C₁-C₆-Alkyl- oder SO₂-C₁-C₆-Alkylrest oder einen gegebenenfalls substituierten Hetaryl, Hetarylalkyl, Arylalkyl, C₃-C₇-Cycloalkyl-, CO-O-Alkylen-Aryl-, CO-Alkylen-Aryl-, CO-Aryl, SO₂-Aryl-, CO-Hetaryl- oder SO₂-Alkylen-Arylrest,
R_{E}¹⁷ Wasserstoff, eine Hydroxygruppe, CN, Halogen, einen verzweigten oder unverzweigten, gegebenenfalls substituierten C₁-C₆-Alkylrest, einen gegebenenfalls substituierten C₃-C₇-Cycloalkyl-, Aryl-, Heteroaryl oder Arylalkylrest, einen gegebenenfalls mit C₁-C₄-Alkyl oder Aryl substituierten C₂-C₆-Alkinyl- oder C₂-C₆-Alkenylrest, einen gegebenenfalls substituierten C₆-C₁₂-Bicycloalkyl-, C₁-C₆-Alkylen-C₆-C₁₂-Bicycloalkyl-, C₇-C₂₀-Tricycloalkyl- oder C₁-C₆-Alkylen-C₇-C₂₀-Tricycloalkylrest, oder einen mit bis zu drei gleichen oder verschiedenen Resten substituierten, 3- bis 8-gliedrigen, gesättigten oder ungesättigten Heterocyclus, der bis zu drei verschiedene oder gleiche Heteroatome O, N, S enthalten kann, wobei zwei Reste zusammen einen anellierten, gesättigten, ungesättigten oder aromatischen Carbocyclus oder Heterocyclus, der bis zu drei verschiedene oder gleiche Heteroatome O, N, S enthalten kann, darstellen können und der Cyclus gegebenenfalls substituiert oder an diesem Cyclus ein weiterer, gegebenenfalls substituierter, gesättigter, ungesättigter oder aromatischer Cyclus ankondensiert sein kann, oder der Rest R_{E}¹⁷ bildet zusammen mit R_{w}² oder R_{W}²* einen gesättigten oder ungesättigten C₃-C₇-Heterocyclus, der gegebenenfalls bis zu zwei weitere Heteroatome, ausgewählt aus der Gruppe O, S oder N enthalten kann,
R_{E}¹¹, R_{E}¹¹* unabhängig voneinander Wasserstoff, einen verzweigten oder unverzweigten, gegebenenfalls substituierten C₁-C₆-Alkyl-, C₁-C₆-Alkoxyalkyl-, C₂-C₆-Alkenyl-, C₂-C₁₂-Alkinyl-, CO-C₂-C₆-Alkyl-, CO-O-C₁-C₆-Alkyl-, CO-NH-C₁-C₆-Alkoxalkyl-, CO-NH-C₁-C₆-Alkyl-
oder SO₂-C₁-C₆-Alkylrest oder einen gegebenenfalls substituierten Hetaryl, Arylalkyl-, C₃-C₇-Cycloalkyl-, CO-O- Alkylen-Aryl-, CO-NH-Alkylen-Aryl-, CO-Alkylen-Aryl-, CO-Aryl, CO-NH-Aryl, SO₂-Aryl-, CO-Hetaryl-, SO₂-Alkylen-Aryl-, SO₂-Hetaryl- oder SO₂-Alkylen-Hetarylrest,
R_{E}¹² Wasserstoff, einen verzweigten oder unverzweigten, gegebenenfalls substituierten C₁-C₆-Alkyl-, C₂-C₆-Alkenyl-, C₂-C₈-Alkinyl-, einen gegebenenfalls substituierten C₃-C₇-Cycloalkyl-, Hetaryl-, Arylalkyl- oder Hetarylalkyl Rest oder einen Rest CO-R_{E}¹⁶, COOR_{E}¹⁶ oder SO₂-R_{E}¹⁶,
R_{E}¹³, R_{E}¹⁴ unabhängig voneinander Wasserstoff, eine Hydroxygruppe, einen verzweigten oder unverzweigten, gegebenenfalls substituierten C₁-C₆-Alkyl-, C₁-C₄-Alkoxy-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest,
R_{E}¹⁵ Wasserstoff, einen verzweigten oder unverzweigten, gegebenenfalls substituierten C₁-C₆-Alkyl-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest,
R_{E}¹⁶ Wasserstoff, eine Hydroxygruppe, einen verzweigten oder unverzweigten, gegebenenfalls substituierten C₁-C₆-Alkyl-, C₂-C₆-Alkenyl-, C₂-C₆-Alkinyl- oder C₁-C₅-Alkylen-C₁-C₄-Alkoxyrest, oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-C₃-C₇-Heterocycloalkyl-, C₁-C₄-Alkylen-C₃-C₇-Heterocycloalkenyl- oder Hetarylalkylrest
bedeuten.

Der Koeffizient c bedeutet vorzugsweise 0 oder 1, der Koeffizient d vorzugsweise 1 oder 2, die Koeffizienten f, g, h unabhängig voneinander vorzugsweise 0 oder 1.

Unter einem gegebenfalls substituierten 4 bis 11-gliedrigen mono- oder polycyclischen aliphatischen oder aromatischen Kohlenwasserstoff, der bis zu 6 Doppelbindungen und bis zu 6 gleiche oder verschiedene Heteroatome, ausgewählt aus der Gruppe N, O, S, enthalten kann, wobei die Ringkohlenstoffe oder Ringstickstoffe gegebenenfalls substituiert sein können werden für Q_{E} und X_{E} unabhängig voneinander vorzugsweise gegebenenfalls substituiertes Arylen, wie beispielsweise gegebenenfalls substituiertes Phenylen oder Naphtylen, gegebenfalls substituiertes Hetarylen wie beispielsweise die Reste 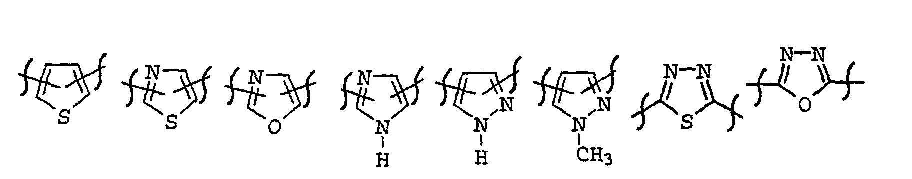 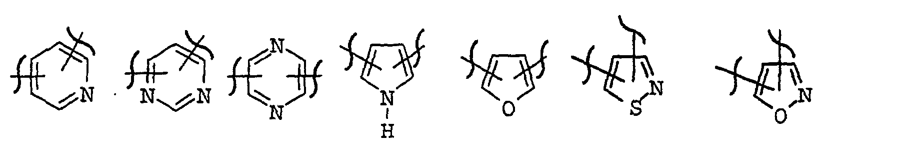 sowie deren substituierte oder anellierte Derivate, oder Reste der Formeln I_{E}¹ bis I_{E}¹¹ verstanden, 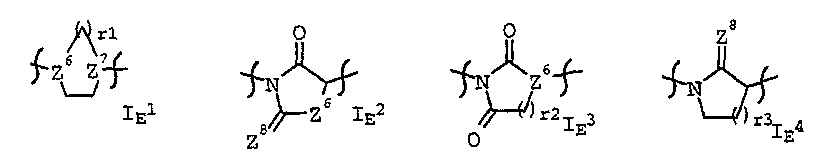 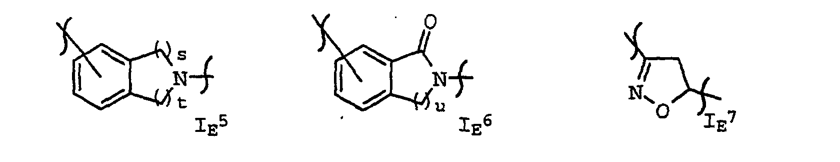 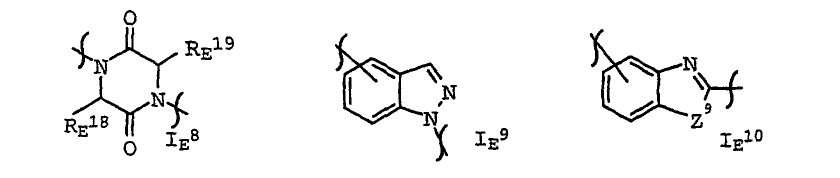 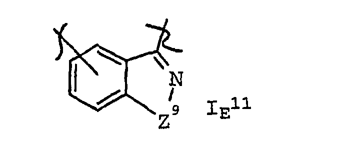 wobei der Einbau der Reste in beiden Orientierungen erfolgen kann. Unter aliphatischen Kohlenwasserstoffen werden beispielsweise gesättigte und ungesättigte Kohlenwasserstoffe verstanden.

Z⁶ und Z⁷ bedeuten unabhängig voneinander CH oder Stickstoff.

Z⁸ bedeutet Sauerstoff, Schwefel oder NH

Z⁹ bedeutet Sauerstoff, Schwefel oder NR_{E}²⁰.

r1, r2, r3 und t bedeuten unabhängig voneinander 0, 1, 2 oder 3.

s und u bedeuten unabhängig voneinander 0, 1 oder 2.

Besonders bevorzugt bedeuteten X_{E} und Q_{E} unabhängig voneinander gegebenenfalls substituiertes Phenylen, einen Rest 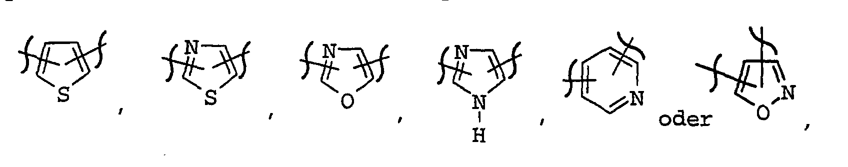 sowie deren substituierte oder anellierte Derivate, oder Reste der Formeln I_{E}¹, I_{E}², I_{E}³, I_{E}⁴ und I_{E}⁷, wobei der Einbau der Reste in beiden Orientierungen erfolgen kann.

R_{E}¹⁸ und R_{E}¹⁹ bedeuten unabhängig voneinander Wasserstoff, -NO₂, -NH₂, -CN, -COOH, eine Hydroxygruppe, Halogen einen verzweigten oder unverzweigten, gegebenenfalls substituierten C₁-C₆-Alkyl-, C₁-C₄-Alkoxy-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest, wie jeweils vorstehend beschrieben.

R_{E}²⁰ bedeutet unabhängig voneinander Wasserstoff, einen verzweigten oder unverzweigten, gegebenenfalls substituierten C₁-C₆-Alkyl-, C₁-C₆-Alkoxyalkyl, C₃-C₁₂-Alkinyl-, CO-C₁-C₆-Alkyl-, CO-O-C₁-C₆-Alkyl- oder SO₂-C₁-C₆-Alkylrest oder einen gegebenenfalls substituierten C₃-C₇-Cycloalkyl-, Aryl, Arylalkyl-, CO-O-Alkylen-Aryl-, CO-Alkylen-Aryl-, CO-Aryl, SO₂-Aryl-, Hetaryl, CO-Hetaryl- oder SO₂-Alkylen-Arylrest, vorzugsweise Wasserstoff oder einen verzweigten oder unverzweigten, gegebenenfalls substituierten C₁-C₆-Alkylrest.

Y_{E} und Z_{E} bedeuten unabhängig voneinander CO, CO-NR_{E}¹², NR_{E}¹²-CO, Schwefel, SO, SO₂, SO₂-NR_{E}¹², NR_{E}¹²-SO₂, CS, CS-NR_{E}¹², NR_{E}¹²-CS, CS-O, O-CS, CO-O, O-CO, Sauerstoff, Ethinylen, CR_{E}¹³-O-CR_{E}¹⁴, C(=CR_{E}¹³R_{E}¹⁴), CR_{E}¹³=CR_{E}¹⁴, -CR_{E}¹³(OR_{E}¹⁵)-CHR_{E}¹⁴- oder -CHR_{E}¹³-CR_{E}¹⁴(OR_{E}¹⁵)-, vorzugsweise CO, SO₂ und Sauerstoff.

R_{E}¹² bedeutet Wasserstoff, einen verzweigten oder unverzweigten, gegebenenfalls substituierten C₁-C₆-Alkyl-, C₂-C₆-Alkenyl- oder C₂-C₈-Alkinylrest oder einen gegebenenfalls substituierten C₃-C₇-Cycloalkyl-, Hetaryl-, Arylalkyl- oder Hetarylalkyl Rest, wie beispielsweise entsprechend vorstehend für R_{G}⁷ beschrieben oder einen Rest CO-R_{E}¹⁶, COOR_{E}¹⁶ oder SO₂-R_{E}¹⁶, vorzugsweise Wasserstoff, Methyl, Allyl, Propargyl und Cyclopropyl.

Unter einem verzweigten oder unverzweigten, gegebenenfalls substituierten C₁-C₆-Alkyl-, C₂-C₆-Alkenyl- oder C₂-C₆-Alkinylrest oder einen gegebenfalls substituierten C₃-C₇-Cycloalkyl-, Aryl-, Arylalkyl-, Hetaryl- oder Hetarlyalkylrest, werden für R_{E}¹³, R_{E}¹⁴ oder R_{E}¹⁵ unabhängig voneinander beispielsweise die entsprechenden, vorstehend für R_{G}⁷ beschriebenen Reste verstanden.

Unter einem verzweigten oder unverzweigten, gegebenenfalls substituierten C₁-C₄-Alkoxyrest werden für R_{E}¹³ oder R_{E}¹⁴ unabhängig voneinander beispielsweise die vorstehend für R_{A}¹⁴ beschriebenen C₁-C₄-Alkoxyreste verstanden.

Bevorzugte Alkylen-Cycloalkylreste sind für R_{E}¹³, R_{E}¹⁴ oder R_{E}¹⁵ unabhängig voneinander beispielsweise die vorstehend für R_{G}⁷ beschriebenen C₁-C₄-Alkylen-C₃-C₇-Cycloalkylreste.

Unter einem verzweigten oder unverzweigten, gegebenenfalls substituierten C₁-C₆-Alkyl-, C₂-C₆-Alkenyl-, C₂-C₆-Alkinyl- oder C₁-C₅-Alkylen-C₁-C₄-Alkoxyrest, oder einem gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-C₃-C₇-Heterocycloalkyl-, C₁-C₄-Alkylen-C₃-C₇-Heterocycloalkenyl- oder Hetarylalkylrest werden für R_{E}¹⁶ beispielsweise die entsprechenden, vorstehend für R_{G}¹¹ beschriebenen Reste verstanden.

Unter einem verzweigten oder unverzweigten, gegebenenfalls substituierten C₁-C₆-Alkyl-, C₂-C₆-Alkenyl-, C₂-C₆-Alkinyl- oder Alkylen-Cycloalkylrest oder einen gegebenenfalls substituierten C₃-C₇-Cycloalkyl-, Aryl-, Arylalkyl-, Hetaryl- oder Hetarylalkylrest werden für R_{E}¹, R_{E}², R_{E}³, R_{E}⁴, R_{E}⁵, R_{E}⁶, R_{E}⁷, R_{E}⁸, R_{E}⁹ oder R_{E}¹⁰ unabhängig voneinander beispielsweise die entsprechenden, vorstehend für R_{G}⁷ erwähnten Reste verstanden.

Ferner können jeweils unabhängig voneinander zwei Reste R_{E}³ und R_{E}⁴ oder R_{E}⁵ und R_{E}⁶ oder R_{E}⁷ und R_{E}⁸ oder R_{E}⁹ und R_{E}¹⁰ zusammen einen 3- bis 7-gliedrigen, gegebenenfalls substituierten, gesättigten oder ungesättigten Carbo- oder Heterocyclus, der bis zu drei Heteroatome aus der Gruppe O, N oder S enthalten kann, bilden.

Der Rest -(CH₂)ₓ-(W_{E})_{z}-R_{E}¹⁷ setzt sich aus einem C₀-C₄-Alkylenrest, gegebenenfalls einem Bindungselement W_{E} ausgewählt aus der Gruppe -CO-, -CO-N(R_{w}²)-, -N(R_{w}²)-CO-, -N(R_{w}²)-CO-N(R_{w}²*)-, -N(R_{w}²)-CO-O-, -O-, -S-, -SO₂-, -SO₂-N(R_{w}²)-, -SO₂-O-, -CO-O-, -O-CO-, -O-CO-N(R_{w}²⁾-, -N(R_{w}²)- oder -N(R_{w}²)-SO₂-, vorzugsweise ausgewählt aus der Gruppe -CO-N(R_{w}²)-, -N(R_{w}²)-CO-, -O-, -SO₂-N(R_{w}²)-, -N(R_{w}²)- oder -N(R_{w}²)-SO₂-, und dem Rest R_{E}¹⁷ zusammen, wobei
R_{w}² und R_{w}²*
unabhängig voneinander Wasserstoff, einen verzweigten oder unverzweigten, gegebenenfalls substituierten C₁-C₆-Alkyl-, C₂-C₆-Alkenyl-, C₂-C₈-Alkinyl-, CO-C₁-C₆-Alkyl-, CO-O-C₁-C₆-Alkyl- oder SO₂-C₁-C₆-Alkylrest oder einen gegebenenfalls substituierten Hetaryl, Hetarylalkyl, Arylalkyl, C₃-C₇-Cycloalkyl-, CO-O-Alkylen-Aryl-, CO-Alkylen-Aryl-, CO-Aryl, SO₂-Aryl-, CO-Hetaryl- oder SO₂-Alkylen-Arylrest, vorzugsweise unabhängig voneinander Wasserstoff, Methyl, Cyclopropyl, Allyl, Propargyl, und
R_{E}¹⁷
Wasserstoff, eine Hydroxygruppe, CN, Halogen, einen verzweigten oder unverzweigten, gegebenenfalls substituierten C₁-C₆-Alkylrest, einen gegebenenfalls substituierten C₃-C₇-Cycloalkyl-, Aryl-, Heteroaryl oder Arylalkylrest, einen gegebenenfalls mit C₁-C₄-Alkyl oder Aryl substituierten C₂-C₆-Alkinyl- oder C₂-C₆-Alkenylrest, einen gegebenenfalls substituierten C₆-C₁₂-Bicycloalkyl-, C₁-C₆-Alkylen-C₆-C₁₂-Bicycloalkyl-, C₇-C₂₀-Tricycloalkyl- oder C₁-C-Alkylen-C₇-C₂₀-Tricycloalkylrest, oder einen mit bis zu drei gleichen oder verschiedenen Resten substituierten, 3- bis 8-gliedrigen, gesättigten oder ungesättigten Heterocyclus, der bis zu drei verschiedene oder gleiche Heteroatome O, N, S enthalten kann, wobei zwei Reste zusammen einen anellierten, gesättigten, ungesättigten oder aromatischen Carbocyclus oder Heterocyclus, der bis zu drei verschiedene oder gleiche Heteroatome O, N, S enthalten kann, darstellen können und der Cyclus gegebenenfalls substituiert oder an diesem Cyclus ein weiterer, gegebenenfalls substituierter, gesättigter, ungesättigter oder aromatischer Cyclus ankondensiert sein kann, wie beispielsweise gegebenenfalls substituiertes 2-Pyridyl, 3-Pyridyl, 4-Pyridyl, 2-Furyl, 3-Furyl, 2-Pyrrolyl, 3-Pyrrolyl, 2-Thienyl, 3-Thienyl, 2-Thiazolyl, 4-Thiazolyl, 5-Thiazolyl, 2-Oxazolyl, 4-Oxazolyl, 5-Oxazolyl, 2-Pyrimidyl, 4-Pyrimidyl, 5-Pyrimidyl, 6-Pyrimidyl, 3-Pyrazolyl, 4-Pyrazolyl, 5-Pyrazolyl, 3-Isothiazolyl, 4-Isothiazolyl, 5-Isothiazolyl, 2-Imidazolyl, 4-Imidazolyl, 5-Imidazolyl, 3-Pyridazinyl, 4-Pyridazinyl, 5-Pyridazinyl, 6-Pyridazinyl, 2-(1,3,4-Thiadiazolyl), 2-(1,3,4)-Oxadiazolyl, 3-Isoxazolyl, 4-Isoxazolyl, 5-Isoxazolyl oder Triazinyl,
bedeuten.

Ferner können R_{E}¹⁷ und R_{w}² oder R_{w}²* zusammen einen gesättigten oder ungesättigten C₃-C₇-Heterocyclus bilden, der gegebenenfalls bis zu zwei weitere-Heteroatome, ausgewählt aus der Gruppe O, S oder N enthalten kann.

Vorzugsweise bilden die Reste R_{E}¹⁷ und R_{W}² oder R_{W}²* zusammen ein cyclisches Amin als C₃-C₇-Heterocyclus, für den Fall, daß die Reste am gleichen Stickstoffatom gebunden sind, wie beispielsweise N-Pyrrolidinyl, N-Piperidinyl, N-Hexahydroazepinyl, N-Morpholinyl oder N-Piperazinyl, wobei bei Heterocyclen die freie Aminprotonen tragen, wie beispielsweise N-Piperazinyl die freien Aminprotonen durch gängige Aminschutzgruppen, wie beispielsweise Methyl, Benzyl, Boc (tert.-Butoxycarbonyl), Z (Benzyloxycarbonyl), Tosyl, -SO₂-C₁-C₄-Alkyl, -SO₂-Phenyl oder -SO₂-Benzyl ersetzt sein können.

Bevorzugte Reste für R_{E}¹, R_{E}², R_{E}³, R_{E}⁴, R_{E}⁵, R_{E}⁶, R_{E}⁷, R_{E}⁸, R_{E}⁹ oder R_{E}¹⁰ sind unabhängig voneinander Wasserstoff, Halogen, ein verzweigter oder unverzweigter, gegebenenfalls substituierter C₁-C₆-Alkylrest, gegebenenfalls substituiertes Aryl oder der Rest -(CH₂)ₓ-(W_{E})_{z}-R_{E}¹⁷.

Besonders bevorzugte Reste für R_{E}¹, R_{E}², R_{E}³, R_{E}⁴, R_{E}⁵, R_{E}⁶, R_{E}⁷, R_{E}⁸, R_{E}⁹ oder R_{E}¹⁰ sind unabhängig voneinander Wasserstoff, F, ein verzweigter oder unverzweigter, gegebenenfalls substituierter C₁-C₄-Alkylrest, insbesondere Methyl.

Unter einem verzweigten oder unverzweigten, gegebenenfalls substituierten C₁-C₆-Alkyl-, C₁-C₆-Alkoxyalkyl-, C₂-C₆-Alkenyl-, C₂-C₁₂-Alkinyl- oder Arylalkylrest oder einem gegebenenfalls substituierten Aryl, Hetaryl oder C₃-C₇-Cycloalkyl werden für R_{E}¹¹ und R_{E}¹¹* in Strukturelement E unabhängig voneinander beispielsweise die entsprechenden vorstehend für R_{G}⁷ beschriebenen Reste verstanden.

Die verzweigten oder unverzweigten, gegebenenfalls substituierten Reste CO-C₁-C₆-Alkyl, CO-O-C₁-C₆-Alkyl, CO-NH-C₁-C₆-Alkoxalkyl, CO-NH-C₁-C₆-Alkyl oder SO₂-C₁-C₆-Alkylrest oder die gegebenenfalls substituierten Reste CO-O-Alkylen-Aryl, CO-NH-Alkylen-Aryl, CO-Alkylen-Aryl, CO-Aryl, CO-NH-Aryl, SO₂-Aryl, CO-Hetaryl, SO₂-Alkylen-Aryl, SO₂-Hetaryl oder SO₂-Alkylen-Hetaryl setzen sich für R_{E}¹¹ und R_{E}¹¹* unabhängig voneinander beispielsweise aus den entsprechenden Gruppen CO, COO, CONH oder SO₂ und den entsprechenden vostehend erwähnten Resten zusammen.

Bevorzugte Reste für R_{E}¹¹ oder R_{E}¹¹* sind unabhängig voneinander Wasserstoff, ein verzweigter oder unverzweigter, gegebenenfalls substituierter C₁-C₆-Alkyl-, C₁-C₆-Alkoxy-, C₂-C₆-Alkenyl-, C₂-C₁₂-Alkinyl- oder Arylalkylrest, oder ein gegebenenfalls substituierter Hetaryl oder C₃-C₇-Cycloalkylrest.

Besonders bevorzugte Reste für R_{E}¹¹ oder R_{E}¹¹* sind Wasserstoff, Methyl, Cyclopropyl, Allyl oder Propargyl.

In einer besonders bevorzugten Ausführungsform des Strukturelements E₁ stellt das Strukturelement E₁ einen Rest -CH₂-CH₂-CO-, -CH₂-CH₂-CH₂-CO- oder einen C₁-C₅-Alkylenrest dar.

In einer besonders bevorzugten Ausführungsform des Strukturelements E verwendet man als Spacer-Strukturelement E ein Strukturelement der Formel I_{E1E2}.

-E₂-E₁- I_{E1E2}

wobei die Strukturelemente E₂ und E₁ die vorstehend beschriebene Bedeutung haben.

Bevorzugte Strukturelemente E setzen sich aus mindestens einem bevorzugten Rest der zum Strukturelement E gehörenden Reste zusammen, während die restlichen Reste breit variabel sind.

Besonders bevorzugte Strukturelemente E setzen sich aus den bevorzugten Resten des Strukturelements E zusammen.

Bevorzugte Strukturelemente B setzen sich entweder aus dem bevorzugten Strukturelement A zusammen, während E weit variabel ist oder aus dem bevorzugten Strukturelement E zusammen, während A weit variabel ist.

In einer weiteren bevorzugten Ausführungsform verwendet man als Strukturelement E das nachstehend bei den neuen verbindungen der Formel I' beschriebene Strukturelement E'.

In einer weiteren bevorzugten Ausführungsform verwendet man als Strukturelement G das nachstehend bei den neuen verbindungen der Formel I' beschriebene Strukturelement G'.

Die Verbindungen der Formel I und auch die Zwischenprodukte zu ihrer Herstellung, können ein oder mehrere asymmetrische substituierte Kohlenstoffatome besitzen. Die Verbindungen können als reine Enantiomere bzw. reine Diastereomere oder als deren Mischung vorliegen. Bevorzugt ist die Verwendung einer enantiomerenreinen Verbindung als Wirkstoff.

Die Verbindungen der Formel I können auch in anderen tautomeren Formen vorliegen.

Die Verbindungen der Formel I können auch in Form von physiologisch verträglichen Salzen vorliegen.

Die Verbindungen der Formel I können auch als Prodrugs in einer Form vorliegen, in der die Verbindungen der Formel I unter physiologischen Bedingungen freigesetzt werden. Beispielhaft sei hier auf die Gruppe T in Strukturelement L verwiesen, die teilweise Gruppen enthält, die unter physiologischen Bedingungen zur freien Carbonsäuregruppe hydrolisierbar sind. Es sind auch derivatisierte Strukturelemente B, bzw. A geeignet, die das Strukturelement B bzw. A unter physiologischen Bedingungen freisetzen.

Bei bevorzugten Verbindungen der Formel I weist jeweils eines der drei Strukturelemente B, G oder L den bevorzugten Bereich auf, während die restlichen Strukturelemente weit variabel sind.

Bei besonders bevorzugten Verbindungen der Formel I weisen jeweils zwei der drei Strukturelemente B, G oder L den bevorzugten Bereich auf, während die restlichen Strukturelemente weit variabel sind.

Bei ganz besonders bevorzugten Verbindungen der Formel I weisen jeweils alle drei Strukturelemente B, G oder L den bevorzugten Bereich auf, während das restliche Strukturelement weit variabel ist.

Bevorzugte Verbindungen der Formel I weisen beispielsweise das bevorzugte Strukturelement G auf, während die Strukturelemente B und L weit variabel sind.

Bei besonders bevorzugte Verbindungen der Formel I ist beispielsweise B durch das Strukturelement A-E- ersetzt und die Verbindungen weisen beispielsweise das bevorzugte Strukturelement G und das bevorzugte Strukturelement A auf, während die Strukturelemente E und L weit variabel sind.

Weitere besonders bevorzugte Verbindungen der Formel I weisen beispielsweise das bevorzugte Strukturelement G und das bevorzugte Strukturelement A auf, während die Strukturelemente E und L weit variabel sind.

Die Erfindung betrifft ferner die Verwendung des Strukturelements der Formel I_{GL}

-G-L I_{GL}

zur Herstellung von Verbindungen, die an Integrinrezeptoren binden.

Die Verbindungen der Formel I binden an Integrinrezeptoren. Die Verbindungen der Formel I eignen sich deshalb vorzugsweise als Integrin-Rezeptorliganden und zur Herstellung von Arzneimitteln zur Behandlung von Krankheiten in denen ein Integrinrezeptor involviert ist, insbesondere zur Behandlung von Krankheiten, bei denen die Wechselwirkung zwischen Integrinen und ihren natürlichen Liganden fehlreguliert, also überhöht oder erniedrigt ist.

Unter Integrinrezeptorliganden werden Agonisten und Antagonisten verstanden.

Unter einer überhöhten oder erniedrigten Wechselwirkung wird sowohl eine überhöhte oder erniedrigte Expression des natürlichen Liganden oder und/oder des Integrinrezeptors und damit eine überhöhte oder erniedrigte Menge an natürlichen Liganden oder und/oder Integrinrezeptor oder eine erhöhte oder erniedrigte Affinität des natürlichen Liganden an den Integrinrezeptor verstanden.

Die Wechselwirkung zwischen Integrinen und ihren natürlichen Liganden ist dann gegenüber dem Normalzustand fehlreguliert, also überhöht oder erniedrigt, wenn diese Fehlregulierung nicht dem physiologischen Zusand entspricht. Eine erhöhte oder erniedrigte Wechselwirkung kann zu pathophysiologischen Situationen führen.

Die Höhe der Fehlregulierung die zu einer pathophysiologischen Situation führt ist vom individuellen Organismus und vom Ort und der Art der Erkrankung abhängig.

Bevorzugte Integrinrezeptoren, für die die erfindungsgemäßen Verbindungen der Formel I verwendet werden können, sind die α₅β₁-, α₄β₁-, αᵥβ₅- und αᵥβ₃-Integrinrezeptoren.

Besonders bevorzugt binden die Verbindungen der Formel I an den α_{V}β₃-Integrinrezeptor und können somit besonders bevorzugt als Liganden des α_{V}β₃-Integrinrezeptors und zur Behandlung von Krankheiten, bei denen die Wechselwirkung zwischen α_{V}β₃-Integrinrezeptor und seinen natürlichen Liganden überhöht oder erniedrigt ist, verwendet werden.

Die Verbindungen der Formel I werden bevorzugt zur Behandlung folgender Krankheiten verwendet:
Kardiovaskuläre Erkrankungen wie Atherosklerose, Restenose nach Gefäßverletzung oder Stentimplantation, und Angioplastie (Neointimabildung, Glattmuskelzellmigration und Proliferation),
akutes Nierenversagen,
Angiogenese-assoziierte Mikroangiopathien wie beispielsweise diabetische Angiopathien oder Retinopathie oder rheumatische Arthritis,
Blutplättchen vermittelter Gefäßverschluß, arterielle Thrombose,
Schlaganfall, Reperfusionsschäden nach Myokardinfarkt oder Schlaganfall,
Krebserkrankungen, wie beispielsweise bei der Tumormetastasierung oder beim Tumorwachstum (tumorinduzierte Angiogenese),
Osteoporose (Knochenresorption nach Chemotaxis und Adhäsion von Osteoclasten an Knochenmatrix),
Bluthochdruck, Psoriasis, Hyperparathyroismus, Paget'sche Erkrankung, maligne Hypercalcemie, metastatische osteolytische Läsionen, Entzündung, Wundheilung, Herzinsuffizienz, Kongestives Herzversagen CHF, sowie bei
   anti-viraler, anti-mykotischer, anti-parasitärer oder anti-bakterieller Therapie und Prophylaxe (Adhäsion und Internalisierung).

Weiterhin betrifft die Erfindung insbesondere die Verwendung der Verbindungen der Formel I als Liganden des α_{V}β₃-Integrinrezeptors.

Die Erfindung betrifft weiterhin die neuen Verbindungen der Formel I'

A-E'-G'-L I'

wobei die Strukturelemente A und L die vorstehend beschriebene Bedeutung haben. Bevorzugte Strukturelemente A und L sind vorstehend beschrieben.

Strukturelement E' wird aus zwei bis vier Teilstrukturelementen, ausgewählt aus der Gruppe E¹ und E² zusammengesetzt, wobei die Reihenfolge der Verknüpfung der Teilstrukturelemente beliebig ist und E¹ und E² folgende Bedeutung haben:
- E¹: ein Teilstrukturelement der Formel T_{E1}

-(Y_{E})ₖ₁-(CR_{E}¹-R_{E}²)_{c}- (Q_{E})ₖ₂-(CR_{E}³R_{E}⁴)_{d-} I_{E1}

und
- E²: ein Teilstrukturelement der Formel I_{E2}

-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2},
wobei alle Reste und Koeffizienten von Strukturelement E' die vorstehend beschriebene Bedeutung von Strukturelement E haben, mit der Maßgabe, daß für den Fall,
daß Y_{E} oder Z_{E} = CO bedeuten und ein Rest X_{E} oder Q_{E} oder ein aromatischer oder heteroaromatischer Rest aus dem Strukturelement A direkt an Y_{E} oder Z_{E} gebunden ist, eine direkte Atombindung von Y_{E} oder Z_{E} an das Strukturelement G' ausgeschlossen ist.

In einer weiteren bevorzugten Ausführungsform von Strukturelement E' verwendet man als Strukturelement E' ein Strukturelement der Formel I_{E1E2}

-E₂-E₁- I_{E1E2}

wobei E¹ und E² folgende Bedeutung haben:
- E¹: ein Teilstrukturelement der Formel I_{E1}

-(Y_{E})ₖ₁-(CR_{E}¹R_{E}²)_{c}-(Q_{E})ₖ₂-(CR_{E}³R_{E}⁴)_{d-} I_{E1}

und
- E²: ein Teilstrukturelement der Formel I_{E2}

-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2},
wobei alle Reste und Koeffizienten von Strukturelement E' die vorstehend beschriebene Bedeutung von Strukturelement E haben, mit der Maßgabe, daß für den Fall,
Y_{E} = CO,
k1 und k5 = 1 und
h und k6 = 0
die Summe der Indizes c, k2 und d von 0 verschieden sein muß
und für den Fall daß ein aromatischer oder heteroaromatischer Rest aus dem Strukturelement A direkt an Y_{E} oder Z_{E} gebunden ist, eine direkte Atombindung von Y_{E} oder Z_{E} an das Strukturelement G' ausgeschlossen ist.

Weitere bevorzugte Ausführungsformen des Strukturelements E' entsprechen den bevorzugten Ausführungsformen von Strukturelement E mit der vorstehend beschriebenen Maßgabe.

Strukturelement G' ist mit Strukturelement G, wie vorstehend beschrieben, bis auf die Reste R_{G}¹² und R_{G}¹³ identisch. In Strukturelement G' sind die Reste R_{G}¹² und R_{G}¹³ von Strukturelement G durch die Reste R_{G'}¹² und R_{G'}¹³ ersetzt.

Die Reste R_{G'}¹² und R_{G'}¹³ haben in Strukturelement G' folgende Bedeutung:
Unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NRG¹¹RG¹¹* oder -CO-R_{G}¹⁴,
wobei R_{G}¹⁴ Wasserstoff, einen verzweigten oder unverzweigten, gegebenenfalls substituierten C₁-C₈-Alkyl-, C₂-C₆-Alkenyl-, C₂-C₆-Alkinyl- oder C₁-C₅-Alkylen-C₁-C₄-Alkoxy-rest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest bedeutet.

Bevorzugte Reste von R_{G'}¹² und R_{G'}¹³ sind die entsprechenden für R_{G}¹² und R_{G}¹³ vorstehend beschriebenen Reste.

Weitere bevorzugte Ausführungsformen des Strukturelements G' entsprechen den bevorzugten Ausführungsformen von Strukturelement G.

Die Verbindungen der Formel I' und auch die Zwischenprodukte zu ihrer Herstellung, können ein oder mehrere asymmetrische substituierte Kohlenstoffatome besitzen. Die Verbindungen können als reine Enantiomere bzw. reine Diastereomere oder als deren Mischung vorliegen. Bevorzugt ist die Verwendung einer enantiomerenreinen Verbindung als Wirkstoff.

Die Verbindungen der Formel I' können auch in anderen tautomeren Formen vorliegen.

Die Verbindungen der Formel I' können auch in Form von physiologisch verträglichen Salzen vorliegen.

Die Verbindungen der Formel I' können auch als Prodrugs in einer Form vorliegen, in der die Verbindungen der Formel I' unter physiologischen Bedingungen freigesetzt werden. Beispielhaft sei hier auf die Gruppe T in Strukturelement L verwiesen, die teilweise Gruppen enthält, die unter physiologischen Bedingungen zur freien Carbonsäuregruppe hydrolisierbar sind. Es sind auch derivatisierte Strukturelemente A geeignet, die das Strukturelement A unter physiologischen Bedingungen freisetzen.

Analog zu den Verbindungen der Formel I gilt für die Verbindungen der Formel I' wie vorstehend erwähnt:

Bei bevorzugten Verbindungen der Formel I' weist jeweils eines der vier Strukturelemente A, E', G' oder L den bevorzugten Bereich auf, während die restlichen Strukturelemente weit variabel sind.

Bei besonders bevorzugten Verbindungen der Formel I' weisen jeweils zwei der vier Strukturelemente A, E', G' oder L den bevorzugten Bereich auf, während die restlichen Strukturelemente weit variabel sind.

Bei weiter besonders bevorzugten Verbindungen der Formel I' weisen jeweils drei der vier Strukturelemente A, E', G' oder L den bevorzugten Bereich auf, während das restliche Strukturelement weit variabel ist.

Bei ganz besonders bevorzugten Verbindungen der Formel I' weisen alle vier Strukturelemente A, E', G' oder L den bevorzugten Bereich auf.

Bevorzugte Verbindungen der Formel I' weisen beispielsweise das bevorzugte Strukturelement G' auf, während die Strukturelemente A, E' und L weit variabel sind.

Weitere besonders bevorzugte Verbindungen der Formel I' weisen beispielsweise das bevorzugte Strukturelement G' und das bevorzugte Strukturelement A auf, während die Strukturelemente E' und L weit variabel sind.

Ganz besonders bevorzugte Verbindungen der Formel I' sind im folgenden aufgelistet, wobei die Zahl vor dem Textblock für die Nummer einer individualisierten Verbindung der Formel I' steht, und im Textblock A-E'-G'-L die Abkürzungen getrennt durch einen Bindungsstrich jeweils für ein einzelnes Strukturelement A, E', G' oder L stehen und die Bedeutung der Abkürzungen der Strukturelemente nach der Tabelle erläutert wird.

| Nr. | A-E'-G'-L |
|---|---|
| 1 | bhs-edia3-phen-es |
| 2 | 2py-inda2-phen-es |
| 3 | bhs-35thima2-meph-es |
| 4 | bim-dibema2-dmeph-es |
| 5 | 2py-bam2-4clph-es |
| 6 | 2py-dibema2-dmeph-es |
| 7 | bhs-a24thima2-dmeph-es |
| 8 | bhs-aaf-phen-es |
| 9 | bhs-a24thima2-4clph-es |
| 10 | bim-me42thiaz2-phen-es |
| 11 | 2py-edia2-phen-es |
| 12 | bim-a24thima2-hdb-es |
| 13 | 2py-apma2-4clph-es |
| 14 | gua-chex2-phen-es |
| 15 | bhs-dibema2-4clph-es |
| 16 | bhs-bam2-phen-es |
| 17 | bim-a23thima2-4clph-es |
| 18 | bim-dibema2-phen-es |
| 19 | bim-bam2-4clph-es |
| 20 | 2py-pipa2-4clph-es |
| 21 | 2py-me25thima2-phen-es |
| 22 | gua-a24thima2-ioph-es |
| 23 | imhs-apma2-phen-es |
| 24 | 2py-apma2-ioph-es |
| 25 | gua-edia3-phen-es |
| 26 | bhs-a23thima2-4clph-es |
| 27 | 2py-a24thima2-2pyph-es |
| 28 | 2py-bam2-thoph-es |
| 29 | gua-apma2-phen-es |
| 30 | 2py-a24thima2-reph-es |
| 31 | gua-hexa-phen-es |
| 32 | dimethpym-apma2-phen-es |
| 33 | 2py-dibema2-meph-es |
| 34 | bhs-apma2-phen-es |
| 35 | bim-edia3-phen-es |
| 36 | gua-apma2-meph-es |
| 37 | bim-apma2-reph-es |
| 38 | 2py-a24thima2-dmeph-es |
| 39 | gua-aaf-phen-es |
| 40 | gua-apma2-yrph-es |
| 41 | gua-pipeme2-phen-es |
| 42 | 2py-35thima2-phen-es |
| 43 | gua-a24thima2-reph-es |
| 44 | bim-bam2-phen-es |
| 45 | gua-a24thima2-phen-ms |
| 46 | gua-apma2-reph-es |
| 47 | mam2py-a24thima2-phen-es |
| 48 | 2py-a24thima2-phen-gs |
| 49 | 2py-apma2-phen-nes |
| 50 | 2py-a24thima2-4clph-es |
| 51 | bhs-penta-phen-es |
| 52 | gua-35thima2-dmeph-es |
| 53 | bim-dibema2-thoph-es |
| 54 | bim-a24thima2-reph-es |
| 55 | 2py-a23thima2-4clph-es |
| 56 | gua-a23thima2-phen-es |
| 57 | dhim-a24thima2-phen-es |
| 58 | gua-penta-phen-es |
| 59 | bhs-a24thima2-reph-es |
| 60 | 2py-a23thima2-meph-es |
| 61 | gua-prodia2-phen-es |
| 62 | bhs-apma2-reph-es |
| 63 | 2py-apma2-meph-es |
| 64 | gua-bam2-4clph-es |
| 65 | 2py-me35thima2-phen-es |
| 66 | gua-apma2-2pyph-es |
| 67 | 2py-35thima2-thoph-es |
| 68 | clim-a24thima2-phen-es |
| 69 | 2py-buta-phen-es |
| 70 | am2py-apma2-phen-es |
| 71 | gua-a24thima2-dmeph-es |
| 72 | bhs-apma2-hdb-es |
| 73 | bhs-dibema2-thoph-es |
| 74 | bim-dibema2-meph-es |
| 75 | bhs-bam2-meph-es |
| 76 | bhs-apma2-yrph-es |
| 77 | bhs-apma2-dmeph-es |
| 78 | gua-me25thima2-phen-es |
| 79 | 2py-a24thima2-meph-es |
| 80 | gua-inda2-phen-es |
| 81 | bhs-mepipe2-phen-es |
| 82 | bhs-a24thima2-phen-as |
| 83 | bim-pipa2-thoph-es |
| 84 | bhs-bam2-4clph-es |
| 85 | bhs-a24thima2-phen-es |
| 86 | bhs-35thima2-phen-es |
| 87 | bim-penta-phen-es |
| 88 | bhs-apma2-dbph-es |
| 89 | gua-42thiaz2-phen-es |
| 90 | bhs-a24thima2-24pym-es |
| 91 | 2py-dibema2-phen-es |
| 92 | bim-a24thima2-dm -es |
| 93 | bhs-pipa2-4clph-es |
| 94 | 2py-apma2-phen-ps |
| 95 | 2py-apma2-dmeph-es |
| 96 | 2py-mepipe2-phen-es |
| 97 | bhs-35thima2-4clph-es |
| 98 | gua-apma2-phen-mals |
| 99 | gua-35thima2-4clph-es |
| 100 | mam2py-apma2-phen-es |
| 101 | gua-buta-phen-es |
| 102 | 2py-a23thima2-thoph-es |
| 103 | 2py-pyma2-phen-es |
| 104 | gua-apma2-phen-gs |
| 105 | bim-apma2-phen-as |
| 106 | bhs-apma2-4clph-es |
| 107 | bhs-a24thima2-4pyph-es |
| 108 | thpym-apma2-phen-es |
| 109 | gua-pipa2-dmeph-es |
| 110 | amim-a24thima2-phen-es |
| 111 | bim-aepi2-phen-es |
| 112 | bim-a23thima2-thoph-es |
| 113 | bhs-a23thima2-thoph-es |
| 114 | gua-pdagk-phen-es |
| 115 | 2py-hexa-phen-es |
| 116 | bhs-a24thima2-meph-es |
| 117 | gua-bam2-meph-es |
| 118 | 2py-35thima2-meph-es |
| 119 | 2py-pipa2-meph-es |
| 120 | bhs-a23thima2-phen-es |
| 121 | bim-pipeme2-phen-es |
| 122 | bhs-buta-phen-es |
| 123 | bhs-pipa2-dmeph-es |
| 124 | bhs-pipa2-meph-es |
| 125 | 2py-35thima2-dmeph-es |
| 126 | bim-bam2-thoph-es |
| 127 | gua-35thima2-thoph-es |
| 128 | bim-edia2-phen-es |
| 129 | bim-apma2-phen-nes |
| 130 | gua-bam2-thoph-es |
| 131 | gua-bam2-phen-es |
| 132 | pippy-a24thima2-phen-es |
| 133 | gua-35thima2-phen-es |
| 134 | bim-a23thima2-phen-es |
| 135 | gua-dibema2-dmeph-es |
| 136 | bhs-apma2-phen-gs |
| 137 | bhs-apma2-thoph-es |
| 138 | 2py-apma2-phen-es |
| 139 | im-a24thima2-phen-es |
| 140 | gua-aepi2-phen-es |
| 141 | 2py-mea2-phen-es |
| 142 | gua-a24thima2-phen-es |
| 143 | 2py-a24thima2-thaph-es |
| 144 | gua-apma2-4clph-es |
| 145 | bhs-apma2-phen-f2es |
| 146 | bhs-inda2-phen-es |
| 147 | bim-a24thima2-dbph-es |
| 148 | bim-apma2-phen-ms |
| 149 | gua-a23thima2-thoph-es |
| 150 | pippy-apma2-phen-es |
| 151 | bhs-apma2-meph-es |
| 152 | 2py-apma2-phen-as |
| 153 | gua-mea2-phen-es |
| 154 | bhs-me35thima2-phen-es |
| 155 | bhs-pdagk-phen-es |
| 156 | bim-42thiaz2-phen-es |
| 157 | 2py-pipeme2-phen-es |
| 158 | bim-me25thima2-phen-es |
| 159 | gua-dibema2-phen-es |
| 160 | 2py-apma2-oxph-es |
| 161 | bhs-a24thima2-3clph-es |
| 162 | bim-pyma2-phen-es |
| 163 | bhs-edia2-phen-es |
| 164 | imhs-a24thima2-phen-es |
| 165 | gua-dibema2-thoph-es |
| 166 | bim-a24thima2-4clph-es |
| 167 | bim-apma2-phen-ps |
| 168 | gua-apma2-dm -es |
| 169 | 2py-a24thima2-phen-mals |
| 170 | 2py-dibema2-4clph-es |
| 171 | bhs-dibema2-meph-es |
| 172 | bim-aof-phen-es |
| 173 | bhs-pipeme2-phen-es |
| 174 | gua-35thima2-meph-es |
| 175 | bim-aaf-phen-es |
| 176 | bim-dibema2-4clph-es |
| 177 | bim-a24thima2-2pyph-es |
| 178 | bim-a24thima2-yrph-es |
| 179 | bim-35thima2-4clph-es |
| 180 | bhs-aepi2-phen-es |
| 181 | bim-pipa2-phen-es |
| 182 | gua-a23thima2-dmeph-es |
| 183 | 2py-a24thima2-yrph-es |
| 184 | gua-a24thima2-dmeoph-es |
| 185 | bhs-42thiaz2-phen-es |
| 186 | bim-mepipe2-phen-es |
| 187 | 2py-chex2-phen-es |
| 188 | bhs-prodia2-phen-es |
| 189 | gua-bam2-dmeph-es |
| 190 | bhs-me25thima2-phen-es |
| 191 | thpym-a24thima2-phen-es |
| 192 | bim-pipa2-dmeph-es |
| 193 | bhs-dibema2-phen-es |
| 194 | gua-a24thima2-thoph-es |
| 195 | 2py-pipa2-thoph-es |
| 196 | clim-apma2-phen-es |
| 197 | bhs-chex2-phen-es |
| 198 | gua-a24thima2-phen-nes |
| 199 | gua-a24thima2-meph-es |
| 200 | bhs-a24thima2-dmeoph-es |
| 201 | 2py-apma2-24pym-es |
| 202 | 2py-a24thima2-phen-pms |
| 203 | gua-a24thima2-4pyph-es |
| 204 | bim-apma2-3clph-es |
| 205 | gua-apma2-hdb-es |
| 206 | 2py-bam2-meph-es |
| 207 | bhs-a24thima2-phen-nes |
| 208 | gua-pyma2-phen-es |
| 209 | bim-a24thima2-thoph-es |
| 210 | gua-mepipe2-phen-es |
| 211 | bim-apma2-4clph-es |
| 212 | 2py-42thiaz2-phen-es |
| 213 | bim-apma2-24pym-es |
| 214 | bhs-a23thima2-dmeph-es |
| 215 | gua-apma2-dbph-es |
| 216 | gua-me35thima2-phen-es |
| 217 | bim-35thima2-phen-es |
| 218 | gua-apma2-thaph-es |
| 219 | bim-35thima2-thoph-es |
| 220 | gua-apma2-phen-f2es |
| 221 | 2py-apma2-3clph-es |
| 222 | gua-apma2-thoph-es |
| 223 | bim-pipa2-meph-es |
| 224 | 2py-aof-phen-es |
| 225 | bhs-35thima2-dmeph-es |
| 226 | bhs-hexa-phen-es |
| 227 | bim-pipa2-4clph-es |
| 228 | bhs-apma2-phen-pms |
| 229 | bim-a23thima2-dmeph-es |
| 230 | 2py-me42thiaz2-phen-es |
| 231 | bim-a24thima2-phen-gs |
| 232 | bim-apma2-dmeph-es |
| 233 | gua-a23thima2-4clph-es |
| 234 | bim-a24thima2-phen-mals |
| 235 | 2py-apma2-phen-ms |
| 236 | bhs-a24thima2-ioph-es |
| 237 | bim-a24thima2-phen-es |
| 238 | 2py-pdagk-phen-es |
| 239 | gua-a24thima2-phen-ps |
| 240 | 2py-pipa2-phen-es |
| 241 | 2py-aepi2-phen-es |
| 242 | 2py-a24thima2-thoph-es |
| 243 | bhs-bam2-dmeph-es |
| 244 | bim-hexa-phen-es |
| 245 | bim-a24thima2-meph-es |
| 246 | bhs-me42thiaz2-phen-es |
| 247 | am2py-a24thima2-phen-es |
| 248 | bim-apma2-meph-es |
| 249 | bim-me35thima2-phen-es |
| 250 | gua-pipa2-phen-es |
| 251 | bhs-a24thima2-oxph-es |
| 252 | 2py-pipa2-dmeph-es |
| 253 | 2py-apma2-4pyph-es |
| 254 | bhs-35thima2-thoph-es |
| 255 | gua-me42thiaz2-phen-es |
| 256 | bim-a24thima2-thaph-es |
| 257 | gua-a24thima2-phen-as |
| 258 | 2py-a24thima2-phen-f2es |
| 259 | 2py-a24thima2-dbph-es |
| 260 | bim-35thima2-meph-es |
| 261 | bim-apma2-phen-es |
| 262 | bim-a24thima2-phen-pms |
| 263 | bim-chex2-phen-es |
| 264 | bim-a24thima2-dmeph-es |
| 265 | bim-mea2-phen-es |
| 266 | 2py-apma2-thoph-es |
| 267 | dimethpym-a24thima2-phen-es |
| 268 | 2py-dibema2-thoph-es |
| 269 | 2py-apma2-dmeoph-es |
| 270 | gua-dibema2-4clph-es |
| 271 | bhs-pipa2-phen-es |
| 272 | gua-edia2-phen-es |
| 273 | gua-apma2-dmeph-es |
| 274 | 2py-edia3-phen-es |
| 275 | gua-a23thima2-meph-es |
| 276 | bim-pdagk-phen-es |
| 277 | gua-apma2-phen-pms |
| 278 | bhs-mea2-phen-es |
| 279 | bim-35thima2-dmeph-es |
| 280 | bhs-aof-phen-es |
| 281 | 2py-prodia2-phen-es |
| 282 | bim-inda2-phen-es |
| 283 | bhs-bam2-thoph-es |
| 284 | bim-apma2-4pyph-es |
| 285 | 2py-aaf-phen-es |
| 286 | 2py-bam2-phen-es |
| 287 | bhs-apma2-dm -es |
| 288 | 2py-penta-phen-es |
| 289 | gua-aof-phen-es |
| 290 | im -apma2-phen-es |
| 291 | gua-pipa2-4clph-es |
| 292 | bim-apma2-ioph-es |
| 293 | bim-bam2-meph-es |
| 294 | gua-pipa2-meph-es |
| 295 | bhs-apma2-thaph-es |
| 296 | bhs-apma2-2pyph-es |
| 297 | bim-apma2-dmeoph-es |
| 298 | amim-apma2-phen-es |
| 299 | dhim-apma2-phen-es |
| 300 | bhs-a24thima2-phen-ps |
| 301 | 2py-a23thima2-dmeph-es |
| 302 | gua-pipa2-thoph-es |
| 303 | bim-a23thima2-meph-es |
| 304 | 2py-bam2-dmeph-es |
| 305 | bhs-a24thima2-thoph-es |
| 306 | bhs-apma2-phen-mals |
| 307 | bhs-a23thima2-meph-es |
| 308 | bim-buta-phen-es |
| 309 | 2py-apma2-reph-es |
| 310 | gua-dibema2-meph-es |
| 311 | 2py-a24thima2-hdb-es |
| 312 | gua-a24thima2-4clph-es |
| 313 | bhs-pipa2-thoph-es |
| 314 | gua-a24thima2-3clph-es |
| 315 | gua-a24thima2-oxph-es |
| 316 | bim-bam2-dmeph-es |
| 317 | bim-apma2-thoph-es |
| 318 | bim-apma2-oxph-es |
| 319 | 2py-a24thima2-phen-es |
| 320 | bhs-a24thima2-phen-ms |
| 321 | bim-prodia2-phen-es |
| 322 | 2py-a24thima2-dm -es |
| 323 | bhs-pyma2-phen-es |
| 324 | bim-a24thima2-phen-f2es |
| 325 | 2py-a23thima2-phen-es |
| 326 | gua-a24thima2-24pym-es |
| 327 | 2py-35thima2-4clph-es |
| 328 | bhs-dibema2-dmeph-es |

In der vorstehenden Liste werden die folgenden Abkürzungen für die Bausteine A, E', G' und L verwendet.

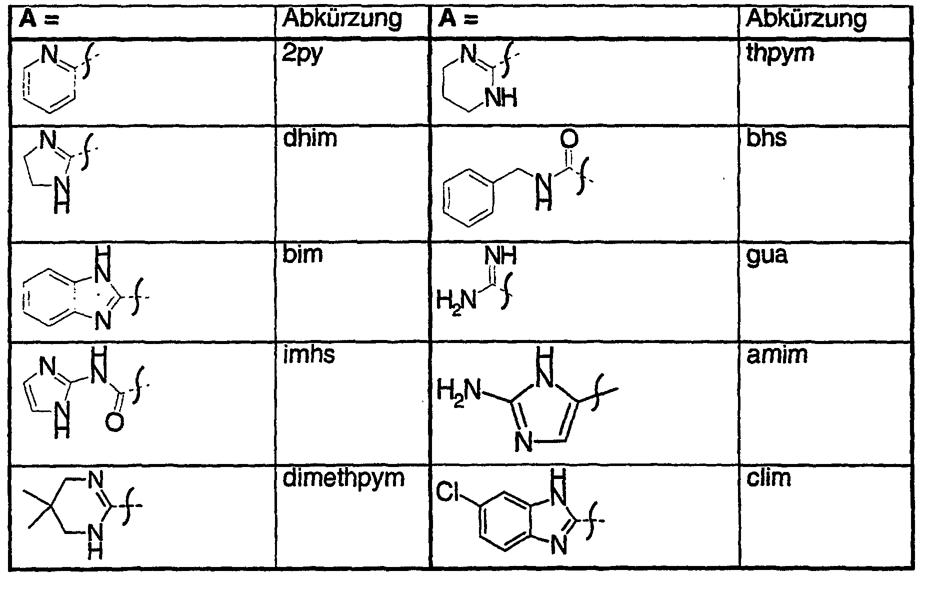

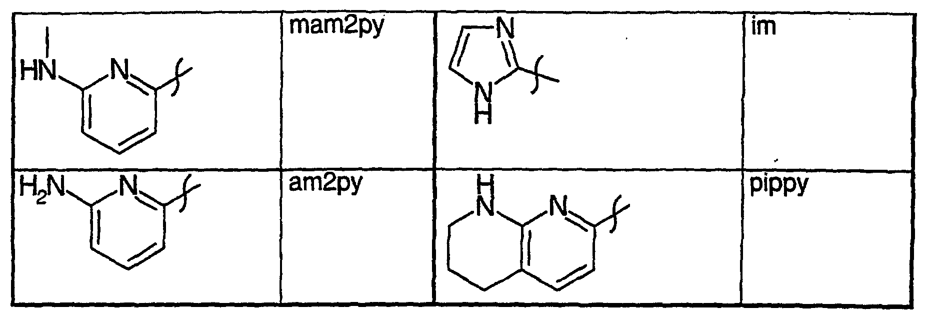

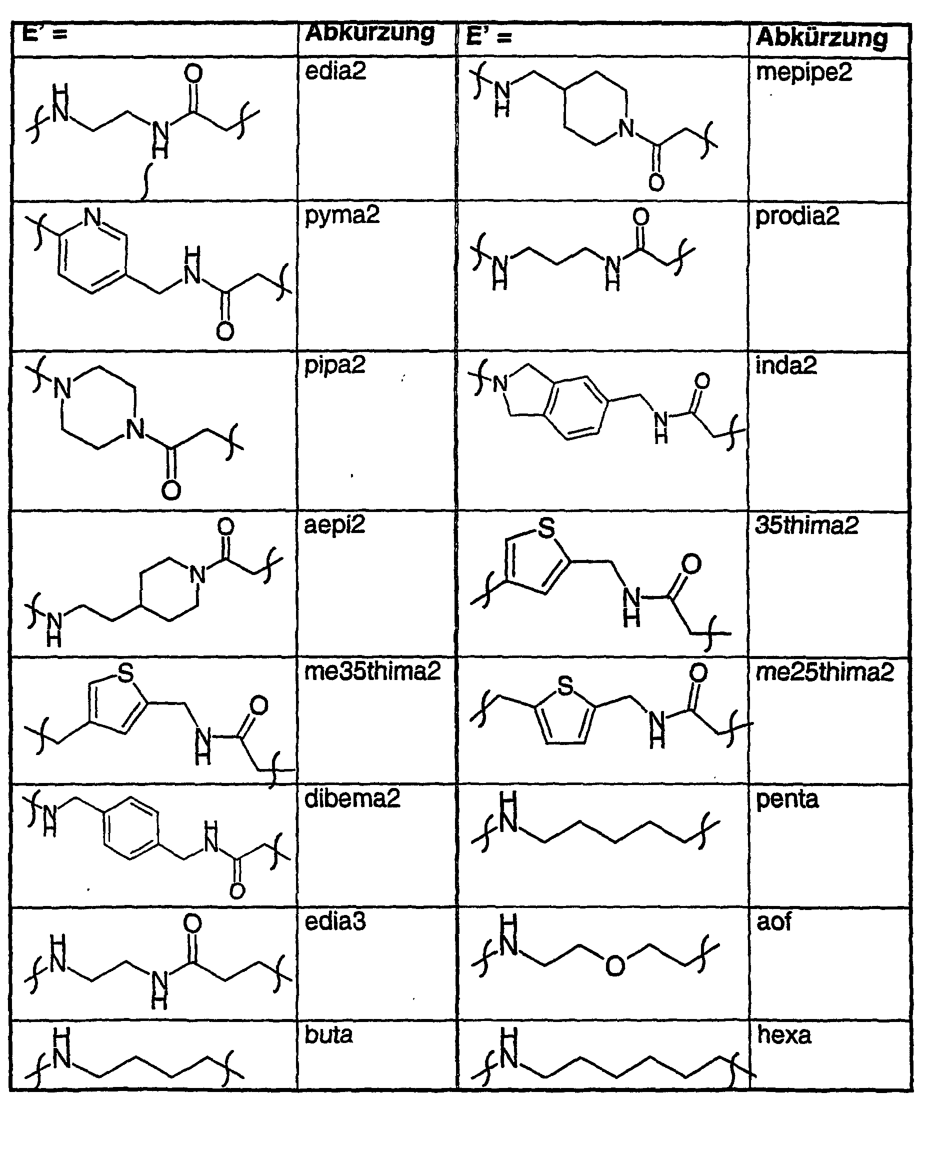

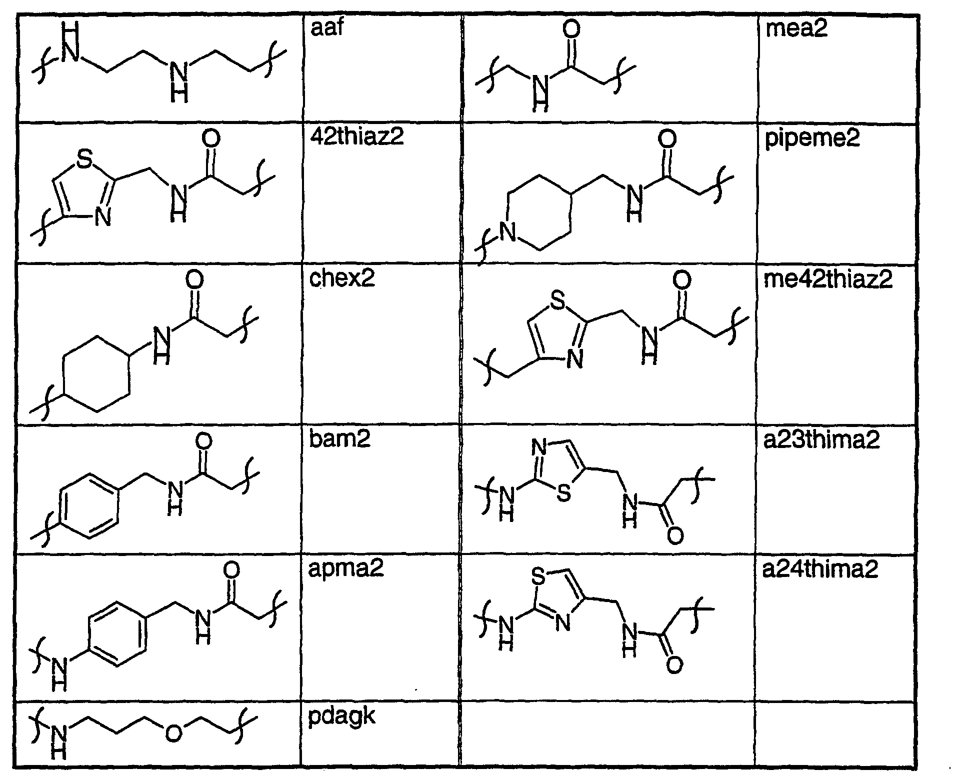

Die Bindung zum Baustein L soll in verbindung mit L = as als Doppelbindung verstanden werden.

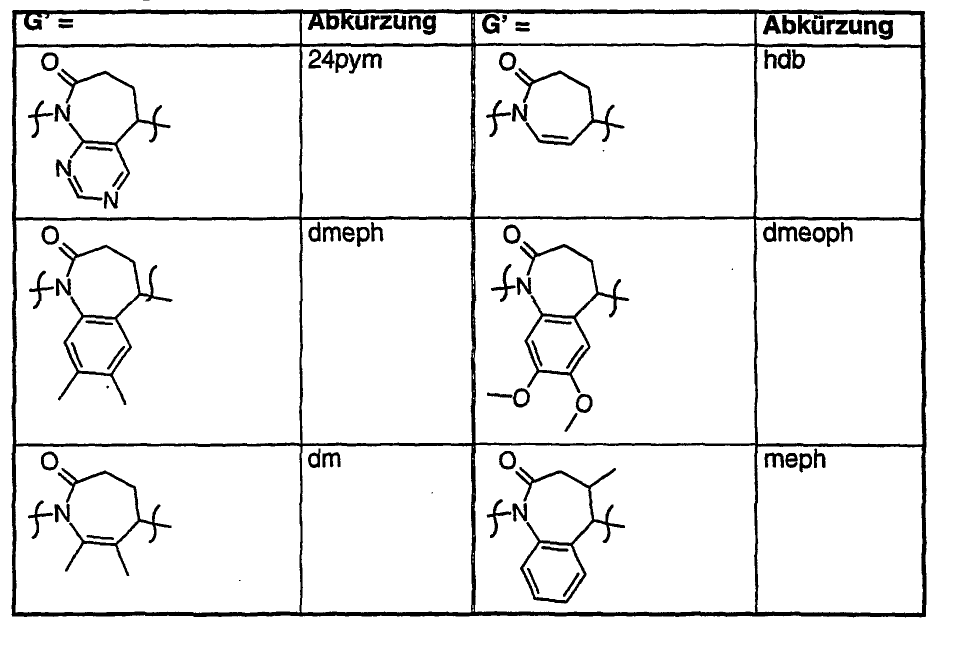

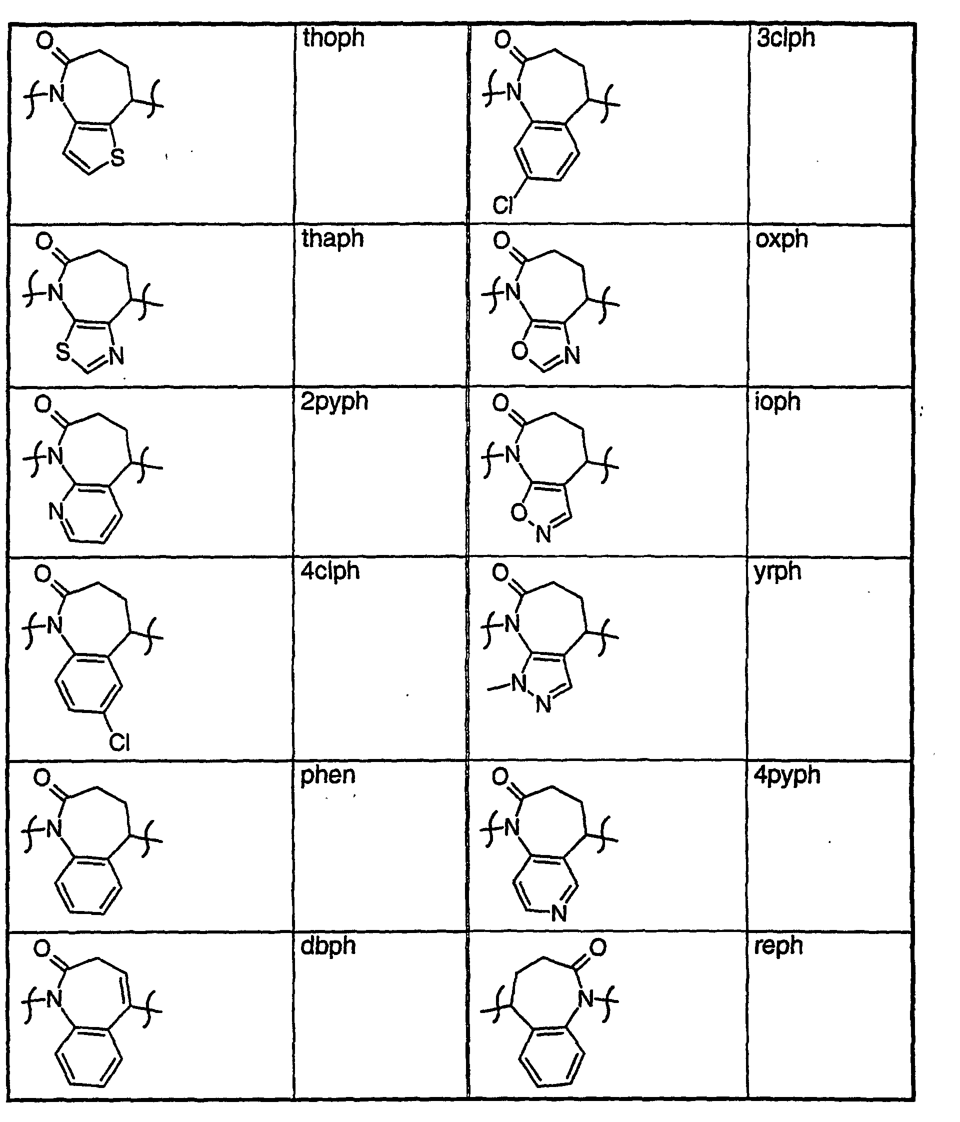

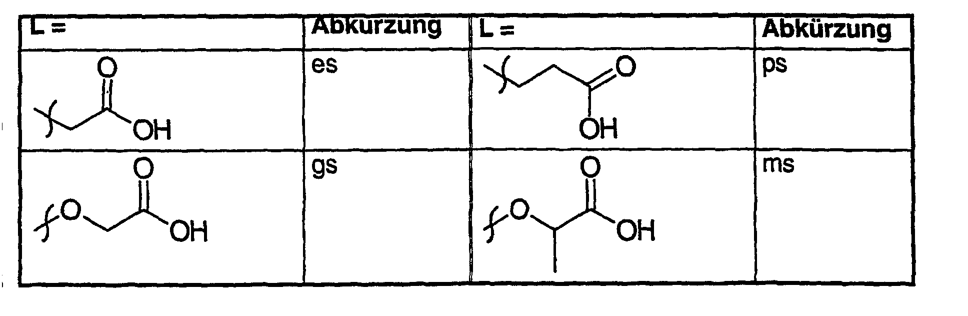

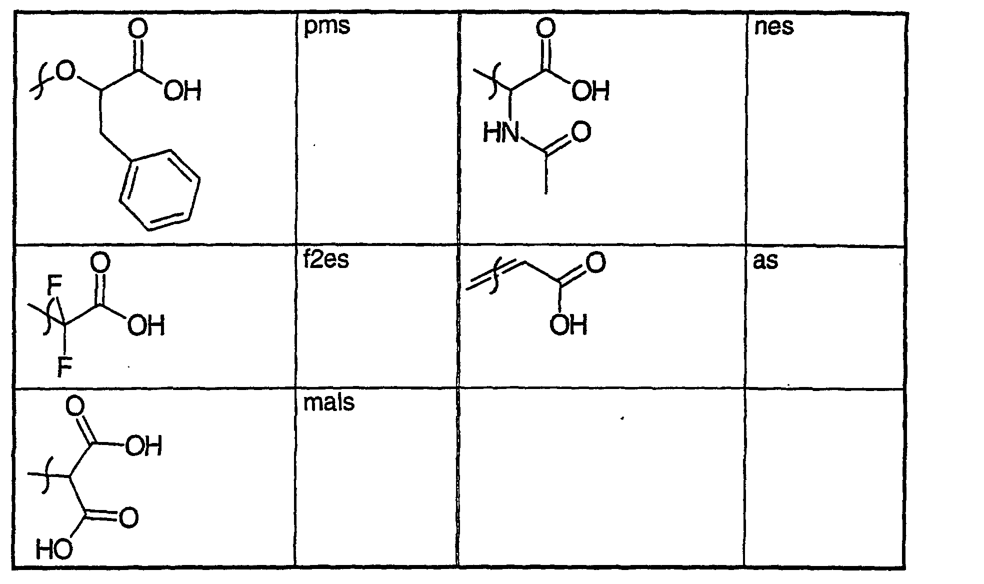

Die Verbindungen der allgemeinen Formel I und damit auch die Verbindungen der Formel I' sowie die zu ihrer Herstellung verwendeten Ausgangsstoffe lassen sich nach dem Fachmann bekannten Methoden der organischen Chemie herstellen, wie es in Standardwerken wie z.B. Houben-Weyl, "*Methoden der Organischen Chemie*", Thieme-Verlag, Stuttgart, oder March "*Advanced Organic Chemistry*", 4^{th} Edition, Wiley & Sons, beschrieben ist. Weitere Herstellungsmethoden sind auch in R. Larock, "Comprehensive Organic Transformations", Weinheim 1989 beschrieben, insbesondere die Herstellung von Alkenen, Alkinen, Halogeniden, Aminen, Ethern, Alkoholen, Phenolen, Aldehyden, Ketonen, Nitrilen, Carbonsäuren, Estern, Amiden und Säurechloriden.

Die Synthese von Verbindungen der Formel I kann entweder nach "klassischer" Methode in Lösung oder an einem polymeren Träger durchgeführt werden, wobei jeweils Reaktionsbedingungen verwendet wurden, wie sie für die jeweiligen Umsetzungen bekannt und geeignet sind. Dabei kann auch von an sich bekannten, hier nicht erwähnten Varianten Gebrauch gemacht werden.

Die allgemeine Synthese von Verbindungen der Formel I ist in den Schemata 1-7 beschrieben. Sofern nicht anders angegeben, sind sämtliche Ausgangsmaterialien und Reagenzien käuflich, oder lassen sich aus käuflich erhältlichen Vorprodukten nach gängigen Methoden herstellen.

Anelierte 2,3,4,5-Tetrahydro-1H-azepindione vom Typ **II** sind bekannt und lassen sich nach bekannten Methoden, z.B. ausgehend von Anthranilsäureestern bzw. den entsprechenden Heterocyclen-Analogen über Dieckmann-Kondensation und anschließende Decarboxylierung darstellen, wie es in folgenden Publikationen beschrieben ist: J. Am. Chem. Soc. 80, **1958**, 2172-2178; J. Chem. Soc. **1959**, 3111; J. Chem. Soc. **1934**, 1326; Arch. Pharm. 324, **1991**, 579-581. Die Herstellung von 3,4-Dihydro-1*H*-azepin-2,5-dion ist in Heterocycles 8, **1977**, 345-350, beschrieben.

Die Überführung in Verbindungen des Typs **III** erfolgt generell nach dem.Fachmann bekannten Methoden, wie sie z.B. in Larock, "Comprehensive Organic Transformations", Weinheim 1989, S. 167ff beschrieben sind, wobei auch hier nicht erwähnte Methoden zur Anwendung kommen können. Bevorzugt lassen sich Verbindungen der allgemeinen Formel **III** durch Umsetzung der Ketone **II** mit einem Phosphonester der allgemeinen Formel (EtO)₂P(=O) (X_{L})ₐ(CR_{L}¹R_{L}²)_{b}-COOSG1 in Gegenwart einer Base herstellen.

Die Reaktion findet bevorzugt in einem polaren aprotischen Lösungsmittel statt, wie z.B. Tetrahydrofuran, Dioxan; Dimethylformamid (DMF), Dimethylacetamid oder Acetamid; Dimethylsulfoxid, Sulfolan; N-Methylpyrrolidon, 2,3-Dimethyltetrahydro-2(1*H*)-pyrimidinon (DMPU), 1,3-Dimethyl-2-imidazolidinon; in einem Temperaturbereich -je nach Art des verwendeten Solvens- von -40°C bis zum Siedepunkt des entsprechenden Lösungsmittels.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, ein Alkoholat wie z.B. Natriummethanolat, Kalium*tert*.butanolat, eine metallorganische Verbindung wie Butyllithium oder Alkaliamide wie Lithiumdiisopropylamid, Lithium-, Natrium- oder Kaliumbis-(trimethylsilyl)-amid dienen.

Die Umsetzung zu **IV** wird durch Hydrierung der Doppelbindung unter Standardbedingungen durchgeführt. Auch hier kann von an sich bekannten aber nicht erwähnten Varianten Gebrauch gemacht werden. Bevorzugt wird die Hydrierung in Gegenwart eines Edelmetallkatalysators, wie z.B Pd auf Aktivkohle, Pt, PtO₂, Rh auf Al₂O₃ in einem inerten Lösungsmittel bei einer Temperatur von 0-150°C und einem Druck von 1-200bar durchgeführt; der Zusatz einer Säure wie z.B. Essigsäure oder Salzsäure kann vorteilhaft sein. Besonders bevorzugt ist die Hydrierung in Gegenwart von 5-10% Pd auf Aktivkohle.

Als Lösungsmittel können alle gängigen inerten Lösungsmittel verwendet werden wie z.B. Kohlenwasserstoffe wie Hexan, Heptan, Petrolether, Toluol, Benzol oder Xylol; chlorierte Kohlenwasserstoffe wie Trichlorethylen, 1,2-Dichlorethan, Tetrachlorkohlenstoff, Chloroform, Dichlormethan; Alkohole wie Methanol, Ethanol, Isopropanol, n-Propanol, n-Butanol oder tert.-Butanol; Ether wie Diethylether, Methyl-tert.butylether, Diisopropylether, Tetrahydrofuran, Dioxan; Glycolether wie Ethylenglycolmonomethylether oder -monoethylether, Ethylenglycoldimethylether; Ketone wie Aceton, Butanon; Amide wie Dimethylformamid (DMF), Dimethylacetamid oder Acetamid; Sulfoxide wie Dimethylsulfoxid, Sulfolan; Pyridin, N-Methylpyrrolidon, 1,3-Dimethyltetrahydro-2(1*H*)-pyrimidinon (DMPU), 1,3-Dimethyl-2-imidazolidinon, Wasser oder Gemische der genannten Lösungsmittel.

Die Darstellung von Verbindungen des Typs **V** erfolgt durch Umsetzung mit Verbindungen der allgemeinen Formel A-E-X¹ **(VI),** wobei der Rest X¹ für eine übliche Abgangsgruppe steht, beispielsweise Halogen wie Chlor, Brom, Iod oder gegebenenfalls durch Halogen, Alkyl oder Halogenalkyl substituiertes Aryl- oder Alkylsulfonyl wie z.B. Toluolsulfonyl, Trifluormethansulfonyl und Methylsulfonyl oder eine andere äquivalente Abgangsgruppe. Die Reaktion findet bevorzugt in einem inerten Lösungsmittel statt (wie zuvor beschrieben) unter Zusatz einer geeigneten Base, d.h. einer Base, die eine Deprotonierung des Zwischenproduktes **IV** bewirkt, in einem Temperaturbereich von -40°C bis zum Siedepunkt des entsprechenden Lösungsmittels.

Als Base kann ein Alkali- oder Erdalkalimetallhydrid wie Natriumhydrid, Kaliumhydrid oder Calciumhydrid, ein Carbonat wie Alkalimetallcarbonat, z.B. Natrium- oder Kaliumcarbonat, ein Alkali- oder Erdalkalimetallhydroxid wie Natrium- oder Kaliumhydroxid, ein Alkoholat wie z.B. Natriummethanolat, Kalium*tert.*butanolat, eine metallorganische Verbindung wie Butyllithium oder Alkaliamide wie Lithiumdiisopropylamid, Lithium-, Natrium- oder Kaliumbis-(trimethylsilyl)-amid dienen.

Abspaltung der Schutzgruppe SG1 nach Standardbedingungen (s. unten) führt zu den Verbindungen der allgemeinen Formel **I**. Für den Fall SG1 gleich C1-4-Alkyl oder Benzyl entsprechen die Verbindungen der allgemeinen Formel **V** direkt den Verbindungen des Typs **I.**

Alternativ zu dieser Synthesestrategie lassen sich Verbindungen des Typs **I** auch über **VII** als Zwischenprodukt herstellen, wobei auch hier Reaktionsbedingungen verwendet werden, wie sie dem Fachmann bekannt und in Standardwerken beschrieben sind. Die Herstellung der Verbindung **VII** erfolgt durch Umsetzung von Verbindungen des Typs **IV** mit Resten der allgemeinen Formel D_{E}-E-X² (**VIII**) unter Reaktionsbedingungen, wie sie für die Darstellung von **V** aus **IV** und **VI** schon beschrieben wurden. X² steht hier für eine geeignete Abgangsgruppe, wie sie schon für X¹ beschrieben wurde, und D_{E} für CN, N₃ oder eine geschützte Amino- oder Säurefunktion der allgemeinen Formel NSG3 oder COOSG2. Der Aufbau der Fragmente D_{E}-E bzw. A-E erfolgt -abhängig von der eigentlichen Struktur von E- durch Abspaltung der Schutzgruppen und Ankopplung der restlichen Fragmente nach Standardmethoden, z.B. Amidkupplungen. Die Einführung von A erfolgt dann analog zu den in den Schemata **3-7** beschriebenen Umsetzungen.

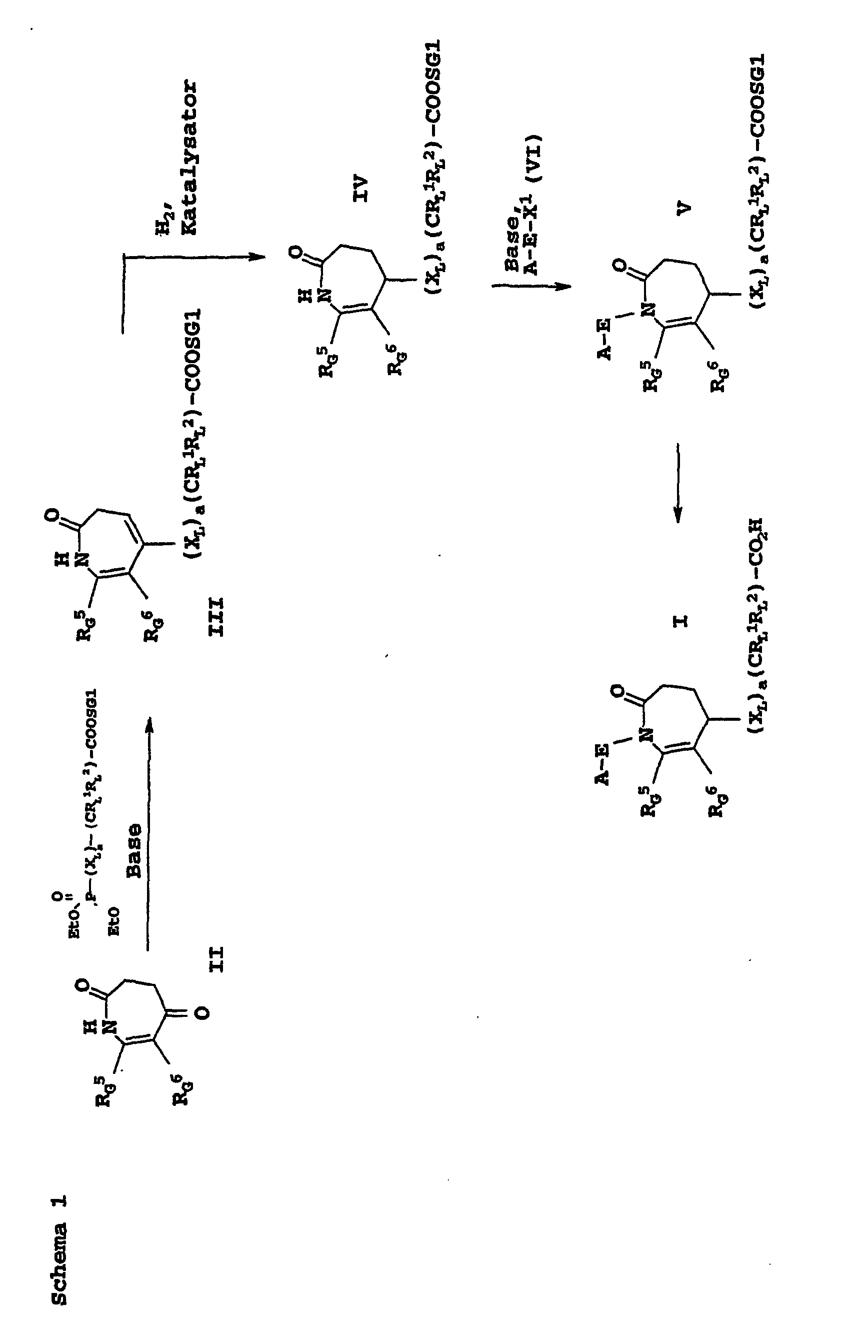

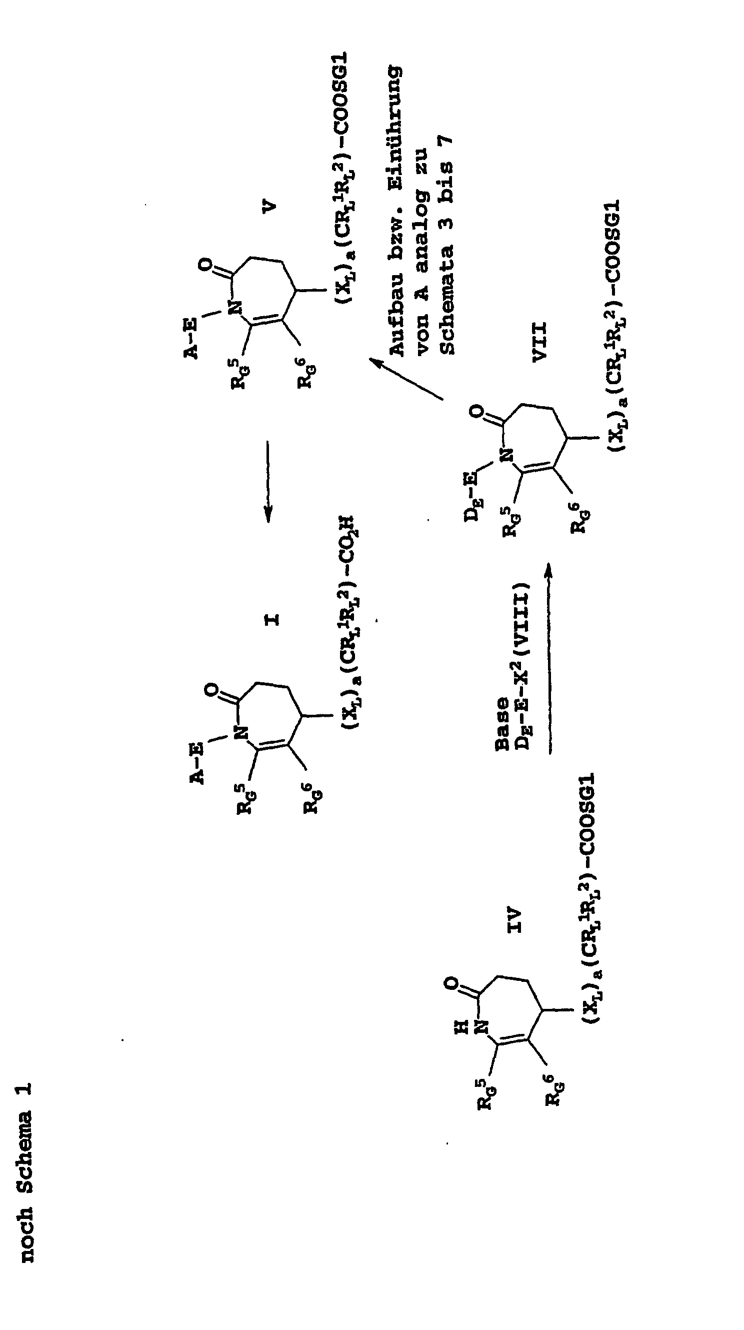

Verbindungen der Formel I, in denen in Strukturelement G W_{G} für ein Strukturelement der Formel I_{WG}¹ steht, lassen sich gemäß Schema 2 herstellen.

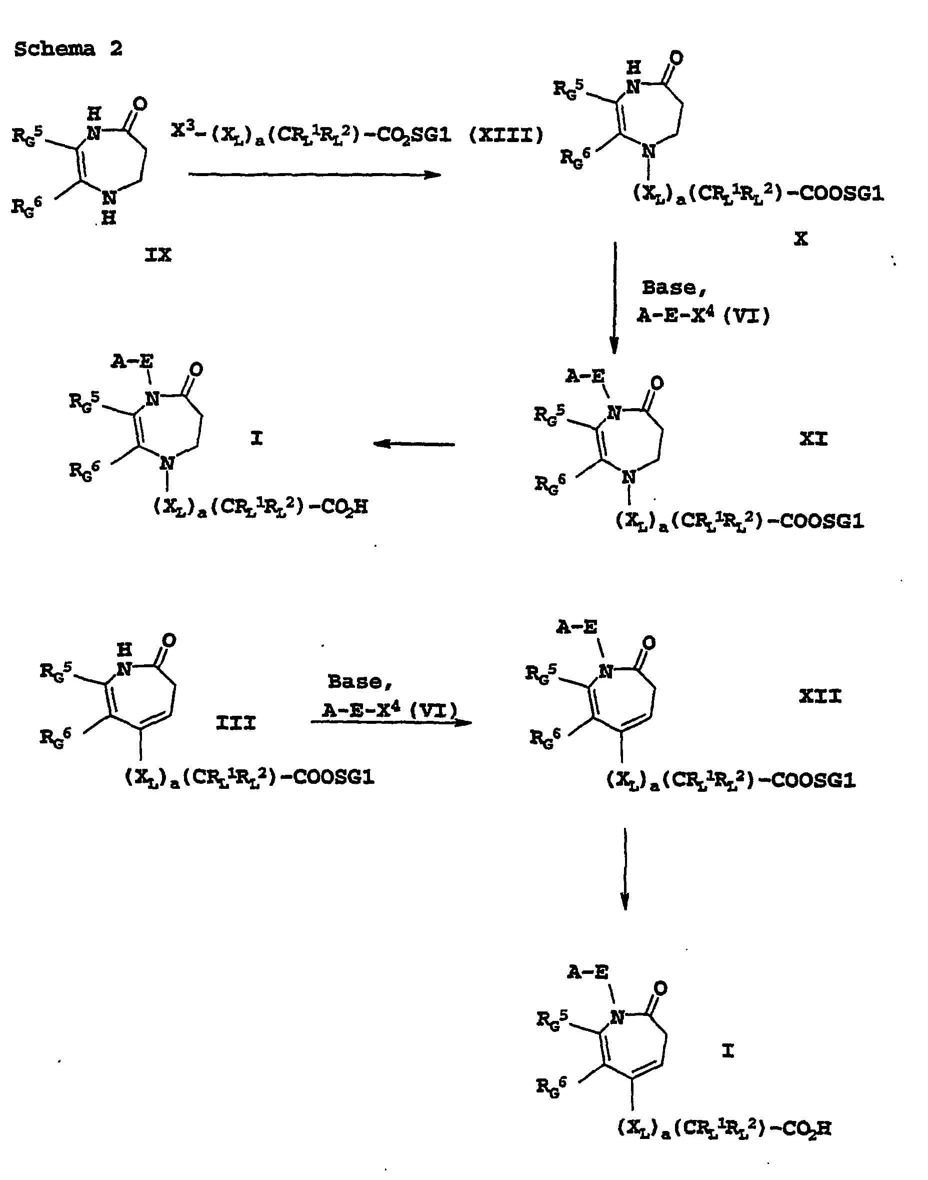

Ausgangspunkt der Synthese sind Verbindungen des Typs **IX,** die entweder bekannt sind bzw. dem Fachmann nach bekannten Methoden zugänglich sind, wie es z.B. in J. Am. Chem. Soc. 71, **1949**, 1985 beschrieben ist. Alkylierung mit einer Verbindung der allgemeinen Formel **XIII** (X³, X⁴ = übliche Abgangsgruppe) unter üblichen Reaktionsbedingungen führt zu **X**. Die weiteren Umsetzungen zu **I** verlaufen dann analog zu Schema 1 über Verbindungen des Typs **XI**.

Für den Fall, daß in Strukturelement G W_{G} für ein Strukturelement der Formel I_{WG}³ steht, können Verbindungen des Typs **III** analog zur Herstellung von **V** in Verbindungen des Typs **XII** und anschließend in **I** überführt werden (Schema 2).

Die Kupplung der einzelnen Fragmente und die Abspaltung der Schutzgruppen kann nach bekannten Verfahren erfolgen (s. Larock, "Comprehensive Organic Transformations; Schutzgruppen: Greene, T., "Protective Groups in Organic Synthesis", New York 1991), im Falle von Amidbindungen auch analog den Methoden der Peptidsynthese, wie in Standardwerken z.B. in Bodanszky "*The Practice of Peptide Synthesis*", 2^{nd} Edition, Springer-Verlag 1994, und Bodanszky "*Principles of Peptide Synthesis*", Springer-Verlag 1984, beschrieben ist. Eine allgemeine Übersicht der gängigen Methoden zur Peptidsynthese und eine Auflistung geeigneter Reagenzien ist weiterhin zu finden in NOVABIOCHEM **1999** *"Catalog and Peptide Synthesis Handbook"*.

Die genannten Amidkupplungen können mithilfe gängiger Kupplungsreagenzien unter Verwendung von geeignet geschützten Amino- und Carbonsäure-Derivaten durchgeführt werden. Eine andere Methode besteht in der Verwendung voraktivierter Carbonsäure-Derivate, vorzugsweise von Carbonsäurehalogeniden, symmetrischen oder gemischten Anhydriden oder sogenannter Aktivester, die üblicherweise zur Acylierung von Aminen verwendet werden. Diese aktivierten Carbonsäure-Derivate können auch in-situ hergestellt werden.

Die Kupplungen lassen sich in der Regel in inerten Lösungsmitteln in Gegenwart eines säurebindenden Mittels durchführen, vorzugsweise einer organischen Base wie z.B. Triethylamin, Pyridin, Diisopropylethylamin, N-Methylmorpholin, Chinolin; auch der Zusatz eines Alkali- oder Erdalkalimetallhydroxids, -carbonats oder -hydrogencarbonats oder eines anderen Salzes einer schwachen Säure der Alkali- oder Erdalkalimetalle, vorzugsweise des Kaliums, Natriums, Calciums oder Caesiums kann günstig sein.

Die Reaktionszeit liegt je nach verwendeten Bedingungen zwischen Minuten und 14 Tagen, die Reaktionstemperatur zwischen -40°C und 140°C, vorzugsweise zwischen -20°C und 100°C.

Als inerte Lösungsmittel eignen sich z.B. Kohlenwasserstoffe wie Hexan, Heptan, Petrolether, Toluol, Benzol oder Xylol; chlorierte Kohlenwasserstoffe wie Trichlorethylen, 1,2-Dichlorethan, Tetrachlorkohlenstoff, Chloroform, Dichlormethan; Alkohole wie Methanol, Ethanol, Isopropanol, n-Propanol, n-Butanol oder tert.-Butanol; Ether wie Diethylether, Methyl-tert.butylether, Diisopropylether, Tetrahydrofuran, Dioxan; Glycolether wie Ethylenglycolmonomethylether oder -monoethylether, Ethylenglycoldimethylether; Ketone wie Aceton, Butanon; Amide wie Dimethylformamid (DMF), Dimethylacetamid oder Acetamid; Nitrile wie Acetonitril; Sulfoxide wie Dimethylsulfoxid, Sulfolan; N-Methylpyrrolidon, 1,3-Dimethyltetrahydro-2(1*H*)-pyrimidinon (DMPU), 1,3-Dimethyl-2-imidazolidinon, Nitroverbindungen wie Nitromethan oder Nitrobenzol; Ester wie Ethylacetat (Essigester); Wasser; oder Gemische der genannten Lösungsmittel.

Als Schutzgruppen SG können alle dem Fachmann aus der Peptidsynthese bekannten und gängigen Schutzgruppen verwendet werden, wie sie auch in den oben genannten Standardwerken beschrieben sind. Die Abspaltung der Schutzgruppen in den Verbindungen der Formel **V, VII, XI** und **XII** erfolgt ebenfalls nach Bedingungen, wie sie dem Fachmann bekannt sind und z.B. von Greene und Wuts *in "Protective Groups in Organic Synthesis"*, 2^{nd} Edition, Wiley & Sons, 1991, beschrieben sind.

Bei Schutzgruppen wie SG3 handelt es sich um sogenannte N-terminale Aminoschutzgruppen; bevorzugt sind hier Boc, Fmoc, Benzyloxycarbonyl (Z), Acetyl, Mtr.

SG1 und SG2 stehen für Säureschutzgruppen bevorzugt sind hier C1-4-Alkyl wie z.B. Methyl, Ethyl, tert-Butyl, oder auch Benzyl oder Trityl, oder auch polymer gebundene Schutzgruppen in Form der handelsüblichen Poylstyrol-Harze wie z.B. 2-Chlortritylchloridharz oder Wang-Harz (Fa. Bachem, Fa. Novabiochem).

Die Abspaltung säurelabiler Schutzgruppen (z.B. Boc, tert.Butyl, Mtr, Trityl) kann -je nach verwendeter Schutzgruppe- mit organischen Säuren wie Trifluoressigsäure (TFA), Trichloressigsäure, Perchlorsäure, Triflurethanol; aber auch anorganischen Säuren wie Salzsäure oder Schwefelsäure, Sulfonsäuren wie Benzol- oder p-Toluolsulfonsäure erfolgen, wobei die Säuren generell im Überschuß eingesetzt werden. Bevorzugt werden HCl oder TFA verwendet. Im Falle von Trityl kann der Zusatz von Thiolen wie z.B. Thioanisol oder Thiophenol vorteilhaft sein. Die Anwesenheit eines zusätzlichen inerten Lösungsmittels ist möglich, aber nicht immer erforderlich. Als inerte Lösungsmittel eignen sich vorzugsweise organische Lösungsmittel, beispielsweise Carbonsäuren wie Essigsäure; Ether wie THF oder Dioxan; Amide wie DMF oder Dimethylacetamid; halogenierte Kohlenwasserstoffe wie Dichlormethan; Alkohole wie Methanol, Isopropanol; oder Wasser. Es kommen auch Gemische der genannten Lösungsmittel in Frage. Die Reaktionstemperatur für diese Umsetzungen liegt zwischen -10°C und 50°C, vorzugsweise arbeitet man in einem Bereich zwischen 0°C und 30°C.

Basenlabile Schutzgruppen wie Fmoc werden durch Behandlung mit organischen Aminen wie Dimethylamin, Diethylamin, Morpholin, Piperidin als 5-50% Lösungen in CH₂Cl₂ oder DMF gespalten. Die Reaktionstemperatur für diese Umsetzungen liegt zwischen -10°C und 50°C, vorzugsweise arbeitet man in einem Bereich zwischen 0°C und 30°C.

Säureschutzgruppen wie Methyl oder Ethyl werden bevorzugt durch basische Hydrolyse in einem inerten Lösungsmittel gespalten. Als Basen werden bevorzugt Alkali- oder Erdalkalimetallhydroxide, vorzugsweise NaOH, KOH oder LiOH verwendet; als Lösungsmittel kommen alle gängigen inerten Lösungsmittel wie z.B. Kohlenwasserstoffe wie Hexan, Heptan, Petrolether, Toluol, Benzol oder Xylol; chlorierte Kohlenwasserstoffe wie Trichlorethylen, 1,2-Dichlorethan, Tetrachlorkohlenstoff, Chloroform, Dichlormethan; Alkohole wie Methanol, Ethanol, Isopropanol, n-Propanol, n-Butanol oder tert.-Butanol; Ether wie Diethylether, Methyl-tert.butylether, Diisopropylether, Tetrahydrofuran, Dioxan; Glycolether wie Ethylenglycolmonomethylether oder -monoethylether, Ethylenglycoldimethylether; Ketone wie Aceton, Butanon; Amide wie Dimethylformamid (DMF), Dimethylacetamid oder Acetamid; Nitrile wie Acetonitril; Sulfoxide wie Dimethylsulfoxid, Sulfolan; ; N-Methylpyrrolidon, 1,3-Dimethyltetrahydro-2(1*H*)-pyrimidinon (DMPU), 1,3-Dimethyl-2-imidazolidinon; Nitroverbindungen wie Nitromethan oder Nitrobenzol; Wasser oder Gemische der genannten Lösungsmittel zum Einsatz. Der Zusatz eines Phasentransferkatalysators kann -je nach verwendetem Lösungsmittel bzw. -gemischs von Vorteil sein. Die Reaktionstemperatur für diese Umsetzungen liegt generell zwischen -10°C und 100°C.

Hydrogenolytisch abspaltbare Schutzgruppen wie Benzyloxycarbonyl (Z) oder Benzyl können z.B. durch Hydrogenolyse in Gegenwart eines Katalysators (z.B. eines Edelmetallkatalysators auf Aktivkohle als Träger) abgespalten werden. Als Lösungsmittel eignen sich die oben angegebenen, insbesondere Alkohole wie Methanol, Ethanol; Amide wie DMF oder Dimethylacetamid; Ester wie Ethylacetat. Die Hydrogenolyse wird in der Regel bei einem Druck von 1-200bar und Temperaturen zwischen 0° und 100°C durchgeführt; der .Zusatz einer Säure wie z.B. Essigsäure oder Salzsäure kann vorteilhaft sein. Als Katalysator wird bevorzugt 5-10% Pd auf Aktivkohle verwendet.

Der Aufbau von Bausteinen des Typs E erfolgt generell nach dem Fachmann bekannten Methoden; die verwendeten Bausteine sind entweder käuflich oder nach literaturbekannten Methoden zugänglich. Die Synthese einiger dieser Bausteine ist exemplarisch im experimentellen Teil beschrieben.

Für den Fall, daß die in den Verbindungen des Typs **VI** und **VIII** enthaltenden Fragmente Q_{E} bzw. **X**_{**E**} für einen Hetaryl-Rest stehen, so sind die verwendenten Bausteine entweder käuflich oder nach dem Fachmann bekannten Methoden zugänglich. Eine Vielzahl Herstellungsmethoden sind in Houben-Weyls "Methoden der organischen Chemie" ausführlich beschrieben (Bd. E6: Furane, Thiophene, Pyrrole, Indole, Benzothiophene, -furane, -pyrrole; Bd. E7: Chinoline, Pyridine, Bd. E8: Isoxazole, Oxazole, Thiazole, Pyrazole, Imidazole und deren benzoannellierte Vertreter, sowie Oxadiazole, Thiadiazole und Triazole; Bd. E9: Pyridazine, Pyrimidine, Triazine, Azepine und deren benzoannelierte Vertreter sowie Purine). Auch die Verknüpfung dieser Fragmente zu E kann, je nach Struktur von E, über die Amino- oder Säurefunktion nach Methoden erfolgen, die dem Fachmann bekannt sind.

Der Aufbau von Strukturen der allgemeinen Formel A-E-D_{E} erfolgt nach dem Fachmann bekannten Methoden, die z.B. in WO 97/08145 beschrieben sind. Beispiele hierfür sind die Überführung von Verbindungen der allgemeinen Formel:

HNR_{E}¹¹ -E_{A1}-D_{E} (XIV)

NC -E_{A2}-D_{E} (XV)

in Verbindungen der allgemeinen Formel:

A-HNR_{E}¹¹-E _{A1}-D_{E} (XVI)

A-E-D_{E} (XVII)

Die Gruppierungen **E**_{**A1**} und **E**_{**A2**} in den Formeln **XIV-XVIII** stehen für Strukturfragmente, die nach einer entsprechenden Modifikation (z.B. Umsetzung mit geeigneten Reagenzien oder Kupplung mit entsprechenden Bausteinen) in der Gesamtheit das Strukturfragment **A-E** bilden. Diese Bausteine können dann entweder direkt -im Fall der entsprechenden freien Amine bzw. Carbonsäuren- oder nach Abspaltung der Schutzgruppen- zu Verbindungen der allgemeinen Formel **I** (Schema **1** und **2**) umgesetzt werden. Prinzipiell kann A jedoch auch, wie in Schema **1** beschrieben, in Verbindungen des Typs **IV** eingeführt werden, wobei die angeführten Reaktionsbedingungen genauso wie hier nicht beschriebene Varianten zum Einsatz kommen können.

In den Schemata **3-7** sind eine Reihe der Methoden zur Einführung von **A** exemplarisch beschrieben, wobei jeweils Reaktionsbedingungen verwendet wurden, wie sie für die jeweiligen Umsetzungen bekannt und geeignet sind. Dabei kann auch von an sich bekannten, hier nicht erwähnten Varianten Gebrauch gemacht werden.

Harnstoffe bzw. Thioharnstoffe (**AE-1** bis **AE-3**) lassen sich nach gängigen Methoden der organischen Chemie herstellen, z.B. durch Umsetzung eines Isocyanats bzw. eines Thioisocyanats mit einem Amin, gegebenenfalls in einem inerten Lösungsmittel unter Erwärmen (Houben-Weyl Band VIII, 157ff.) (Schema **3**):

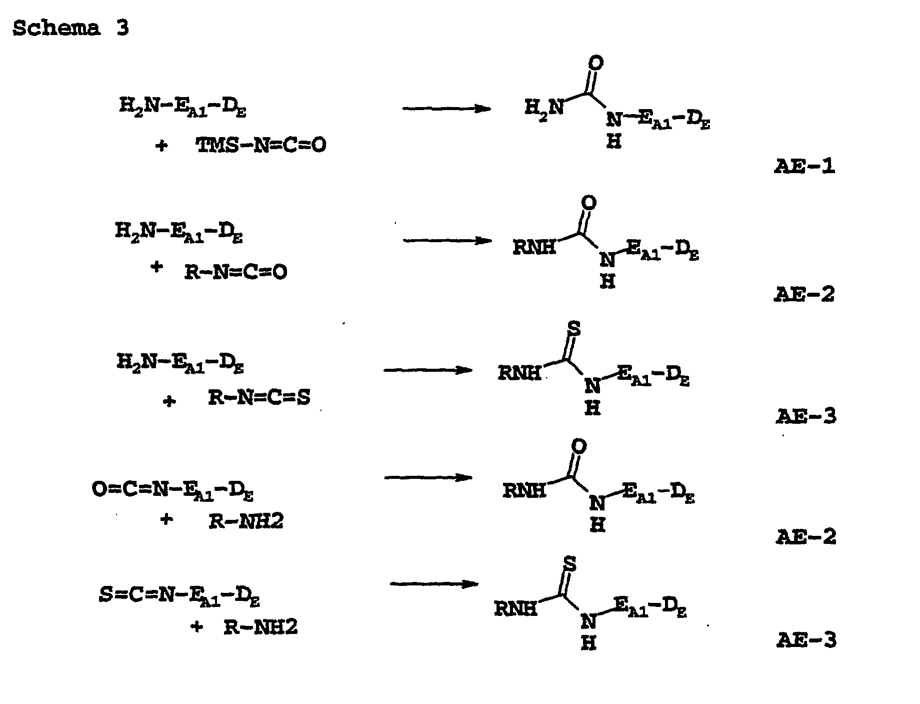

Schema **4** zeigt beispielhaft die Darstellung von Verbindungen des Typs **AE-4**, wie es z.B. von Blakemoore et al, in *Eur. J. Med. Chem*. **1987** (22) 2, 91-100, oder von Misra et al. in *Bioorg. Med. Chem. Lett*. **1994** 4 (18), 2165-2170 beschrieben ist.

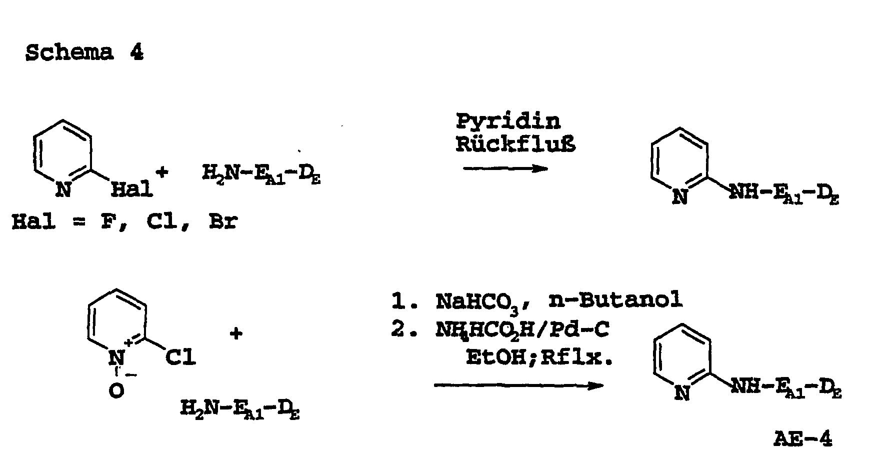

Unsubstituierte oder cycl. Guanidin-Derivate der allgemeinen Formel **AE-5** und **AE-6** lassen sich mittels käuflicher oder einfach zugänglichen Reagenzien herstellen, wie z.B. in *Synlett* ***1990***, 745, *J. Org. Chem.* ***1992***, 57, 2497, *Bioorg. Med. Chem*. ***1996***, 6, 1185-1208; *Bioorg. Med.* Chem. ***1998***, 1185, oder *Synth*. Comm. ***1998***, 28, 741-746, beschrieben (Schema 5).

Die Darstellung von Verbindungen der allgemeinen Formel **AE-7** kann analog zu US 3,202,660, Verbindungen der Formel **AE-9, AE-10, AE-11** und **AE-12** analog zu WO 97/08145 erfolgen. Verbindungen der Formel **AE-8** lassen sich, wie in Schema 6 gezeigt, z.B. gemäß der von Perkins et al., *Tetrahedron Lett*. **1999**, 40, 1103-1106, beschrieben Methode herstellen. Schema **5** gibt eine Übersicht über die Synthese der genannten Verbindungen an, wobei der Kreis in AE-8 für ein ankondensiertes cyclisches System, wie beispielsweise Aryl oder Hetaryl steht.

Verbindungen der allgemeinen Formel **AE-13** lassen sich analog zu Froeyen et al., *Phosphorus Sulfur Silicon Relat. Elem*. **1991**, 63, 283-293, **AE-14** analog zu Yoneda et al., *Heterocycles* **1998**, 15 N'-1, Spec. Issue, 341-344 (Schema 6) herstellen. Die Darstellung entsprechender Verbindungen kann auch analog WO 97/36859 erfolgen.

Verbindungen der allgemeinen Formel **AE-15** lassen sich gemäß Synthesis **1981**, 963-965 bzw. *Synth. Comm*. **1997**, 27 (15), 2701-2707, **AE-16** analog zu J. Org. Chem. **1991**, 56 (6), 2260-2262 herstellen (Schema 7), wobei der Kreis ein ankondensiertes cyclisches Sastem, wie beispielsweise Aryl, Hetaryl oder Cycloalkyl bedeutet.

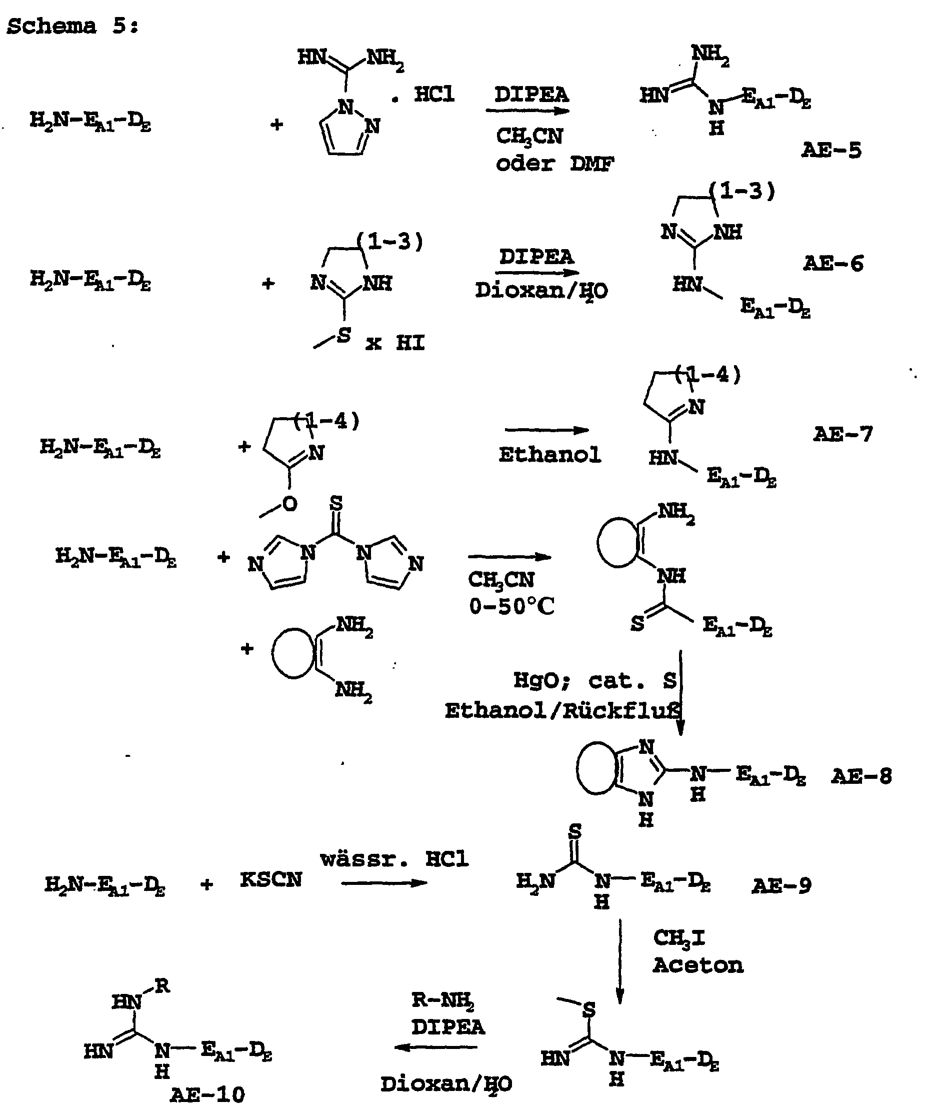

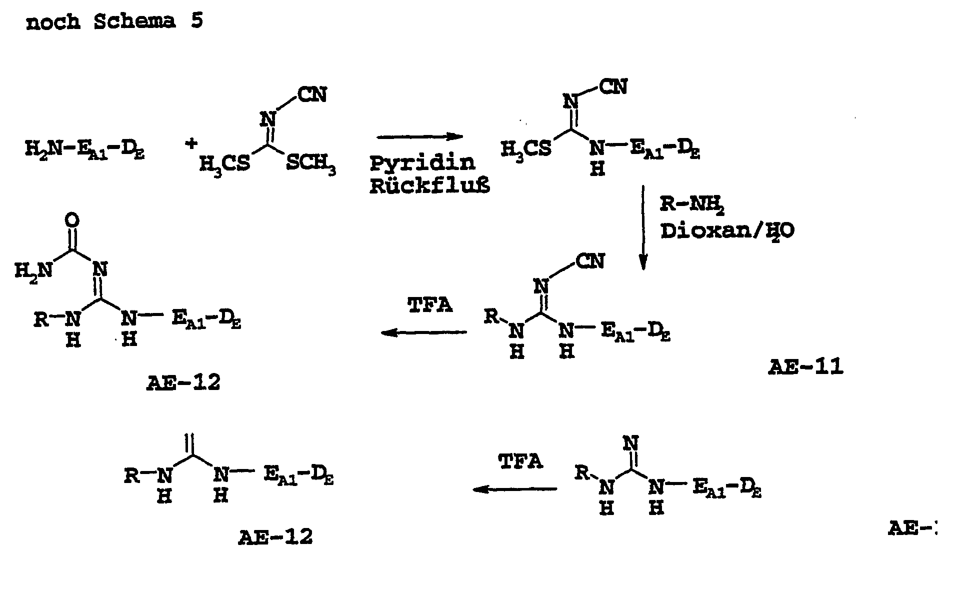

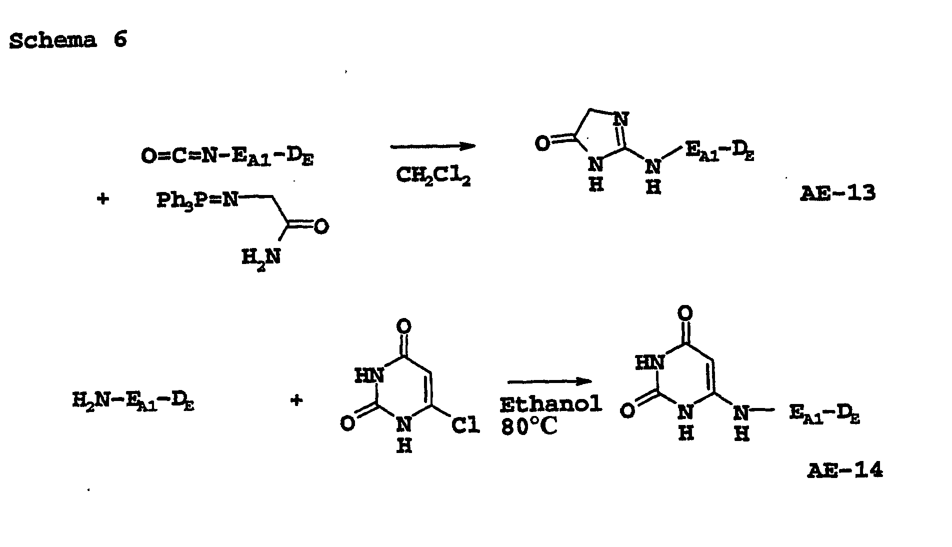

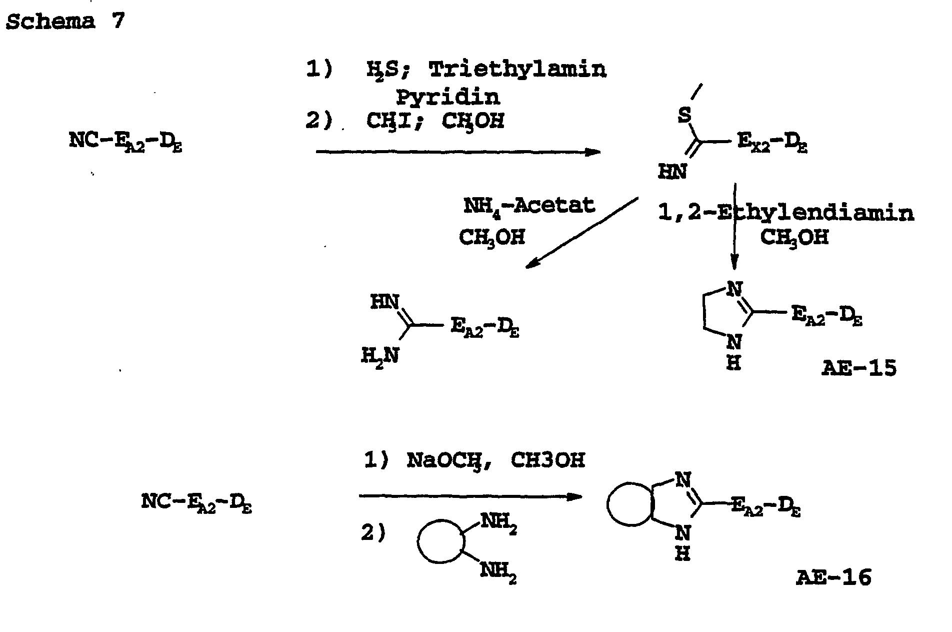

Die Erfindung betrifft ferner Arzneimittelzubereitungen, enthaltend neben den üblichen Arzneimittelhilfsstoffen mindestens eine Verbindung der Formel I'.

Die erfindungsgemäßen Verbindungen können in üblicher Weise oral oder parenteral (subkutan, intravenös, intramuskulär, intraperetoneal) verabreicht werden. Die Applikation kann auch mit Dämpfen oder Sprays durch den Nasen-Rachenraum erfolgen. Ferner können die erfindungsgemäßen Verbindungen durch direkten Kontakt mit dem betroffenen Gewebe eingebracht werden.

Die Dosierung hängt vom Alter, Zustand und Gewicht des Patienten sowie von der Applikationsart ab. In der Regel beträgt die tägliche Wirkstoffdosis zwischen etwa 0,5 und 50 mg/kg Körpergewicht bei oraler Gabe und zwischen etwa 0,1 und 10 mg/kg Körpergewicht bei parenteraler Gabe.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1991). Die so erhaltenen Applikationsformen enthalten den Wirkstoff normalerweise in einer Menge von 0,1 bis 90 Gew.-%.

Ferner betrifft die Erfindung die Verwendung der Verbindungen der Formel I' zur Herstellung von Arzneimitteln zur Behandlung von Krankheiten. Die Verbindungen der Formel I' können zur Behandlung von humanen und tierischen Krankheiten verwendet werden. Die Verbindungen der Formel I', die die neuen Verbindungen der Formel I darstellen, binden, wie vorstehend erwähnt an Integrinrezeptoren. Sie eignen sich deshalb, wie vorstehend erwähnt, vorzugsweise als Integrin-Rezeptorliganden und zur Herstellung von Arzneimitteln zur Behandlung von Krankheiten in denen ein Integrinrezeptor involviert ist, insbesondere zur Behandlung von Krankheiten, bei denen die Wechselwirkung zwischen Integrinen und ihren natürlichen Liganden fehlreguliert, also überhöht oder erniedrigt ist, wie vorstehend beschrieben.

Vorteilhafterweise können die Verbindungen der Formel I, vorzugsweise die Verbindungen der Formel I' in Kombination mit mindestens einer weiteren Verbindung verabreicht werden, um in einer Reihe von Indikationen eine verbesserte Heilwirkung zu erreichen. Diese weiteren Verbindungen können den gleichen oder einen anderen Wirkmechanismus wie die Verbindungen der Formel I aufweisen.

Die Arzneimittelzubereitungen können daher neben den Verbindungen der Formel I, vorzugsweise neben den Verbindungen der Formel I' und den üblichen Arzneimittelhilfsstoffen mindestens eine weitere Verbindung, abhängig von der Indikation jeweils aus einer der nachstehenden 10 Gruppen ausgewählt, enthalten.
Gruppe 1:
   Inhibitoren der Blutplättchenadhäsion, -aktivierung oder -aggregation, wie beispielsweise Acetylsalicylsäure, Lysinacetylsalicylat, Pilacetym, Dipyridamol, Abciximab, Thromboxane-Antagonisten, Fibrinogen-Antagonisten, wie beispielsweise Tirofiban, oder Inhibitoren der ADP-induzierten Aggregation wie beispielsweise Ticlopidin oder Clopidogrel,
   Antikoagulantien, die die Thrombinaktivität oder -bildung verhindern, wie beispielsweise Inhibitoren von IIa, Xa, XIa, IXa oder VIIa,
   Antagonisten von blutplättchenaktivierenden Verbindungen und Selectin-Antagonisten
   zur Behandlung von blutplättchenvermitteltem vaskulärem Verschluß oder Thrombose, oder
Gruppe 2:
   Inhibitoren der Blutplättchenaktivierung oder -aggregation, wie beispielsweise GPIIb/IIIa-Antagonisten, Thrombin- oder Faktor Xa-Inhibitoren oder ADP-Rezeptor-Antagonisten,
   Serin-Protease Inhibitoren,
   Fibrinogen-senkende Verbindungen,
   Selectin-Antagonisten,
   Antagonisten von ICAM-1 oder VCAM-1
   Inhibitoren der Leukozytenadhäsion
   Inhibitoren der Gefäßwandtransmigration,
   Fibrinolyse-modulierende Verbindungen, wie beispielsweise Streptokinase, tPA, Plasminogenaktivierungs-Stimulantien, TAFI-Inhibitoren, XIa Inhibitoren oder PAI-1-Antagonisten,
   Inhibitoren von Komplementfaktoren,
   Endothelinrezeptor-Antagonisten,
   Tyrosinkinase-Inhibitoren,
   Antioxidantien und
   Interleukin 8 Antagonisten
   zur Behandlung von Myokardinfarkt oder Schlaganfall, oder
Gruppe 3:
   Endothelinantagonisten,
   ACE-Inhibitoren,
   Angiotensinrezeptorantagonisten,
   Endopeptidase Inhibitoren,
   Beta-Blocker,
   Kalziumkanal-Antagonisten,
   Phosphodiesterasehemmer und
   Caspaseinhibitoren
   zur Behandlung von kongestiven Herzversagen, oder
Gruppe 4:
   Thrombininhibitoren,
   Inhibitoren des Faktors Xa,
   Inhibitoren des Koagulationsweges der zur Thrombinbildung führt, wie beispielsweise Heparin oder niedermolekulare Heparine, Inhibitoren der Blutplättchenadhäsion, -aktivierung oder -aggregation, wie beispielsweise GPIIb-IIIa-Antagonisten oder Antagonisten der durch vWF oder GPIb vermittelten Blutplättchenadhäsiion und Aktivierung,
   Endothelinrezeptor-Antagonisten,
   Stickstoffoxidsynthasehemmer,
   CD44-Antagonisten,
   Selectin-Antagonisten,
   MCP-1-Antagonisten,
   Inhibitoren der Signaltransduktion in proliferierenden Zellen,
   Antagonisten der durch EGF, PDGF, VEGF oder bFGF vermittelten Zellantwort und
   Antioxidantien
   zur Behandlung von Restenose nach Gefäßverletzung oder Stentimplantation, oder
Gruppe 5:
   Antagonisten der durch EGF, PDGF, VEGF oder bFGF vermittelten Zellantwort,
   Heparin oder niedermolekulare Heparine oder weitere GAGs,
   Inhibitoren von MMPs,
   Selectin-Antagonisten,
   Endothelin-Antagonisten,
   ACE-Inhibitoren,
   Angiotensinrezeptor-Antagonisten und
   Glycosilierungshemmer oder AGE-Bildungs-Inhibitoren oder AGE-Breaker und Antagonisten Ihrer Rezeptoren, wie beispielsweise RAGE,
   zur Behandlung von diabetischen Angiopathien oder
Gruppe 6:
   fettsenkende Verbindungen,
   Selectin-Antagonisten,
   Antagonisten von ICAM-1 oder VCAM-1
   Heparin oder niedermolekulare Heparine oder weitere GAGs,
   Inhibitoren von MMPs,
   Endothelinantagonisten,
   Apolipoprotein A1-Antagonisten,
   Cholesterol-Antagonisten,
   HMG CoA Reduktase-Inhibitoren,
   ACAT Inhibitoren,
   ACE Inhibitoren,
   Angiotensinrezeptorantagonisten,
   Tyrosinkinaseinhibitoren,
   Proteinkinase C-Inhibitoren,
   Kalzium-Kanal-Antagonisten,
   LDL-Rezeptor-Funktionsstimulantien,
   Antioxidantien
   LCAT-Mimetika und
   Freie Radikal-Fänger
   zur Behandlung von Atherosklerose oder
Gruppe 7:
   cytostatische oder antineoplastische Verbindungen,
   Verbindungem die die Proliferation inhibieren, wie beispielsweise Kinaseinhibitoren und
   Heparin oder niedermolekulare Heparine oder weitere GAGs
   zur Behandlung von Krebs, vorzugsweise zur Inhibierung von Tumorwachstum oder -metastase, oder
Gruppe 8:
   Verbindungen zur Anti-resorptiven Therapie,
   Verbindungen zur Hormon-Austausch-Therapie, wie beispielsweise Östrogen- oder Progesteron-Antagonisten,
   Rekombinantes humanes Wachstumshormon,
   Bisphosphonate, wie beispielsweise Alendronate
   Verbindungen zur Calcitonintherapie,
   Calcitoninstimulantien,
   Kalzium-Kanal-Antagonisten,
   Knochenbildungsstimulantien, wie beispielsweise Wachstumsfaktoragonisten,
   Interleukin-6-Antagonisten und
   Src Tyrosinkinase-Inhibitoren
   zur Behandlung von Osteoporose oder
Gruppe 9:
   TNF-Antagonisten,
   Antagonisten von VLA-4 oder VCAM-1,
   Antagonisten von LFA-1, Mac-1 oder ICAMs,
   Komplementinhibitoren,
   Immunosuppressiva,
   Interleukin-1-, -5- oder -8-Antagonisten und
   Dihydrofolatreduktase-Inhibitoren
   zur Behandlung von rheumatoider Arthritis oder
Gruppe 10:
   Collagenase,
   PDGF-Antagonisten und
   MMPs
zur verbesserten Wundheilung.

Unter einer Arzneimittelzubereitungen, enthaltend mindestens eine Verbindung der Formel I, vorzugsweise enthaltend mindestens eine Verbindung der Formel I', gebenenfalls Arzneimittelhilfsstoffe und mindestens eine weitere Verbindung, abhängig von der Indikation jeweils aus einer der vorstehenden Gruppen ausgewählt, wird eine kombinierte Verabreichung mindestens einer der Verbindungen der Formel I. vorzugsweise einer der Verbindungen der Formel I' mit mindestens einer weiteren Verbindung jeweils ausgewählt aus einer der vorstehend beschriebenen Gruppen und gegebenenfalls Arzneimittelhilfstoffen, verstanden.

Die kombinierte Verabreichung kann durch ein Stoffgemisch, enthaltend mindestens eine Verbindung der Formel I, vorzugsweise der Formel I', gebenenfalls Arzneimittelhilfsstoffe und mindestens eine weitere Verbindung, abhängig von der Indikation jeweils aus einer der vorstehenden Gruppen ausgewählt, aber auch räumlich und/oder zeitlich getrennt erfolgen.

Bei der räumlich und/oder zeitlich getrennten Verabreichung erfolgt die Verabreichung der Komponenten der Arzneimittelzubereitung, die Verbindungen der Formel I, vorzugsweise der Formel I' und die Verbindungen ausgewählt aus einer der vorstehend erwähnten Gruppen räumlich und/oder zeitlich getrennt.

Zur Behandlung von Restenose nach Gefäßverletzung oder *Stenting* kann die Verabreichungen der Verbindungen der Formel I, vorzugsweise der Formel I' alleine oder in Kombination mit mindestens einer Verbindung ausgewählt aus der Gruppe 4 lokal auf die betroffenen Stellen erfolgen. Auch kann es vorteilhaft sein, die Stents mit diesen Verbindungen zu überziehen.

Zur Behandlung von Osteoporose kann es vorteilhaft sein, die Verabreichung der Verbindungen der Formel I, vorzugsweise der Formel I' in Kombination mit einer antiresorptiven oder Hormonaustausch-Therapie durchzuführen.

Die Erfindung betrifft demnach die Verwendung der vorstehend erwähnten Arzneimittelzubereitungen zur Herstellung von Arzneimittel zur Behandlung von Krankheiten.

In einer bevorzugten Ausführungsform betrifft die Erfindung die Verwendung der vorstehend erwähnten kombinierten Arzneimittelzubereitungen zur Herstellung von Arzneimitteln zur Behandlung von
Blutplättchen vermitteltem vaskulärem Verschluß oder Thrombose
   bei Verwendung von Verbindungen der Gruppe 1,
Myokardinfarkt oder Schlaganfall
   bei Verwendung von Verbindungen der Gruppe 2,
kongestivem Herzversagen
   bei Verwendung von Verbindungen der Gruppe 3,
Restenose nach Gefäßverletzung oder Stentimplantation
   bei Verwendung von Verbindungen der Gruppe 4,
diabetischen Angiopathien
   bei Verwendung von Verbindungen der Gruppe 5,
Atherosklerose
   bei Verwendung von Verbindungen der Gruppe 6,
Krebs
   bei Verwendung von Verbindungen der Gruppe 7,
Osteoporose
   bei Verwendung von Verbindungen der Gruppe 8,
Rheumatoider Arthritis
   bei Verwendung von Verbindungen der Gruppe 9,
Wundheilung
   bei Verwendung von Verbindungen der Gruppe 10.

Die folgenden Beispiele erläutern die Erfindung, wobei die Auswahl dieser Beispiele nicht limitierend ist.

### I. Synthesebeispiele

### I.A Vorstufen

### (2-Oxo-2,3-dihydro-1H-1-benzazepin-5-yl)essigsäure-t.butylester (1)

Zu einer Suspension aus 3.27g NaH (60%; entölt) in 35ml DMF wurden bei 0°C 22.3g (80mMol) Diethylphosphonessigsäure-t.butylester (95%) zugetropft. Die Mischung wurde bis zur Bildung einer klaren Lösung gerührt und anschließend bei 0°C 12.4g (70.9mMol) 3,4-Dihydro-1H-1-benzazepin-2,5-dion (Darstellung nach *Arch. Pharm. 1991, 324, 579*) in 90ml DMF zugetropft. Das Reaktionsgemisch blieb dann ca. 3Tage lang bei RT stehen. Zur Aufarbeitung wurde die Mischung in 700ml kalte 5% NaCl-Lösung eingegossen, der entstandene gelbe Niederschlag abgesaugt und mit H₂O nachgewaschen. Der feuchte Rückstand wurde in CH₂Cl₂ aufgenommen, mit 5% NaHCO₃-Lösung gewaschen und über Na₂SO₄ getrocknet. Der nach dem Eindampfen verbliebene Rückstand wurde in der Wärme mit 150ml Cyclohexan behandelt, nach Abkühlen, Absaugen und Waschen mit n-Hexan verblieben 17.5g (90.5%) weiße Kristalle; Fp.: 136-138°C.

### (2-Oxo-2,3,4,5-tetrahydro-1H-1-benzazepin-5-yl)essigsäure-t.butylester (2)

Eine Suspension von 3g 10% Pd/C in 50ml Ethanol wurde vorhydriert, anschließend eine Lösung von Verbindung **1** (14.7g; 53.8mMol) in 125ml Ethanol und 75ml Dioxan zugegeben, und bis zur Beendigung der Wasserstoffaufnahme unter Normalbedingungen hydriert. Nach Absaugen und Auswaschen des Katalysators mit Ethanol wurde im Vakuum eingeengt, der ölige Rückstand in Diethylether gelöst und die beginnende Kristallisation durch Zugabe von n-Hexan vervollständigt. Nach Absaugen des Niederschlags und Nachwaschen mit n-Hexan verblieben 14.2g (96%) weiße Kristalle; Fp.: 101-103°C.

### [5-(2-t.Butoxy-2-oxoethyl)-2-oxo-2,3,4,5-tetrahydro-1H-benzazepin-1-yl]essigsäure (3)

a.) Zu einer Suspension aus 2.6g NaH (60%, entölt) in 35ml DMF wurde bei 10-20°C eine Lösung von Verbindung **2** (16.8g; 61.1mMol) in 60ml DMF zugetropft und bis zum Auftreten einer fast klaren gelblichen Lösung gerührt. Anschließend wurde Bromessigsäure-methylester (10g; 63.4mmol) zugetropft und über Nacht nachgerührt. Zur Aufarbeitung wurde die Reaktionsmischung in 400ml 5% kalte NaCl-Lösung eingegossen und 3x mit je 100ml eines Diethylether/n-Hexan-Gemischs extrahiert.
   Die vereinigten Extrakte wurden dann mit H₂O, 10% NaHCO₃-Lösung und NaCl-Lösung nachgewaschen, über Na₂SO₄ getrocknet, filtriert und eingedampft. Das verbliebene gelbliche Öl wurde ohne weitere Reinigung weiter umgesetzt; FAB-MS: 348 [M+-⁺].
b.) Rohprodukt **3a** wurde in 100ml Dioxan gelöst und unter Rühren bei RT 65ml 1n NaOH zugetropft. Nach ca. 45' wurde die Reaktionsmischung mit 1n KHSO₄-Lösung auf pH7 gestellt, das Dioxan im Vakuum weitgehend abdestilliert, der Rückstand mit H₂O verdünnt, mit 1n NaOH auf pH9 gestellt und 3x mit Diethylether extrahiert. Die wässrige Phase wurde dann mit 1n KHSO4-Lösung sauer gestellt, die sich abscheidende Säure mit einem Gemisch aus Diethylether/n-Hexan 4:1 extrahiert, die organische Phase mit H₂O, 1n NaOH- und NaCl-Lösung gewaschen und über Na₂SO₄ getrocknet. Filtration und Eindampfen ergaben einen öligen Rückstand, der sich durch Behandlung mit Diethylether/n-Hexan 1:4 (wassergesättigt) kristallisieren ließ. Absaugen, Waschen mit n-Hexan und Trocknen ergab 17.8g (87.5%) weiße Kristalle: Fp.: 117-119°C.

### N-[4-(Aminomethyl)phenyl]-1H-benzimidazol-2-amin (Hydrochlorid) (4)

a.) Zu einer Lösung von 24.5g Thiocarbonyldiimidazol und 1.56g Imidazol in 600ml CH₃CN wurden bei 0°C 2.0g *tert*-Butyl-4-aminobenzylcarbamat (89.97mmol) 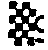 gelöst in 100ml CH₃CN- zugetropft und über Nacht bei RT gerührt. Anschließend wurden 19.5g 1,2-Phenylendiamin zugesetzt und erneut 2h bei RT gerührt. Zur Aufarbeitung wurde die Reaktionsmischung im Vakuum eingedampft, der Rückstand in CH₂Cl₂ aufgenommen, 7x mit 10% Citronensäure-und 2x mit ges. NaCl-Lösung gewaschen, über Na₂SO₄ getrocknet, filtriert und eingeengt. Das so erhaltene Rohprodukt (31.78g; brauner Schaum) wurde direkt ohne weitere Reinigung direkt umgesetzt; ESI-MS [M+H⁺] = 373.15.
   ^{*1*}*H-NMR (360 MHz, DMSO) δ ppm: 9.5 und 9. 05 (je s, 1H), 7.45 (d, 2H) , 7.35 (m, 1H), 7.20 (d, 1H), 7.15, 6.95, 6.75, 6.60 (je m, 1H), 4.85 (s, 2H), 4.10 (d, 2H), 1.35 (s, 9H)*.
b.) Rohprodukt **4a** wurde zusammen mit 36.7g HgO (gelb) und 0.4g Schwefel in 750ml Ethanol gelöst und 2h auf Rückfluß erhitzt. Die Reaktionsmischung wurde anschließend zweimal über Celite filtriert und zur Trockene eingedampft; 20.7g, ESI-MS [M+H⁺] = 339.15.
c.) 7g des Rohprodukts **4b** wurden in 70ml CH₂Cl₂ vorgelegt, 35ml HCl in Diethylether (ges. bei 0°C) zugesetzt und 2h bei RT nachgerührt. Der entstandene Niederschlag wurde abgesaugt, mit CH₂Cl₂ nachgewaschen und getrocknet.
   6.7g brauner amorpher Feststoff; ESI-MS [M+H⁺] = 239.15
   ^{*1*}*H-NMR (360 MHz, DMSO) δ ppm: 11.6 (s breit, 1H), 8.4 (s breit, 3H) , 8.25 (s breit, 1H), 7.65 und 7.55 (je d, 2H), 7.45 und 7.3 (j e m, 2H), 4.19 (m, 2H).*

### N-[5-(Aminomethyl)-1,3-thiazol-2-yl]guanidin (Dihydrochlorid) (5)

a.) 31g (130mmol) 2-Chloro-3-(1,3-dioxo-1,3-dihydro-2*H*-isoindol-2-yl)propanal (Herstellung gemäß *THL 39 (1998) , 8085-8088*) und 15.4g Amidinothioharnstoff wurden in 200ml n-Butanol 75Min. lang auf 110°C erhitzt, danach die Mischung eingedampft und der Rückstand mit CH₂Cl₂ 2 und konz. NH₃ versetzt. Eindampfen der organischen Phase, Reinigung des Rückstands durch Chromatographie an Kieselgel (CH₂Cl₂/CH₃OH 0-5%) und Kristallisation aus Aceton ergaben 12.3g *N*-{5-[(1,3-Dioxo-1,3-dihydro-2*H*-isoindol-2-yl)methyl]-1,3-thiazol-2-yl}guanidin.
b.) 1g **5a** in 20ml CH₃OH wurde mit 0.81ml Hydrazinhydrat versetzt und 2h lang bei RT gerührt. Anschließend wurde die Mischung auf 0°C abgekühlt, filtriert, das Filtrat eingeengt und mit verdünnter HCl verrührt. Dieser Vorgang wurde mehrmals wiederholt, und das auf diese Weise erhaltene Rohprodukt anschließend mit Ethanol verrührt; 0.92g weiße Festkörper, ESI-MS [M+H⁺] = 172.05.

### N-[4-(Aminomethyl)phenyl]-N'-benzylharnstoff (6)

a.) 4-Aminobenzylamin (10.0 g, 81.85 mmol) in 150ml CH₂Cl₂ wurde mit Triethylamin (6.8 g, 67.12 mmol) und dann bei 0°C mit Di-t.Butyldicarbonat (18.6 g, 85.0 mmol) versetzt. Die Mischung wurde 1h bei 0°C und dann 2h bei RT nachgerührt. Zur Aufarbeitung wurden 150ml einer 1% wässrigen Citronensäure-Lösung zugegeben, die Phasen getrennt und die wässrige Phase 2 mal mit CH₂Cl₂ (150 mL) nachextrahiert. Erneutes Waschen mit H₂O, Trocknen der vereinigten organische Phasen mit Na₂SO₄ und Eindampfen ergaben einen Feststoff, der mit wenig Diisopropylether ausgerührt, abgesaugt und getrocknet wurde.
   13.0g; ESI-MS [M+H⁺-^{t}Bu] = 167.05.
   ^{*1*}*H-NMR (360 MHz, CDCl*_{*3*}*) δ (ppm) : 7.04 (2H, d), 6.61 (2H, d) , 4.78 (1H, s br.), 4.17 (2H, d) , 3.67 (2H, s br.), 1.46 (9H, s)*.
b.) Zu einer Lösung des geschützten Amins **6a** (4.0 g, 17.99 mmol) und Triethylamin (1.82 g, 18.0 mmol) in 220ml Toluol/DMF 10:1 wurde unter Eiskühlung Benzylisocyanat (2.40 g, 18.0 mmol) zugegeben. Die Reaktionsmischung wurde über Nacht bei RT gerührt. Ein Teil des gebildeten Harnstoffs konnte direkt als Niederschlag abfiltriert und getrocknet werden. Das Filtrat wurde 2x mit H₂O, verdünnter Weinsäure bis pH 3 und erneut 2mal mit H₂O bis pH 5 gewaschen, die organische Phase dann getrocknet und eingedampft. Insgesamt wurden so 6.0 g erhalten; ESI-MS [M+H⁺- ^{t}Bu] = 300.15.
c.) Der so erhaltene Harnstoff **6b** wurde in 90ml CH₂Cl₂ vorgelegt, bei 0°C TFA (2.24 g, 196.25 mmol) -gelöst in 90ml CH₂Cl₂- zugetropft. Nach 3h wurden erneut 1ml TFA zugegeben, dann über Nacht bei RT gerührt. Nach erneuter Zugabe von 1ml TFA wurden noch 5 h gerührt, dann die Mischung auf Eiswasser gegossen und mit Ethylacetat (2x50 ml) extrahiert. Die Wasserphase wurde mit 2n NaOH-Lösung basisch gestellt und mit CH₂Cl₂ (2x50 ml) extrahiert. Der unlösliche Anteil zwischen den Phasen wurde abfiltriert und getrocknet.
   4g; ESI-MS [2M+H⁺] = 511.35
   ^{*1*}*H-NMR (200 MHz, DMSO) δ (ppm) : 8.52 (1H, s), 7.39-7.07 (9H, m), 6.62 (1H, t), 4.27 (2H, d) , 3.61 (2H, s)*.

### [4-(1H-Benzimidazol-2-yl)pheayl]methanamin (Hydrochlorid) (7)

a.) Di(*tert*-butyl)-4-cyanobenzylimidodicarbonat (10g, 30.08mmol; Herstellung gemäß *Synth. Comm. 28, 23, 1998, 4419ff*) in 200ml Pyridin wurden mit 45ml Triethylamin versetzt und 1.5h lang bei 0°C mit H₂S gesättigt. Die Reaktionsmischung wurde über Nacht bei RT stehen gelassen und anschließend eingedampft. Der so erhaltene Rückstand wurde dann mit Diethylether verrührt, abgesaugt und getrocknet (8.5g).
b.) 6g des Thioamids **7a** (16.37mmol) in 40ml trockenem CH₂Cl₂ wurden mit 23.2g CH₃I über Nacht bei RT alkyliert und die Mischung anschließend eingedampft. Der so erhaltene Rückstand wurde in 40ml CH₃OH aufgenommen, 1. 95g 1, 2-Phenylendiamin zugegeben und erneut über Nacht gerührt. Eindampfen der Reaktionsmischung und Verrühren des Feststoffs mit n-Pentan ergaben 6.9g des gewünschten Benzimidazols. Fp.: >170°C (Zersetzung); ESI-MS: [M+H⁺] = 424.25
c.) 1g der Bis-Boc-Verbindung **7b** wurde in 5ml CH₂Cl₂ gelöst, bei 0°C 5ml TFA zugesetzt und 1h bei Raumtemperatur gerührt. Eindampfen der Reaktionsmischung, Versetzen mit HCl in Diethylether und Verrühren des isolierten Feststoffs mit Diethylether ergaben 0.6g des Amins als Hydrochlorid; ESI-MS: [M+H⁺] = 224.05.

### N¹-(1H-Benzimidazol-2-yl)pentan-1,5-diamin (Hydrochlorid) (8)

Die Darstellung erfolgte analog zur Herstellung von Verbindung **4** ausgehend von 7g N-Boc-1,5-Diaminopentan-Hydrochlorid (29.3mmol). Nach Umsetzung analog zu **4a** wurden 10.3g N-Boc 5-{[(2-aminoanilino)carbothioyl]amino}pentan-1-amin erhalten; ESI-MS [M+H⁺]= 353.25. Cyclodesulfurierung und anschließende Abspaltung der Boc-Gruppe mit TFA ergab ein öliges Rohprodukt, das in CH₃OH aufgenommen und mit 250ml etherischer HCl (gesättigt bei 0°C) in das entsprechende Hydrochlorid überführt wurde. Verrühren der erhaltenen Festkörper mit einer Mischung aus CH₃OH/Methylt.butylether ergab 1.8g eines rötlichen amorphen Feststoffs.
^{*1*}*H-NMR (360 MHz, DMSO) δ ppm: 9.30 (t, 1H), 8.15 (s breit, 3H), 7.40 und 7.25 (je* *m, 2H) , 3.35 (m, 2H überlagert mit H*_{*2*}*O-Peak), 2.80 (m, 2H) , 1. 65 (m, 4H), 1.45 (m, 2H)*.

### N¹-(1H-Benzimidazol-2-yl)butan-1,4-diamin (Trifluoracetat) (9)

Die Darstellung erfolgte analog zur Herstellung von Verbindung **4** ausgehend von 9.87g N-Boc-1,4-Diaminobutan (52.3mmol). Nach Umsetzung analog zu **4a** wurden 17.08g 3g N-Boc 4-{[(2-aminoanilino)carbothioyl]amino}butan-1-amin erhalten; ESI-MS [M+H⁺]= 338.99.
Nachfolgende Cyclodesulfurierung und Boc-Abspaltung mit TFA ergab einen braunen Feststoff, der mehrmals mit n-Pentan verrührt und dann aus einer Mischung aus CH₃OH/Methyl-t.butylether umkristallisiert wurde; 14.35g, ESI-MS [M+H⁺] = 205.15.
^{*1*}*H-NMR (360 MHz, DMSO) δ ppm: 9.20 (t, 1H), 7.80 (s breit, 3H), 7.35 und 7.20 (je m, 2H), 3.40 (m, 2H teilweise überlagert mit H*_{*2*}*O-Peak), 2.80 (m, 2H), 1.65 (m, 4H)*.

### trans-N-[(4-Aminocyclohexyl)methyl]-1H-benzimidazol-2-amin (Dihydrochlorid) (10)

Die Herstellung erfolgte analog zu Verbindung **4** ausgehend von 5.4g tert.Butyl-4-(Aminomethyl)cyclohexylamincarbamat (WO 9603374; Bioorg. Med. Chem. Lett. **1997**, 7 (1), 67). Nach Abspaltung der Boc-Gruppe wurden 3.3g weißes Dihydrochlorid; FAB-MS [M+H⁺]: 245.

### trans-N-{[4-(Aminomethyl)cyclohexyl]}-1H-benzimidazol-2-amin (Dihydrochlorid) (11)

Die Herstellung erfolgte analog zu Verbindung 4 ausgehend von 10g Benzyl-{4-[(*tert*-butoxycarbonyl)amino]cyclohexyl}methylcarbamat (EP 669317) durch Abspaltung der Boc-Gruppe mit 4n HCl in Dioxan, Aufbau des Benzimidazols und nachfolgender Hydrogenolyse. Es wurden 3.6g weißes Dihydrochlorid isoliert; FAB-MS [M+H⁺] : 245.

### [6-(1H-Benzimidazol-2-yl)pyridin-3-yl]methanamin (Trifluoracetat) (12)

a.) Die Herstellung erfolgte analog zu **7** ausgehend von *tert*-Butyl-(6-cyanopyridin-3-yl)methylcarbamat (6.0g, 25.72 mmol); Kristallisation des Rohprodukts aus Ethanol ergab 5.15g; ESI-MS [M+H⁺] = 325.
b.) 0.55g des Boc-geschützten Amins **7a** in 10ml CH₂Cl₂ wurden mit 5ml TFA versetzt und 2h bei RT gerührt. Eindampfen der Reaktionsmischung ergab 0.95g eines weißen Feststoffs; ESI-MS [M+H⁺] =225.25.

### N- [4-(Aminoanethyl)phenyl]-2-pyridinamin (13)

*tert*-Butyl-4-aminobenzylcarbamat (2g; 9mmol) wurden mit 8.74g 2-Fluorpyridin 32h lang auf Rückfluß erhitzt. Die Reaktionsmischung wurde im Vakuum eingedampft und der erhaltene Rückstand mit n-Pentan verrührt (1.9g). Die Spaltung der Boc-Gruppe erfolgte mit TFA, das erhaltene Rohprodukt wurde als Hydrochlorid aus Diethylether gefällt und anschließend mit NH₃ in die freie Base überführt (0.8g); ESI-MS [M+H⁺] :200.25.

### N- [4- (Aminomethyl)benzyl]-2-pyridinamin (14)

a.) 20g 2-Aminopyridin wurden in 100 ml CH₃OH gelöst, mit isopropanolischer HCl auf pH 6 eingestellt und mit 36g p-Cyanobenzaldehyd versetzt. 9.35g Natriumcyanoborhydrid wurden portionsweise in 1 h zugegeben und über Nacht gerührt. Zur Aufarbeitung wurde die Suspension eingeengt, der Rückstand in 100 ml Wasser aufgenommen und mit KOH auf pH > 10 eingestellt. Die wässrige Phase wurde mit NaCl gesättigt und 3x mit Diethylether extrahiert. Die Etherphase wurde nach Filtration eines Niederschlags noch 3x mit FeSO₄-Lösung gewaschen, getrocknet und eingeengt. Reinigung des Rückstands durch Chromatographie an Kieselgel (Heptan/Ethylacetat 1:1) ergab 28.15g 4-[(2-Pyridinylamino)methyl]benzonitril.
b.) 10g 4-[(2-Pyridinylamino)methyl]benzonitril wurden in 280ml ammoniakalkalischem CH₃OH gelöst, mit 10g Raney-Nickel versetzt und 24h hydriert. Es wurde abfiltriert, eingeengt und der Rückstand an Kieselgel (Ethylacetat/Ethanol 1:3) chromatographiert.
   5.18g, ESI-MS: [M+H⁺] = 214.

### [5- (1H-Benzimidazol-2-yl)thien-2-yl]methanamin (15)

Die Synthese erfolgte ausgehend von 5-(Aminomethyl)thiophen-2-carbonitril (Herstellung gemäß WO 95/23609), das nach Standardmethoden in das entsprechende Boc-Derivat überführt wurde.
*tert*-Butyl-5-cyanothien-2-ylcarbamat (25g; 104.9mmol) in 330ml CH₃OH wurde mit 1.1eq. Natriummethanolat-Lsg. versetzt und über nacht bei RT, dann 2h bei 40-50°C gerührt. Anschließend wurden 18.95g Phenylendiaminbishydrochlorid zugegeben und erneut bei RT gerührt. Zur Aufarbeitung wurde mit Wasser versetzt, der entstandene Niederschlag abfiltriert und sorgfältig getrocknet. 19.6g gelber Feststoff; ESI-MS: [M+H⁺] = 330. Nachfolgende Spaltung der Boc-Gruppe mit TFA ergab ein Rohprodukt, das in Wasser gelöst wurde, 2x mit Diethylether extrahiert, die wässrige Phase auf pH 10-11 gebracht und anschließend 2x mit Ethylacetat extrahiert. Die wässrige Phase wurde mit NaCl gesättigt und nochmals mit Ethylacetat extrahiert. Die vereinigten organische Phasen werden getrocknet und eingeengt (6.3g); ESI-MS [M+H⁺] = 230.1.

### tert-Butyl-2-[4-(1H-Benzimidazol-2-yl)phenyl]ethylcarbamat (16)

Die Herstellung erfolgte analog zur Synthese von [4-(1*H*-Benzimidazol-2-yl)phenyl]methanamin (Hydrochlorid) (7) ausgehend von *tert*-Butyl-2-(4-cyanophenyl)ethylcarbamat. Das nach Umsetzung mit H₂S, Alkylierung mit CH₃I und Reaktion mit 1,2-Phenylendiamin erhaltene Rohprodukt wurde durch Chromatographie an Kieselgel (CH₂Cl₂/CH₃OH 4-50%) gereinigt (4.8g); ESI-MS [M+H⁺] = 338.15. Das zur weiteren Umsetzung benötigte Amin wurde durch Abspaltung der Boc-Gruppe mit TFA (unter Standardbedingungen) erhalten; das isolierte TFA-Salz dann direkt in den entsprechenden Kupplungen eingesetzt.

### N-(Piperidin-4-ylmethyl)-1H-benzimidazol-2-amin (Trifluoracetat) (17)

a.) Zu 6.75g Thiocarbonyldiimidazol und 0.5g Imidazol in 100ml CH₃CN wurde bei 0°C eine Lösung von *tert*.Butyloxycarbonyl-4-(aminomethyl)-1-piperidin (5.39g; 25mmol) in 25ml CH₃CN zugetropft und 3h bei RT nachgerührt. Anschließend wurde 1,2-Phenylendiamin (5.5g; 50.86mmol) zugesetzt und ca. 1h lang auf 60°C erhitzt. Der beim Abkühlen entstandene Feststoff wurde abgesaugt und getrocknet.
   6.79g; ESI-MS [M+H⁺-^{t}Bu] = 309.15
b.) *tert*-Butyoxycarbonyl-4-({[(2-aminoanilino)carbothioyl]amino}methyl)1-piperidin (5g; 13.72mmol), 5.94g HgO (gelb) und 0.6g Schwefel in 150ml Ethanol wurden 1h lang auf Rückfluss erhitzt. Die Mischung wurde 2x über Celite filtriert, eingedampft und das erhaltene Rohprodukt durch Chromatographie an Kieselgel (CH₂Cl₂/CH₃OH 5→25%) gereinigt.
   2.65g; ESI-MS [M+H⁺] = 331.25
   ¹H-NMR (360 MHz, DMSO) δ ppm: 7.15 und 6.9 (je m, 2H) , 3.95 (d, 2H), 3.2 (m 2H), 2.7 (br m; 2H), 1.8 (m, 1H), 1.7 (m, 2H), 1.35 (s, 9H), 1.05 (m, 2H).
c.) tert-Butyloxycarbonyl-4-[(1H-benzimidazol-2-ylamino)methyl]-1-piperidin (2.65g; 8.02mmol) wurde nach Standardbedingungen mit 10ml TFA behandelt. Einengen und Verrühren des Rohproduktes mit n-Pentan ergaben 2.3g; ESI-MS [M+H⁺] = 231.15.
   ¹H-NMR (360 MHz, DMSO) δ ppm: 13.25 (s, 1H), 9.35 (m, 1H), 8.8 und 8.5 (je br s, 1H), 7.4 und 7.20 (je m, 2H), 3.3 (m, 4H), 2.85 (m, 2H), 1.9 (m, 3H), 1.35 (m, 2H).

### N-{[5-(Aminomethyl)thien-3-yl]methyl}pyridin-2-amin (Trifluoracetat) (18)

a.) Eine Lösung von tert-Butyl-(4-cyanothien-2-yl)methylcarbamat (7g; 29.4mmol) in 120ml Ethanol wurde mit NH₃ gesättigt und dann in Gegenwart von Ra-Ni (9g wässrige Suspension; mit Ethanol abdekantiert) unter Standardbedingungen hydriert. Filtration der Reaktionsmischung, Eindampfen und Chromatographie des erhaltenen Rückstands an Kieselgel (CH₂Cl₂/CH₃OH plus wässr. NH₃) ergaben 4.4g des Amins als gelbliches Öl.
b.) 1.2g des Amins 18a (4.3mmol), 0.6g Ethyldiisopropylamin und 15g 2-Fluorpyridin wurden 20h auf Rückfluß erhitzt. Der nach Eindampfen der Mischung erhaltene Rückstand wurde in CH₂Cl₂ aufgenommen, mit 0.1n HCl- und ges. NaCl-Lösung gewaschen, getrocknet und erneut eingedampft.
   1g; ESI-MS [M+H⁺] = 320.15
c.) 0.9g des Boc-geschützten Amins 18b wurden in 10ml CH₂Cl₂ gelöst, bei 0°C 5ml TFA zugesetzt und 1h bei Raumtemperatur gerührt. Eindampfen der Reaktionsmischung ergab 1.65g eines bräunlichen Öls, das ohne weitere Reinigung direkt umgesetzt wurde (ESI-MS [M+H⁺] = 220.05).

### 3-Amino-N-(1H-imidazol-2-yl)propanamid (19)

a.) Z-β-Alanin (10g; 44.8mmol) wurden in 200ml DMF gelöst und 15.86g (3.5 eq) N-Methylmorpholin und 5.9g (0.5eq) 2-Aminoimidazolsulfat zugegeben. Bei -10°C wurden 7.87g (1.3eq) HOBt und 11.16g (1.3eq) N'-(Dimethylaminopropyl)-N-ethylcarbodiimid zugegeben, und 1h unter Erwärmung auf RT und dann 18h gerührt. Es wurden 150ml Diethylether hinzugegeben, worauf ein weißer Feststoff ausfiel, der abgesaugt wurde. Der Rückstand wurde mit kaltem Diethylether gewaschen, in Ethylacetat suspendiert und mit 1n HCl bis zur sauren Reaktion versetzt. Die wässrige Lösung wurde 1x mit Ethylacetat extrahiert, dann die wässrige Phase mit 10% NaOH bei 4°C auf basischen pH gebracht. Der entstehende Niederschlag wurde abgesaugt und mit Wasser gewaschen. 5.4g; ESI-MS [M+H⁺] = 289.05
b.) 5.3g der Z-Verbindung 19a wurden in 250 ml Ethanol suspendiert und 530mg 10% Pd auf Aktivkohle zugegeben. Es wurde 18h bei RT mit H₂ hydriert, anschließend mit CH₃OH verdünnt und die Suspension aufgekocht, worauf der Produktniederschlag sich auflöste. Filtrieren und Einengen der Lösung ergaben 1.5g; ESI-MS [M+H⁺] = 155.05.

### N-[4-(Aminomethyl)phenyl]-4,5-dihydro-1H-imidazol-2-amin (Hydrochlorid) (20)

tert-Butyl-4-aminobenzylcarbamat (2g; 9mmol), 2.2g Ethyldiisoppropylamin und 4.4g 2-(3,5-Dimethylpyrazol)-4,5-dihydroimidazol x HBr in 15ml DMF wurden bei 110°C gerührt. Nach beendeter Umsetzung wurde die Mischung eingeengt, der Rückstand in Ethylacetat aufgenommen, je 2x mit ges. NaHCO₃- und NaCl-Lösung gewaschen, getrocknet und erneut eingedampft. Die basische wässr. Phase wurde ebenfalls eingedampft, mit Aceton verrührt, der Niederschlag abgesaugt und die Mutterlauge eingeengt. Die vereinigten Rückstände wurden mittels MPLC (Kieselgel: Fa. Bischoff Prontoprep 60-2540-C18E, 32µm; Fließmittel: CH₃CN/H₂O + 0.1% Essigsäure) gereinigt. 0.22g; ESI-MS [M+H⁺] = 291.15.
Das so erhaltene Produkt wurde 2h lang bei RT mit 4n HCl in Dioxan behandelt. Der entstandene Niederschlag wurde abgesaugt, mit Pentan nachgewaschen und getrocknet.110mg; ESI-MS [M+H⁺] = 191.15.
¹H-NMR (360 MHz, d₆-DMSO) δ ppm: 11.85 (s, 1H), 8.45 (s breit, 3H) , 8.40 (s, 1H), 7.60 und 7.30 (je d, 2H), 4.05 (m, 2H), 3.70 (s, 4H).

### N-{[5-(Aminomethyl)thien-3-yl]methyl}-1H-benzimidazol-2-amin (Hydrochlorid) (21)

Amin 18a (6.5g; 23.31mmol) wurde analog zur Darstellung von 17 durch Umsetzung mit Thiocarbonyldiimidazol, Imidazol und anschließend Phenylendiamin in das entsprechenden Aminobenzimidazol überführt. 1.6g; ESI-MS [M+H⁺] = 359.15. Anschließende Spaltung der Boc-Gruppe mittels 4n HCl in Dioxan ergab 1.3g leicht gelbe Festkörper; ESI-MS [M+H⁺] = 191.15.

### N¹-Pyridin-2-ylpentan-1,5-diamin (Hydrochlorid) (22)

Herstellung analog zu 18b durch Umsetzung von N-1-Boc-1,5-diaminonpentan x HCl (5g; 20.94mmol) und 20.3g 2-Fluorpyridin, 5.64g klares Öl; ESI-MS [M+H⁺] = 280.15. Abspaltung der Boc-Gruppe mit 4n HCl in Dioxan ergab 3.46g weiße Festkörper; ESI-MS [M+H⁺] = 180.20.
¹H-NMR (360 MHz, d₆-DMSO) δ ppm: 9.10 (s breit, 1H), 8.05 (s breit, 3H), 7.85 (m, 2H), 7.20 (m, 2H) , 6.80 (m, 1H), 3.45 (m, überlagert mit H2O), 2.80 (m, 2H), 1.65 (m, 4H) , 1.45 (m, 2H).

### N¹-(4,5-Dihydro-1H-imidazol-2-yl)pentan-1,5-diamin (Hydrochlorid) (23)

N-1-Boc-1,5-diaminopentan x HCl (5g; 20.94mmol), 5.4g Ethylidiisopropylamin und 5.11g 2-(Methylsulfanyl)-4,5-dihydro-1H-imidazol x HI in 30ml DMF wurden über Nacht bei RT gerührt. Die Reaktionsmischung wurde eingedampft, in CH₂Cl₂ aufgenommen, mit H₂O und ges. NaCl-Lösung gewaschen, getrocknet und erneut eingeengt. 5.05g klares Öl, ESI-MS [M+H⁺]: 271.18. Abspaltung der Boc-Gruppe mit 4n HCl in Dioxan und Reinigung des Rohprodukts mittels MPLC ergab 2.57g; ESI-MS [M+H⁺]: 171.15.
^{1 3}C-NMR (90.55 MHz, d₆-DMSO) δ ppm: 160.3, 43.3 (2 Signale überlagert) , 42.85, 39.70, 28.90, 27.2, 23.60.

### N-[4-(Aminomethyl)phenyl]-1H-imidazol-2-amin (24)

a.) Zu einer Mischung aus tert-Butyl-4-aminobenzylcarbamat (10g; 44.99mmol) und 11.07g Natriumacetat in 100ml CH₃OH wurden bei 0°C 5.24g BrCN 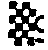 gelöst in 50ml CH₃OH- zugetropft, anschließend 3h lang bei 0°C und über Nacht bei RT gerührt. Die Mischung wurde eingedampft, der erhaltene Rückstand in Wasser aufgenommen und 2x mit Methyl-tert.butylether extrahiert. Trocknen und Einengen der org. Phasen ergab 12.9g eines gelborangen Öls.
b.) 7g tert-Butyl-4-[(iminomethylen)amino]benzylcarbamat in 50ml Pyridin wurden mit 28.6g Triethylamin versetzt, bei 0°C 1h lang H₂S eingegast und die Mischung 48h stehen gelassen. Eindampfen ergab 9.79g eines rosa Schaums; ESI-MS [M+H⁺] : 282.05.
   ¹H-NMR (360 MHz, d₆-DMSO) δ ppm: 9.70 (s, 1H), 7.35 (m, 4H), 7.20 (m, 2H), 4.15 (d, 2H), 1.45 (s, 9H).
c.) 5g dieses Thioamids in 50ml CH₃OH wurden mit 5.05g CH₃I methyliert, und das erhaltene Rohprodukt direkt mit 1.73g Aminoacetaldehyd-diethylacetal in 7.5ml CH₃CN 3h lang bei RT umgesetzt. Einengen der Reaktionsmischung ergab 6.36g eines rötlichen Öls (ESI-MS [M+H⁺]: 381.25), das in 50ml 6n HCl gelöst und bei 0°C 3h lang gerührt wurde. Anschließend wurde mit 25% NaOH-Lösung ein pH von 12 eingestellt und erneut 48h bei RT gerührt. Die Mischung wurde 4x mit Ethylacetat extrahiert, die vereinigten org. Phasen getrocknet und eingeengt. Das so erhaltene Öl wurde 2x mit mit Methyl-tert.butylether verrührt, die erhaltenen Feststoffe abgesaugt und getrocknet. 0.6g rote Festkörper; ESI-MS [M+H⁺] : 189.15.
   ¹³C-NMR (90.55 MHz, d₆-DMSO) δ ppm: 145.5, 141.60, 130.75, 128.4, 119.8, 115.6, 44.85.

### N¹-(1,4,5,6-Tetrahydropyrimidin-2-yl)pentan-1,5-diamin (Hydrochlorid) (25)

Herstellung erfolgte analog zu 23 ausgehend von N-1-Boc-1,5-diaminonpentan x HCl (5g; 20.94mmol) und 5.4g 2-(Methylsulfanyl)-1,4,5,6-tetrahydropyrimidin X HI. Nach Aufarbeitung wurden 1.3g eines gelbliches Öl erhalten; [M+H⁺] :282..2. Abspaltung der Boc-Gruppe mit 4n HCl in Dioxan und Reinigugn mittels MPLC ergab 0.46g; ESI-MS [M+H⁺]: 185.15.

### N-[4-(Aminomethyl)cyclohexyl]pyridin-2-amin (Hydrochlorid) (26)

Benzyl (4-aminocyclohexyl)methylcarbamat (TFA-Salz) (5g; 13.28mmol) 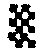 Herstellung durch TFA-Spaltung ausgehend von Benzyl-{4-[(tert-butoxycarbonyl)amino]cyclohexyl}-methylcarbamat (EP 669317)- wurde analog zu 18 mit 1.71g Ethyldiisopropylamin in 50ml 2-Fluorpyridin auf Rückfluß erhitzt. Übliche Aufarbeitung und Kristallisation des Rohprodukts aus Methyl-tert.butylether/Methanol ergab 4.15g; ; ESI-MS [M+H⁺]: 340.29.
¹H-NMR (360 MHz, d₆-DMSO) δ ppm: 8.75 (d breit, 1H), 7.85 (m, 2H), 7.35 (m, 5H), 7.05 (d, 1H), 6.85 (m, 1H), 5.05 (s, 2H), 2.90 (m, 2H), 1.95 und 1.75 (jeweils m, 2H) , 1.45-0.90 (m, 6H).
Abspaltung der Z-Gruppe unter Standardbedingungen (H₂; Pd-Aktivkohle), Fällung des entstandenen Amins als Hydrochlorid und Trocknen des erhaltenen Niederschlags ergab 1.5g; ESI-MS [M+H⁺] : 206.15.

### tert-Butyl-2,3,4,5-tetrahydro-1H-1-benzazepin-5-ylacetat (27)

Zu einer Lösung von (2-Oxo-2,3,4,5-tetrahydro-1H-1-benzazepin-5-yl)essigsäure-t.butylester (2) (10g; 36.32mmol) in 100ml THF wurden 75ml einer 1.0m BH₃-THF-Lösung gegeben und bei RT gerührt. Zur Aufarbeitung wurde vorsichtig Wasser zugesetzt, 2x mit Diethylether extrahiert und anschließend die vereinigten org. Phasen je 2x mit Wasser gewaschen. Einengen und Trocknen ergab 9.3g; ESI-MS [M+H⁺]: 262.04.

### [5-(2-tert-Butoxy-2-oxoethyl)-2,3,4,5-tetrahydro-1H-1-benzazepin-1-yl]essigsäure (28)

a.) Eine Lösung von tert-Butyl-2,3,4,5-tetrahydro-1H-1-benzazepin-5-ylacetat (27) in 50ml DMF wurde mit 3g K₂CO₃, 0.05g KI und 5g Methylbromacetat versetzt und 12h bei 90°C gerührt. Danach wurden erneut 4g Methylbromacetat zugegeben und noch 5h bei 120°C nachgerührt. Zur Aufarbeitung wurde die Mischung aufkonzentriert, mit CH₂Cl₂ verdünnt, mit fes. NaCl-Lösung gewaschen, getrocknet und erneut eingeengt. Chromatographie an Kieselgel (CH₂Cl₂/CH₃OH 1-5%) ergab 4.6g eines hellgelben Öls; ESI-MS [M+H⁺]: 334.12.
b.) 4.1g des Methylesters in 20ml Dioxan/15ml H₂O wurden mit 1g KOH bei RT verseift. Zur Aufarbeitung wurde die Mischung aufkonzentriert, mit 2n HCl auf einen pH von 2 gestellt und mit CH₂Cl₂ extrahiert. Die vereinigten org. Phasen wurden getrocknet, eingedampft und das erhaltene Rohprodukt durch Chromatographie an Kieselgel (CH₂Cl₂/CH₃OH 2-7%) gereinigt. 2.4g Öl, ESI-MS [M+H⁺]: 320.15.
   ¹H-NMR (360 MHz, CDCl₃) δ ppm: 7.20-7.10 (m, 1H), 6.90 und 6.80 (je m, 1H), 4.0 (s, 2H), 3.55 (m, 1H), 3.10 (m, 2H), 2.85 Und 2.70 (je m, 1H), 2.90-2.50 (m, 4H), 1.35 (s, 9H).

### N-[4-(Aminomethyl)cyclohexyl]-1H-imidazol-2-amin (Hydrobromid) (29)

Synthese analog zur Herstellung von N-[4-(Aminomethyl)phenyl]-1H-imidazol-2-amin (23) ausgehend von Benzyl-(4-aminocyclohexyl)methylcarbamat (TFA-Salz) (3g; 7.97mmol).
a.) Umsetzung mit BrCN und anschließende Reinigung (1.52g; ESI-MS [M+H⁺] : 288.15)
   ¹H-NMR (360 MHz, d₆-DMSO) δ ppm: 7.45-7.25 (m, 5H) , 6.75 (d, 1H), 5.05 (d, 2H), 2.85 (m, 3H), 1.85 und 1.70 (je m, 2H), 1.35 (m, 1H), 1.20 und 0.95 (je m, 2H).
b.) Überführung in den entsprechenden Thioharnstoff und anschließende Methylierung (1.48g; ESI-MS [M+H⁺]: 336.15).
c.) Umsetzung mit Aminoacetaldehyd-diethylacetal und nachfolgende Cyclisierung ergab 0.79g; ESI-MS [M+H⁺]: 329.15.
   ¹H-NMR (360 MHz, d₆-DMSO) δ ppm: 7.45-7.25 (m, 5H), 6.45 (s, 2H), 5.35 (d, 1H), 5.05 (s, 2H), 2.90 (m, 2H), 1.95 und 1.70 (je m, 2H), 1.35 (m, 1H), 1.15 und 0.95 (je m, 2H).
d.) Zur Abspaltung der Z-Gruppe wurde in 30ml HBr/Eisessig gelöst und 3h bei RT gerührt. Zur Aufarbeitung wurde die Mischung eingedampft und mehrmals mit Aceton koevaporiert.0.89g; ESI-MS [M+H⁺]:195.15.

### tert-Butyloxycarbonyl-4-[(2-pyridinylamino)methyl]-1-piperidin (30)

tert.Butyloxycarbonyl-4-(aminomethyl)-1-piperidin (3g; 14mmol) und 10ml 2-Fluorpyridin wurden für 4h auf Rückfluß erhitzt. Einengen und Verrühren des Rohprodukts in n-Pentan ergaben 3g eines weißen Feststoffes, Smp: 126-130°C; ESI-MS [M+H⁺] = 292.15.
Das zur weiteren Umsetzung benötigte Amin wurde durch Abspaltung der Boc-Gruppe mit HCl in Dioxan (unter Standardbedingungen) erhalten; das isolierte HCl-Salz dann direkt in eingesetzt.

### Ethyl-2-{[5-(2-tert-butoxy-2-oxoethyl)-2-oxo-2,3,4,5-tetrahydro-1H-1-benzazepin-1-yl]methyl}-1,3-thiazol-4-carboxylat (31)

a.) Zu einer Suspension aus 1.28g NaH (60%; entölt) in 10ml DMF wurde bei 5°C eine Lösung von (2-Oxo-2,3,4,5-tetrahydro-1H-1-benzazepin-5-yl)essigsäure-t.butylester (2) in 20ml DMF zugetropft und 1hnachgerührt. Anschließend wurden 3.49g Bromacetonitril 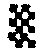 gelöst in 20ml DMF- zugetropft und 4h lang bei RT gerührt. Zur Aufarbeitung wurde vorsichtig mit Wasser versetzt, mit CH₂Cl₂ verdünnt, mehrmals mit H₂O und ges. NaCl-Lösung gewaschen, getrocknet und eingeengt. Reinigung des Rohprodukts durch Chromatographie an Kieselgel (CH₂Cl₂/CH₃OH 5%) ergab 7.61g; ESI-MS [M+H⁺-tBu] : 259.05.
b.) 5g tert-Butyl-[1-(cyanomethyl)-2-oxo-2,3,4,5-tetrahydro-1H-1-benzazepin-5-yl]acetat in 70ml Pyridin wurden 1h lang mit H₂S gesättigt und dann über Nacht bei RT stehen gelassen. Eindampfen der Mischung und Verrühren des erhaltenen Rückstands mit Pentan ergab 5.5g eeines rosa Feststoffs, der direkt weiter umgesetzt wurde.
c.) Eine Mischung aus 2g tert-Butyl-[1-(2-amino-2-thioxoethyl)-2-oxo-2,3,4,5-tetrahydro-1H-1-benzazepin-5-yl]acetat, 1.62g Ethylpyruvat und 0.86g KHCO₃ in 30ml Dioxan wurde 2.5h bei RT gerührt. Verdünnen mit CH₂Cl₂, Waschen mit ges. NaCl-Lösung, Trocknen und Einengen ergab 2.65g eines gelben Öls; ESI-MS [M+H⁺] : 445.15.
   ¹H-NMR (360 MHz, d₆-DMSO) δ ppm: 8.50 (s, 1H), 7.50 und 7.35 (je m, 1H), 7.30-7.20 (m, 2H), 7.35 (d, 1H), 5.20 (breit, 1H), 4.30 (q, 2H), 4.20 (m, 1H), 3.65-3.50 (m, 2H), 2.70 (m, 2H), 2.35-2.10 (m, 3H), 1.70-1.50 (m, 2H), 1.30 (s, 9H; überlagert mit t, 3H).

### 2-{[5-(2-tert-Butoxy-2-oxoethyl)-2-oxo-2,3,4,5-tetrahydro-1H-1-benzazepin-1-yl]methyl}-1,3-thiazol-4-carbonsäure (32)

2.6g Ethyl-2-{[5-(2-tert-butoxy-2-oxoethyl)-2-oxo-2,3,4,5-tetrahydro-1H-1-benzazepin-1-yl]methyl}-1,3-thiazol-4-carboxylat (31) wurde in einer Mischung aus 31ml Dioxan und 4ml H₂O vorgelegt, 1.5eq. KOH zugesetzt und auf Rückfluß erhitzt. Nach 5h wurde erneut 1eq. KOH zugegeben und noch 12h bei RT gerührt. Zur Aufarbeitung wurde aufkonzentriert, der Rückstand in Wasser aufgenommen, mit 2n HCl ein pH von 4-5 eingestellt und mehrmals mit CH₂Cl₂ extrahiert. Die vereinigten org. Phasen wurden mit ges. NaCl-Lösung gewaschen, getrocknet und eingeengt. Verrühren mit n-Pentan ergab 2.1g eines weißen Feststoffs; ESI-MS [M+H⁺]: 417.15.
¹H-NMR (360 MHz, d₆-DMSO) δ ppm: 8.40 (breit, 1H), 7.5 (m, 1H), 7.35 (m, 1H), 7.30-7.20 (m, 3H), 5.25 (m, 2H), 2.70 (m, 2H), 2.35-2.10 (m, 6H), 1.70 (m, 1H), 1.30 (s, 9H).

### 2-Ammonio-6-(ammoniomethyl)pyridinium trichlorid (33)

a) 2-Amino-6-methylpyridin (0.14mol, 15.0g) und Phtalanhydrid (0.14mol, 20.55g) wurden am Wasserabscheider auf 190°C erhitzt. Verteilen zwischen H₂O und CH₂Cl₂, Einengen der org. Phase und Umkristallisieren des Rückstands (Diethylether) ergaben 28.25g eines leicht gelblichen Feststoffs; ESI-MS [M+H⁺] = 239.15.
   ¹H-NMR (270 MHz, CDCl₃) δ (ppm) : 7.95 (2H, m), 7.78 (3H, m), 7.23 (2H, m) , 2.64 (3H, s).
b.) N-Bromosuccimid (25.18mmol, 4.48g) wurde portionsweise zu einer kochenden Suspension von 2-(6-Methyl-2-pyridinyl)-1H-isoindole-1,3(2H)-dione (33a, 20.99mmol, 5.0g), AIBN (2.10mmol, 0.35g) und Dibenzoylperoxid (2.10mmol, 0.51g) in CCl₄ gegeben. Das Reaktionsgemisch wurde 20h gekocht, filtriert und das Filtrat eingeengt. Chromatographie (CH₂Cl₂) ergab 3.12g des Zielprodukts und 1.20g der Dibromo-Verbindung; ESI-MS 318.95, 316.95.
   ¹H-NMR (270 MHz, CDCl₃) δ (ppm): 7.98 (2H, m), 7.95 (1H, t), 7.81 (2H, m), 7.58 (1H, d), 7.35 (1H, d), 4.60 (2H, s).
c) Kaliumphtalimid (9.46mmol, 1.75g) wurde zu einer Lösung von 2-[6-(Bromomethyl)-2-pyridinyl]-1H-isoindol-1,3(2H)-dion (33b, 6.31mmol, 2.0g) in DMF (30ml) gegeben, das Reaktionsgemisch 15h lang auf 60°C erhitzt, 24h bei RT weitergerührt, mit Wasser (60ml) versetzt und 2h bei 0°C nachgerührt. Der Rückstand wurde filtriert und mit einer Mischung H₂O-DMF und anschließend mit Diethylether gewaschen; 2.12g; ESI-MS [M+H⁺] = 384.05.
   ¹H-NMR (270 MHz, CDCl₃) δ (ppm): 7.98-7.91 (4H, m), 7.88 (1H, t), 7.85-7.75 (4H, m), 7.38-7.32 (2H, m), 5.10 (2H, s).
d) 2-{6-[(1,3-Dioxo-1,3-dihydro-2H-isoindol-2-yl)methyl]-2-pyridinyl}-1H-isoindol-1,3(2H)-dion (33c, 5.22mmol, 2.0g) wurde mit Hydraziniumhydroxid (13.04mmol, 0.65g) in Methanol (50ml) 3h lang auf Rückfluss erhitzt. Zur Aufarbeitung wurde mit Wasser versetzt, eingeengt und die wässrige Phase mit konz. HCl angesäuert. Erneutes Einengen und Umkristallisieren (Ethanol) ergaben 1.20g eines weißen Feststoffs; ESI-MS [M+H⁺] = 124.05.
   ¹H-NMR (270 MHz, D₂O) δ (ppm): 7.94 (1H, t) , 7.08 (1H, d), 6.99 (1H, d), 4.33 (2H, s).

### trans-N-[4-(Aminomethyl)cyclohexyl]-N'-benzylharnstoff (34)

Die Herstellung erfolgte analog zu Verbindung 6 ausgehend von Benzyl-{4-[(tertbutoxycarbonyl)amino]cyclohexyl}methylcarbamat (EP 669317) durch Abspaltung der Boc-Gruppe mit 4n HCl in Dioxan. Aufbau des Benzylharnstoffs durch Umsetzung mit Benzylisocyanat und Triethylamin in DMF und nachfolgender Hydrogenolyse ergaben 0.55g Zielprodukt; ESI-MS [M+H⁺] = 262.20.

### 7-(4-Aminobutyl)-1,2,3,4-tetrahydro[1,8]naphthyridin (Bistrifluoroacetat) (35)

a.) Eine Lösung von 5-tert.-Butoxycarbonylaminovaleriansäure (50.0mmol, 10.86g), O,N-Dimethylhydroxylamin Hydrochlorid (50mmol, 4.88g), N-Methylmorpholin (0.30mol, 30.35g), HOBT (53.90mmol, 8.42g) und EDCI*HCl (55.0mmol, 10.54g) in CH₃CN (200ml) wurde 2Tage lang bei RT gerührt. Nach Eindampfen wurde der Rückstand in Ethylacetat aufgenommen, nacheinander mit Wasser, einer 10% KHSO₄-Lsg, ges. wässr. NaHCO₃ Lösung und ges. wässr. NaCl-Lösung gewaschen. Trocknen und Eindampfen der org. Phase ergab 6.96g gelbliches Öl; ESI-MS: [2M+Na⁺] = 543.3, [M+Na⁺] = 283.1, 205.1, 161.1.
   ¹H-NMR (270 MHz, CDCl₃) δ (ppm): 4.63 (1H, s. br.), 3.68 (3H, s), 3.21-3.05 (3+2H, m), 2.44 (2H, t), 1.76-1.48 (2+2H, m), 1.43 (9H, s).
b.) Zu einer Lösung von tert-Butyl 5-[methoxy(methyl)amino]-5-oxopentylcarbamat (35a, 30.0mmol, 6.9g) in THF (120ml) wurde Methylmagnesiumbromid (60.0mmol, 17.30ml einer 3M Lösung in Et₂O) bei 0°C zugetropft und 5h bei 0°C nachgerührt. Die Reaktionsmischung wurde dann vorsichtig mit einer 10% KHSO₄-Lsg angesäuert, mit Ethylacetat extrahiert und die org. Phase anschließend mit ges. wässr. NaHCO₃- und ges. wässr. NaCl-Lösung gewaschen, getrocknet und eingedampft: 5.5g gelbliches Öl; ESI-MS: [M-BOC+H⁺] = 116.15.
c.) Eine Mischung aus tert-Butyl 5-oxohexylcarbamat (35b, 9.29mmol, 2.0g), 2-Aminonicotinaldehyd (Heterocycl. 1993, 36, 2518; 11.20mmol, 1.37g) und KOH (0.37ml einer 20% wässr. Lsg) wurde 8h auf Rückfluss erhitzt. Eindampfen und Säulenchromatographie ergaben 1.60g des Zielprodukts; ESI-MS: [M+H⁺] = 302.15.
d.) Eine Suspension aus tert-Butyl 4-[1,8]naphthyridin-2-ylbutylcarbamat (35c, 5.31mmol, 1.60g) und Pd/C (10%, 1.5g) in Ethanol (40 ml) wurde über Nacht unter H₂-Atmosphäre gerührt, dann über Celite filtriert und mit Ethanol nachgewaschen. Säulenchromatographie ergab 290mg; ESI-MS: [M+H⁺] = 306.25.
   ¹H-NMR (360 MHz, CDCl₃) δ (ppm): 7.04 (1H, d), 6.29 (1H, d), 4.97 (1H, s.br.), 4.81 (1H, s.br.), 3.37 (2H, m sym.), 3.12 (2H, q br.), 2.65 (2H, t), 2.53 (2H, t), 1.89 (2H, quint.), 1.67 (2H, quint.), 1.51 (2H, quint.), 1.43 (9H, s).
e.) Zu einer Lösung von tert-Butyl 4-(5,6,7,8-tetrahydro[1,8]naphthyridin-2-yl)butylcarbamat (35d, 0.92mmol, 0.28g) in CH₂Cl₂ (8ml) wurde TFA (18.30mmol, 2.09g) zugesetzt, die Lösung 20h nachgerührt und eingedampft: 380mg; ESI-MS: 206.1, 130.7.
   ¹H-NMR (400 MHz, CDCl₃) δ (ppm) : 7.07 (1H, d), 6.31 (1H, d) , 5.58 (1H, s.br.), 3.39 (2H, m sym.), 2.96 (2H, s br.), 2.76 (2H, t) , 2.68 (2H, t), 2.56 (2H, t) , 1.88 (2H, quint.), 1.69 (2H, quint.), 1.51 (2H, quint.).

### [1-(2-Hydroxyethyl)-2-oxo-2,3,4,5-tetrahydro-1H-1-benzazepin-5-yl] essigsäure-tert-butylester (36)

Zu einer Lösung aus Diisopropylamin (11.0mmol, 1.11g) und Butyllithium (11.0mmol, 6.91ml einer Lösung 15% in Hexan) in THF (100ml) wurde bei 0°C (2-Oxo-2,3,4,5-tetrahydro-1H-1-benzazepin-5-yl)essigsäure-t.butylester (2) (10.9mmol, 3.0g) - gelöst in THF- zugetropft und die Lösung 1h weitergerührt. Anschließend wurden ca. 100ml Ethylenoxid zugegeben und die Mischung über Nacht bei RT nachgerührt. Die Lösung wurde zwischen ges. NH₄Cl und Ethylacetat verteilt, die organische Phase mit Wasser gewaschen und eingedampft; 2.7 g; ESI-MS: [2M+Na⁺] = 661.3, [M+K⁺] = 358.1, 321.1, [M+H⁺] = 320.1, 264.0.

### [2-Oxo-1-(2-oxoethyl)-2,3,4,5-tetrahydro-1H-1-benzazepin-5-yl]essigsäure-tert-butylester (37)

Zu einer Lösung aus Oxalylchlorid (7.93mmol, 1.0g) und DMSO (16.59mmol, 1.26g) in wenig CH₂Cl₂ wurde [1-(2-Hydroxyethyl)-2-oxo-2,3,4,5-tetrahydro-1H-1-benzazepin-5-yl]essigsäure-tert-butylester (36, 6.26mmol, 2.00g) gelöst in CH₂Cl₂ innerhalb 10 min. zugetropft. Nach 30min. wurde Triethylamin (38.22mmol, 3.87g) zugegeben, 5min. weitergerührt, auf RT kommen lassen und über Nacht bei RT gerührt. Zur Aufarbeitung wurde mit Wasser versetzt, die Mischung mit CH₂Cl₂ extrahiert und die org. Phase mit ges. NaCl-, 1%-H₂SO₄- und mit 5%-NaHCO₃-Lösung gewaschen. Einengen ergab 1.8g Zielprodukt; ESI-MS: 693.2, [M+K⁺] = 358.1, 319.1, [M+H⁺] = 318.1, 262.0.

### 2-Amino-5-chlorbenzoesäure-methylester (38)

Zu einer Lösung aus 2-Chlor-5-aminobenzoesäure (0.23mmol, 40.0g) in Methanol (400ml) wurde Thionylchlorid (0.47mmol, 55.46g) bei 0°C zugetropft und die Mischung auf 50-60°C erhitzt. Nach beendeter Umsetzung wurde mit Wasser versetzt und mit Ethylacetat extrahiert. Anschließend wurde die org. Phase nacheinander mit 1n NaOH- und verd. HCl-Lösung (pH 1-2) gewaschen und eingeengt; 23.0 g; ESI-MS: [M+H⁺] = 186.05.

### 4-Chlor-2-[(4-ethoxy-4-oxobutanoyl)amino]benzoesäuremethylester (39)

Zu einer Lösung aus 2-Amino-5-chlorbenzoesäure-methylester (38, 123.9mmol, 23.0g) und Pyridin (0.26mol, 20.58g) in Toluol (40ml) wurde Bernsteinsäureethylesterchlorid (0.14mol, 22.44g) in Toluol (15 ml) bei 0°C zugetropft. Die Lösung wurde über Nacht bei RT nachgerührt mit Wasser versetzt und mit Ethylacetat extrahiert. Die organische Phase wurde mit 1N HCl-Lsg, mit Wasser, mit einer ges. NaHCO₃-Lsg, mit einer ges. NaCl-Lsg gewaschen. Einengen, umkristallisieren (Methanol) ergaben 34.1 g Zielprodukt; ESI-MS: [2M+Na⁺] = 649.0, [M+K⁺] = 352.0, [M+H⁺] = 314.05;
¹H-NMR (270 MHz, CDCl₃) δ (ppm) : 11.06 (1H, s br.), 8.68 (1H, d), 7.99 (1H, m), 7.47 (1H, dd), 4.16 (2H, q), 3.92 (3H, s), 2.74 (4H, m), 1.24 (3H, t).

### Ethyl-7-chloro-5-hydroxy-2-oxo-2,3-dihydro-1H-1-benzazepin-4-carboxylat (40)

4-Chlor-2-[(4-ethoxy-4-oxobutanoyl)amino]benzoesäuremethylester (39, 0.16mol, 50.20g) in DMSO (250ml) wurde bei 15°C zu einer Suspension aus entöltem NaH (0.27mol) in THF (50ml) und DMSO (80 ml) zugetropft und die Mischung 2h bei RT nachgerührt. Bei 0°C wurde Eisessig (24ml) zugegeben und 20min nachgerührt. Wasser (25ml) wurde zugegeben, der entstandene Niederschlag abfiltriert, mit Wasser gewaschen, in CH₂Cl₂ aufgenommen, mit Wasser extrahiert und die org. Phase eingeengt. Der Rückstand wurde anschließend mit Diethylether (50ml) ausgerührt, filtriert und getrocknet; 32g einer 6:4-Mischung Methyl-/Ethyl-ester, die nicht getrennt wurde; ESI-MS (Me-Ester): [M+K⁺] = 307.9, [M+Na⁺] = 290.0, [M+H⁺] = 268.0; ESI-MS (Et-Ester): [M+K⁺] = 321.9, [M+Na⁺] = 304.0, [M+H⁺] = 282.0.

### 7-Chloro-3,4-dihydro-1H-1-benzazepine-2,5-dion (41)

Ethyl-7-chloro-5-hydroxy-2-oxo-2,3-dihydro-1H-1-benzazepin-4-carboxylat (40, 0.11mol, 32.0g) wurde in DMSO (500ml) und Wasser (0.23mol, 4.09g) auf 150°C erhitzt und 2h gerührt. Wasser wurde bei 100°C zugegeben, die Mischung auf 0°C gekühlt und der entstandene Niederschlag abfiltriert. Trocknen ergab 19.0g; ESI-MS: [M+Na⁺] = 251.1, [M+H⁺] = 211.9, 209.95, 130.1.

### (2E,Z)-(7-Chlor-2-oxo-1,2,3,4-tetrahydro-5H-1-benzazepin-5-yliden)ethansäure-tert-butylester (42)

Zu einer Suspension aus entöltem NaH (0.10 mol) in DMF (40ml) wurde bei 0°C Diethylphosphonessigsäure-t-butylester (0.10mol, 25.84g) zugetropft und gerührt, bis eine klare Lösung entsteht. Bei 0°C wurde 7-Chloro-3,9-dihydro-1H-1-benzazepin-2,5-dion (41, 90.63mmol, 19.0g) in DMF (185ml) zugetropft und bei RT gerührt. Das Reaktionsgemisch wurde mit Wasser versetzt, 1h nachgerührt und der entstandene gelbe Rückstand abgesaugt, mit Wasser gewaschen und in CH₂Cl₂ aufgenommen. Die organische Phase wurde mit einer 5%-NaHCO₃-Lösung gewaschen und eingeengt. Umkristallisieren ergab 23.5g Zielprodukt; ESI-MS: [2M+H⁺] = 615.2, [M+Na⁺] = 330.0, 293.0, 254.1, 252.1.
¹H-NMR (400 MHz, CDCl₃) δ (ppm) : 9.59 (1H, s br.), 7.45 (1H, m), 7.25 (1H, m), 7.06 (1H, m), 5.99 (1H, t), 3.43 (2H, s), 2.84 (2H, d), 1.32 (9H, s).

### (7-Chloro-2-oxo-2,3,4,5-tetrahydro-1H-1-benzazepin-5-yl)essigsäure-tert-butylester (43)

(2E,Z)-(7-Chloro-2-oxo-1,2,3,4-tetrahydro-5H-1-benzazepin-5-yliden)ethansäure-tert-butylester (42, 75.0mmol, 23.08g) in Ethanol/Dioxan (250ml/100ml) wurde 4Tage lang mit Pt/Kohle (5%, 4.1g) unter Standardbedingungen hydriert Das Reaktionsgemisch wurde mit Wasser versetzt, 1h nachgerührt, der gelbe Rückstand abgesaugt, mit Wasser gewaschen und in CH₂Cl₂ aufgenommen. Die organische Phase wurde mit einer 5%-NaHCO₃-Lösung gewaschen und eingeengt. Umkristallisieren ergab 23.5g eines Feststoffs (Mischung aus Zielprodukt und der entsprechenden dechlorierten Verbindung; die Mischung wurde direkt weiter umgesetzt); ESI-MS: [2M+H⁺] = 618.94, [M+K⁺] = 350.66, 309.75, 254.11.

### [5-(2-tert-Butoxy-2-oxoethyl)-7-chloro-2-oxo-2,3,4,5-tetrahydro-1H-1-benzazepin-1-yl]essigsäure-methylester (44)

Zu entöltem NaH (69.00 mmol) in DMF (5 ml) wurde (7-Chloro-2-oxo-2,3,4,5-tetrahydro-1H-1-benzazepin-5-yl)essigsäure-tert-butylester (43, 60.00mmol, 18.59g) in DMF (10ml) bei 15°C zugetropft und die Mischung über Nacht bei RT nachgerührt. Das Reaktionsgemisch wurde mit Eiswasser versetzt, mit Ethylacetat (2x) extrahiert und die org. Phase mit einer 10% CH₃COOH-Lsg, Wasser und anschließend mit 1n NaOH gewaschen. Einengen ergab 20.4g Rohprodukt, das ohne weitere Reinigung umgesetzt wurde.

### [5-(2-tert-Butoxy-2-oxoethyl)-7-chloro-2-oxo-2,3,4,5-tetrahydro-1H-1-benzazepin-1-yl]essigsäure (45)

[5-(2-tert-Butoxy-2-oxoethyl)-7-Chloro-2-oxo-2,3,4,5-tetrahydro-1H-1-benzazepin-1-yl]essigsäure-methylester (44, 50.28mmol, 19.2g) gelöst in Dioxan (250ml) wurde mit KOH (80.45mmol, 4.51g) in Wasser (150ml) versetzt und 1h bei RT gerührt. Das Reaktionsgemisch wurde eingeengt, mit Wasser (100ml) versetzt und mit Ethylacetat (2x) extrahiert. Nach dem Einengen wurde der Rückstand in Diethylether aufgenommen und durch Zugabe von n-Pentan gefällt. Umkristallisieren (Diisopropylether, 2x) ergab 4.8g (enthält ca. 15% der entsprechenden Deschloro-Verbindung); ESI-MS: [M+Na⁺] = 390.0, 314.0, 312.0.

### [4-(Aminomethyl)phenyl]guanidin (Bishydrochlorid) (46)

p-Aminobenzylamin (6.7g; 54.84mmol) wurde in 20ml 6n HCl suspendiert und unter Rückfluß 5.3g Cyanamid -gelöst 5ml H₂0 - langsam zugetropft. Nach beendeter Reaktion wurde die Lösung bei 0°C mit 50% NaOH-Lösung versetzt, der entstandene Niederschlag abgesaugt, in 50ml Ethanol aufgekocht und filtriert. Einengen der Mutterlauge und Verrühren des erhaltenen Rückstands mit Diethylether ergab 1.4g gelbe Festkörper; Fp.: 255°C.

### 7,8-Dimethoxy-3,4-dihydro-1H-1-benzazepin-2,5-dion (47)

Analog zur Herstellung der Bausteine 39, 40 und 41 wurde zuerst Ethyl-2-amino-4,5-dimethoxybenzoat (20g; 88.8mmol) mit Bernsteinsäureethylester-chlorid in das entsprechende Amid überführt. Nach Verrühren mit n-Pentan wurden 30.4g Festkörper erhalten; ESI-MS [M+H+]: 354.15.
¹H-NMR (400 MHz, DMSO) δ (ppm) : 10.65 (s, 1H), 8.10 und 7.40 (jeweils s, 1H), 4.30 und 4.10 (jeweils q, 2H), 3.85 und 3.80 (jeweils s, 3H), 2.70 (m, 4H), 1.30 und 1.20 (jeweils t, 3H).
Anschließende Cyclisierung mit 25g des erhaltenen Amids unter Verwendung von NaH analog zu 40 und übliche Aufarbeitung ergab 19.5g weiße Festkörper; ESI-MS [M+H⁺]: 308.05.
¹H-NMR (400 MHz, DMSO) δ (ppm): 13.3 (s, 1H), 10.10 (s, 1H), 7.25 und 6.7.5 (je s, 1H), 4.30 (q, 2H), 3.80 (s, 6H), 2.95 (s, 2H), 1.35 (t, 3H).
Decarboxylierung analog zu 41 ausgehend von 17g ergab 10.5g des Zielprodukts als Feststoff; ESI-MS [M+H⁺]: 236.15.
¹H-NMR (400 MHz, DMSO) δ (ppm) : 9.90 (s, 1H), 7.35 und 6.80 (jeweils s, 1H), 3.80 und 3.75 (je s, 3H), 2.85 und 2.65 (je m, 2H).

### tert-Butyl-(7,8-dimethoxy-2-oxo-2,3,4,5-tetrahydro-1H-1-benzazepin-5-yl)acetat (48)

Herstellung analog zu Bausteinen 42 und 43 durch Wittig-Horner-Reaktion und anschließende Hydrierung; nach Verrühren mit n-Pentan wurden 8.16g Festkörper erhalten; ESI-MS [M+H⁺-tBu]: 280.15.

### [5-(2-tert-Butoxy-2-oxoethyl)-7,8-dimethoxy-2-oxo-2,3,4,5-tetrahydro-1H-1-benzazepin-1-yl]acetat (49)

Analog zu 44 und 45 ausgehend von 4g tert-Butyl-(7,8-dimethoxy-2-oxo-2,3,4,5-tetrahydro-1H-1-benzazepin-5-yl)acetat wurden 2.6g des Zielprodukts als heller Schaum isoliert; [M+H⁺-tBu]: 338.15.
¹H-NMR (400 MHz, DMSO) δ (ppm): 6.85 und 6.75 (je s, 1H), 4.35 (s breit, 2H), 3.80 und 3.75 (je s, 3H), 3.60 (s, 2H) , 2.70 (m, 2H), 2.25 (m, 1H), 2.15 (m, 2H),1.60 (m, 1H)*,* 1.35 (s, 9H).

### [1-(2-Methoxy-2-oxoethyl)-2-oxo-2,3-dihydro-1H-1-benzazepin-5-yl]essigsäure (50)

a.) Eine Lösung von 37g (135.3mmol) (2-Oxo-2,3-dihydro-1H-1-benzazepin-5-yl)essigsäure-t.butylester (1) und 3.7g Tetrabutylammoniumbromid in 370ml DMF wurde bei Raumtemperatur mit 56.1g (406mmol) gepulvertem K₂CO₃ versetzt, 22.7g (148.9mmol)Bromessigsäuremethylester zugetropft, anschließend 3h bei 40°C und über Nacht bei Raumtemperatur gerührt. Das Reaktionsgemisch wurde in 1000ml Wasser-Eis-Gemisch gegossen, und 2x mit je 200ml Methyl-tert.butylether extrahiert. Die vereinigten Extrakte wurden mit H₂O, 5% NaHCO₃- und NaCl-Lösung gewaschen, über Na₂SO₄ getrocknet, filtriert und eingeengt. Das verbliebene gelbliche Öl (ca. 52g, Reinheit ca. 90%) wurde ohne weitere Aufreinigung umgesetzt; ESI-MS [M+H⁺]: 346.
b.) 9.2g (26.6mmol) des Rohprodukts 50a wurden in 66.6ml 4n HCl in Dioxan gelöst und 24h bei 50°C gerührt, anschließend das Dioxan weitgehend abdestilliert, der Rückstand mit 5% NaHCO₃-Lösung und Diethylether versetzt, die wässrige Phase erneut mit Diethylether gewaschen und mit 1n KHSO₄-Lösung sauer gestellt. Die sich abscheidende Säure wurde mit Diethylether extrahiert, die Etherphase mit NaCl-Lösung gewaschen, über Na₂SO₄ getrocknet, abgesaugt und eingeengt. Es verblieben 3.2g leicht gelbliches Öl; ESI-MS [M+H⁺]: 290.

### trans-[4-(1H-Benzimidazol-2-yl)cyclohexyl]methanamin (Hydrochlorid) (51)

39.3g (0.25mol) trans-4-(Aminomethyl)cyclohexancarbonsäure und 27.0g (0.25mol) 1,2-Phenylendiamin wurden analog zu J. Heterocycl. Chem. 26, 541 (1989) in einem Gemisch aus 167ml konz. Salzsäure und 333ml H2O 18h lang auf Rückfluss erhitzt. Die grüne Reaktionslösung wurde bis zum Auftreten eines gelben Kristallbreis eingeengt und mit 400ml Isopropanol ausgerührt, abfiltriert, mit 90% Isopropanol und zuletzt mit Diethylether ausgewaschen. Nach 2maliger Umkristallisation aus einem Isopropanol-Wasser-Gemisch (70/30) verblieben 30g weißes Monohydrochlorid; ESI-MS [M+H⁺]: 230.

### (2-Oxo-2,3,4,5-tetrahydro-1H-1,5benzodiazepin-1-yl)essigsäuremethylester (52)

Zu einer eisgekühlten Lösung von 12.2g (75.3mmol) 1,3,4,5-Tetrahydro-2H-1,5-benzodiazepin-2-on (Herstellung: J. Am. Chem. Soc. 1949, 71, 1985) in 350ml DMF gab man 1.9g (79.8mmol) NaH (60% Dispersion in Mineralöl), ließ 30min. bei 0-5°C und 10min. bei Raumtemperatur nachrühren. Anschließend wurden bei 0°C 11.5g (75.3mmol) Bromessigsäuremethylester zugetropft und dann bei gleicher Temperatur 30min. nachgerührt. Die Reaktionslösung wurde auf 600ml Eiswasser gegossen und 3x mit je 150ml Ethylacetat extrahiert. Die organische Phase wurde mit NaCl-Lösung gewaschen, über MgSO₄ getrocknet und Ethylacetat abdestilliert. Der Rückstand wurde säulenchromatographisch gereinigt (Eluent: CH₂Cl₂/CH₃OH 9/1). Es wurden 9.6g gelbliches Öl isoliert; ESI-MS [M+H⁺]: 235.

### (2-Oxo-2,3,4,5-tetrahydro-1H-1,5-benzodiazepin-1-yl)essigsäure-tert.butylester (53)

Die Herstellung erfolgte analog zu Baustein 52 durch Umsetzung von 11.9g (73mmol) 1,3,4,5-Tetrahydro-2H-1,5-benzodiazepin-2-on mit 14.3g (73mmol) Bromessigsäure-tert.butylester. Es wurden 17g eines schwach gelblichen Öls isoliert; ESI-MS [M+H⁺]: 277.

### [5-(2-tert-Butoxy-2-oxoethyl)-2-oxo-2,3,4,5-tetrahydro-1H-1,5-benzodiazepin-1-yl]essigsäure (54)

a.) Zu einer Lösung von 9.6g (41mmol) Verbindung 52 und 8.0g (41mmol) Bromessigsäure-tert.butylester in 90ml DMF gab man bei 0°C 14.2g (102mmol) gepulvertes K₂CO₃ , ließ 1h bei 0° C und dann 14h bei Raumtemperatur nachrühren. Das Reaktionsgemisch wurde auf 300ml Eiswasser gegossen und 3x mit je 100ml Methyl-tert.butylether extrahiert. Die vereinigten organischen Phasen wurden mehrmals mit NaCl-Lösung gewaschen, über MgSO₄ getrocknet und zur Trockene eingedampft. Der Rückstand wurde säulenchromatographisch gereinigt (Eluent: Ethylacetat/Cyclohexan 7/3). Es wurden 7.0g eines schwach gelblichen Öls isoliert; ESI-MS [M+H⁺]: 349.
b.) Die alkalische Verseifung des Methylesters wurde analog zu 3b durchgeführt. Es wurden 3.8g weiße Kristalle erhalten; Fp.: 140-142°C; ESI-MS [M+H⁺]: 335.

### [5-(2-tert-Butoxy-2-oxoethyl)-4-oxo-2,3,4,5-tetrahydro-1H-1,5-benzodiazepin-1-yl]essigsäure (55)

Analog Baustein 54a wurde (2-Oxo-2,3,4,5-tetrahydro-1H-1,5-benzodiazepin-1-yl)essigsäure-tert.butylester (53) mit Bromessigsäuremethylester umgesetzt und anschließend analog zu 3b alkalisch verseift. Nach säulenchromatographischer Reinigung wurden 2.9g weiße Kristalle erhalten; Fp.: 82-84°C; ESI-MS [M+H⁺] : 335.

### [1-(2-Methoxy-2-oxoethyl)-2-oxo-2,3,4,5-tetrahydro-1H-1-benzazepin-5-yl]essigsäure (56)

14.5g (42mmol) Baustein 3a wurden in 105ml 4n HCl in Dioxan gelöst und 2 Tage bei 50°C gerührt. Nach Abziehen des Lösungsmittels wurde der Rückstand in 5% NaHCO₃-Lösung gelöst, 2x mit Methyl-tert.butylether extrahiert, anschließend die wässrige Phase 1n KHSO₄-Lösung sauer gestellt und die sich abscheidende Säure mit Methyl-tert.butylether extrahiert. Nach Trocknen über MgSO₄, Abziehen des Lösungsmittels und säulenchromatographischer Reinigung (Eluent: CH₂Cl₂/CH₃OH/Eisessig 45/5/1) verblieben 1.6g zähes, leicht gelbliches Öl; ESI-MS [M+H⁺] : 292.

### [(5R)-5-(2-tert-Butoxy-2-oxoethyl)-2-oxo-2,3,4,5-tetrahydro-1H-1-benzazepin-1-yl]essigsäure (57)

14.2g (42.6mmol) [5-(2-t.Butoxy-2-oxoethyl)-2-oxo-2,3,4,5-tetrahydro-1H-benzazepin-1-yl]essigsäure (3) wurden in 170ml Diethylether suspendiert und durch Zugabe von 7.3g (42.64mmol) (1S)-(-)-1-Napthyl)ethylamin in Lösung gebracht. Die gelbe Lösung wurde mit (1S)-1-Napthyl)ethanaminium[(5R)-5-(2-tert.butoxy-2-oxoethyl)-2-oxo-2,3,4,5-tetrahydro-1H-benzazepin-1-yl]acetat 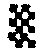 hergestellt durch präparative HPLC-Trennung von Verbindung 3 mittels chiraler Säuel (Chiralpak AD 500x; 50mm; 20µm) und nachfolgender Salzbildung- angeimpft, der ausgeschiedene Niederschlag nach 3.5h abgesaugt und 3x aus einem Ethylacetat/Isopropanol-Gemisch umkristallisiert. Die Enantiomerenreinheit wurde mittels chiraler HPLC überprüft. 3.5g des Salzes wurden in 30ml eines Diethylether/Hexan-Gemisches 10/3 suspendiert und nach Zugabe von 50ml einer 5% wässrigen Amidosulfonsäure-Lösung bis zum Auftreten einer klaren Phase gerührt. Nach Abtrennen der wässrigen Phase wurde die organische Phase 3x mit 5ml Amidosulfonsäure- und dann NaCl-Lösung gewaschen, über Na₂SO₄ getrocknet und eingeengt. 2.25g amorpher Rückstand; ESI-MS [M+H⁺] : 505.

### [(5S)-5-(2-tert-Butoxy-2-oxoethyl)-2-oxo-2,3,4,5-tetrahydro-1H-1-benzazepin-1-yl]essigsäure (58)

Aus den vereinigten Mutterlaugen von Baustein 57 wurde wie beschrieben mit Amidosulfonsäurelösung 8g der Säure als gelblich amorpher Rückstand isoliert, diese mit (1R)-(+)-1-Napthyl)ethylamin in das diastereomere Salz überführt und bis zum Vorliegen von Enantiomerenreinheit umkristallisiert. Analog Beispiel 57 wurden daraus 2.5g der rechtsdrehenden Säure als amorpher Rückstand isoliert; ESI-MS [M+H⁺]: 505.
Eine Probe der Säure wurde mit 4-Brombenzylamin in das gut kristallisierende Amid überführt und mittels Röntgenstrüktur-Analyse die absolute Konfiguration ermittelt.

### tert-Butyl-(5-oxo-5,6-dihydro-4H-thieno[3,2-b]azepin-8-yl)acetat (59)

Eine Lösung von 5.3g (29.2mmol) 6,7-Dihydro-4H-thieno[3,2-b]azepin-5,8-dion (Arch. Pharm. 1991 324, 579) und 12g (32.2mmol) (tert.Butoxycarbonylmethylen)-triphenylphosphoran in 15ml Toluol wurde 10h lang auf Rückfluss erhitzt, anschließend Toluol abdestilliert und der schwarze Rückstand säulenchromatographisch gereinigt (Eluent: Ethylacetat/Cyclohexan 7/3). Die einheitliche Fraktion wurde nochmals mit 40ml siedendem Cyclohexan digeriert, abgekühlt und abgesaugt. 3g gelbliche Kristalle; ESI-MS [M+H⁺] : 280.

### tert-Butyl-(5-oxo-5,6,7,8-tetrahydro-4H-thieno[3,2-b]azepin-8-yl)acetat (60)

3g (11mol) tert-Butyl-(5-oxo-5,6-dihydro-4H-thieno[3,2-b]azepin-8-yl)acetat wurden analog Baustein 2 in Gegenwart von 10% Pd/C hydriert. Da nach 6h laut HPLC noch Edukt vorhanden war, wurde der Katalysator abgesaugt und nach Zugabe von neuem Katalysator nochmals 6h hydriert. Nach Aufarbeitung und chromatographischer Reinigung (Eluent: Ethylacetat/Cyclohexan 7/3) wurden 1.4g gelbliche Kristalle isoliert, die gemäß HPLC noch ca. 2.5% Ausgangsmaterial enthielten; ESI-MS [M+H⁺] : 282.

### [8-(2-tert-Butoxy-2-oxoethyl)-5-oxo-5,6,7,8-tetrahydro-4H-thieno[3,2-b]azepin-4-yl]essigsäure (61)

Die Herstellung erfolgte analog zu Baustein 50.
Esterstufe: 1.5g gelbliche Kristalle; ESI-MS [M+H⁺]: 354.
Zielprodukt: 1.2g gelbliche Kristalle; ESI-MS [M+H⁺]: 340.
I.B Verbindungen der Formel I bzw. I'

### Beispiel I:

### [1-(2-{[(2-{[Amino(imino)methyl]amino}-1,3-thiazol-5-yl)methyl]amino}-2-oxoethyl)-2-oxo-2,3,4,5-tetrahydro-1H-1-benzazepin-5-yl]essigsäure-t.butylester

Zu 1.55g (4.65mmol) [5-(2-t.Butoxy-2-oxoethyl)-2-oxo-2,3,4,5-tetrahydro-1Hbenzazepin-1-yl]essigsäure **(3)** und 1.22g (4.8mmol) *N*-[5-(Aminomethyl)-1,3-thiazol-2-yl]guanidin-dihydrochlorid **(5)** in 20ml DMF wurden bei 0°C 1.1g N-Methylmorpholin zugetropft, und anschließend über 35' lang 1.55g (4.7mmol) TOTU (O-[(Ethoxycarbonyl)cyanomethylenamino]-N,N,N',N'-tetrafluoroborat) portionsweise eingetragen. Die gelbe Reaktionslösung wurde 1h lang bei 0°C nachgerührt und anschließend im Vakuum weitgehend eingedampft. Der Rückstand wurde dann mehrmals mit H₂O digeriert, in einer Mischung aus 120ml Ethylacetat und 40ml Diethylether aufgenommen, mit 10% K₂CO₃- und NaCl-Lösung gewaschen, über Na₂SO₄ getrocknet und eingeengt, wobei das Rohprodukt durchkristallisierte. Reinigung durch Chromatographie an Kieselgel (CH₂Cl₂/CH₃OH/NH₃ 42:8:0.1) und Kristallisation aus Ethylacetat/n-Hexan ergaben 1.45g (65%) weiße Kristalle.
Fp.: 190-193°C (Zers.); FAB-MS [M+H⁺]: 487.

### Beispiel II:

### [1-(2-{[(2-{[Amino(imino)methyl]amino}-1,3-thiazol-5-yl)methyl]amino}-2-oxoethyl)-2-oxo-2,3,4,5-tetrahydro-1H-1-benzazepin-5-yl]essigsäure

1.2g des t.Butylesters aus Beispiel I wurden in 70ml CH₂Cl₂ suspendiert, mit 45ml 4n HCl in Dioxan versetzt und über Nacht bei RT gerührt. Die Lösung wurde eingedampft, der Rückstand mehrmals mit CH₂Cl₂ digeriert und anschließend getrocknet. Auf diese Weise wurden 1.07g eines leicht gelblichen amorphen Pulvers isoliert; FAB-MS [M+H⁺]: 432.

### Beispiel III:

### [1-(2-{[4-(1H-Benzimidazol-2-ylamino)benzyl]amino}-2-oxoethyl)-2-oxo-2,3,4,5-tetrahydro-1H-1-benzazepin-5-yl]essigsäure

Die Herstellung erfolgte analog zu Beispiel I durch Umsetzung von ) [5- (2-t.Butoxy-2-oxoethyl)-2-oxo-2,3,4,5-tetrahydro-1H-benzazepin-1-yl]essigsäure **(3)** mit *N*-[4-(Aminomethyl)phenyl]-1*H*-benzimidazol-2-amin-hydrochlorid **(4)** und nachfolgender Spaltung der t.Butylgruppe analog Beispiel II. Es wurde ein leicht gelblicher amorpher Rückstand erhalten, FAB-MS [M-H⁺]: 498.

### Beispiel IV:

### [1-(2-{[(2-{[Amino(imino)methyl]amino}-1,3-thiazol-5-yl)methyl]amimo}-2-oxoethyl)-2-oxo-2,3-dihydro-1H-1-benzazepin-5-yl]essigsäure-t.butylester

Analog zur Herstellung von Verbindung **3a** wurden 0.9g (3.3mmol) (2-Oxo-2,3-dihydro-1H-1-benzazepin-5-yl)essigsäure-t.butylester **(1)** mit Bromessigsäuremethylester alkyliert (FAB-MS [M-H⁺]: 346) und dann analog zu **3b** verseift (0.44g; FAB-MS [M-H⁺]: 332).
Kupplung von 0.57g (1.72mmol) [5-(2-t.Butoxy-2-oxoethyl)-2-oxo-2,3-dihydro-1*H*-1-benzazepin-1-yl]essigsäure mit *N-*[5- (Aminomethyl)-1,3-thiazol-2-yl]guanidindihydrochlorid **(5)** analog Beispiel I ergab die Titelverbindung als leicht gelbliches Pulver; FAB-MS [M+H⁺]: 485.

### Beispiel V:

### [1-(2-{[(2-{[Amino(imino)methyl]amino}-1,3-thiazol-5-yl)methyl]amino}-2-oxoethyl)-2-oxo-2,3-dihydro-1H-1-benzazepin-5-yl]essigsäure

Die Abspaltung des t.Butylesters erfolgte analog zu Beispiel II und ergab 0.42g der Titelverbindung als leicht gelbliches Pulver; ; FAB-MS [M+H⁺] : 429.

### Beispiel VI:

### (1-{2-[(4-{[(Benzylamino)carbonyl]amino}benzyl)amino]-2-oxoethyl}-2-oxo-2,3,4,5-tetrahydro-1H-1-benzazepin-5-yl)essigsäure

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure **(3)** mit *N*-[4-(Aminomethyl) phenyl]-*N*'-benzylharnstoff **(6)** und nachfolgender Spaltung der t.Butylgruppe analog Beispiel II. Reinigung des Rohprodukts durch Elution über eine Kieselgel-Kartusche (Fa. Chromasorb; CH₂Cl₂/CH₃OH 0-20%) ergab 27mg als amorphen Feststoff; ESI-MS [M+H⁺]: 515.2; [M+K⁺] : 553.2.

### Beispiel VII:

### [1-(2-{[4-(1H-Benzimidazol-2-yl)benzyl]amino}-2-oxoethyl)-2-oxo-2,3,4,5-tetrahydro-1H-1-benzazepin-5-yl]essigsäure

Die Herstellung erfolgte analog zu Beispiel I durch Umsetzung von [5- (2-t.Butoxy-2-oxoethyl)-2-oxo-2,3,4,5-tetrahydro-1H-benzazepin-1-yl]essigsäure **(3)** mit [4-(1*H*-Benzimidazol-2-yl)phenyl]methanamin **(7)** und nachfolgender Spaltung der t.Butylgruppe analog Beispiel II. Reinigung des Rohprodukts durch Elution über eine Kieselgel-Kartusche (Fa. Chromasorb; CH₂Cl₂/CH₃OH 0-20%) ergab 9mg als amorphen Feststoff; ESI-MS [M+H⁺] : 483.25.

### Beispiel VIII:

### [1-(2-{[4-(1H-Benzimidazol-2-ylamino)butyl]amino}-2-oxoethyl)-2-oxo-2,3,4,5-tetrahydro-1H-1-benzazepin-5-yl]essigsäure

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure **(3)** mit N¹-(1*H*-Benzimidazol-2-yl)butan-1,4-diamin (Trifluoracetat) **(9)** und nachfolgender Spaltung der der t.Butylgruppe analog Beispiel II. Nach chromatographischer Reinigung über Kieselgel (Eluent: CH₂Cl₂/CH₃OH/50% Essigsäure 42:8:0.7) wurden 0.5g als schwach gelbliches amorphes Pulver isoliert; FAB-MS [M+H⁺] : 464.

### Beispiel IX:

### [1-(2-{[5-(1H-Benzimidazol-2-ylamino)pentyl]amino}-2-oxoethyl)-2-oxo-2,3,4,5- tetrahydro-1H-1-benzazepin-5-yl]essigsäure

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure (3) mit N¹-(1*H*-Benzimidazol-2-yl)pentan-1,5-diamin (Hydrochlorid) **(8)** und nachfolgender Spaltung der der t.Butylgruppe analog Beispiel II. Nach chromatographischer Reinigung über Kieselgel (Eluent: CH₂Cl₂/CH₃OH/50% Essigsäure 42:8:0.7) wurden 0.48g als schwach gelbliches amorphes Pulver isoliert; FAB-MS [M+H⁺] : 478.

### Beispiel X:

### {1-[2-({4-[(1H-Benzimidazol-2-ylamino)methyl]cyclohexyl}amino)-2-oxoethyl]-2-oxo-2,3,4,5-tetrahydro-1H-1-benzazepin-5-yl}essigsäure

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure (3) mit *trans-N*-[(4-Aminocyclohexyl)methyl]-1*H*-benzimidazol-2-amin (Dihydrochlorid) **(10)** und nachfolgender Spaltung der der t.Butylgruppe analog zu Beispiel II. Nach chromatographischer Reinigung über Kieselgel wurden 0.7g schwach gelbliches amorphes isoliert; FAB-MS [M+H⁺]: 504.

### Beispiel XI:

### {1-[2-({[4-(1H-Benzimidazol-2-ylamino)cyclohexyl]methyl}amino)-2-oxoethyl]-2-oxo-2,3,4,5-tetrahydro-1H-1-benzazepin-5-yl}essigsäure

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure (3) *trans*-*N*-{[4-(Aminomethyl)cyclohexyl]}-1*H*-benzimidazol-2-amin (Dihydrochlorid) (11) und nachfolgender Spaltung der der t.Butylgruppe analog zu Beispiel II. Nach chromatographischer Reinigung über Kieselgel wurden 0.5g schwach gelbliches amorphes isoliert; FAB-MS [M+H⁺]: 504.

### Beispiel XII:

### [2-Oxo-1-(2-oxo-2-{[4-(pyridin-2-ylamino)benzyl]amino}ethyl)-2,3,4,5-tetrahydro-1H-1-benzazepin-5-yl]essigsäure

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure **(3)** mit *N*-[4-(Aminomethyl)phenyl]-2-pyridinamin **(13)** und nachfolgender Spaltung der der t.Butylgruppe analog zu Beispiel II.4mg; ESI-MS [M+H⁺] :459.15.

### Beispiel XIII:

### {1-[2-({[6-(1H-Benzimidazol-2-yl)-pyridin-3-yl]methyl}amino)-2-oxoethyl]-2-oxo-2,3,4,5-tetrahydro-1H-1-benzazepin-5-yl}essigsäure (Bishydrochlorid)

Die Herstellung erfolgte analog zu Beispiel I durch Umsetzung von [5-(2-t.Butoxy-2-oxoethyl)-2-oxo-2,3,4,5-tetrahydro-1H-benzazepin-1-yl]essigsäure **(3)** [6-(1*H*-Benzimidazol-2-yl)pyridin-3-yl]methanamin (Trifluoracetat) **(12)** und nachfolgender Spaltung der der t.Butylgruppe analog zu Beispiel II. 13mg; ESI-MS [M+H⁺] :484.15.

### Verbindungen der allgemeinen Formel I

analog zu Beispiel II wurden hergestellt:

### Beispiel 14:

### {2-Oxo-1-[2-oxo-2-({4-[(pyridin-2-ylamino)methyl]benzyl}amino)ethyl]-2,3,4,5-tetrahydro-1H-1-benzazepin-5-yl}essigsäure

Unter Verwendung von N-[4-(Aminomethyl)benzyl]-2-pyridinamin (14) als Edukt wurden nach MPLC 207mg als amorpher Feststoff erhalten; ESI-MS [M+H⁺] : 473.28.
¹H-NMR (360 MHz, d₆-DMSO) δ ppm: 8.5 (m, 1H), 7.95 (d, 1H), 7.45 (m, 1H), 7.35-72.0 (m, 9H), 6.60 (m, 2H), 4.45 (m, 3H), 4.25 (m, 3H), 3.55 (m, 1H), 2.70 (m, 2H), 2.35 (m, 1H), 2.20 (m, 2H), 1.65 (m 1H).

### Beispiel 15:

### {1-[2-({[5-(1H-Benzimidazol-2-yl)thien-2-yl]methyl}amino)-2-oxoethyl]-2-oxo-2,3,4,5-tetrahydro-1H-1-benzazepin-5-yl}essigsäure

Unter Verwendung von [5-(1H-Benzimidazol-2-yl)thien-2-yl]methanamin (15) als Edukt wurden nach MPLC 210mg als amorphe weiße Festkörper erhalten; ESI-MS [M+H⁺] : 489.27.
¹H-NMR (360 MHz, d₆-DMSO) δ ppm: 8.80 (m, 1H), 7.70 (m, 1H), 7.65 (m, 2H), 7.35-7.15 (m, 6H), 7.05 (m, 1H), 4.55 (m, 3H), 4.20 (m, 1H), 3.55 (m, 1H), 2.75 (m , 2H), 2.35 (m, 1H), 2.15 (m, 2H), 1.65 (m, 1H).

### Beispiel 16:

### {1-[2-({2-[4-(1H-Benzimidazol-2-yl)phenyl]ethyl}amino)-2-oxoethyl]-2-oxo-2,3,4,5-tetrahydro-1H-1-benzazepin-5-yl}essigsäure

Unter Verwendung von tert-Butyl-2-[4-(1H-Benzimidazol-2-yl)phenyl]ethylcarbamat (16) als Edukt wurden nach MPLC 190mg als amorphe weiße Festkörper erhalten; ESI-MS [M+H⁺]: 497.15.
¹H-NMR (360 MHz, d₆-DMSO) δ ppm: 8.15 (d, 2H), 7.65 (d, 2H), 7.45 (m, 2H), 7.30-7.15 (m, 6H), 4.45 und 4.15 (je m, 1H), 3.6-3.25 (m, überlagert mit H2O), 2.80 (m, 2H) , 2.70 (m, 2H) , 2.35 (m, 1H), 2.20 (m, 2H) , 1.70 (m, 1H).

### Beispiel 17:

### [1-(2-{4-[(1H-Benzimidazol-2-ylamino)methyl]piperidin-1-yl}-2-oxoethyl)-2-oxo-2,3,4,5-tetrahydro-1H-1-benzazepin-5-yl]essigsäure

Unter Verwendung von (3 N-(Piperidin-4-ylmethyl)-1H-benzimidazol-2-amin (Trifluoracetat) (17) als Edukt wurden nach MPLC 134mg als amorphe weiße Festkörper erhalten; ESI-MS [M+H⁺]: 490.29.

### Beispiel 18:

### [2-Oxo-1-(2-oxo-2-{[2-(pyridin-2-ylamino)ethyl]amino}ethyl)-2,3,4,5-tetrahydro-1H-1-benzazepin-5-yl]essigsäure

Unter Verwendung von N¹-Pyridin-2-ylethan-1,2-diamin, nachfolgende MPLC ergab 278mg als amorphe weiße Festkörper; ESI-MS [M+H⁺]: 397.25.
¹H-NMR (360 MHz, d₆-DMSO) δ ppm: 8.20 (m, 1H), 7.95 (m, 1H), 7.70 (m, 2H), 7.35-7.20 (m, 4H), 6.80 und 6.70 (jeweils m, 1H), 4.45 und 4.15 (je m, 1H), 3.70-3.0 (m, überlagert mit H2O), 2.70 (m, 2H), 2.30 (m, 1H), 2.15 (m, 2H), 1.60 (m, 1H).

### Beispiel 19:

### (2-Oxo-1-{2-oxo-2-[({4-[(pyridin-2-ylamino)methyl]thien-2-yl}methyl)amino]ethyl}-2,3,4,5-tetrahydro-1H-1-benzazepin-5-yl)essigsäure

Unter Verwendung von N-{[5-(Aminomethyl)thien-3-yl]methyl}pyridin-2-amin (Trifluoracetat) (18), nachfolgende MPLC ergab 135mg als amorphe weiße Festkörper; ESI-MS [M+H⁺]: 479.15.
¹H-NMR (360 MHz, d₆-DMSO) δ ppm: 8.65 (m, 1H), 8.0 (m, 1H), 7.45 (m, 1H), 7.35-7.15 (m, 6H), 6.95 (s, 1H), 6.60 und 6.55 (je m, 1H), 4.60-4.30 (m, 5H) , 4.15 (m 1H), 3.50 (m, 1H), 2.80-2.60 (m, 2H), 2.35 (m, 1H), 2.15 (m, 2H), 1.70 (m, 1H).

### Beispiel 20:

### [1-(2-{[3-(1H-Imidazol-2-ylamino)-3-oxopropyl]amino}-2-oxoethyl)-2-oxo-2,3,4,5-tetrahydro-1H-1-benzazepin-5-yl]essigsäure

Unter Verwendung von 3-Amino-N-(1*H*-imidazol-2-yl)propanamid (19) und nachfolgender Reinigung des Rohprodukts durch MPLC wurden 50mg als amorphe weiße Festkörper erhalten; ESI-MS [M+H⁺]: 414.25.
¹H-NMR (360 MHz, d₆-DMSO) δ ppm: 8.20 (m, 1H), 7.30-7.20 (m, 7H), 4.45 und 4.15 (jeweils m, 1H), 3.75-3.25 (m, überlagert mit H2O), 2.80-2.65 (m, 4H), 2.35 (m, 1H), 2.15 (m, 2H), 1.60 (m, 1H).

### Beispiel 21:

### [1-(2-{[4-(4,5-Dihydro-1H-imidazol-2-ylamino)benzyl]amino}-2-oxoethyl)-2-oxo-2,3,4,5-tetrahydro-1H-1-benzazepin-5-yl]essigsäure

Unter Verwendung von N-[4-(Aminomethyl)-phenyl]-4,5-dihydro-1H-imidazol-2-amin (Hydrochlorid) (20) als Edukt; nach MPLC wurden 12mg als amorphe weiße Festkörper erhalten; ESI-MS [M+H⁺] : 450.3.

### Beispiel 22:

### (1-{2-[({4-[(1H-Benzimidazol-2-ylamino)methyl]thien-2-yl}methyl)amino]-2-oxoethyl}-2-oxo-2,3,4,5-tetrahydro-1H-1-benzazepin-5-yl)essigsäure

Verwendung von N-{[5-(Aminomethyl)thien-3-yl]methyl}-1H-benzimidazol-2-amin (Hydrochlorid) (21) als Edukt; Reinigung per MPLC ergab 90mg; ESI-MS [M+H⁺] : 518.29.

### Beispiel 23:

### [2-Oxo-1-(2-oxo-2-{[5-(pyridin-2-ylamino)pentyl]amino}ethyl)-2,3,4,5-tetrahydro-1H-1-benzazepin-5-yl]essigsäure

Verwendung von N¹-Pyridin-2-ylpentan-1,5-diamin (Hydrochlorid) (22) als Edukt; nach MPLC wurden 210mg weiße Festkörper erhalten; ESI-MS [M+H⁺]: 439.15.
¹H-NMR (360 MHz, d₆-DMSO) δ ppm: 8.05 (m, 1H), 7.95 (m, 1H), 7.75 (m breit, 1H), 7.65 (m, 1H), 7.30-7.15 (m, 4H), 6.75 und 6.60 (jeweils m, 1H), 4.45 und 4.15 (je m, 1H), 3.70-3.20 (m, überlagert mit H2O), 2.70 (m, 2H), 2.35 (m, 1H), 2.15 (m, 2H), 1.60, 1.45 und 1.30 (jeweils m, 2H).

### Beispiel 24:

### N-[5-({[5-(Carboxymethyl)-2-oxo-2,3,4,5-tetrahydro-1H-1-benzazepin-1-yl]acetyl}amino)pentyl]-4,5-dihydro-1H-imidazol-2-amin (Acetat)

Verwendung von N¹-(4,5-Dihydro-1H-imidazol-2-yl)pentan-1,5-diamin (Hydrochlorid) (23) als Edukt; nach MPLC wurden 22mg erhalten; ESI-MS [M+H⁺] : 430.15.

### Beispiel 25:

### [1-(2-{[4-(1H-Imidazol-2-ylamino)benzyl]amino}-2-oxoethyl)-2-oxo-2,3,4,5-tetrahydro-1H-1-benzazepin-5-yl]essigsäure

Verwendung von N-[4-(Aminomethyl)phenyl]-1H-imidazol-2-amin (24) als Edukt; Reinigung mittels MPLC ergab 40mg; ESI-MS [M+H⁺] : 448.15.
¹H-NMR (360 MHz, d₆-DMSO) δ ppm: 8.95 (s, 1H), 8.45 (m, 1H), 7.35-7.10 (m, 8H), 6.75 (s, 2H), 4.50 (m, 1H), 4.20 (m, 3H), 3.5-3.1 (m, überlagert mit H2O), 2,70 (m, 1H), 2.35 (m 1H), 2.20 (m, 2H), 1.65 (m, 1H).

### Beispiel 26:

### [2-Oxo-1-(2-oxo-2-{[5-(1,4,5,6-tetrahydropyrimidin-2-ylamino)pentyl]amino}ethyl)-2,3,4,5-tetrahydro-1H-1-benzazepin-5-yl]essigsäure

Verwendung von N¹-(1,4,5,6-Tetrahydropyrimidin-2-yl)pentan-1,5-diamin (Hydrochlorid) (26) als Edukt; nach MPLC wurden 40mg erhalten; ESI-MS [M+H⁺] : 444.9.

### Beispiel 27:

### {2-Oxo-1-[2-oxo-2-({[4-(pyridin-2-ylamino)cyclohexyl]methyl}amino)ethyl]-2,3,4,5-tetrahydro-1H-1-benzazepin-5-yl}essigsäure

Verwendung von N- [4-(Aminomethyl)cyclohexyl]pyridin-2-amin (Hydrochlorid) (26) , Reinigung mittels Elution über eine Chromabond-C18-Kartusche ergaben 85mg; ESI-MS [M+H⁺]: 465.15.
¹H-NMR (360 MHz, d₆-DMSO) δ ppm: 8.55 (s breit, 1H), 8.05 (m, 1H), 7.90 (d, 1H), 7.85 (m, 1H), 7.35 (m, 2H), 7.25 (m, 2H), 6.95 (d, 1H), 6.80 (m, 1H), 4.40 und 4.20 (jeweils m, 1H), 3.55 (m, überlagert mit H2O), 2.95 (m, 2H), 2.70 (m, 2H), 2.35 (m, 1H), 2.15, 1.95 und 1.75 (jeweils m, 2H), 1.60 und 1.40 (je m, 1H), 1.25 (m, 2H) , 1. 05 (m, 2H).

### Beispiel 28:

### Ethyl-{2-oxo-1-[2-oxo-2-({[4-(pyridin-2-ylamino)cyclohexyl]methyl}amino)ethyl]-2,3,4,5-tetrahydro-1H-1-benzazepin-5-yl}acetat

100mg {2-Oxo-1-[2-oxo-2-({[4-(pyridin-2-ylamino)cyclohexyl]methyl}amino)ethyl]-2,3,4,5-tetrahydro-1H-1-benzazepin-5-yl}essigsäure in 10ml EtOH wurden mit 30ml 4n HCl in Dioxan versetzt und 48h bei RT gerührt. Einengen und Elution über eine Chromabond-C18-Kartusche ergaben 26mg; ; ESI-MS [M+H⁺]: 493.25.

### Beispiel 29:

### (1-{4-[4-(1H-Benzimidazol-2-ylamino)phenyl]butyl}-2-oxo-2,3,4,5-tetrahydro-1H-1-benzazepin-5-yl)essigsäure

a.) Zu einer Suspension von 0.16g NaH (60%; entölt) in 10ml DMF wurden bei 0°C eine Lösung (2-Oxo-2,3,4,5-tetrahydro-1H-1-benzazepin-5-yl)essigsäure-t.butylester (2) (0.8g; 2.91mmol) in 20ml DMF zugetropft und 1h nachgerührt. Anschließend wurde ebenfalls bei 0°C eine Spatelspitze KI zugegeben, 1g 1-(4-Bromobutyl)-4-nitrobenzol (Darstellung nach J. Med. Chem. 38, 13 (1995), 2418-2420) 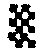 gelöst in 10ml DMF- langsam zugetropft und bei RT 2h lang gerührt. Zur Aufarbeitung wurde die Reaktionsmischung vorsichtig mit Wasser versetzt, mit CH₂Cl₂ 2 verdünnt und mehrmals mit ges. NaCl-Lösung extrahiert. Nach Trocknen und Eindampfen wurde das erhaltene Rohprodukt durch Chromatographie an Kieselgel gereinigt (CH₂Cl₂/CH₃OH 2-10%); 0.42g gelbes Öl, ESI-MS [M+H⁺-Boc]: 397.15.
b.) Hydrierung der Nitrogruppe in 75ml CH₃OH mit 100mg 10% Pd auf Aktivkohle unter Standardbedingungen ergab 0.36mg eines hellen Öls; ESI-MS [M+H⁺]: 423.25.
c.) Der Aufbau des entsprechende Aminobenzimidazols erfolgte analog zur Darstellung von 17 durch Umsetzung mit Thiocarbonyldiimidazol, Imidazol und anschließend Phenylendiamin. Chromatographie an Kieselgel (CH₂Cl₂/ CH₃OH 2-4%) ergab 220mg eines hellen Schaums; ESI-MS [M+H⁺]: 539.25.
d.) 200mg tert-Butyl-(1-{4-[4-(1H-benzimidazol-2-ylamino)phenyl]butyl}-2-oxo-2,3,4,5-tetrahydro-1H-1-benzazepin-5-yl)acetat wurden bei RT mit 10ml TFA behandelt. Einengen und Lyophilisieren des erhaltenen Öls ergab 213mg eines Feststoffs; ESI-MS [M+H⁺]: 483.25.

### Beispiel 30:

### 2-(4-{[{[5-(Carboxymethyl)-2-oxo-2,3,4,5-tetrahydro-1H-1-benzazepin-1-yl]acetyl}(methyl)amino]methyl}anilino)-1H-benzimidazol (Trifluoroacetat)

a.) Standardkupplung von [5-(2-t.Butoxy-2-oxoethyl)-2-oxo-2,3,4,5-tetrahydro-1H-benzazepin-1-yl]essigsäure (3) (2.2g; 6.6mmol) mit 1.32g N-Methyl(4-nitrophenyl)-methanamin (J. Am. Chem. Soc. 65 (1943), 1984-1990) in 40ml CH₂Cl₂ unter Verwendung von HATU als Kupplungsreagenz und Ethyldiisopropylamin als Base. Nachfolgende Chromatographie des Rohprodukts an Kieselgel (CH₂Cl₂/CH₃OH 2-4%) ergab 2.26g eines leicht gelben Öls; ; ESI-MS [M+H⁺] : 482.03.
   ¹H-NMR (360 MHz, d₆-DMSO) δ ppm (Rotamere) : 8.25/8.20 (d, 2H), 7.60/7.50 (d, 2H), 7.35-7.20 (m, 4H), 4.85-4.5 (m, 4H), 3.05/2.85 (s, 3H), 2.70 (m, 1H), 2.30 (m, 1H), 2.15 (m, 2H) , 1. 65 (m, 1H), 1.30 (s, 9H).
b.) Zu 1.26g Nitroverbindung a und 15mg FeCl₃ x 6H₂O in 20ml CH₃OH wurden bei 60°C 5ml Hydrazinhydrat zugesetzt und 1h lang bei 60°C gerührt. Die Mischung wurde über Celite filtriert und das Filtrat eingedampft. 1.17g; ESI-MS [M+H⁺]: 452.17.
c.) Der Aufbau des entsprechende Aminobenzimidazols erfolgte analog zur Darstellung von 17 durch Umsetzung mit Thiocarbonyldiimidazol, Imidazol und anschließend Phenylendiamin. Chromatographie an Kieselgel (CH₂Cl₂/CH₃OH 4-7.5%) ergab 0.9g eines leicht beigen Schaums; ESI-MS [M+H⁺]: 568.25.
d.) Spaltung des t-Butylesters mit 50ml TFA und anschließende Reinigung durch Chromatographie an Kieselgel (CH₂Cl₂/CH₃OH 8-10%) ergab 0.88g; ESI-MS [M+H⁺]: 512.25.
   ¹H-NMR (360 MHz, d₆-DMSO) δ ppm (Rotamere): 11.05 (breit, 1H), 7.60-7.20 (m, 12H), 4.85 (m, 1H), 4.75-4.45 (m, 3H), 3.65 (m, 1H), 3.20 (s, 3H), 2.70 (m, 2H), 2.35 (m, 1H), 2.20 (m, 2H), 1.65 (m, 1H).

### Beispiel 31:

### tert-Butyl-[1-(2-{[4-(1H-benzimidazol-2-ylamino)benzyl]amino}-2-oxoethyl)-2,3,4,5-tetrahydro-1H-1-benzazepin-5-yl]acetat

Kupplung von [5-(2-tert-Butoxy-2-oxoethyl)-2,3,4,5-tetrahydro-1H-1-benzazepin-1-yl]essigsäure (28) mit N-[4-(Aminomethyl)phenyl]-1H-benzimidazol-2-amin (Hydrochlorid) (4) analog zu I und Reinigung durch Chromatographie an Kieselgel (CH₂Cl₂/CH₃OH 2-3%) ergab 200mg eines beigen Schaums; ESI-MS [M+H⁺] : 540.25.

### Beispiel 32:

### [1-(2-{[4-(1H-Benzimidazol-2-ylamino)benzyl]amino}-2-oxoethyl)-2,3,4,5-tetrahydro-1H-1-benzazepin-5-yl]essigsäure (Trifluoracetat)

TFA-Spaltung von tert-Butyl-[1-(2-{[4-(1H-benzimidazol-2-ylamino)benzyl]amino}-2-oxoethyl)-2,3,4,5-tetrahydro-1H-1-benzazepin-5-yl]acetat ergab 240mg; ESI-MS [M+H⁺]: 484.36.
¹H-NMR (360 MHz, d₆-DMSO) δ ppm (Rotamere): 11.10 (s, 1H), 8.30 (t, 1H), 7.5-7.25 (m, 8H), 7.15 (m, 2H), 6.85 (m, 2H), 4.40 (m, 2H), 3.75-3.55 (m, überlagert mit H2O), 3.5 (m, 1H), 3.0 (m, 1H), 2.70 (m, 2H), 1.70 (m, 2H), 1.45 (m, 1H).

### Beispiel 33:

### tert-Butyl-{1-[2-({[4-(1H-benzimidazol-2-ylamino)cyclohexyl]methyl}amino)-2-oxoethyl]-2,3,4,5-tetrahydro-1H-1-benzazepin-5-yl}acetat

Herstellung analog zu Beispiel 31 ausgehend von [5- (2-tert-Butoxy-2-oxoethyl)-2,3,4,5-tetrahydro-1H-1-benzazepin-1-yl]essigsäure (28) und trans-N-{[4-(Aminomethyl)cyclohexyl)}-1H-benzimidazol-2-amin (Dihydrochlorid) (11). 410mg weißer Schaum; ESI-MS [M+H⁺]: 546.35.

### Beispiel 34:

### {1-[2-({[4-(1H-Benzimidazol-2-ylamino)cyclohexyl]methyl}amino)-2-oxoethyl]-2,3,4,5-tetrahydro-1H-1-benzazepin-5-yl}essigsäure (Trifluoracetat)

Analog zu Beispiel 32; 420mg weiße Festkörper; ESI-MS [M+H⁺]: 490.47.
¹H-NMR (360 MHz, d₆-DMSO) δ ppm: 9.0 (d, 1H), 7.65 (m, 1H), 7.40 und 7.30 (jeweils m, 2H), 7.15 und 6.85 (je m, 2H), 3.80 (m, 2H), 3.50 (m, 1H), 3.0 (m, 4H), 2.75 (m, 2H), 2.0 (m, 2H), 1.75-1.50 (m, 5H), 1.50-1.20 (m, 5H), 0.95 (m, 2H) .

### Beispiel 35:

### tert-Butyl-[1-(2-{[5-(1H-benzimidazol-2-ylamino)pentyl]amino}-2-oxoethyl)-2,3,4,5-tetrahydro-1H-1-benzazepin-5-yl] acetat

Herstellung analog zu Beispiel 31 ausgehend von [5-(2-tert-Butoxy-2-oxoethyl)-2,3,4,5-tetrahydro-1H-1-benzazepin-1-yl]essigsäure (28) und N¹-(1H-Benzimidazol-2-yl)pentan-1,5-diamin (Hydrochlorid) (8). 300mg Schaum; ESI-MS [M+H⁺] : 520.39.

### Beispiel 36:

### [1-(2-{[5-(1H-Benzimidazol-2-ylamino)pentyl]amino}-2-oxoethyl)-2,3,4,5-tetrahydro-1H-1-benzazepin-5-yl]essigsäure

Spaltung des t-Butylesters aus Beispiel 35 mit 15ml 4nHCl in Dioxan ergab 300mg Feststoff; ESI-MS [M+H⁺]: 464.25.
¹H-NMR (360 MHz, d₆-DMSO) δ ppm: 9.10 (m, 1H), 7.70 (m, 1H), 7.35, 7.25, 7.10 und 6.85 (jeweils m, 2H), 4.75 (m 2H), 3.30, 3.15, 2.95, 2.70 (jeweils m, 2H), 1.65 (m, 5H), 1.45 und 1.30 (jeweils m, 2H).

### Beispiel 37

### tert-Butyl-{1-[2-({[4-(1H-imidazol-2-ylamino)cyclohexyl]methyl}amino)-2-oxoethyl]-2-oxo-2,3,4,5-tetrahydro-1H-1-benzazepin-5-yl}acetat

Herstellung analog zu Beispiel I unter Verwendung von N- [4-(Aminomethyl)cyclohexyl]-1H-imidazol-2-amin (Hydrobromid) (29) als Edukt; Chromatographie an Kieselgel (CH₂Cl₂/CH₃OH 4-15%) ergab 530mg festen Schaum; ESI-MS [M+H⁺]: 510.35.

### Beispiel 38:

### {1-[2-({[4-(1H-Imidazol-2-ylamino)cyclohexyl]methyl}amino)-2-oxoethyl]-2-oxo-2,3,4,5-tetrahydro-1H-1-benzazepin-5-yl}essigsäure (Acetat)

Spaltung des t-Butylesters aus Beispiel 37 mit TFA und anschließende Reinigung mittels Chromatographie an Kieselgel (CH₂Cl₂/CH₃OH 10-15% + 2% Eisessig) ergab 570mg; ESI-MS [M+H⁺]:454.25.
¹H-NMR (360 MHz, d₆-DMSO) δ ppm: 8.0 (breit, 1H), 7.30 und 7.25 (je m, 2H), 6.80 (breit, 1H), 6.70 (s, 2H), 4.40 und 4.20 (je breit, 1H), 4.75-4.25 (überlagert durch H2O), 3.0 (m, 2H), 2.65 (m, 2H), 2.35 (m, 1H), 2.15 (m, 2H), 1.95 (m, 1H), 1.75 (m, 2H), 1.60 und 1.35 (je m, 1H), 1.20 (m, 4H), 0.95 (m, 2H).

### Beispiel 39:

### tert-Butyl-(2-oxo-1-{[4-({4-[(pyridin-2-ylamino)methyl]piperidin-1-yl}carbonyl)-1,3-thiazol-2-yl]methyl}-2,3,4,5-tetrahydro-1H-1-benzazepin-5-yl)acetat

Standardkupplung 2-{[5-(2-tert-Butoxy-2-oxoethyl)-2-oxo)-2,3,4,5-tetrahydro-1H-1-benzazepin-1-yl]methyl}-1,3-thiazol-4-carbonsäure (32) (0.5g; 1.2mmol) mit N-(Piperidin-4-ylmethyl)pyridin-2-amin (Hydrochlorid; zugänglich aus 29) in 15ml CH₂Cl₂ unter Verwendung von HATU als Kupplungsreagenz und Ethyldiisopropylamin als Base. Nachfolgende Chromatographie des Rohprodukts an Kieselgel (CH₂Cl₂/CH₃OH 3-20%) ergab 0.44g eines leicht gelben Öls; ESI-MS [M+H⁺] : 590.35.

### Beispiel 40:

### (2-Oxo-1-{[4-({4-[(pyridin-2-ylamino)methyl]piperidin-1-yl}carbonyl)-1,3-thiazol-2-yl]methyl}-2,3,4,5-tetrahydro-1H-1-benzazepin-5-yl)essigsäure

Spaltung des t-Butylesters aus Beispiel 31 mit TFA ergab 385mg; ESI-MS [M+H⁺]: 534.25.
¹H-NMR (360 MHz, d₆-DMSO) δ ppm: 8.05 (s, 1H), 7.90 (m, 2H), 7.55 (m, 1H), 7.35 (m, 1H), 7.25 (m, 2H), 7.05 und 6.80 (je m, 1H), 5.40-5.20 (m, 2H), 4.20 und 4.0 (je m, 1H), 3.25 (m, 2H), 2.95 (m, 1H), 2.80-2.60 (m, 3H) , 2.30 (m, 1H), 2.25-2.05 (m, 2H) , 1.80 und 1.65 (je m, 2H), 1.20-1.10 (m, 3H).

### Beispiel 41:

### tert-Butyl-[1-({4-[({[4-(1H-benzimidazol-2-ylamino)cyclohexyl]methyl}amino)carbonyl]-1,3-thiazol-2-yl}methyl)-2-oxo-2,3,4,5-tetrahydro-1H-1-benzazepin-5-yl]acetat

Analog zu Beispiel 29 unter Verwendung von trans-N-{[4-(Aminomethyl)cyclohexyl]}-1H-benzimidazol-2-amin (Dihydrochlorid) (11) als Edukt; Chromatographie des Rohprodukts an Kieselgel (CH₂Cl₂/CH₃OH 5-8%) ergab 350mg eines hellgelben Öls; ESI-MS [M+H⁺] : 643.45.

### Beispiel 42:

### [1-({4-[({[4-(1H-Benzimidazol-2-ylamino)cyclohexyl]methyl}amino)carbonyl]-1,3-thiazol-2-yl}methyl)-2-oxo-2,3,4,5-tetrahydro-1H-1-benzazepin-5-yl] essigsäure

Spaltung des t-Butylesters aus Beispiel 31 und Reinigung durch Elution über Chromabond-C18 (Acetonitril/H₂O 10-80% + 0.1% Eisessig) ergab 300mg; ESI-MS [M+H⁺]: 587.35.
¹H-NMR (360 MHz, d₆-DMSO) δ ppm: 8.40 (breit, 1H), 8.20 (m, 1H), 8.20 (s, 1H), 7.35-7.20 (m,5H), 7.15 (m, 2H), 5.40 und 5.25 (jeweils d, 1H), 3.15 (m, überlagert mit H2O), 2.80-2.60 (m, 2H), 2.35 (m, 1H), 2.20 (m, 2H), 2.05 und 1.80 (je m, 2H), 1.65, 1.30 und 1.15 (je m, 2H).

### Beispiel 43:

### tert-Butyl-(1-{[4-({4-[(1H-benzimidazol-2-ylamino)methyl]piperidin-1-yl}carbonyl)-1,3-thiazol-2-yl]methyl}-2-oxo-2,3,4,5-tetrahydro-1H-1-benzazepin-5-yl)acetat

Analog zu Beispiel 29 unter Verwendung von N-(Piperidin-4-ylmethyl)-1H-benzimidazol-2-amin (Trifluoracetat) (17) und nachfolgender Reinigung wurden 430mg erhalten; ESI-MS [M+H⁺] : 629.45.

### Beispiel 44:

### (1-{[4-({4-[(1H-Benzimidazol-2-ylamino)methyl]piperidin-1-yl}carbonyl)-1,3-thiazol-2-yl]methyl}-2-oxo-2,3,4,5-tetrahydro-1H-1-benzazepin-5-yl}essigsäure

Spaltung des t-Butylesters aus Beispiel 43 und Reinigung durch Elution über Chromabond-C18 (Acetonitril/H₂O 10-80% + 0.1% Eisessig) ergab 340mg; ESI-MS [M+H⁺]: 573.35.
¹H-NMR (360 MHz, d₆-DMSO) δ ppm: 9.20 (m, 1H), 7.95 (s, 1H), 7.45 (m, 1H), 7.40 (m, 2H), 7.30-7.15 (m, 5H), 5.35-5.10 (m, 2H), 4.45 (m, 1H), 4.0-3.25 (m, überlagert mit H20), 2.95 (m, 1H), 2.8-2.60 (m, 2H), 2.30 und 2.20 (je m, 1H), 2.0-1.75 (m, 3H), 1.70-1.50 (m, 2H), 1.20 (m, 3H).

### Beispiel 45:

### tert-Butyl-{2-oxo-1-[(4-{[({4-[(pyridin-2-ylamino)methyl]thien-2-yl}methyl)amino]carbonyl}-1,3-thiazol-2-yl)methyl]-2,3,4,5-tetrahydro-1H-1-benzazepin-5-yl}acetat

Analog zu Beispiel 39 unter Verwendung von N-{[5-(Aminomethyl)thien-3-yl]methyl}pyridin-2-amin (Trifluoracetat) (18) und Reinigung wurden 330mg erhalten; ESI-MS [M+H⁺] : 618.25.

### Beispiel 46:

### {2-Oxo-1-[(4-{[({4-[(pyridin-2-ylamino)methyl]thien-2-yl}methyl)amino]carbonyl}-1,3-thiazol-2-yl)methyl]-2,3,4,5-tetrahydro-1H-1-benzazepin-5-yl}essigsäure

Spaltung des t-Butylesters aus Beispiel 45 und Reinigung mittels MPLC ergab 150mg; ; ESI-MS [M+H⁺]: 562.25.
¹H-NMR (360 MHz, d₆-DMSO) δ ppm: 8.80 (m, 1H), 8.20 (m, 1H), 8.0 (d, 1H), 7.50 (d, 1H), 7.30-7.20 (m, 4H), 7.10 und 6.95 (jeweils s, 1H), 6.85 (m, 1H), 6.50 (m, 2H), 5.40 und 5.20 (je d, 1H), 4.55 und 4.35 (je m, 2H), 2.80-2.55 (m, 2H), 2.30 (m, 1H), 2.20 (m, 2H) , 1.65 (m, 1H).

### Beispiel 47:

### tert-Butyl-[1-({4-[4-(1H-benzimidazol-2-ylamino)phenyl]-1,3-thiazol-2-yl}methyl)-2-oxo-2,3,4,5-tetrahydro-1H-1-benzazepin-5-yl]acetat

a.) Eine Mischung aus 1.5g tert-Butyl-[1-(2-amino-2-thioxoethyl)-2-oxo-2,3,4,5-tetrahydro-1H-1-benzazepin-5-yl]acetat, 1.36g 2-Brom-4-nitroacetophenon und 0.65g KHCO₃ in 30ml Dioxan wurde 12h bei RT gerührt. Aufarbeitung analog zu Baustein 30c und Verrühren des Rohprodukts mit n-Pentan ergab 2.1g brauen Festkörper; ESI-MS [M+H⁺]: 494.15.
   ¹H-NMR (360 MHz, d₆-DMSO) δ ppm: 8.40 (s, 1H), 8.30 und 8.20 (je m, 2H), 7.60 und 7.35 (je m, 1H), 7.25 (m, 2H), 5.50 und 5.30 (je d, 1H), 2.70 (m, 2H), 2.30 (m, 1H), 2.20 (m, 3H), 1.65 (m, 1H), 1.25 (s, 9H).
b.) Reduktion der Nitrogruppe analog zu Beispiel 30b (1.6g; ESI-MS [M+H⁺]: 464.15) und Aufbau des Aminobenzimidazols wie bereits beschrieben ergab nach Chromatographie an Kieselgel (CH₂Cl₂/CH₃OH 2-4%) 0.58g eines leicht gelben Schaums; ESI-MS [M+H⁺]: 614.35.

### Beispiel 48:

### [1-({4-[4-(1H-Benzimidazol-2-ylamino)phenyl]-1,3-thiazol-2-yl}methyl)-2-oxo-2,3,4,5-tetrahydro-1H-1-benzazepin-5-yl]essigsäure

Spaltung des t-Butylesters aus Beispiel XXXXVII und Reinigung mittels MPLC ergab 40mg; ESI-MS [M+H⁺]: 524.25.
¹H-NMR (360 MHz, d₆-DMSO) δ ppm: 11.95 und 9.60 (je breit, 1H), 7.85-7.75 (m, 4H), 7.65 (m, 1H), 7.40-7.25 (m, 5H), 7.0 (m, 2H), 5.40 und 5.30 (je d, 1H), 3.50 (m, 1H), 2.75 (m, 1H), 2.45 (m, 2H), 2.20 (m, 2H), 1.70 (m, 1H).

### Beispiel 49:

### (1-{2-[(4-{[Amino(imino)methyl]amino}benzyl)amino]-2-oxoethyl}-2-oxo-2,3,4,5-tetrahydro-1H-1-benzazepin-5-yl)essigsäure-tert-butylester

Ethyldiisopropylamin (0.42 mmol, 54.50 mg) und HATU (0.50 mmol, 190.11 mg) wurden bei 0°C zu einer Lösung von [5-(2-t.Butoxy-2-oxoethyl)-2-oxo-2,3,4,5-tetrahydro-1H-benzazepin-1-yl]essigsäure (3) (0.42mmol, 140.59mg) in CH₂Cl₂ (10mL) gegeben, die Mischung dann 1h bei 0°C nachgerührt und [4-(Aminomethyl)phenyl]guanidin (Bishydrochlorid) (46) (0.42mmol, 100mg), Ethyldiisopropylamin (0.63mmol, 81.76mg) zugesetzt. Die Mischung wurde 1h bei 0°C und über Nacht bei RT nachgerührt. Nach Einengen wurde der Rückstand in CH₂Cl₂/Wasser aufgenommen, mit wäßr. NaHCO₃-, 5%-Citronensäure- und zuletzt mit wässr. gesättigter NaCl-Lösung gewaschen. Einengen und Chromatographie an Kieselgel (CH₂Cl₂/ CH₃OH 0-100%) ergaben 6.0mg Zielprodukt; ESI-MS [M+H⁺]: 426.1, 425.1.

### Beispiel 50:

### {4-[({[5-(Carboxymethyl)-2-oxo-2,3,4,5-tetrahydro-1H-1-benzazepin-1-yl]acetyl}amino)methyl]anilino}(imino)methanamin (Trifluoracetat)

(1-{2-[(4-{[Amino(imino)methyl]amino}benzyl)amino]-2-oxoethyl}-2-oxo-2,3,4,5-tetrahydro-1H-1-benzazepin-5-yl)essigsäure-tert-butylester (Beispiel 49, 0.01mmol, 6mg) wurde in 5ml CH₂Cl₂ gelöst, bei 0°C TFA (1.25 mmol, 142.53 mg) zugesetzt und über Nacht bei Raumtemperatur gerührt. Nach Einengen wurde der Rückstand in CHCl₃/Wasser aufgenommen und die wässr. Phase mit CHCl₃ gewaschen; Einengen der wässr. Phase ergibt 4.8mg Zielprodukt; ESI-MS [M+H⁺] = 425.1.

### Beispiel 51:

### [1-(2-{[(6-Amino-2-pyridinyl)methyl]amino}-2-oxoethyl)-2-oxo-2,3,4,5-tetrahydro-1H-1-benzazepin-5-yl]essigsäure-tert-butylester

Analog zu Beispiel 49 unter Verwendung von 2-Ammonio-6-(ammoniomethyl)pyridinium trichlorid (33) (0.30mmol, 69.76mg) und anschließender Reinigung (Chromatographie; CH₂Cl₂/MeOH 0-100%) ergaben 120mg Zielprodukt; ESI-MS: [M+H⁺] = 439.28, 383.36.

### Beispiel 52:

### [1-(2-{[(6-Amino-2-pyridinyl)methyl]amino}-2-oxoethyl)-2-oxo-2,3,4,5-tetrahydro-1H-1-benzazepin-5-yl]essigsäure (Hydrochlorid)

Spaltung des t-Butylesters, Chromatographie des Rohprodukts (CHCl₃/MeOH 0-5%) und Umsalzen mit HCl in Diethylether ergaben 15.0mg; Zielprodukt als Hydrochlorid; ESI-MS [M+H⁺] = 383.1.

### Beispiel 53:

### [1-(2-{[4-(1H-Benzimidazol-2-ylamino)benzyl]amino}ethyl)-2-oxo-2,3,4,5-tetrahydro-1H-1-benzazepin-5-yl]essigsäure-tert-butylester

N- [4-(Aminomethyl)phenyl]-1H-benzimidazol-2-amin (Hydrochlorid) (4) (0.63mmol, 196.10mg) in Methanol (40 mL) wurden mit 2 Tropfen einer Lösung von HCL in Diethylether versetzt und 5min. bei RT gerührt. [2-Oxo-1-(2-oxoethyl)-2,3,4,5-tetrahydro-1H-1-benzazepin-5-yl]essigsäure-tert-butylester (37, 0.63mmol, 0.20g) wurde zugesetzt, nach 10min. NaBH₃CN (3mmol, 188.5mg) portionsweise zugegeben und die Mischung 17h bei RT nachgerührt. Nach Einengen wurde der Rückstand in Ethylacetat aufgenommen und mit NaHCO₃- (pH 8-9), ges. NaCl- (1x) und einer 10% FeSO4-Lösung (2x) gewaschen. Chromatographie (CHCl₃/MeOH 0-5%) ergab 84mg des Zielprodukts; ESI-MS [M+H⁺] = 540.24, 270.75.

### Beispiel 54:

### [1-(2-{[4-(1H-Benzimidazol-2-ylamino)benzyl]amino}ethyl)-2-oxo-2,3,4,5-tetrahydro-1H-1-benzazepin-5-yl]essigsäure

Spaltung des t-Butylesters und Chromatographie (CHCl₃/MeOH 0-100%) ergaben 4mg des Zielprodukts; ESI-MS [M+H⁺] = 484.1.

### Beispiel 55:

### [1-(2-{[(4-{[(Benzylamino)carbonyl]amino}cyclohexyl)methyl]amino}-2-oxoethyl) -2-oxo-2,3,4,5-tetrahydro-1H-1-benzazepin-5-yl]essigsäure-tert-butylester

Analog zu Beispiel 49 unter Verwendung von N-[4-(Aminomethyl)cyclohexyl]-N'-benzylharnstoff (34) (0.50mmol, 130mg) ergaben 320mg; ESI-MS [M+H⁺] = 577.11.

### Beispiel 56:

### [1-(2-{[(4-{[(Benzylamino)carbonyl]amino}cyclohexyl)methyl]amino}-2-oxoethyl)-2-oxo-2,3,4,5-tetrahydro-1H-1-benzazepin-5-yl]essigsäure

Analoge Spaltung des t-Butylesters ergab ein Rohprodukt, das nach Eindampfen zwischen Ethylacetat/Wasser verteilt wurde. Die wässr. Phase wurde dann mit 0.1n NaOH auf pH 10 gestellt und mit Ethylacetat extrahiert. Anschließend wurde mit 1nN HCl auf pH4 gestellt, mit CH₂Cl₂ extrahiert, gewaschen und eingeengt; 80mg; ESI-MS [M+H⁺] = 521.3.

### Beispiel 57:

### [1-(2-{[5-(1H-Benzimidazol-2-ylamino)pentyl]amino}ethyl)-2-oxo-2,3,4,5-tetrahydro-1H-1-benzazepin-5-yl]essigsäure-tert-butylester

Herstellung analog zu Beispiel 53 unter Verwendung von N¹-(1H-Benzimidazol-2-yl)pentan-1,5-diamin (Hydrochlorid) (8) (0.63mmol, 183.47mg) und [2-Oxo-1-(2-oxoethyl)-2,3,4,5-tetrahydro-1*H*-1-benzazepin-5-yl]essigsäure-tert-butylester (37, 0.63mmol, 0.20g) ergab 50.0mg Zielprodukt; ESI-MS [M+H⁺] = 520.41, 260.79.

### Beispiel 58:

### [1-(2-{[5-(1H-Benzimidazol-2-ylamino)pentyl]amino}ethyl)-2-oxo-2,3,4,5-tetrahydro-1H-1-benzazepin-5-yl]essigsäure (Trifluoracetat)

[1-(2-{[5-(1H-Benzimidazol-2-ylamino)pentyl]amino}ethyl)-2-oxo-2,3,4,5-tetrahydro-1H-1-benzazepin-5-yl]essigsäure-tert-butylester (Beispiel 57, 0.04mmol, 20.00 mg) wurde in 5ml CH₂Cl₂ gelöst, bei 0°C TFA (65.34 mmol, 7.45 g) zugesetzt und über Nacht bei Raumtemperatur gerührt. Eindampfen der Reaktionsmischung, Chromatographie (CHCl₃/MeOH 0-100%) ergaben 10mg Zielprodukt; ESI-MS [M+H⁺] = 464.25.

### Beispiel 59:

### {2-Oxo-1-[2-({[4-({4-[(1Z)-1-propenyl]-5-vinyl-1H-imidazol-2-yl}amino)cyclohexyl]methyl}amino)ethyl]-2,3,4,5-tetrahydro-1H-1-benzazepin-5-yl}essigsäure-tert-butylester

Herstellung analog zu Beispiel 53 unter Verwendung von Ethyldiisopropylamin und EDCl*HCl ausgehend von trans-N-{[4-(Aminomethyl)cyclohexyl]-1H-benzimidazol-2-amin (Dihydrochlorid) (11) (0.63 mmol, 0.20g) ergab 84mg; ESI-MS [M+H⁺] = 546.32, 273.79.

### Beispiel 60:

### {1-[2-({[4-(1H-Benzimidazol-2-ylamino)cyclohexyl]methyl}amino)ethyl]-2-oxo-2,3,4,5-tetrahydro-1H-1-benzazepin-5-yl}essigsäure (Trifluoracetat)

{2-Oxo-1-[2-({[4-({4-[(1Z)-1-propenyl]-5-vinyl-1H-imidazol-2-yl}amino)cyclohexyl]methyl}amino)ethyl]-2,3,4,5-tetrahydro-1H-1-benzazepin-5-yl}essigsäure-tert-butylester (Beispiel 59, 0.01mmol, 8mg) wurde in 5ml CH₂Cl₂ gelöst, bei 0°C TFA (65.34mmol, 7.45g) zugesetzt und über Nacht bei Raumtemperatur gerührt. Die Reaktionsmischung wurde eingeengt, die wässr. Phase mit einer 3:1 Mischung CHCl₃/EtOH gewaschen. Einengen der wässr. Phase ergab 6.0 mg Zielprodukt; ESI-MS [M+H⁺] = 490.25.

### Beispiel 61:

### [1-(2-{[4-(1H-Benzimidazol-2-ylamino)benzyl]amino}-2-oxoethyl)-7-chloro-2-oxo-2,3,4,5-tetrahydro-1H-1-benzazepin-5-yl]essigsäure (Trifluoracetat)

Kupplung analog zu Beispiel 49 ausgehend von N-[4-(Aminomethyl)phenyl]-1Hbenzimidazol-2-amin (Hydrochlorid) (4) (1.70mmol, 467.08mg) und [5-(2-tert-Butoxy-2-oxoethyl)-7-chloro-2-oxo-2,3,4,5-tetrahydro-1H-1-benzazepin-1-yl]essigsäure (45) (1.63mmol, 600mg). Das erhaltene Rohprodukt wurde bei 0°C mit TFA (1.73mmol, 197.72mg) versetzt und über Nacht bei Raumtemperatur gerührt. Nach Einengen wurde der Rückstand zwischen Ethylacetat und Wasser verteilt, die wässr. Phase mit 2n NaOH auf pH10 gebracht und mit Ethylacetat extrahiert; Reinigung mittels MPLC ergab 90mg Zielprodukt als TFA-Salz; ESI-MS [M+K⁺] = 570.2, 534.2, 532.2, 266.5, 101.1.

### Beispiel 62

### [1-(2-{[5-(1H-Benzimidazol-2-ylamino)pentyl]amino}-2-oxoethyl)-7-chloro-2-oxo-2,3,4,5-tetrahydro-1H-1-benzazepin-5-yl]essigsäure (Trifluoracetat)

Kupplung analog zu Beispiel 49 ausgehend von N¹-(1H-Benzimidazol-2-yl)pentan-1,5-diamin (Hydrochlorid) (8) (1.7mmol, 433.10mg) und [5-(2-tert-Butoxy-2-oxoethyl)-7-chloro-2-oxo-2,3,4,5-tetrahydro-1H-1-benzazepin-1-yl]essigsäure (45) (1.63 mmol, 600.00 mg). Behandlung des Rohprodukts mit TFA analog zu Beispiel 61 ergab 48mg als TFA-Salz; ESI-MS: 514.2, 512.2, 101.2.

### Beispiel 63

### [1-(2-{[4-(1H-Benzimidazol-2-ylamino)butyl]amino}-2-oxoethyl)-7-chloro-2-oxo-2,3,4,5-tetrahydro-1H-1-benzazepin-5-yl]essigsäure

Kupplung analog zu Beispiel 49 ausgehend von N¹-(1H-Benzimidazol-2-yl)butan-1,4-diamin (Trifluoroacetat) (9) (1.7mmol, 541.1mg) und [5-(2-tert-Butoxy-2-oxoethyl)-7-chloro-2-oxo-2,3,4,5-tetrahydro-1H-1-benzazepin-1-yl]essigsäure (45) (1.63mmol, 600mg). Behandlung des Rohprodukts mit TFA analog zu Beispiel 61 ergab 15mg als TFA-Salz; ESI-MS: 536.2, 500.1, 498.2, 105.1, 101.2.

### Beispiel 64

### [7-Chloro-1-(2-{[5-(4,5-dihydro-1H-imidazol-2-ylamino)pentyl]amino}-2-oxoethyl)-2-oxo-2,3,4,5-tetrahydro-1H-1-benzazepin-5-yl]essigsäure (Trifluoracetat)

Kupplung analog zu Beispiel 49 ausgehend von N¹-(4,5-Dihydro-1H-imidazol-2-yl)pentan-1,5-diamin (Hydrochlorid) (23) (1.70mmol, 351.43mg) und [5-(2-tert-Butoxy-2-oxoethyl)-7-chloro-2-oxo-2,3,4,5-tetrahydro-1H-1-benzazepin-1-yl]essigsäure (45) (1.63mmol, 600mg). Behandlung des Rohprodukts mit TFA analog zu Beispiel 61 ergab 85mg als TFA-Salz; ESI-MS: 464.15.

### Beispiel 65

### tert-Butyl 7-[4-({[5-(2-tert-butoxy-2-oxoethyl)-2-oxo-2,3,4,5-tetrahydro-1H-1-benzazepin-1-yl]acetyl}amino)butyl]-3,4-dihydro[1,8]naphthyridine-1(2H)-carboxylat

Ethyldiisopropylamin (2.08 mmol, 268.41 mg) und EDCI*HCl (0.78 mmol, 150.44 mg) wurden bei 0°C zu einer Lösung von [5-(2-t.Butoxy-2-oxoethyl)-2-oxo-2,3,4,5-tetrahydro-1H-benzazepin-1-yl]essigsäure (3) (0.46mmol, 0.15g) in CH₃CN (45ml) zugegeben. Nach 1h bei 0°C wurde 7-(4-Aminobutyl)-1,2,3,4-tetrahydro[1,8]naphthyridin (Bistrifluoroacetat) (35) (0.46mmol, 0.2g) zugesetzt, 1h bei 0°C und über Nacht bei RT nachgerührt. Da durch Chromatographie keine Reinigung zu erzielen war, wurde das erhaltene nach Standardmethoden in das entsprechende Boc-Derivat überführt. Chromatographie (Heptan/CH₂Cl₂ 0-100%, CH₂Cl₂/MeOH 0-100%) ergab 15mg Zielprodukt (ca. 95% Reinheit); ESI-MS [M+Na⁺]: 643.5, 622.5, [M+H⁺] = 621.5.

### Beispiel 66

### 7-[4-({[5-(Carboxymethyl)-2-oxo-2,3,4,5-tetrahydro-1H-1-benzazepin-1-yl]acetyl}amino)butyl]-1,2,3,4-tetrahydro[1,8]naphthyridin (Trifluoroacetat)

tert-Butyl 7-[4-({[5-(2-tert-butoxy-2-oxoethyl)-2-oxo-2,3,4,5-tetrahydro-1H-1-benzazepin-1-yl]acetyl}amino)butyl]-3,4-dihydro[1,8]naphthyridine-1(2H)-carboxylat (Beispiel 65; 0.02mmol, 15mg) wurde in 2ml CH₂Cl₂ gelöst, bei 0°C TFA (0.53mmol, 60.7mg) zugesetzt und über Nacht bei Raumtemperatur gerührt. Übliche Aufarbeitung ergab 9.3mg des Zielprodukts als TFA-Salz; ESI-MS: 466.2, [M+H⁺] = 465.2, 233.3.

### Beispiel 67

### N-{4-[({[5-(Carboxymethyl)-7-chloro-2-oxo-2,3,4,5-tetrahydro-1H-1-benzazepin-1-yl]acetyl}amino)methyl]cyclohexyl}-1H-benzimidazol (Acetat)

Kupplung analog zu Beispiel 49 ausgehend von trans-N-{[4-(Aminomethyl)cyclohexyl]-1H-benzimidazol-2-amin (Dihydrochlorid) (11) (2.99mmol, 948.78mg) und [5-(2-tert-Butoxy-2-oxoethyl)-7-chloro-2-oxo-2,3,4,5-tetrahydro-1H-1-benzazepin-1-yl]essigsäure (45) (2.72 mmol, 1.00 g) ergab 760mg Rohprodukt. Behandlung des Rohprodukts mit TFA analog zu Beispiel 61 und Reinigung des Rohprodukts mittels MPLC ergab 400mg; ESI-MS: [M+H⁺] = 540.3, 538.25, 269.6, 101.1.

### Beispiel 68:

### Ethyl-{1-[2-({[4-(1H-benzimidazol-2-ylamino)cyclohexyl]methyl}amino)-2-oxoethyl]-2,3,4,5-tetrahydro-1H-1-benzazepin-5-yl}acetat

300mg {1-[2-({[4-(1H-Benzimidazol-2-ylamino)cyclohexyl]methyl}amino)-2-oxoethyl]-2,3,4,5-tetrahydro-1H-1-benzazepin-5-yl}essigsäure in 30ml Ethanol wurden mit 15ml HCl in Diethylether (gesättigt bei 0°C) versetzt und 3 Tage bei Raumtemperatur stehen gelassen. Die Mischung wurde eingedampft, der verbliebene Rückstand durch Chromatographie an Kieselgel (CH2Cl2/CH3OH 5% + 1% Eisessig) gereinigt und lyophilisiert. 230mg; ESI-MS: [M+H⁺]= 518.35.

### Beispiel 69:

### {1-[2-({[4-(1H-Benzimidazol-2-ylamino)cyclohexyl]methyl}amino)-2-oxoethyl]-7,8-dimethoxy-2-oxo-2,3,4,5-tetrahydro-1H-1-benzazepin-5-yl}essigsäure (Acetat)

Standardkupplung von [5-(2-tert-Butoxy-2-oxoethyl)-7,8-dimethoxy-2-oxo-2,3,4,5-tetrahydro-1H-1-benzazepin-1-yl]acetat (Baustein 49) (1g; 2.54mmol) mit trans-N-{[4-(Aminomethyl)cyclohexyl]}-1H-benzimidazol-2-amin (Dihydrochlorid) (11); nach Chromatographie an Kieselgel (CH₂Cl₂/CH₃OH 5-7%) wurden 1.03g als weißes Glas isoliert; ESI-MS: [M+H⁺]= 620.35. Spaltung des Esters unter Verwendung von TFA und anschließende Reinigung des Rohprodukts durch Chromatographie an Kieselgel (CH₂Cl₂/CH₃OH 10% + 2% Eisessig) ergaben 0.8g des Zielprodukts; ESI-MS: [M+H⁺] = 564.25.

### Beispiel 70:

### [1-(2-{[4-(1H-Benzimidazol-2-ylamino)benzyl]amino}-2-oxoethyl)-7,8-dimethoxy-2-oxo-2,3,4,5-tetrahydro-1H-1-benzazepin-5-yl]essigsäure

Standardkupplung von [5-(2-tert-Butoxy-2-oxoethyl)-7,8-dimethoxy-2-oxo-2,3,4,5-tetrahydro-1H-1-benzazepin-1-yl]acetat (Baustein 49) (0.5g; 1.27mmol) mit N- [4-(Aminomethyl)phenyl]-1H-benzimidazol-2-amin (Hydrochlorid) (4); nach Chromatographie an Kieselgel (CH₂Cl₂/CH₃OH 3-5%) wurden 0.67g als Schaum isoliert; ESI-MS: [M+H⁺]= 614.35. Spaltung des Esters unter Verwendung von TFA und Lyophilisieren des erhaltenen Produkts ergab 0.61g; ESI-MS: [M+H⁺] = 558.35.

### Beispiel 80:

### {5-(2-({[4-(1H-Benzimidazol-2-yl)cyclohexyl]methyl}amino)-2-oxoethyl]-2-oxo-2,3-dihydro-1H-1-benzazepin-1-yl}essigsäure

0.4g (1.4mmol) [1-(2-Methoxy-2-oxoethyl)-2-oxo-2,3-dihydro-1H-1-benzazepin-5-yl]essigsäure (50) und 0.37g (1.4mmol) [4-(1H-Benzimidazol-2-yl)cyclohexyl]methanamin (Hydrochlorid) (51) wurden analog zu Beispiel I umgesetzt, das Reaktionsprodukt anschließend mittels präparativer Dickschichtchromatographie gereinigt (Eluent: CH₂Cl₂/CH₃OH/konz. NH₃ 45/5/0.2) und anschließend der Methylester mit 0.8ml 1n NaOH in 8ml Dioxan bei Raumtemperatur gespalten. Nach Neutralisation mit 1n HCl, Abziehen des Lösungsmittels, Extraktion mit Ethylacetat und Trocknen mit Na₂SO₄ wurde das Rohprodukt chromatographisch gereinigt (CH₂Cl₂/CH₃OH/50% Essigsäure 20/5/1). Nach Lyophilisieren verblieben 0.22g weißer amorpher Rückstand; ESI-MS: [M+H⁺] = 487.

### Beispiel 81:

### [5-(2-{[5-(1H-Benzimidazol-2-ylamino)pentyl]amino}-2-oxoethyl)-2-oxo-2,3-dihydro-1H-1-benzazepin-1-yl]essigsäure

Analog zu Beispiel I wurden 0.4g (1.4mmol) [1-(2-Methoxy-2-oxoethyl)-2-oxo-2,3-dihydro-1H-1-benzazepin-5-yl]essigsäure (50) und 0.36g (1.4mmol) N¹-(1H-Benzimidazol-2-yl)pentan-1,5-diamin (Hydrochlorid) (8) umgesetzt. Reinigung des Rohproduktes, alkalische Verseifung des Methylesters und Reinigung der Endstufe erfolgte analog zu Beispiel 80. 0.3g weißer amorpher Rückstand; ESI-MS: [M+H⁺] = 476.

### Beispiel 82:

### [5-(2-{4-[(1H-Benzimidazol-2-ylamino)methyl]piperidin-1-yl}-2-oxoethyl)-2-oxo-2,3-dihydro-1H-1-benzazepin-1-yl]essigsäure

Analog zu Beispiel I wurden 0.4g (1.4mmol) [1-(2-Methoxy-2-oxoethyl)-2-oxo-2,3-dihydro-1H-1-benzazepin-5-yl]essigsäure (50) mit 0.63g (1.4mmol) N- (Piperidin-4-ylmethyl)-1H-benzimidazol-2-amin (Trifluoracetat) (17) umgesetzt. Nach Reinigung, alkalischer Esterhydrolyse und chromatographischer Reinigung des Endprodukts (CH₂Cl₂/CH₃OH/50% Essigsäure 20/5/1) wurden 0.3g weißes amorphes Pulver erhalten; ESI-MS: [M+H⁺]= 488.

### Beispiel 83:

### [5-(2-{4-[(1H-Benzimidazol-2-ylamino)methyl]piperidin-1-yl}-2-oxoethyl)-2-oxo-2,3-dihydro-1H-1-benzazepin-1-yl]essigsäure

Analog zu Beispiel I wurden 0.4g (1.4mmol) [1-(2-Methoxy-2-oxoethyl)-2-oxo-2,3-dihydro-1H-1-benzazepin-5-yl]essigsäure (50) mit 0.43g trans-N-{[4-(Aminomethyl)cyclohexyl]}-1H-benzimidazol-2-amin (Dihydrochlorid) (11) umgesetzt. Nach Reinigung des Esters (Eluent: CH₂Cl₂/CH₃OH/25% NH₃ 45/5/0.2), alkalischer Esterhydrolyse und chromatographischer Reinigung des Endprodukts (CH₂Cl₂/CH₃OH/50% Essigsäure 20/5/1) wurden 0.18g weißes amorphes Pulver erhalten; ESI-MS: [M+H⁺]= 502.

### Beispiel 84:

### [5-(2-{[5-(1H-Benzimidazol-2-ylamino)pentyl]amino}-2-oxoethyl)-4-oxo-2,3,4,5-tetrahydro-1H-1,5-benzodiazepin-1-yl]essigsäure (Hydrochlorid)

Analog zu Beispiel I wurden 0.65g (2mmol) [5-(2-tert-Butoxy-2-oxoethyl)-2-oxo-2,3,4,5-tetrahydro-1H-1,5-benzodiazepin-1-yl]essigsäure (54) mit N¹-(1H-Benzimidazol-2-yl)pentan-1,5-diamin (Hydrochlorid) (8) umgesetzt, das Reaktionsprodukt mittels präparativer Dickschichtchromatographie gereinigt (Eluent: CH₂Cl₂/CH₃OH/konz. NH₃ 45/5/0.2) ; ESI-MS: [M+H⁺] = 535. Anschließend wurde der tert.-Butylester mit 4n HCl in Dioxan gespalten, und nach chromatographischer Reinigung 40mg weißes amorphes Pulver isoliert; ESI-MS: [M+H⁺] = 479.

### Beispiel 85

### {5-[2-({[4-(1H-Benzimidazol-2-ylamino)cyclohexyl]methyl}amino)-2-oxoethyl]-4-oxo-2,3,4,5-tetrahydro-1H-1,5-benzodiazepin-1-yl}essigsäure (Hydrochlorid)

Herstellung und Reinigung erfolgte analog zu Beispiel 84 durch Umsetzung mit mit trans-N-{[4-(Aminomethyl)cyclohexyl]}-1H-benzimidazol-2-amin (Dihydrochlorid) (11); Esterstufe ESI-MS: [M+H⁺] = 561. Reinigung nach Esterspaltung erfolgte mittels präparativer Dickschichtchromatographie (CH₂Cl₂/CH₃OH/50% Essigsäure 12/3/1); 0.35g weißes amorphes Pulver; ESI-MS: [M+H⁺] = 505.

### Beispiel 86:

### [5-(2-{[4-(1H-Benzimidazol-2-ylamino)benzyl]amino}-2-oxoethyl)-4-oxo-2,3,4,5-tetrahydro-1H-1,5-benzodiazepin-1-yl]essigsäure (Hydrochlorid)

Herstellung und Reinigung erfolgte analog zu Beispiel 84 durch Umsetzung mit N- [4-(Aminomethyl)phenyl]-1H-benzimidazol-2-amin (Hydrochlorid) (4); Esterstufe ESI-MS: [M+H⁺] = 555; Zielprodukt: 0.4g weißes amorphes Pulver; ESI-MS: [M+H⁺] = 499.

### Beispiel 87:

### [5-(2-{[4-(1H-Benzimidazol-2-ylamino)benzyl]amino}-2-oxoethyl)-2-oxo-2,3,4,5-tetrahydro-1H-1,5-benzodiazepin-1-yl]essigsäure

Analog zu Beispiel I wurden 1.2g (3.6mmol) [5-(2-tert-Butoxy-2-oxoethyl)-4-oxo-2,3,4,5-tetrahydro-1H-1,5-benzodiazepin-2-yl]essigsäure (55) mit 0.86g (3.6mmol) N-[4-(Aminomethyl)phenyl]-1H-benzimidazol-2-amin (Hydrochlorid) (4) umgesetzt, das Reaktionsprodukt mittels präparativer Dickschichtchromatographie gereinigt (Eluent: CH₂Cl₂/CH₃OH/konz. NH₃ 45/5/0.1); ESI-MS: [M+H⁺]=: 555. Anschließende Spaltung des tert.Butylesters mit 4n HCl in Dioxan ergab 0.8g weißes amorphes Pulver; ESI-MS [M+H⁺]= 499.

### Beispiel 88:

### {5-[2-({[4-(1H-Benzimidazol-2-ylamino)cyclohexyl]methyl}amino)-2-oxoethyl]-2-oxo-2,3,4,5-tetrahydro-1H-1,5-benzodiazepin-1-yl}essigsäure

Herstellung und Reinigung analog zu Beispiel 87; Esterstufe ESI-MS: [M+H⁺] = 561; es wurden 0.9g des Zielprodukts als weißes amorphes Pulver erhalten; ESI-MS [M+H⁺]= 505.

### Beispiel 89:

### (1-{2-[4-(1H-Benzimidazol-2-yl)piperidin-1-yl]-2-oxoethyl}-2-oxo-2,3,4,5-tetrahydro-1H-1-benzazepin-5-yl)essigsäure (Acetat)

Analog zu Beispiel I wurden 0.5g (1.5mmol) [5-(2-t.Butoxy-2-oxoethyl)-2-oxo-2,3,4,5-tetrahydro-1H-benzazepin-1-yl]essigsäure (3) und 0.36g (1.5mmol) 2-(4-Piperidinyl)-1H-benzimidazol (J. Heterocycl. Chem. 1989, 26, 541) umgesetzt, mittels präparativer Dickschichtchromatographie gereinigt (Eluent: CH₂Cl₂/CH₃OH/konz. NH₃ 45/5/0.2); ESI-MS: [M+H⁺] = 517. Anschließende Spaltung des tert.Butylesters mit 4n HCl in einem Dioxan/Eisessig-Gemisch und chromatographische Reinigung ergaben 0.25g weißes amorphes Pulver; ESI-MS [M+H⁺] =: 461.

### Beispiel 90:

### (1-{2-[[3-(1H-Benzimidazol-2-yl)propyl](methyl)amino]-2-oxoethyl}-2-oxo-2,3,4,5-tetrahydro-1H-1-benzazepin-5-yl)essigsäure (Hydrochlorid)

Herstellung analog zu Beispiel I durch Umsetzung mit N-[3-(1H-Benzimidazol-2-yl)propyl]-N-methylamin (WO 9849148) ; nach chromatographischer Reinigung wurden 0.6g als weißes amorphes Pulver erhalten; ESI-MS [M+H⁺] =: 449.

### Beispiel 91:

### (1-{2-[4-(1H-Benzimidazol-2-ylamino)piperidin-1-yl]-2-oxoethyl}-2-oxo-2,3,4,5-tetrahydro-1H-1-benzazepin-5-yl)essigsäure

Herstellung analog zu Beispiel I durch Umsetzung mit N-Piperidin-4-yl-1H-benzimidazol-2-amin (J. Med. Chem. 1985, 28, 1925); nach chromatographischer Reinigung wurden 0.45g als weißes amorphes Pulver erhalten; ESI-MS [M+H⁺] =: 476.

### Beispiel 92:

### [5-(2-{[4-(1H-Benzimidazol-2-ylamino)benzyl]amino}-2-oxoethyl)-2-oxo-2,3,4,5-tetrahydro-1H-1-benzazepin-1-yl]essigsäure

Analog zu Beispiel I wurden 0.4g (1.4mmol) [1-(2-Methoxy-2-oxoethyl)-2-oxo-2,3,4,5-tetrahydro-1H-1-benzazepin-5-ylessigsäure (56) und 0.33g (1.4mmol) N-[4-(Aminomethyl)phenyl]-1H-benzimidazol-2-amin (Hydrochlorid) (4) umgesetzt und mittels präparativer Dickschichtchromatographie gereinigt (Eluent: CH₂Cl₂/CH₃OH/konz. NH₃ 45/5/0.2); ESI-MS: [M+H⁺] = 512. Anschließende Spaltung des Methylesters mit 1n NaOH in Dioxan und chromatographische Reinigung des Rückstandes (CH₂Cl₂/CH₃OH/50% Essigsäure 20/5/1) ergaben 0.15g als weißes amorphes Pulver isoliert; ESI-MS [M+H⁺]= 498.

### Beispiel 93:

### {5-[2-({[4-(1H-Benzimidazol-2-yl)cyclohexyl]methyl}amino)-2-oxoethyl]-2-oxo-2,3,4,5-tetrahydro-1H-1-benzazepin-1-yl}essigsäure

Darstellung und Reinigung analog zu Beispiel 92; Esterstufe ESI-MS [M+H⁺] = 503; Chromatographische Reinigung nach Spaltung des Esters ergab 0.3g des Zielprodukts als weißes amorphes Pulver; ESI-MS [M+H⁺]= 489.

### Beispiel 94:

### [5-(2-{[5-(1H-Benzimidazol-2-ylamino)pentyl]amino}-2-oxoethyl)-2-oxo-2,3,4,5-tetrahydro-1H-1-benzazepin-1-yl]essigsäure

Darstellung und Reinigung analog zu Beispiel 92; Esterstufe ESI-MS [M+H⁺] = 492. Chromatographische Reinigung nach Spaltung des Esters ergab 20mg als weißes amorphes Pulver; ESI-MS [M+H⁺]= 478.

### Beispiel 95:

### {5-[2-({[4-(1H-Benzimidazol-2-ylamino)cyclohexyl]methyl}amino)-2-oxoethyl]-2-oxo-2,3,4,5-tetrahydro-1H-1-benzazepin-1-yl}essigsäure

Darstellung und Reinigung analog zu Beispiel 92; Esterstufe ESI-MS [M+H⁺] = 518. Chromatographische Reinigung nach Spaltung des Esters ergab 0.15g als weißes amorphes Pulver; ESI-MS [M+H⁺]= 504.

### Beispiel 96:

### Ethyl-{1-[2-({[4-(1H-benzimidazol-2-ylamino)cyclohexyl]methyl}amino)-2-oxoethyl]-2-oxo-2,3,4,5-tetrahydro-1H-1-benzazepin-5-yl}acetat

Zu einer Lösung von 0.8g (1.6mmol) der Säure aus Beispiel XI, 0.15g (3.2mmol) Ethanol, 0.2g (1.6mmol) 4-Dimethylaminopyridin und 0.4g (4mmol) Triethylamin in 60ml CH₂Cl₂ wurden 0.6g (2.4mmol) N, N-Bis(2-oxo-3-oxazolidinyl)phosphorylchlorid gegeben und über Nacht nachgerührt. Nach beendeter Umsetzung wurden der Reaktionslösung 2g Eisessig und 3.7g Ethanol zugesetzt und weitere 20h bei Raumtemperatur nachgerührt. Zur Aufarbeitung wurde die Mischung mit 50ml H₂O versetzt, die organische Phase mit 10% K₂CO₃-Lösung und H₂O gewaschen, über MgSO₄ getrocknet und eingeengt. Chromatographische Reinigung des Rückstands (Eluent: CH₂Cl₂/Ethanol/50% Essigsäure 15/5/1) verblieben 0.35g weißes amorphes Pulver; ESI-MS [M+H⁺]= 532.

Analog zu Beispiel 96 wurden hergestellt:

### Beispiel 97:

### Cyclohexyl-{1-[2-({[4-(1H-benzimidazol-2-ylamino)cyclohexyl]methyl}amino)-2-oxoethyl]-2-oxo-2,3,4,5-tetrahydro-1H-1-benzazepin-5-yl}acetat

0.26g; ESI-MS [M+H⁺]= 586.

### Beispiel 98:

### Neopentyl-{1-[2-({[4-(1H-benzimidazol-2-ylamino)cyclohexyl]methyl}amino)-2-oxoethyl]-2-oxo-2,3,4,5-tetrahydro-1H-1-benzazepin-5-yl}acetat

0.21g; ESI-MS [M+H⁺]= 574.

### Beispiel 99

### tert-Butyl-[1-(2-{[4-(1H-benzimidazol-2-ylamino)benzyl]amino}-2-oxoethyl)-2-oxo-2,3,4,5-tetrahydro-1H-1-benzazepin-5-yl]acetat

Die Darstellung erfolgte analog zu Beispiel I durch Umsetzung von [5- (2-tert-Butoxy-2-oxoethyl)-2-oxo-2,3,4,5-tetrahydro-1H-1-benzazepin-1-yl]essigsäure (3) mit N-[4-(Aminomethyl)phenyl]-1H-benzimidazol-2-amin (Hydrochlorid) (4). Nach chromatographischer Reinigung (Eluent: CH₂Cl₂/Ethanol/50% Essigsäure 45/5/0.3) wurde der Rückstand in 70ml CH₂Cl₂ und 5ml CH₃OH gelöst, mit 5% K₂CO₃-Lösung und H₂O gewaschen, über Na₂SO₄ getrocknet und zur Trockene eingedampft. Der amorphe Rückstand wurde mit 25ml CH₃OH in der Wärme verrührt. 0.51g weiße Kristalle; Fp.: 231°C (Zersetzung); ESI-MS [M+H⁺] = 554.

### Beispiel 100:

### Cyclohexyl-[1-(2-{[4-(1H-benzimidazol-2-ylamino)benzyl]amino}-2-oxoethyl)-2-oxo-2,3,4,5-tetrahydro-1H-1-benzazepin-5-yl]acetat

0.64g (1.28mmol) [1-(2-{[4-(1*H*-Benzimidazol-2-ylamino)benzyl]amino}-2-oxoethyl)-2-oxo-2,3,4,5-tetrahydro-1H-1-benzazepin-5-yl]essigsäure (Säure des Beispiels III) wurden in 30ml Cyclohexanol suspendiert, unter Rühren 1.0g HCl-Gas eingeleitet und die entstandene gelbliche Lösung 4 Tage bei Raumtemperatur stehen gelassen. Da chromatographisch noch Säure nachweisbar war, wurde 7h lang auf 40-50°C erwärmt. Zur Aufarbeitung wurde mit 100ml Diethylether versetzt, mit K₂CO₃-Lösung und H₂O gewaschen, über Na₂SO₄ getrocknet und das Lösungsmittel 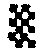 zuletzt zur Entfernung von Cyclohexanol unter Ölpumpenvakuum und einer Badtemperatur von 50°C 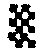 abdestilliert. Der Rückstand wurde chromatographisch (Eluent: CH₂Cl₂/Aceton/Methanol/50% Essigsäure 45/5/4/0.3) gereinigt. 0.65g leicht beigefarbenes Pulver; ESI-MS [M+H⁺]= 580.

### Beispiel 101:

### Ethyl-[1-(2-{[4-(1H-benzimidazol-2-ylamino)benzyl]amino}-2-oxoethyl)-2-oxo-2,3,4,5-tetrahydro-1H-1-benzazepin-5-yl]acetat

0.61g (1.23mmol) [1-(2-{[4-(1H-Benzimidazol-2-ylamino)benzyl]amino}-2-oxoethyl)-2-oxo-2,3,4,5-tetrahydro-1H-1-benzazepin-5-yl]essigsäure (Säure des Beispiels III) wurden in 30ml Ethanol suspendiert, 0.4g HCl-Gas eingeleitet und 4 Tage bei Raumtemperatur stehen gelassen. Das Ethanol wurde im Vakuum abdestilliert, der Rückstand in Ethylacetat aufgenommen, mit 5% NaHCO₃- und NaCl- Lösung gewaschen, über Na₂SO₄ getrocknet und eingedampft. Nach dickschichtchromatographischer Reinigung (Eluent: CH₂Cl₂/Ethanol/50% Essigsäure 43/7/0.5) wurde das Eluat mit etwas CH₂Cl₂ verdünnt und zur Entfernung der Essigsäure mit 50% NaHCO3-Lösung gewaschen. Nach Trocknen über Na₂SO₄ wurde eingeengt und der Rückstand mittels Diethylether/n-Hexan in ein absaugbares amorphes Pulver umgewandelt; 0.55g; ESI-MS [M+H⁺] = 526.

### Beispiel 102:

### 1-{[(Cyclohexyloxy)carbonyl]oxy}ethyl [1-(2-{[4-(1H-benzimidazol-2-ylamino)benzyl]amino}-2-oxoethyl)-2-oxo-2,3,4,5-tetrahydro-1H-1-benzazepin-5-yl]acetat

Zu einer Lösung von 0.6g (1.2mmol) [1-(2-{[4-(1H-Benzimidazol-2-ylamino)benzyl]amino}-2-oxoethyl)-2-oxo-2,3,4,5-tetrahydro-1H-1-benzazepin-5-yl]essigsäure (Säure des Beispiels III) in 10ml DMF gab man bei 3°C unter Rühren 0.3g K₂CO₃, einige Kristalle 18-Krone-6, tropfte 0.4g Cyclohexyl-1-jodethylcarbonat (Herstellung aus Cyclohexyl-1-chlorethylcarbonat und NaI analog J. Antibiot. 1987, 40 (1), 81-90) gelöst in 5ml CH₃CN zu und ließ 20 Min. nachrühren. Nach Zugabe von 100ml kalter NaCl-Lösung wurde mehrmals mit Ethylacetat extrahiert, die vereinigten organischen Phasen mit NaCl-Lösung gewaschen, über Na₂SO₄ getrocknet und eingeengt. Der Rückstand wurde säulenchromatographisch gereinigt (Eluent: CH₂Cl₂/Aceton/Methanol/50% Essigsäure 45/5/5/0.3), nach Abziehen des Lösungsmittels in 50ml CH₂Cl₂ aufgenommen, mit 5% NaHCO₃-Lösung gewaschen, über Na₂SO₄ getrocknet und erneut eingeengt. 90mg weißes amorphes Pulver; ESI-MS [M+H⁺]= 668.

### Beispiel 103

### [(5R)-2-(2-{[4-(1H-Benzimidazol-2-ylamino)benzyl]amino}-2-oxoethyl)-2-oxo-2,3,4,5-tetrahydro-1H-1-benzazepin-5-yl]essigsäure

1.0g (3.0mmol) der linksdrehenden Säure (Baustein 57) und 0.72g (3.0mmol) N-[4-(Aminomethyl)phenyl]-1H-benzimidazol-2-amin (Hydrochlorid) (4) wurden analog zu Beispiel I umgesetzt und das Reaktionsprodukt säulenchromatographisch gereinigt (Eluent: CH₂Cl₂/Aceton/Methanol/50% Essigsäure 45/5/4/0.3); Esterstufe ESI-MS [M+H⁺] = 554. Spaltung des tert.Butylesters mit 4n HCl in Dioxan ergab 0.82g eines weißen amorphen Pulvers; ESI-MS [M+H⁺] = 498; [α]_{D}^{2 0} = -107.7° (K⁺-Salz, c=1 in H₂O).

### Beispiel 104

### [(5S)-1-(2-{[4-(1H-Benzimidazol-2-ylamino)benzyl]amino}-2-oxoethyl)-2-oxo-2,3,4,5-tetrahydro-1H-1-benzazepin-5-yl]essigsäure

Herstellung analog zu Beispiel 103 ausgehend von der rechtsdrehenden Säure Baustein 58.
Esterstufe: 1.5g amorphes Pulver; ESI-MS [M+H⁺] = 554.
Zielprodukt: 0.79g weißes amorphes Pulver; ESI-MS [M+H⁺] = 498.

### Beispiel 105:

### {(5R)-1-[2-({[4-(1H-Benzimidazol-2-ylamino)cyclohexyl]methyl}amino)-2-oxoethyl]-2-oxo-2,3,4,5-tetrahydro-1H-1-benzazepin-5-yl}essigsäure

Herstellung analog zu Beispiel I aus der linksdrehenden Säure Baustein 57 und trans-N-{[4-(Aminomethyl)cyclohexyl]}-1H-benzimidazol-2-amin (Dihydrochlorid) (11).
Esterstufe: 0.9g weißes amorphes Pulver; ESI-MS [M+H⁺]= 560.
Zielprodukt: 0.67g weißes amorphes Pulver; ESI-MS [M+H⁺]= 504; [α]_{D}^{2 0} = -104° (K⁺-Salz, c=1 in H₂O).

### Beispiel 106:

### {(5S)-1-[2-({[4-(1H-Benzimidazol-2-ylamino)cyclohexyl]methyl}amino)-2-oxoethyl]-2-oxo-2,3,4,5-tetrahydro-1H-1-benzazepin-5-yl}essigsäure

Herstellung analog zu Beispiel 103 ausgehend von der rechtsdrehenden Säure Baustein 58.
Esterstufe: 0.72g amorphes Pulver; ESI-MS [M+H⁺] = 560.
Zielprodukt: 0.56g weißes amorphes Pulver; ESI-MS [M+H⁺] = 504; [α]_{D}^{2 0} = +101.6° (K⁺-Salz, c=1 in H₂O).

### Beispiel 107:

### [4-(2-{[4-(1H-Benzimidazol-2-ylamino)benzyl]amino}-2-oxoethyl)-5-oxo-5,6,7,8-tetrahydro-4H-thieno[3,2-b]azepin-8-yl]essigsäure

Analog zu Beispiel I wurden 0.6g (1.8mmol) [8-(2-tert-Butoxy-2-oxoethyl)-5-oxo-5,6,7,8-tetrahydro-4H-thieno[3,2-b]azepin-4-yl]essigsäure (61) mit 0.42g (1.8mmol) N-[4-(Aminomethyl)phenyl]-1H-benzimidazol-2-amin (Hydrochlorid) (4) umgesetzt und das Reaktionsprodukt durch Dickschichtchromatographie gereinigt ((Eluent: CH₂Cl₂/Methanol/konz. NH3 45/5/0.2); ESI-MS [M+H⁺] = 560. Spaltung der t.Butylgruppe mit 4n HCl in Dioxan ergab 0.34g eines leicht gelblichen Pulvers; ESI-MS [M+H⁺] = 504.

### Beispiel 108:

### {4-[2-({[4-(1H-Benzimidazol-2-ylamino)cyclohexyl]methyl}amino)-2-oxoethyl]-5-oxo-5,6,7,8-tetrahydro-4H-thieno[3,2-b]azepin-8-yl}essigsaure

Analog zu Beispiel 107.
Esterstufe: 100mg weißes amorphes Pulver; ESI-MS [M+H⁺] = 566.
Zielprodukt: 98mg weißes amorphes Pulver; ESI-MS [M+H⁺] = 510.

### Beispiel 1

### Integrin αᵥβ₃-Assay

Zur Identifizierung und Bewertung von Integrin-αᵥβ₃-Liganden wurde ein Testsystem verwendet, das auf einer Kompetition zwischen dem natürlichen Integrin αᵥβ₃-Liganden Vitronectin und der Testsubstanz um die Bindung an Festphasen-gebundenes Integrin-αᵥβ₃ basiert.

### Durchführung

- Microtiterplatten beschichten mit 250 ng/ml Integrin-αᵥβ₃ in 0,05 M NaHCO₃ pH 9,2; 0,1 ml/well;
- Absättigen mit 1 % Milchpulver/Assaypuffer; 0,3 ml/well; 0,5 h/RT
- 3x Waschen mit 0,05 % Tween 20/Assaypuffer
- Testsubstanz in 0,1 % Milchpulver/Assaypuffer, 50 µl/well + 0 µg/ml bzw. 2 µg/ml human Vitronectin (Boehringer Ingelheim T007) in 0,1 % Milchpulver/Assaypuffer, 50 µl/well; 1 h/RT
- 3x Waschen mit 0,05 % Tween 20/Assaypuffer
- 1 µg/ml anti human Vitronectin Antikörper gekoppelt an Peroxidase (Kordia SAVN-APHRP) in 0,1 % Milchpulver/Assaypuffer; 0,1 ml/well; 1 h/RT
- 3x Waschen mit 0,05 % Tween 20/Assaypuffer
- 0,1 ml/well Peroxidasesubstrat
- Reaktion stoppen mit 0,1 ml/well 2 M H₂SO₄
- Messung der Absorption bei 450 nm

Integrin-αᵥβ₃: Human-Placenta wird mit Nonidet solubilisiert und Integrin-αᵥβ₃ an einer GRGDSPK-Matrix affinitätsgereinigt (Elution mit EDTA). Verunreinigungen durch Integrin α_{IIb}β₃ und humanes Serumalbumin sowie das Detergens und EDTA werden durch Anionenaustauschchromatographie entfernt.

Assaypuffer: 50 mM Tris pH 7,5; 100 mM NaCl; 1 mM CaCl₂; 1 mM MgCl₂; 10 µM MnCl₂
Peroxidasesubstrat: 0,1 ml TMB-Lösung (42 mM TMB in DMSO) und 10 ml Substratpuffer (0,1 m Na-Acetat pH 4,9) mischen, dann Zusatz von 14, 7 µl 3 % H₂O₂.

In dem Assay werden verschiedene Verdünnungen der Testsubstanzen eingesetzt und die IC₅₀-Werte bestimmt (Konzentration des Liganden, bei der 50 % des Liganden verdrängt werden). Dabei zeigten die Verbindung aus Beispiel VII das beste Ergebnis.

### Beispiel 2

### Integrin α_{IIb}β₃-Assay

Der Assay basiert auf einer Kompetition zwischen dem natürlichen Integrin-α_{IIb}β₃ Liganden Fibrinogen und der Testsubstanz um Bindung an Integrin-α_{IIb}β₃.

### Durchführung

- Microtiterplatten beschichten mit 10 µg/ml Fibrinogen (Calbiochem 341578) in 0,05 M NaHCO₃ pH 9,2; 0,1 ml/well;
- Absättigen mit 1 % BSA/PBS; 0,3 ml/well; 30 min/RT
- 3x Waschen mit 0,05 % Tween 20/PBS
- Testsubstanz in 0,1 % BSA/PBS; 50 µl/well +
   200 µg/ml Integrin-α_{IIb}β₃ (Kordia) in 0,1 % BSA/PBS; 50 µl/well; 2 bis 4 h/RT
- 3x Waschen wie oben
- biotinylierter anti Integrin-α_{IIb}β₃ Antikörper (Dianova CBL 130 B); 1:1000 in 0,1 % BSA/PBS; 0,1 ml/well; 2 bis 4 h/RT
- 3x Waschen wie oben
- Streptavidin-Peroxidase Komplex (B.M. 1089153) 1:10000 in 0,1 % BSA/PBS; 0,1 ml/well; 30 min/RT
- 3x Waschen wie oben
- 0,1 ml/well Peroxidasesubstrat
- Reaktion stoppen mit 0,1 ml/well 2 M H₂SO₄
- Messung der Absorption bei 450 nm

Peroxidasesubstrat: 0,1 ml TMB-Lösung (42 mM TMB in DMSO) und 10 ml Substratpuffer (0,1 M Na-acetat pH 4,9) mischen, dann Zusatz von 14,7 µl 3 % H₂O₂

In dem Assay werden verschiedene Verdünnungen der Testsubstanzen eingesetzt und die IC₅₀-Werte bestimmt (Konzentration des Antagonisten, bei der 50 % des Liganden verdrängt werden).
Durch Vergleich der IC₅₀-Werte im Integrin α_{IIb}β₃- und Integrin αᵥb₃-Assay kann die Selektivität der Substanzen bestimmt werden.

### Beispiel 3

### CAM-Assay

Der CAM (Chorioallantoinmembran) Assay dient als allgemein anerkanntes Modell zur Beurteilung der in vivo Aktivität von Integrin αᵥβ₃-Antagonisten. Er beruht auf der Inhibition von Angiogenese und Neovaskularisation von Tumorgewebe (Am. J. Pathol. 1975, 79, 597-618; Cancer Res. 1980, 40, 2300-2309; Nature 1987, 329, 630). Die Durchführung erfolgt analog zum Stand der Technik. Das Wachstum der Hühnerembryo-Blutgefäße und des transplantierten Tumorgewebes ist gut zu verfolgen und zu bewerten.

### Beispiel 4

### Kaninchenaugen-Assay

In diesem in vivo Modell kann analog zu Beispiel 3 die Inhibition der Angiogenese und Neovaskularisation in Gegenwart von Integrin αᵥβ₃-Antagonisten verfolgt und bewertet werden. Das Modell ist allgemein anerkannt und beruht auf dem Wachstum der Kaninchenblutgefäße ausgehend vom Rand in die Cornea des Auges (Proc. Natl. Acad. Sci. USA. 1994, 91, 4082-4085; Science 1976, 193, 70-72). Die Durchführung erfolgt analog zum Stand der Technik.

## Patentansprüche

1. Verbindungen der Formel I'
A-E'-G'-L I'
wobei A, E', G' und L folgende Bedeutung haben:
L ein Strukturelement der Formel I_{L}
-U-T I_{L}
wobei
T eine Gruppe COOH, ein zu COOH hydrolisierbarer Rest oder ein zu COOH bioisosterer Rest und
-U- -(X_{L})ₐ-(CR_{L}¹R_{L}²)_{b}-, -CR_{L}¹=CR_{L}²-, Ethinylen oder =CR_{L}¹- bedeuten, wobei
a 0 oder 1,
b 0, 1 oder 2
X_{L} CR_{L}³R_{L}⁴, NR_{L}⁵, Sauerstoff oder Schwefel,
R_{L}¹, R_{L}², R_{L}³, R_{L}⁴ unabhängig voneinander Wasserstoff, -T, -OH, -NR_{L}⁶R_{L}⁷, -CO-NH₂, einen Halogenrest, einen verzweigten oder unverzweigten, gegebenenfalls substituierten C₁-C₆-Alkyl-, C₂-C₆-Alkenyl-, C₂-C₆-Alkinyl-, C₃-C₇-Cycloalkyl-, -CO-NH(C₁-C₆-Alkyl), -CO-N(C₁-C₆-Alkyl)₂ oder C₁-C₄-Alkoxyrest, einen gegebenenfalls substituierten Rest C₁-C₂-Alkylen-T, C₂-Alkenylen-T oder C₂-Alkinylen-T, einen gegebenenfalls substituierten Aryl- oder Arylalkylrest oder jeweils unabhängig voneinander zwei Reste R_{L}¹ und R_{L}² oder R_{L}³ und R_{L}⁴ oder gegebenenfalls R_{L}¹ und R_{L}³ zusammen einen, gegebenenfalls substituierten 3 bis 7 gliedrigen gesättigten oder ungesättigten Carbocyclus oder Heterocyclus, der bis zu drei verschiedene oder gleiche Heteroatome O, N, S enthalten kann,
R_{L}⁵, R_{L}⁶, R_{L}⁷ unabhängig voneinander Wasserstoff, einen verzweigten oder unverzweigten, gegebenenfalls substituierten C₁-C₆-Alkyl-, C₃-C-₇-Cycloalkyl-, CO-O-C₁-C₆-Alkyl-, SO₂-C₁-C₆-Alkyl- oder CO-C₁-C₆-Alkylrest oder einen, gegebenenfalls substituierten CO-O-Alkylen-Aryl-, SO₂-Aryl-, CO-Aryl-, SO₂-Alkylen-Aryl- oder CO-Alkylen-Arylrest,
bedeuten,
G' ein Strukturelement der Formel I_{G} 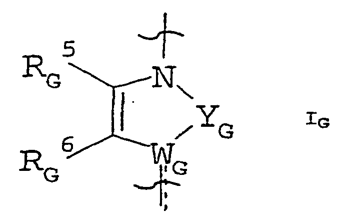
wobei
das Strukturelement E' über den Ringstickstoff und das Strukturelement L über WG an das Strukturelement G' gebunden ist,
Y_{G} CO, CS, C=NR_{G}² oder CR_{G}³R_{G}⁴,
R_{G}² Wasserstoff, eine Hydroxy-Gruppe, einen verzweigten oder unverzweigten, gegebenenfalls substituierten C₁-C₆-Alkyl-, C₁-C₄-Alkoxy-, C₃-C₇-Cycloalkyl- oder -O-C₃-C₇-Cycloalkylrest oder einen gegebenenfalls substituierten Aryl-, -O-Aryl, Arylalkyl- oder -O-Alkylen-Arylrest,
R_{G}³, R_{G}⁴ unabhängig voneinander Wasserstoff oder einen verzweigten oder unverzweigten, gegebenenfalls substituierten C₁-C₆-Alkyl-, C₂-C₆-Alkenyl-, C₂-C₆-Alkinyl- oder C₁-C₄-Alkoxyrest oder beide Reste R_{G}³ und R_{G}⁴ zusammen ein cyclisches Acetal -O-CH₂-CH₂-O- oder -O-CH₂-O- oder beide Reste R_{G}³ und R_{G}⁴ zusammen einen, gegebenenfalls substituierten C₃-C₇-Cycloalkylrest,
R_{G}⁵ und R_{G}⁶ unabhängig voneinander Wasserstoff, eine Hydroxygruppe, einen verzweigten oder unverzweigten, gegebenenfalls substituierten C₁-C₆-Alkyl- oder C₁-C₄-Alkoxyrest, einen gegebenfalls substituierten Aryl- oder Arylalkylrest oder beide Reste R_{G}⁵ und R_{G}⁶ zusammen einen, gegebenenfalls substituierten, anelierten, ungesättigten oder aromatischen 3- bis 10-gliedrigen Carbocyclus oder Heterocyclus, der bis zu drei verschiedene oder gleiche Heteroatome O, N, S enthalten kann,
W_{G} ein Strukturelement ausgewählt aus der Gruppe der Strukturelemente der Formeln I_{WG}¹ bis I_{WG}⁴, 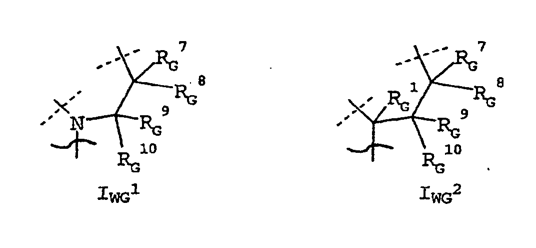 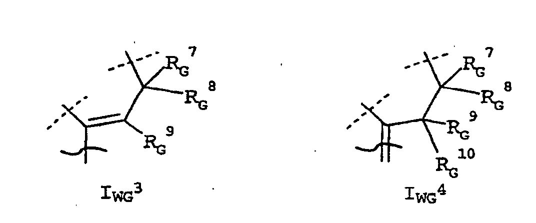
R_{G}¹ Wasserstoff, Halogen, eine Hydroxy-Gruppe oder einen verzweigten oder unverzweigten, gegebenenfalls substituierten C₁-C₆-Alkyl- oder C₁-C₄-Alkoxyrest,
R_{G}⁷, R_{G}⁸, R_{G}⁹, R_{G}¹⁰ unabhängig voneinander Wasserstoff, eine Hydroxygruppe, -CN, Halogen, einen verzweigten oder unverzweigten, gegebenenfalls substituierten C₁-C₆-Alkyl-, C₂-C₆-Alkenyl-, C₂-C₆-Alkinyl-, C₁-C₄-Alkylen-C₃-C₇-Cycloalkyl-, C₁-C₄-Alkylen-C₃-C₇-Heterocycloalkyl- oder C₁-C₄-Alkylen-C₃-C₇-Heterocycloalkenylrest, einen verzweigten oder unverzweigten, gegebenenfalls substituierten Rest C₁-C₄-Alkylen-OR_{G}¹¹, C₁-C₄-Alkylen-CO-OR_{G}¹¹, C₁-C₄-Alkylen-O-CO-R_{G}¹¹, C₁-C₄-Alkylen-CO-R_{G}¹¹, C₁-C₄-Alkylen-SO₂-NR_{G'}¹²R_{G'}¹³, C₁-C₄-Alkylen-CO-NR_{G'}¹²R_{G'}¹³, C₁-C₄-Alkylen-O-CO-NR_{G'}¹²R_{G'}¹³, C₁-C₄-Alkylen-NR_{G'}¹²R_{G'}¹³ oder C₁-C₄-Alkylen-SR_{G}¹¹, C₁-C₄-Alkylen-SO-R_{G}¹¹, einen Rest -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G'}¹²R_{G'}¹³, -SO₂-NR_{G'}¹²R_{G'}¹³, -CO-NR_{G'}¹²R_{G'}¹³, -NR_{G'}¹²R_{G'}¹³ oder CO-R_{G}¹¹, einen gegebenenfalls substituierten C₃-C₇-Cycloalkyl-, C₃-C₇-Heterocycloalkyl-, C₃-C₇-Heterocycloalkenyl-, Aryl-, Hetaryl-, Arylalkyl- oder Hetarylalkylrest oder jeweils unabhängig voneinander zwei Reste R_{G}⁷ und R_{G}⁹ oder R_{G}⁸ und R_{G}¹⁰ oder R_{G}⁷ und R_{G}⁸ oder R_{G}⁹ und R_{G}¹⁰ zusammen einen, gegebenenfalls substituierten, gesättigten oder ungesättigten, nicht aromatischen, 3 bis 7 gliedrigen Carbocyclus oder Heterocyclus der bis zu 3 Heteroatome ausgewählt aus der Gruppe O, N, S und bis zu zwei Doppelbindungen enthalten kann,
R_{G}¹¹ Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest,
R_{G'}¹², R_{G'}¹³ unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NRG¹¹RG¹¹* oder -CO-R_{G}¹⁴ und
R_{G}¹¹* einen von R_{G}¹¹ unabhängigen Rest R_{G}¹¹
R_{G'}¹⁴ Wasserstoff, einen verzweigten oder unverzweigten, gegebenenfalls substituierten C₁-C₈-Alkyl-, C₂-C₆-Alkenyl-, C₂-C₆-Alkinyl- oder C₁-C₅-Alkylen-C₁-C₄-Alkoxy-rest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest,
bedeuten,
E' ein Strukturelement, zusammengesetzt aus zwei bis vier Teilstrukturelementen, ausgewählt aus der Gruppe E¹ und E², wobei die Reihenfolge der Verknüpfung der Teilstrukturelemente beliebig ist und E¹ und E² folgende Bedeutung haben:
E¹ ein Teilstrukturelement der Formel I_{E1}
- ₍Y_{E)k1}-(CR_{E}¹R_{E}²)_{c}-(Q_{E})ₖ₂-(CR_{E}³R_{E}⁴)_{d}- I_{E1}
und
E² ein Teilstrukturelement der Formel I_{E2}
-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2} ,
wobei
c, d, f, g, h unabhängig voneinander 0, 1 oder 2,
k1, k2, k3, k4, k5, k6 unabhängig voneinander 0 oder 1,
X_{E}, Q_{E} unabhängig voneinander einen gegebenenfalls substituierten 4 bis 11-gliedrigen mono- oder polycyclischen, aliphatischen oder aromatischen Kohlenwasserstoff, der bis zu 6 Doppelbindungen und bis zu 6 gleiche oder verschiedene Heteroatome, ausgewählt aus der Gruppe N, O oder S enthalten kann, wobei die Ringkohlenstoffe und/oder die Ringstickstoffe gegebenenfalls substituiert sein können,
Y_{E}, Z_{E} unabhängig voneinander CO, CO-NR_{E}¹², NR_{E}¹²-CO, Schwefel, SO, SO₂, SO₂-NR_{E}¹², NR_{E}¹²-SO₂, CS, CS-NR_{E}¹², NR_{E}¹²-CS, CS-O, O-CS, CO-O, O-CO, Sauerstoff, Ethinylen, CR_{E}¹³-O-CR_{E}¹⁴, C(=CR_{E}¹³R_{E}¹⁴), CR_{E}¹³=CR_{E}¹⁴, -CR_{E}¹³(OR_{E}¹⁵)-CHR_{E}¹⁴- oder -CHR- _{E}¹³-CR_{E}¹⁴(OR_{E}¹⁵)-,
R_{E}¹, R_{E}², R_{E}³, R_{E}⁴, R_{E}⁵, R_{E}⁶, R_{E}⁷, R_{E}⁸, R_{E}⁹, R_{E}¹⁰ unabhängig voneinander Wasserstoff, Halogen, eine Hydroxygruppe, einen verzweigten oder unverzweigten, gegebenenfalls substituierten C₁-C₆-Alkyl-, C₂-C₆-Alkenyl-, C₂-C₆-Alkinyl- oder Alkylen-Cycloalkylrest, einen Rest -(CH₂)ₓ-(W_{E})_{z}-R_{E}¹⁷, einen gegebenenfalls substituierten C₃-C₇-Cycloalkyl-, Aryl-, Arylalkyl-, Hetaryl- oder Hetarylalkylrest oder unabhängig voneinander jeweils zwei Reste R_{E}¹ und R_{E}² oder R_{E}³ und R_{E}⁴ öder R_{E}⁵ und R_{E}⁶ oder R_{E}⁷ und R_{E}⁸ oder R_{E}⁹ und R_{E}¹⁰ zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann,
x 0, 1, 2, 3 oder 4,
z 0 oder 1,
W_{E} -CO-, -CO-N(R_{w}²)-, -N(R_{w}²)-CO-, -N(R_{w}²)-CO-N(R_{w}²*)-, -N(R_{w}²)-CO-O-, -O-, -S-, -SO₂-, -SO₂-N(R_{w}²)-, -SO₂-O-, -CO-O-, -O-CO-, -O-CO-N(R_{w}²)-, -N(R_{w}²)- oder -N(R_{w}²)-SO₂-,
R_{w}^{2,} R_{w}²* unabhängig voneinander Wasserstoff, einen verzweigten oder unverzweigten, gegebenenfalls substituierten C₁-C₆-Alkyl-, C₂-C₆-Alkenyl-, C₂-C₈-Alkinyl-, CO-C₁-C₆-Alkyl-, CO-O-C₁-C₆-Alkyl- oder SO₂-C₁-C₆-Alkylrest oder einen gegebenenfalls substituierten Hetaryl, Hetarylalkyl, Arylalkyl, C₃-C₇-Cycloalkyl-, CO-O-Alkylen-Aryl-, CO-Alkylen-Aryl-, CO-Aryl, SO₂-Aryl-, CO-Hetaryl- oder SO₂-Alkylen-Arylrest,
R_{E}¹⁷ Wasserstoff, eine Hydroxygruppe, CN, Halogen, einen verzweigten oder unverzweigten, gegebenenfalls substituierten C₁-C₆-Alkylrest, einen gegebenenfalls substituierten C₃-C₇-Cycloalkyl-, Aryl-, Heteroaryl oder Arylalkylrest, einen gegebenenfalls mit C₁-C₄-Alkyl oder Aryl substituierten C₂-C₆-Alkinyl- oder C₂-C₆-Alkenylrest, einen gegebenenfalls substituierten C₆-C₁₂-Bicycloalkyl-, C₁-C₆-Alkylen-C₆-C₁₂-Bicycloalkyl-, C₇-C₂₀-Tricycloalkyl- oder C₁-C₆-Alkylen-C₇-C₂₀-Tricycloalkylrest, oder einen mit bis zu drei gleichen oder verschiedenen Resten substituierten, 3- bis 8-gliedrigen, gesättigten oder ungesättigten Heterocyclus, der bis zu drei verschiedene oder gleiche Heteroatome O, N, S enthalten kann, wobei zwei Reste zusammen einen anellierten, gesättigten, ungesättigten oder aromatischen Carbocyclus oder Heterocyclus, der bis zu drei verschiedene oder gleiche Heteroatome O, N, S enthalten kann, darstellen können und der Cyclus gegebenenfalls substituiert oder an diesem Cyclus ein weiterer, gegebenenfalls substituierter, gesättigter, ungesättigter oder aromatischer Cyclus ankondensiert sein kann, oder der Rest R_{E}¹⁷ bildet zusammen mit R_{w}² oder R_{W}²* einen gesättigten oder ungesättigten C₃-C₇-Heterocyclus, der gegebenenfalls bis zu zwei weitere Heteroatome, ausgewählt aus der Gruppe O, S oder N enthalten kann,
R_{E}¹¹, R_{E}¹¹* unabhängig voneinander Wasserstoff, einen verzweigten oder unverzweigten, gegebenenfalls substituierten C₁-C₆-Alkyl-, C₂-C₆-Alkoxyalkyl-, C₂-C₆-Alkenyl-, C₂-C₁₂-Alkinyl-, CO-C₁-C₆-Alkyl-, CO-O-C₁-C₆-Alkyl-, CO-NH-C₁-C₆-Alkoxalkyl-, CO-NH-C₁-C₆-Alkyl- oder SO₂-C₁-C₆-Alkylrest oder einen gegebenenfalls substituierten Hetaryl, Arylalkyl-, C₃-C₇-Cycloalkyl-, CO-O-Alkylen-Aryl-, CO-NH-Alkylen-Aryl-, CO-Alkylen-Aryl-, CO-Aryl, CO-NH-Aryl, SO₂-Aryl-, CO-Hetaryl-, SO₂-Alkylen-Aryl-, SO₂-Hetaryl- oder SO₂-Alkylen-Hetarylrest,
R_{E}¹² Wasserstoff, einen verzweigten oder unverzweigten, gegebenenfalls substituierten C₁-C₆-Alkyl-, C₂-C₆-Alkenyl-, C₂-C₈-Alkinyl-, einen gegebenenfalls substituierten C₃-C₇-Cycloalkyl-, Hetaryl-, Arylalkyl- oder Hetarylalkyl Rest oder einen Rest CO-R_{E}¹⁶, COOR_{E}¹⁶ oder SO₂-R_{E}¹⁶,
R_{E}¹³, R_{E}¹⁴ unabhängig voneinander Wasserstoff, eine Hydroxygruppe, einen verzweigten oder unverzweigten, gegebenenfalls substituierten C₁-C₅-Alkyl-, C₁-C₄-Alkoxy-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest,
R_{E}¹⁵ Wasserstoff, einen verzweigten oder unverzweigten, gegebenenfalls substituierten C₁-C₆-Alkyl-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest,
R_{E}¹⁶ Wasserstoff, eine Hydroxygruppe, einen verzweigten oder unverzweigten, gegebenenfalls substituierten C₁-C₆-Alkyl-, C₂-C₆-Alkenyl-, C₂-C₆-Alkinyl- oder C₁-C₅-Alkylen-C₁-C₄-Alkoxyrest, oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-C₃-C₇-Heterocycloalkyl-, C₁-C₄-Alkylen-C₃-C₇-Heterocycloalkenyl- oder Hetarylalkylrest
bedeuten,
mit der Maßgabe, daß für den Fall,
daß Y_{E} oder Z_{E} = CO bedeuten und ein Rest X_{E} oder Q_{E} oder ein aromatischer oder heteroaromatischer Rest aus dem Strukturelement A direkt an Y_{E} oder Z_{E} gebunden ist, eine direkte Atombindung von Y_{E} oder Z_{E} an das Strukturelement G' ausgeschlossen ist,
A ein Strukturelement ausgewählt aus der Gruppe:
ein 4- bis 8-gliedriger monocyclischer gesättigter, ungesättigter oder aromatischer Kohlenwasserstoff, der bis zu 4 Heteroatome, ausgewählt aus der Gruppe O, N oder S, enthalten kann, wobei jeweils unabhängig voneinander der gegebenenfalls enthaltene Ring-Stickstoff oder die Kohlenstoffe substituiert sein können,
mit der Maßgabe daß mindestens ein Heteroatom, ausgewählt aus der Gruppe O, N oder S im Strukturelement A enthalten. ist,
oder
ein 9- bis 14-gliedriger polycyclischer gesättigter, ungesättigter oder aromatischer Kohlenwasserstoff, der bis zu 6 Heteroatome, ausgewählt aus der Gruppe N, O oder S, enthalten kann, wobei jeweils unabhängig voneinander der gegebenenfalls enthaltene Ring-Stickstoff oder die Kohlenstoffe substituiert sein können,
mit der Maßgabe daß mindestens ein Heteroatom, ausgewählt aus der Gruppe O, N oder S im Strukturelement A enthalten ist,
ein Rest 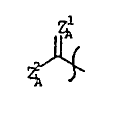
wobei
Z_{A}¹ Sauerstoff, Schwefel oder gegebenenfalls substituierter Stickstoff und
Z_{A}² gegebenenfalls substituierten Stickstoff, Sauerstoff oder Schwefel
bedeuten,
oder ein Rest 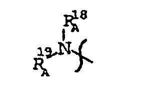 wobei
R_{A}¹⁸, R_{A}¹⁹ unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NR_{G}¹¹R_{G}¹¹* oder -CO-R_{G}¹¹
bedeuten,
sowie die physiologisch verträglichen Salze, Prodrugs und die enantiomerenreinen oder diastereomerenreinen und tautomeren Formen.

2. Verbindung nach Anspruch 1, **dadurch gekennzeichnet daß** man als Strukturelement A ein Strukturelement, ausgewählt aus der Gruppe der Strukturelemente der Formeln I_{A}¹ bis I_{A}¹⁸ verwendet, 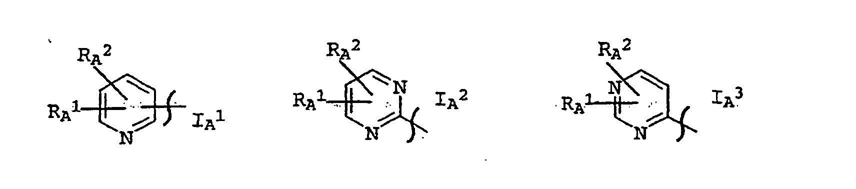 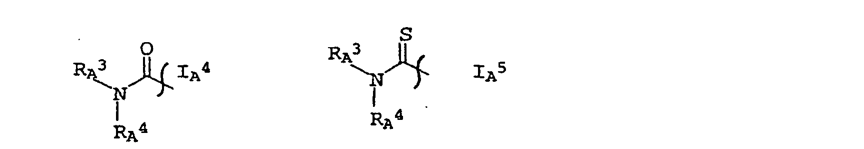 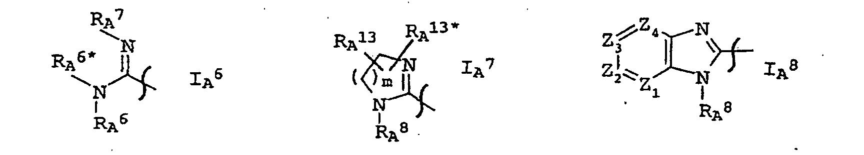 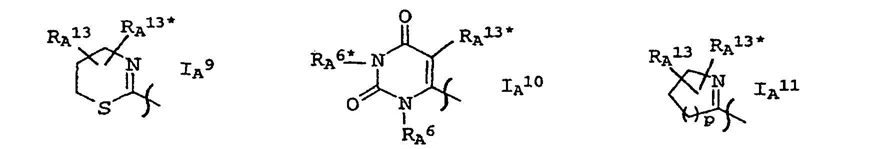 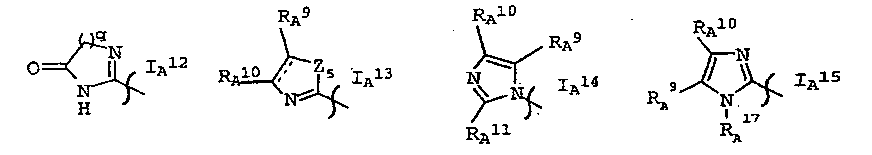 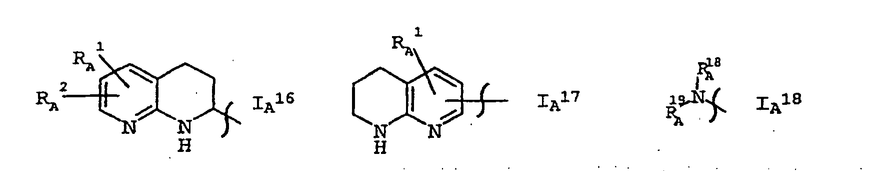 wobei
m,p,q unabhängig voneinander 1,2 oder 3,
R_{A}¹, R_{A}² unabhängig voneinander Wasserstoff, CN, Halogen, einen verzweigten oder unverzweigten, gegebenenfalls substituierten C₁-C₆-Alkyl- oder CO-C₁-C₆-Alkylrest oder einen gegebenenfalls substituierten Aryl-, Arylalkyl-, Hetaryl-, Hetarylalkyl- oder C₃-C₇-Cycloalkylrest oder einen Rest CO-O-R_{A}¹⁴, O-R_{A}¹⁴, S-R_{A}¹⁴, NR_{A}¹⁵R_{A}¹⁶, CO-NR_{A}¹⁵R_{A}¹⁶ oder SO₂NR_{A}¹⁵R_{A}¹⁶ oder beide Reste R_{A}¹ und R_{A}² zusammen einen anellierten, gegebenenfalls substituierten, 5- oder 6-gliedrigen, ungesättigten oder aromatischen Carbocyclus oder Heterocyclus der bis zu drei Heteroatome, ausgewählt aus der Gruppe O, N, oder S enthalten kann,
R_{A}¹³, R_{A}¹³* unabhängig voneinander Wasserstoff, CN, Halogen, einen verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-NR_{A}¹⁵R_{A}¹⁶,
wobei
R_{A}¹⁴ Wasserstoff, einen verzweigten oder unverzweigten, gegebenenfalls substituierten C₁-C₆-Alkyl-, Alkylen-C₁-C₄-Alkoxy-, C₂-C₆-Alkenyl-, C₂-C₆-Alkinyl- oder C₁-C₆-Alkylen-C₃-C₇-Cycloalkylrest oder einen gegebenfalls substituierten C₃-C₇-Cycloalkyl-, Aryl-, Arylalkyl-, Hetaryl- oder Hetarylalkylrest,
R_{A}¹⁵, R_{A}¹⁶, unabhängig voneinander Wasserstoff, einen verzweigten oder unverzweigten, gegebenenfalls substituierten C₁-C₆-Alkyl-, CO-C₁-C₆-Alkyl-, SO₂-C₁-C₆-Alkyl-, COO-C₁-C₆-Alkyl-, CO-NH-C₁-C₆-Alkyl-, Arylalkyl-, COO-Alkylen-Aryl-, SO₂-Alkylen-Aryl-, CO-NH-Alkylen-Aryl-, CO-NH-Alkylen-Hetaryl- oder Hetarylalkylrest oder einen gegebenenfalls substituierten C₃-C₇-Cycloalkyl-, Aryl-, CO-Aryl-, CO-NH-Aryl-, SO₂-Aryl, Hetaryl, CO-NH-Hetaryl-, oder CO-Hetarylrest bedeuten, ,
R_{A}³, R_{A}⁴ unabhängig voneinander Wasserstoff, -(CH₂)ₙ-(X_{A})ⱼ-R_{A}¹², oder beide Reste zusammen einen 3 bis 8 gliedrigen, gesättigten, ungesättigten oder aromatischen N-Heterocyclus der zusätzlich zwei weitere, gleiche oder verschiedene Heteroatome O, N, oder S enthalten kann, wobei der Cyclus gegebenenfalls substituiert oder an diesem Cyclus ein weiterer, gegebenenfalls substituierter, gesättigter, ungesättigter oder aromatischer Cyclus ankondensiert sein kann,
wobei
n 0, 1, 2, oder 3,
j 0 oder 1,
X_{A} -CO-, -CO-N(R_{X}¹)-, -N(R_{X}¹)-CO-, -N(R_{X}¹)-CO-N(R_{X}¹*)-, -N(R_{X}¹)-CO-O-, -O-, -S-, -SO₂-, -SO₂-N(R_{X}¹)-, -SO₂-O-, -CO-O-, -O-CO-, -O-CO-N(R_{X}¹)-, -N(R_{X}¹)- oder -N(R_{X}¹)-SO₂-,
R_{A}¹² Wasserstoff, einen verzweigten oder unverzweigten, gegebenenfalls substituierten C₁-C₆-Alkylrest, einen gegebenenfalls mit C₁-C₄-Alkyl oder Aryl substituierten C₂-C₆-Alkinyl- oder C₂-C₆-Alkenylrest oder einen mit bis zu drei gleichen oder verschiedenen Resten substituierten, 3-6 gliedrigen, gesättigten oder ungesättigten Heterocyclus, der bis zu drei verschiedene oder gleiche Heteroatome O, N, S enthalten kann, C₃-C₇-Cycloalkyl-, Aryl- oder Heteroarylrest, wobei zwei Reste zusammen einen anellierten, gesättigten, ungesättigten oder aromatischen Carbocyclus oder Heterocyclus, der bis zu drei verschiedene oder gleiche Heteroatome O, N, S enthalten kann, darstellen können und der Cyclus gegebenenfalls substituiert oder an diesem Cyclus ein weiterer, gegebenenfalls substituierter, gesättigter, ungesättigter oder aromatischer Cyclus ankondensiert sein kann, oder der Rest R_{A}¹² bildet zusammen mit R_{X}¹ oder R_{X}¹* einen gesättigten oder ungesättigten C₃-C₇-Heterocyclus, der gegebenenfalls bis zu zwei weitere Heteroatome, ausgewählt aus der Gruppe O, S oder N enthalten kann,
R_{X}¹, R_{X}¹* unabhängig voneinander Wasserstoff, einen verzweigten oder unverzweigten, gegebenenfalls substituierten C₁-C₆-Alkyl-, C₁-C₆-Alkoxyalkyl, C₂-C₆-Alkenyl-, C₂-C₁₂-Alkinyl-, CO-C₁-C₆-Alkyl-, CO-O-C₁-C₆-Alkyl- oder SO₂-C₁-C₆-Alkylrest oder einen gegebenenfalls substituierten C₃-C₇-Cycloalkyl-, Aryl, Arylalkyl-, CO-O-Alkylen-Aryl-, CO-Alkylen-Aryl-, CO-Aryl, SO₂-Aryl-, Hetaryl, CO-Hetaryl- oder SO₂-Alkylen-Arylrest,
R_{A}⁶, R_{A}⁶* Wasserstoff, einen verzweigten oder unverzweigten, gegebenenfalls substituierten C₁-C₄-Alkyl-, -CO-O-C₁-C₄-Alkyl-, Arylalkyl-, -CO-O-Alkylen-Aryl-, -CO-O-Allyl-, -CO-C₁-C₄-Alkyl-, -CO-Alkylen-Aryl-, C₃-C₇-Cycloalkyl- oder -CO-Allylrest oder in Strukturelement I_{A}⁷ beide Reste R_{A}⁶ und R_{A}⁶* zusammen einen gegebenenfalls substituierten, gesättigten, ungesättigten oder aromatischen Heterocyclus, der zusätzlich zum Ringstickstoff bis zu zwei weitere verschiedene oder gleiche Heteroatome O, N, S enthalten kann,
R_{A}⁷ Wasserstoff, -OH, -CN, -CONH₂, einen verzweigten oder unverzweigten, gegebenenfalls substituierten C₁-C₄-Alkyl-, C₁-C₄-Alkoxy-, C₃-C₇-Cycloalkyl- oder -O-CO-C₁-C₄-Alkylrest, oder einen gegebenenfalls substituierten Arylalkyl-, -O-Alkylen-Aryl-, -O-CO-Aryl-, -O-CO-Alkylen-Aryl- oder -O-CO-Allylrest, oder beide Reste R_{A}⁶ und R_{A}⁷ zusammen einen gegebenenfalls substituierten, ungesättigten oder aromatischen Heterocyclus, der zusätzlich zum Ringstickstoff bis zu zwei weitere verschiedene oder gleiche Heteroatome O, N, S enthalten kann,
R_{A}⁸ Wasserstoff, einen verzweigten oder unverzweigten, gegebenenfalls substituierten C₁-C₄-Alkyl-, CO-C₁-C₄-Alkyl-, SO₂-C₁-C₄-Alkyl- oder CO-O-C₁-C₄-Alkylrest oder einen gegebenenfalls substituierten Aryl-, CO-Aryl-, SO₂-Aryl, CO-O-Aryl, CO-Alkylen-Aryl-, SO₂-Alkylen-Aryl-, CO-O-Alkylen-Aryl- oder Alkylen-Arylrest,
R_{A}⁹, R_{A}¹⁰ unabhängig voneinander Wasserstoff, -CN, Halogen, einen verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-NR_{A}¹⁵R_{A}¹⁶, oder beide Reste R_{A}⁹ und R_{A}¹⁰ zusammen in Strukturelement I_{A}¹⁴ einen 5 bis 7 gliedrigen gesättigten, ungesättigten oder aromatischen Carbocyclus oder Heterocyclus, der bis zu drei verschiedene oder gleiche Heteroatome O, N, S enthalten kann und gegebenenfalls mit bis zu drei gleichen oder verschiedenen Resten substituiert ist,
R_{A}¹¹ Wasserstoff, -CN, Halogen, einen verzweigten oder unverzweigten, gegebenenfalls substituierten C₁-C₆-Alkylrest oder einen gegebenenfalls substituierten Aryl-, Arylalkyl-, Hetaryl-, C₃-C₇-Cycloalkylrest oder einen Rest CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ oder CO-NR_{A}¹⁵R_{A}¹⁶,
R_{A}¹⁷ Wasserstoff oder in strukturelement I_{A}¹⁵ beide Reste R_{A}⁹ und R_{A}¹⁷ zusammen einen 5 bis 7 gliedrigen gesättigten, ungesättigten oder aromatischen Heterocyclus, der zusätzlich zum Ringstickstoff bis zu drei verschiedene oder gleiche Heteroatome O, N, S enthalten kann und gegebenenfalls mit bis zu drei gleichen oder verschiedenen Resten substituiert ist,
R_{A}¹⁸, R_{A}¹⁹ unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen von R_{G}¹¹ unabhängigen Rest -SO₂-R_{G}¹¹, -CO-OR_{G}¹¹, -CO-NR_{G}¹¹R_{G}¹¹* oder -CO-R_{G}¹¹
Z¹, Z², Z³, Z⁴ unabhängig voneinander Stickstoff, C-H, C-Halogen oder einen verzweigten oder unverzweigten, gegebenenfalls substituieren C-C₁-C₄-Alkyl- oder C-C₁-C₄-Alkoxyrest,
Z⁵ NR_{A}⁸, Sauerstoff oder Schwefel
bedeuten.

3. Verbindung nach Anspruch 1 oder 2, **dadurch gekennzeichnet, daß** man als Strukturelement E' ein Strukturelement der Formel I_{E1E2} verwendet
-E₂-E₁- I_{E1E2}
und E¹ und E² folgende Bedeutung haben:
E¹ ein Teilstrukturelement der Formel I_{E1}
-(Y_{E})ₖ₁-(CR_{E}¹R_{E}²)_{c}-(Q_{E})ₖ₂-(CR_{E}³R_{E}⁴)_{d}- I_{E1}
und
E² ein Teilstrukturelement der Formel I_{E2}
-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2} ,
wobei
c, d, f, g, h unabhängig voneinander 0, 1 oder 2,
k1, k2, k3, k4, k5, k6 unabhängig voneinander 0 oder 1,
X_{E}, Q_{E} unabhängig voneinander einen gegebenenfalls substituierten 4 bis 11-gliedrigen mono- oder polycyclischen, aliphatischen oder aromatischen Kohlenwasserstoff, der bis zu 6 Doppelbindungen und bis zu 6 gleiche oder verschiedene Heteroatome, ausgewählt aus der Gruppe N, O oder S enthalten kann, wobei die Ringkohlenstoffe und/oder die Ringstickstoffe gegebenenfalls substituiert sein können,
Y_{E}, Z_{E} unabhängig voneinander CO, CO-NR_{E}¹², NR_{E}¹²-CO, Schwefel, SO, SO₂, SO₂-NR_{E}¹², NR_{E}¹²-SO₂, CS, CS-NR_{E}¹², NR_{E}¹²-CS, CS-O, O-CS, CO-O, O-CO, Sauerstoff, Ethinylen, CR_{E}¹³-O-CR_{E}¹⁴, C(=CR_{E}¹³R_{E}¹⁴), CR_{E}¹³=CR_{E}¹⁴, -CR_{E}¹³(OR_{E}¹⁵)-CHR_{E}¹⁴- oder -CHR_{E}¹³-CR_{E}¹⁴(OR_{E}¹⁵)-,
R_{E}¹, R_{E}², R_{E}³, R_{E}⁴, R_{E}⁵, R_{E}⁶, R_{E}⁷, R_{E}⁸, R_{E}⁹, R_{E}¹⁰ unabhängig voneinander Wasserstoff, Halogen, eine Hydroxygruppe, einen verzweigten oder unverzweigten, gegebenenfalls substituierten C₁-C₆-Alkyl-, C₂-C₆-Alkenyl-, C₂-C₆-Alkinyl- oder Alkylen-Cycloalkylrest, einen Rest -(CH₂)ₓ-(W_{E})_{z}-R_{E}¹⁷, einen gegebenenfalls substituierten C₃-C₇-Cycloalkyl-, Aryl-, Arylalkyl-, Hetaryl- oder Hetarylalkylrest oder unabhängig voneinander jeweils zwei Reste R_{E}¹ und R_{E}² oder R_{E}³ und R_{E}⁴ oder R_{E}⁵ und R_{E}⁶ oder R_{E}⁷ und R_{E}⁸ oder R_{E}⁹ und R_{E}¹⁰ zusammen einen 3 bis 7-gliedrigen, gegebenenfalls substituierten, gesättigten oder ungesättigten Carbo- oder Heterocyclus, der bis zu drei Heteroatome aus der Gruppe O, N oder S enthalten kann,
x 0, 1, 2, 3 oder 4,
z 0 oder 1,
W_{E} -CO-, -CO-N(R_{w}²)-, -N(R_{w}²)-CO-, -N(R_{w}²)-CO-N(R_{w}²*)-, -N(R_{w}²)-CO-O-, -O-, -S-, -SO₂-, -SO₂-N(R_{w}²)-, -SO₂-O-, -CO-O-, -O-CO-, -O-CO-N(R_{w}²)-, -N(R_{w}²)- oder -N(R_{w}²)-SO₂-,
R_{w}², R_{w}²* unabhängig voneinander Wasserstoff, einen verzweigten oder unverzweigten, gegebenenfalls substituierten C₁-C₆-Alkyl-, C₂-C₆-Alkenyl-, C₂-C₈-Alkinyl-, CO-C₁-C₆-Alkyl-, CO-O-C₁-C₆-Alkyl- oder SO₂-C₁-C₆-Alkylrest oder einen gegebenenfalls substituierten Hetaryl, Hetarylalkyl, Arylalkyl, C₃-C₇-Cycloalkyl-, CO-O-Alkylen-Aryl-, CO-Alkylen-Aryl-, CO-Aryl, SO₂-Aryl-, CO-Hetaryl- oder SO₂-Alkylen-Arylrest,
R_{E}¹⁷ Wasserstoff, eine Hydroxygruppe, CN, Halogen, einen verzweigten oder unverzweigten, gegebenenfalls substituierten C₁-C₆-Alkylrest, einen gegebenenfalls substituierten C₃-C₇-Cycloalkyl-, Aryl-, Heteroaryl oder Arylalkylrest, einen gegebenenfalls mit C₁-C₄-Alkyl oder Aryl substituierten C₂-C₆-Alkinyl- oder C₂-C₆-Alkenylrest, einen gegebenenfalls substituierten C₆-C₁₂-Bicycloalkyl-, C₁-C₆-Alkylen-C₆-C₁₂-Bicycloalkyl-, C₇-C₂₀-Tricycloalkyl- oder C₁-C₆-Alkylen-C₇-C₂₀-Tricycloalkylrest, oder einen mit bis zu drei gleichen oder verschiedenen Resten substituierten, 3- bis 8-gliedrigen, gesättigten oder ungesättigten Heterocyclus, der bis zu drei verschiedene oder gleiche Heteroatome O, N, S enthalten kann, wobei zwei Reste zusammen einen anellierten, gesättigten, ungesättigten oder aromatischen Carbocyclus oder Heterocyclus, der bis zu drei verschiedene oder gleiche Heteroatome O, N, S enthalten kann, darstellen können und der Cyclus gegebenenfalls substituiert oder an diesem Cyclus ein weiterer, gegebenenfalls substituierter, gesättigter, ungesättigter oder aromatischer Cyclus ankondensiert sein kann, oder der Rest R_{E}¹⁷ bildet zusammen mit R_{w}² oder R_{w}²* einen gesättigten oder ungesättigten C₃-C₇-Heterocyclus, der gegebenenfalls bis zu zwei weitere Heteroatome, ausgewählt aus der Gruppe O, S oder N enthalten kann,
R_{E}¹¹, R_{E}¹¹* unabhängig voneinander Wasserstoff, einen verzweigten oder unverzweigten, gegebenenfalls substituierten C₁-C₆-Alkyl-, C₁-C₆-Alkoxyalkyl-, C₂-C₆-Alkenyl-, C₂-C₁₂-Alkinyl-, CO-C₁-C₆-Alkyl-, CO-O-C₁-C₆-Alkyl-, CO-NH-C₁-C₆-Alkoxalkyl-, CO-NH-C₁-C₆-Alkyl- oder SO₂-C₁-C₆-Alkylrest oder einen gegebenenfalls substituierten Hetaryl, Arylalkyl-, C₃-C₇-Cycloalkyl-, CO-O-Alkylen-Aryl-, CO-NH-Alkylen-Aryl-, CO-Alkylen-Aryl-, CO-Aryl, CO-NH-Aryl, SO₂-Aryl-, CO-Hetaryl-, SO₂-Alkylen-Aryl-, SO₂-Hetaryl- oder SO₂-Alkylen-Hetarylrest,
R_{E}¹² Wasserstoff, einen verzweigten oder unverzweigten, gegebenenfalls substituierten C₁-C₆-Alkyl-, C₂-C₆-Alkenyl-, C₂-C₈-Alkinyl-, einen gegebenenfalls substituierten C₃-C₇-Cycloalkyl-, Hetaryl-, Arylalkyl- oder Hetarylalkyl Rest oder einen Rest CO-R_{E}¹⁶, COOR_{E}¹⁶ oder SO₂-R_{E}¹⁶,
R_{E}¹³, R_{E}¹⁴ unabhängig voneinander Wasserstoff, eine Hydroxygruppe, einen verzweigten oder unverzweigten, gegebenenfalls substituierten C₁-C₆-Alkyl-, C₁-C₄-Alkoxy-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest,
R_{E}¹⁵ Wasserstoff, einen verzweigten oder unverzweigten, gegebenenfalls substituierten C₁-C₆-Alkyl-, C₂-C₆-Alkenyl-, C₂-C₆-Alkinyl- oder Alkylen-Cycloalkylrest oder einen gegebenfalls substituierten C₃-C₇-Cycloalkyl-, Aryl-, Arylalkyl-, Hetaryl- oder Hetarylalkylrest,
R_{E}¹⁶ Wasserstoff, eine Hydroxygruppe, einen verzweigten oder unverzweigten, gegebenenfalls substituierten C₁-C₆-Alkyl-, C₂-C₆-Alkenyl-, C₂-C₆-Alkinyl- oder C₁-C₅-Alkylen-C₁-C₄-Alkoxyrest, oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-C₃-C₇-Heterocycloalkyl-, C₁-C₄-Alkylen-C₃-C₇-Heterocycloalkenyl- oder Hetarylalkylrest
bedeuten,
mit der Maßgabe, daß für den Fall
Y_{E} = CO,
k1 und k5 = 1 und
h und k6 = 0
die Summe der Indizes c, k2 und d von O verschieden sein muß
und für den Fall daß ein aromatischer oder heteroaromatischer Rest aus dem Strukturelement A direkt an Y_{E} oder Z_{E} gebunden ist, eine direkte Atombindung von Y_{E} oder Z_{E} an das Strukturelement G ausgeschlossen ist.

4. Verbindung der Formel I
B-G-L I
wobei B, G und L folgende Bedeutung haben:
L ein Strukturelement der Formel I_{L}
-U-T I_{L}
wobei
T eine Gruppe COOH, ein zu COOH hydrolisierbarer Rest oder ein zu COOH bioisosterer Rest und
-U- -(X_{L})ₐ-(CR_{L}¹R_{L}²)_{b}-, -CR_{L}¹=CR_{L}²-, Ethinylen oder =CR_{L}¹- bedeuten, wobei
a 0 oder 1,
b 0, 1 oder 2
X_{L} CR_{L}³R_{L}⁴, NR_{L}⁵, Sauerstoff oder Schwefel,
R_{L}¹, R_{L}², R_{L}³, R_{L}⁴ unabhängig voneinander Wasserstoff, -T, -OH, -NR_{L}⁶R_{L}⁷, -CO-NH₂, einen Halogenrest, einen verzweigten oder unverzweigten, gegebenenfalls substituierten C₁-C₆-Alkyl-, C₂-C₆-Alkenyl-, C₂-C₆-Alkinyl-, C₃-C₇-Cycloalkyl-, -CO-NH(C₁-C₆-Alkyl), -CO-N(C₁-C₆-Alkyl)₂ oder C₁-C₄-Alkoxyrest, einen gegebenenfalls substituierten Rest C₁-C₂-Alkylen-T, C₂-Alkenylen-T oder C₂-Alkinylen-T, einen gegebenenfalls substituierten Aryl- oder Arylalkylrest oder jeweils unabhängig voneinander zwei Reste R_{L}¹ und R_{L}² oder R_{L}3 und R_{L}⁴ oder gegebenenfalls R_{L}¹ und R_{L}³ zusammen einen, gegebenenfalls substituierten 3 bis 7 gliedrigen gesättigten oder ungesättigten Carbocyclus oder Heterocyclus, der bis zu drei verschiedene oder gleiche Heteroatome O, N, S enthalten kann,
R_{L}⁵, R_{L}⁶, R_{L}⁷ unabhängig voneinander Wasserstoff, einen verzweigten oder unverzweigten, gegebenenfalls substituierten C₁-C₆-Alkyl-, C₃-C₇-Cycloalkyl-, CO-O-C₁-C₆-Alkyl-, SO₂-C₁-C₆-Alkyl- oder CO-C₁-C₆-Alkylrest oder einen, gegebenenfalls substituierten CO-O-Alkylen-Aryl-, SO₂-Aryl-, CO-Aryl-, SO₂-Alkylen-Aryl- oder CO-Alkylen-Arylrest,
bedeuten,
G ein Strukturelement der Formel I_{G} 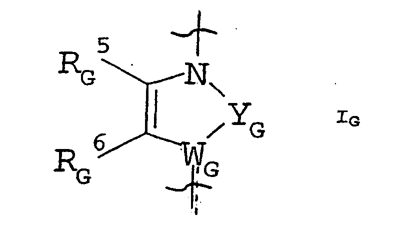 wobei
das Strukturelement B über den Ringstickstoff und das Strukturelement L über W_{G} an das Strukturelement G gebunden ist,
Y_{G} CO, CS, C=NR_{G}² oder CR_{G}³R_{G}⁴,
R_{G}² Wasserstoff, eine Hydroxy-Gruppe, einen verzweigten oder unverzweigten, gegebenenfalls substituierten C₁-C₆-Alkyl-, C₁-C₄-Alkoxy-, C₃-C₇-Cycloalkyl- oder -O-C₃-C₇-Cycloalkylrest oder einen gegebenenfalls substituierten Aryl-, -O-Aryl, Arylalkyl- oder -O-Alkylen-Arylrest,
R_{G}³, R_{G}⁴ unabhängig voneinander Wasserstoff oder einen verzweigten oder unverzweigten, gegebenenfalls substituierten C₁-C₆-Alkyl-, C₂-C₆-Alkenyl-, C₂-C₆-Alkinyl- oder C₁-C₄-Alkoxyrest oder beide Reste R_{G}³ und R_{G}⁴ zusammen ein cyclisches Acetal -O-CH₂-CH₂-O- oder -O-CH₂-O- oder beide Reste R_{G}³ und R_{G}⁴ zusammen einen, gegebenenfalls substituierten C₃-C₇-Cycloalkylrest,
R_{G}⁵ und R_{G}⁶ unabhängig voneinander Wasserstoff, eine Hydroxygruppe, einen verzweigten oder unverzweigten, gegebenenfalls substituierten C₁-C₆-Alkyl- oder C₁-C₄-Alkoxyrest, einen gegebenfalls substituierten Aryl- oder Arylalkylrest oder
beide Reste R_{G}⁵ und R_{G}⁶ zusammen einen, gegebenenfalls substituierten, anelierten, ungesättigten oder aromatischen 3- bis 10-gliedrigen Carbocyclus oder Heterocyclus, der bis zu drei verschiedene oder gleiche Heteroatome O, N, S enthalten kann,
W_{G} ein Strukturelement ausgewählt aus der Gruppe der Strukturelemente der Formeln I_{WG}¹ bis I_{WG}⁴, 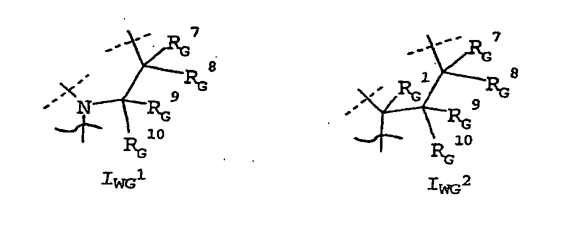 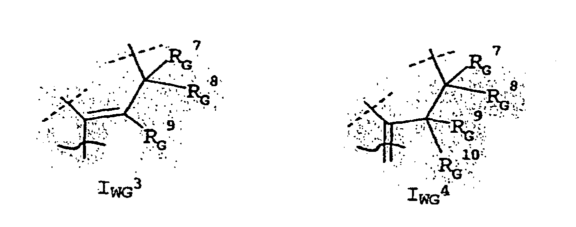
R_{G}¹ Wasserstoff, Halogen, eine Hydroxy-Gruppe oder einen verzweigten oder unverzweigten, gegebenenfalls substituierten C₁-C₆-Alkyl- oder C₁-C₄-Alkoxyrest,
R_{G}⁷, R_{G}⁸, R_{G}⁹, R_{G}¹⁰ unabhängig voneinander Wasserstoff, eine Hydroxygruppe, -CN, Halogen, einen verzweigten oder unverzweigten, gegebenenfalls substituierten C₁-C₆-Alkyl-, C₂-C₆-Alkenyl-, C₂-C₆-Alkinyl-, C₁-C₄-Alkylen-C₃-C₇-Cycloalkyl-, C₁-C₄-Alkylen-C₃-C₇-Heterocycloalkyl- oder C₁-C₄-Alkylen-C₃-C₇-Heterocycloalkenylrest, einen verzweigten oder unverzweigten, gegebenenfalls substituierten Rest C₁-C₄-Alkylen-OR_{G}¹¹, C₁-C₄-Alkylen-CO-OR_{G}¹¹, C₁-C₄-Alkylen-O-CO-R_{G}¹¹, C₁-C₄-Alkylen-CO-R_{G}¹¹, C₁-C₄-Alkylen-SO₂-NR_{G}¹²R_{G}¹³, C₁-C₄-Alkylen-CO-NR_{G}¹²R_{G}¹³, C₁-C₄-Alkylen-O-CO-NR_{G}¹²R_{G}¹³, C₁-C₄-Alkylen-NR_{G}¹²R_{G}¹³ oder C₁-C₄-Alkylen-SR_{G}¹¹, C₁-C₄-Alkylen-SO-R_{G}¹¹, einen Rest -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G}¹²R_{G}¹³, -SO₂-NR_{G}¹²R_{G}¹³, -CO-NR_{G}¹²R_{G}¹³, -NR_{G}¹²R_{G}¹³ oder CO-R_{G}¹¹, einen gegebenenfalls substituierten C₃-C₇-Cycloalkyl-, C₃-C₇-Heterocycloalkyl-, C₃-C₇-Heterocycloalkenyl-, Aryl-, Hetaryl-, Arylalkyl- oder Hetarylalkylrest oder jeweils unabhängig voneinander zwei Reste R_{G}⁷ und R_{G}⁹ oder R_{G}⁸ und R_{G}¹⁰ oder R_{G}⁷ und R_{G}⁸ oder R_{G}⁹ und R_{G}¹⁰ zusammen einen, gegebenenfalls substituierten, gesättigten oder ungesättigten, nicht aromatischen, 3 bis 7 gliedrigen Carbocyclus oder Heterocyclus der bis zu 3 Heteroatome ausgewählt aus der Gruppe O, N, S und bis zu zwei Doppelbindungen enthalten kann,
R_{G}¹¹ Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest,
R_{G}¹², R_{G}¹³ unabhängig voneinander Wasserstoff, einen verzweigten oder unverzweigten, gegebenenfalls substituierten C₁-C₈-Alkyl-, C₂-C₆-Alkenyl-, C₂-C₆-Alkinyl-, C₁-C₅-Alkylen-C₁-C₄-Alkoxy-, mono- und bis-Alkylaminoalkylen- oder Acylaminoalkylenrest oder einen, gegebenenfalls substituierten Aryl-, Heterocycloalkyl-, Heterocycloalkenyl-, Hetaryl, C₃-C₇-Cycloalkyl-, C₁-C₄-Alkylen-C₃-C₇-Cycloalkyl-, Arylalkyl-, C₁-C₄-Alkylen-Heterocycloalkyl-, C₁-C₄-Alkylen-Heterocycloalkenyl- oder Hetarylalkylrest, oder einen Rest -SO₂-R_{G}¹¹, -CO-OR_{G}¹¹, -CO-NRG¹¹RG¹¹* oder -CO-R_{G}¹¹ und
R_{G}¹¹* einen von R_{G}¹¹ unabhängigen Rest R_{G}¹¹
bedeuten,
B ein Strukturelement, enthaltend mindestens ein Atom das unter physiologischen Bedingungen als Wasserstoff-Akzeptor Wasserstoffbrücken ausbilden kann, wobei mindestens ein Wasserstoff-Akzeptor-Atom entlang des kürzestmöglichen Weges entlang des Strukturelementgerüstes einen Abstand von 5 bis 14 Atombindungen zu Strukturelement G aufweist,
sowie die physiologisch vertraglichen Salze, Prodrugs und die enantiomerenreinen oder diastereomerenreinen und tautomeren Formen, zur Anwendung als Arznermittel.

5. Verwendung einer Verbindung der Formel I wie in Anspruch 4 dargestellt, zur Herstellung eines Arzneimittels zur Behandlung von Krankheiten, bei denen die Wechselwirkung zwischen α_{V}β₃-Integrin und seinen natürlichen Liganden überhöht oder erniedrigt ist, nämlich Atherosklerose, rheumatoider Arthritis, Restenose nach Gefäßverletzung oder Stentimplantation, Angioplastie, akutem Nierenversagen, Angiogenese-assoziierte Mikroangiopathien, diabetischen Angiopathien, Blutplättchenvermitteltem vaskulärem Verschluss, arterieller Thrombose, kongestivem Herzversagen, Myokardinfarkt, Schlaganfall, Krebs, Osteoporose, Bluthochdruck, Psoriasis oder viralen, parasitären oder bakteriellen Erkrankungen, Entzündungen, Wundheilung, Hyperparathyroismus, Paget'scher Erkrankung, maligne Hypercalcemie oder metastatische osteolytische Läsionen.

6. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
Inhibitoren der Blutplättchenadhäsion, -aktivierung oder -aggregation, Antikoagulantien, die die Thrombinaktivität oder -bildung verhindern, Antagonisten von blutplättchenaktivierenden Verbindungen oder Selectin-Antagonisten.

7. Verwendung der Arzneimittelzubereitung gemäß Anspruch 6, zur Herstellung eines Arzneimittels zur Behandlung von blutplättchenvermitteltem vaskulärem Verschluss oder Thrombose.

8. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
Inhibitoren der Blutplättchenaktivierung oder -aggregation, Serin-Protease Inhibitoren, Fibrinogen-senkende Verbindungen, Selectin-Antagonisten, Antagonisten von ICAM-1 oder VCAM-1, Inhibitoren der Leukozytenadhäsion, Inhibitoren der Gefäßwandtransmigration, Fibrinolyse-modulierende Verbindungen, Inhibitoren von Komplementfaktoren, Endothelinrezeptor-Antagonisten, Tyrosinkinase-Inhibitoren, Antioxidantien oder Interleukin 8 Antagonisten.

9. Verwendung der Arzneimittelzubereitung gemäß Anspruch 8 zur Herstellung eines Arzneimittels zur Behandlung von Myokardinfarkt oder Schlaganfall.

10. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
Endothelinantagonisten, ACE-Inhibitoren, Angiotensinrezeptorantagonisten, Endopeptidase Inhibitoren, Beta-Blocker, Kalziumkanal-Antagonisten, Phosphodiesterasehemmer oder Caspaseinhibitoren.

11. Verwendung der Arzneimittelzubereitung gemäß Anspruch 10 zur Herstellung eines Arzneimittels zur Behandlung von kongestivem Herzversagen.

12. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe Thrombininhibitoren, Inhibitoren des Faktors Xa, Inhibitoren des Koagulationsweges, der zur Thrombinbildung führt, Inhibitoren der Blutplättchenadhäsion, -aktivierung oder -aggregation, Endothelinrezeptor-Antagonisten, Stickstoffoxidsynthasehemmer, CD44-Antagonisten, Selectin-Antagonisten, MCP-1-Antagonisten, Inhibitoren der Signaltransduktion in proliferierenden Zellen, Antagonisten der durch EGF, PDGF, VEGF oder bFGF vermittelten Zellantwort oder Antioxidantien.

13. Verwendung der Arzneimittelzubereitung gemäß Anspruch 12 zur Herstellung eines Arzneimittels zur Behandlung von Restenose nach Gefäßverletzung oder Stentimplantation.

14. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
Antagonisten der durch EGF, PDGF, VEGF oder bFGF vermittelten Zellantwort, Heparin oder niedermolekulare Heparine oder weitere GAGs, Inhibitoren von MMPs, Selectin-Antagonisten, Endothelin-Antagonisten, ACE-Inhibitoren, Angiotensinrezeptor-Antagonisten, Glycosilierungshemmer oder AGE-Bildungs-Inhibitoren oder AGE-Breaker und Antagonisten ihrer Rezeptoren.

15. Verwendung der Arzneimittelzubereitung gemäß Anspruch 14 zur Herstellung eines Arzneimittels zur Behandlung von diabetischen Angiopathien.

16. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
fettsenkende Verbindungen, Selectin-Antagonisten, Antagonisten von ICAM-1 oder VCAM-1, Heparin oder niedermolekulare Heparine oder weitere GAGs, Inhibitoren von MMPs, Endothelinantagonisten, Apolipoprotein A1-Antagonisten, Cholesterol-Antagonisten, HMG CoA Reduktase-Inhibitoren, ACAT Inhibitoren, ACE Inhibitoren, Angiotensinrezeptorantagonisten, Tyrosinkinaseinhibitoren, Proteinkinase C-Inhibitoren, Kalzium-Kanal-Antagonisten, LDL-Rezeptor-Funktionsstimulantien, Antioxidantien, LCAT-Mimetika oder freie Radikal-Fänger.

17. Verwendung der Arzneimittelzubereitung gemäß Anspruch 16 zur Herstellung eines Arzneimittels zur Behandlung von Atherosklerose.

18. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
cytostatische oder antineoplastische Verbindungen, Verbindungen, die die Proliferation inhibieren oder Heparin oder niedermolekulare Heparine oder weitere GAGs.

19. Verwendung der Arzneimittelzubereitung gemäß Anspruch 18 zur Herstellung eines Arzneimittels zur Behandlung von Krebs.

20. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
Verbindungen zur Anti-resorptiven Therapie, Verbindungen zur Hormon-Austausch-Therapie, Rekombinantes humanes Wachstumshormon, Bisphosphonate, Verbindungen zur Calcitonintherapie, Calcitoninstimulantien, Kalzium-Kanal-Antagonisten, Knochenbildungsstimulantien, Interleukin-6-Antagonisten oder Src Tyrosinkinase-Inhibitoren.

21. Verwendung der Arzneimittelzubereitung gemäß Anspruch 20 zur Herstellung eines Arzneimittels zur Behandlung von Osteoporose.

22. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
TNF-Antagonisten, Antagonisten von VLA-4 oder VCAM-1, Antagonisten von LFA-1, Mac-1 oder ICAMs, Komplementinhibitoren, Immunosuppressiva, Interleukin-1-, -5- oder ―8-Antagonisten oder Dihydrofolatreduktase-Inhibitoren.

23. Verwendung der Arzneimittelzubereitung gemäß Anspruch 22 zur Herstellung eines Arzneimittels zur Behandlung von rheumatoider Arthritis.

24. Arzneimittelzubereitung, enthaltend mindestens eine Verbindung, wie in Anspruch 4 dargestellt, gegebenenfalls Arzneimittelhilfsstoffe und mindestens eine weitere Verbindung, ausgewählt aus der Gruppe
Collagenase, PDGF-Antagonisten oder MMPs.

25. Verwendung der Arzneimittelzubereitung gemäß Anspruch 24 zur Herstellung eines Arzneimittels zur Verbesserung der Wundheilung.

## Claims

1. Compounds of the formula I'
A-E'-G'-L I'
wherein A, E', G' and L have the following meanings:
L denotes a structural element of the formula I_{D}
-U-T I_{D}
wherein
T denotes a group COOH, a radical that can be hydrolysed to COOH or a radical that is bioisoteric to CoOH and
-U- denotes -(X_{L})ₐ-(CR_{L}¹R_{L}²)_{b}-, -CR_{L}¹=CR_{L}²-, ethinylene or -CR_{L}¹-, wherein
a is 0 or 1,
b is 0, 1 or 2,
X_{L} denotes CR_{L}³R_{L}⁴, NR_{L}⁵, oxygen or sulfur,
R_{L}¹, R_{L}², R_{L}³, R_{L}⁴ independently of one another denote hydrogen, -T, -OH, -NR_{L}⁶R_{L}⁷, -CONH₂, a halogen radical, a branched or unbranched, optionally substituted C₁-C₆-alkyl, C₂-C₆-alkenyl, C₂-C₆-alkinyl, C₃-C₇-cycloalkyl,-CO-NH(C₁-C₆-alkyl), -CO-N(C₁-C₆-alkyl)₂ or C₁-C₄-alkoxy radical, an optionally substituted radical C₁-C₂-alkylene-T, C₂-alkenylene-T or C₂-alkinylene-T, an optionally substituted aryl or arylalkyl radical, or in each case independently of one another two radicals R_{L}¹ and R_{L}² or R_{L}³ and R_{L}⁴ or optionally R_{L}¹ and R_{L}³ together form an optionally substituted 3-membered to 7-membered saturated or unsaturated carbocycle or heterocycle, which may contain up to three different or identical heteroatoms O, N, S
R_{L}⁵, R_{L}⁶, R_{L}⁷ independently of one another denote hydrogen, a branched or unbranched, optionally substituted C₁-C₆-alkyl, C₃-C₇-cycloalkyl, CO-O-C₁-C₆-alkyl, SO₂-C₁-C₆-alkyl or CO-C₁-C₆-alkyl radical or an optionally substituted CO-O-alkylenearyl, SO₂-aryl, CO-aryl, SO₂-alkylenearyl or CO-alkylenearyl radical,
G' denotes a structural element of the formula I_{G} 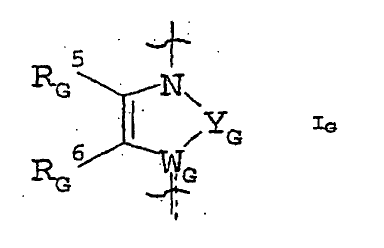
wherein
the structural element E' is bound via the ring nitrogen and the structural element L is bound via WG to the structural element G',
Y_{G} denotes CO, CS, C=NR_{G}² or CR_{G}³R_{G}⁴,
R_{G}² denotes hydrogen, an hydroxy group, a branched or unbranched, optionally substituted C₁-C₆-alkyl, C₁-C₄-alkoxy, C₃-C₇-cycloalkyl or -O-C₃-C₇-cycloalkyl radical or an optionally substituted aryl, -O-aryl, arylalkyl or -O-alkylenearyl radical,
R_{G}³, R_{G}⁴, independently of one another denote hydrogen or a branched or unbranched, optionally substituted C₁-C₆-alkyl, C₂-C₆-alkenyl, C₂-C₆-alkinyl or C₁-C₄-alkoxy radical or both radicals R_{G}³ and R_{G}⁴ together form a cyclic acetal -O-CH₂-CH₂-O- or -O-CH₂-O- or both radicals R_{G}³ and R_{G}⁴ together form an optionally substituted C₃-C₇-cycloalkyl radical,
R_{G}⁵ and R_{G}⁶ independently of one another denote hydrogen, an hydroxy group, a branched or unbranched, optionally substituted C₁-C₆-alkyl or C₁-C₄-alkoxy radical, an optionally substituted aryl or arylalkyl radical or both radicals R_{G}⁵ and R_{G}⁶ together form an optionally substituted, annellated, unsaturated or aromatic 3-membered to 10-membered carbocycle or heterocycle, which may contain up to three different or identical heteroatoms O, N, S,
W_{G} denotes a structural element selected from the group of structural elements of the formulae I_{WG}¹ to I_{WG}⁴, 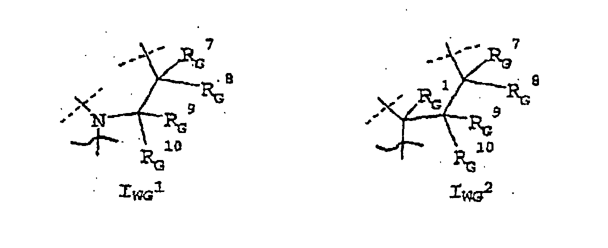 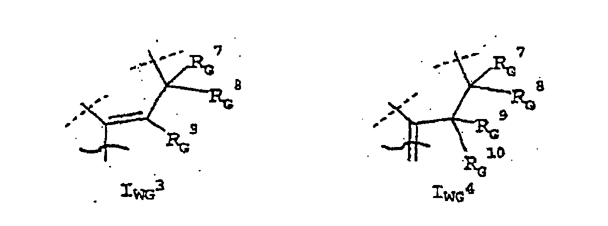
R_{G}¹ denotes hydrogen, halogen, an hydroxy group or a branched or unbranched, optionally substituted C₁-C₆-alkyl or C₁-C₄-alkoxy radical,
R_{G}⁷, R_{G}⁸, R_{G}⁹, R_{G}¹⁰ independently of one another denote hydrogen, a hydroxy group, -CN, halogen, a branched or unbranched, optionally substituted C₁-C₆-alkyl, C₂-C₆-alkenyl, C₂-C₆-alkinyl, C₁-C₄-alkylen-C₃-C₇-cycloalkyl, C₁-C₄-alkylene-C₃-C₇-heterocycloalkyl or C₁-C₄-alkylene-C₃-C₇-heterocycloalkenyl radical, a branched or unbranched, optionally substituted radical C₁-C₄-alkylene-OR_{G}¹¹, C₁-C₄-alkylene-CO-OR_{G}¹¹, C₁-C₄-alkylene-O-CO-R_{G}¹¹, C₁-C₄-alkylene-CO-R_{G}¹¹, C₂-C₄-alkylene-SO₂-NR_{G'}¹²R_{G'}¹³, C₁-C₄-alkylene-CO-NR_{G'}¹²R_{G'}¹³, C₁-C₄-alkylene-O-CO-NR_{G'}¹²R_{G'}¹³, C₁-C₄-alkylene-NR_{G},¹²R_{G'}¹³, C₁-C₄-alkylene-SR_{G}¹¹, C₁-C₄-alkylene-SO-R_{G}¹¹, a radical -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G'}¹²R_{G'}¹³, -SO₂-NR_{G'}¹²R_{G}'¹³, -CO-NR_{G'}¹²R_{G'}¹³, -NR_{G'}¹²R_{G'}¹³ or CO-R_{G}¹¹, an optionally substituted C₃-C₇-cycloalkyl, C₃-C₇-heterocycloalkyl, C₃-C₇-heterocycloalkenyl, aryl, heteroaryl, arylalkyl or heteroarylalkyl radical, or in each case independently of one another two radicals R_{G}⁷ and R_{G}⁹ or R_{G}⁸ and R_{G}¹⁰ or R_{G}⁷ and R_{G}⁸ or R_{G}⁹ and R_{G}¹⁰ together form an optionally substituted, saturated or unsaturated, non-aromatic, 3-membered to 7-membered carbocycle or heterocycle which may contain up to three heteroatoms selected from the group O, N, S and up to two double bonds,
R_{G}¹¹ denotes hydrogen, a branched or unbranched, optionally substituted C₁-C₈-alkyl, C₂-C₆-alkenyl, C₂-C₆-alkinyl, C₁-C₅-alkylene-C₁-C₄-alkoxy, mono- and bis-alkylaminoalkylene or acylaminoalkylene radical or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-heterocycloalkyl, C₁-C₄-alkylene-heterocycloalkenyl or heteroarylalkyl radical,
R_{G'}¹², R_{G'}¹³ independently of one another denote hydrogen, a branched or unbranched, optionally substituted C₁-C₈-alkyl, C₂-C₆-alkenyl, C₂-C₆-alkinyl, C₁-C₅-alkylene-C₁-C₄-alkoxy, mono- and bis-alkylaminoalkylene or acylaminoalkylene radical or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-heterocycloalkyl, C₁-C₄-alkylene-heterocycloalkenyl or heteroarylalkyl radical or a radical -SO₂-R_{G}¹¹, -CO-OR_{G}¹¹, -CO-NR_{G}¹¹R_{G}¹¹* or -CO-R_{G}¹⁴, and
R_{G}¹¹* denotes a radical R_{G}¹¹ independent of R_{G}¹¹
R_{G'}¹⁴ denotes hydrogen, a branched or unbranched, optionally substituted C₁-C₈-alkyl, C₂-C₆-alkenyl, C₂-C₆-alkinyl or C₁-C₅-alkylene-C₁-C₄-alkoxy radical or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-heterocycloalkyl, C₁-C₄-alkylene-heterocycloalkenyl or heteroarylalkyl radical,
E' denotes a structural element composed of two to four partial structural elements selected from the group E¹ and E², in which the sequence of the coupling of the partial structural elements is arbitrary and E¹ and E² have the following meanings:
E¹ denotes a partial structural element of the formula I_{E1}
-(Y_{E})ₖ₁-(CR_{E}¹R_{E}²)_{c}-(Q_{E})ₖ₂- (CR_{E}³R_{E}⁴)_{d}- I_{E1}
and
E² denotes a partial structural element of the formula I_{E2}
NR_{E}¹¹) ₖ₃ - (CR_{E}⁵R_{E}⁶) _{f} - (Z_{E}) ₖ₄ - (CR_{E}⁷R_{E}⁸) _{g} - (X_{E}) ₖ₅ - (CR_{E}⁹R_{E}¹⁰) ₕ- (NR_{E}¹¹*) ₖ₆- I_{E2},
wherein
c, d, f, g, h independently of one another denote 0, 1 or 2,
k1, k2, k3, k4, k5, k6 independently of one another denote 0 or 1,
X_{E}, Q_{E} independently of one another denote an optionally substituted 4-membered to 11-membered monocyclic or polycyclic, aliphatic or aromatic hydrocarbon which may contain up to 6 double bonds and up to 6 identical or different heteroatoms selected from the group N, O or S, wherein the ring carbons and/or the ring nitrogens may optionally be substituted,
Y_{E}, Z_{E} independently of one another denote CO, CO-NR_{E}¹², NR_{E}¹²-CO, sulfur, SO, SO₂, SO₂-NR_{E}¹², NR_{E}¹²-SO₂, CS, CS-NR_{E}¹², NR_{E}¹²-CS, CS-O, O-CS, CO-O, O-CO, oxygen, ethinylene, CR_{E}¹³-O-CR_{E}¹⁴, C (=CR_{E}¹³R_{E}¹⁴), CR_{E}¹³=CR_{E}¹⁴, -CR_{E}¹³(OR_{E}¹⁵) -CHR_{E}¹⁴- or -CHR-_{E}¹³-CR_{E}¹⁴(OR_{E}¹⁵)-,
R_{E}¹,R_{E}², R_{E}³, R_{E}⁴, R_{E}⁵, R_{E}⁶, R_{E}⁷, R_{E}⁸, R_{E}⁹, R_{E}¹⁰ independently of one another denote hydrogen, halogen, an hydroxy group, a branched or unbranched, optionally substituted C₁-C₆-alkyl, C₂-C₆-alkenyl, C₂-C₆-alkinyl or alkylenecycloalkyl radical, a radical -(CH₂)ₓ-(W_{E})_{Z}-R_{E}¹⁷, an optionally substituted C₃-C₇-cycloalkyl, aryl, arylalkyl, heteroaryl or heteroarylalkyl radical, or independently of one another in each case two radicals R_{E}¹ and R_{E}² or R_{E}³ and R_{E}⁴ or R_{E}⁵ and R_{E}⁶ or R_{E}⁷ and R_{E}⁸ or R_{E}⁹ and R_{E}¹⁰ together form a 3-membered to 7-membered, optionally substituted, saturated or unsaturated carbocycle or heterocycle, which may contain up to three heteroatoms from the group O, N or S,
x is 0, 1, 2, 3 or 4,
z is 0 or 1,
W_{E} denotes -CO-, -CO-N(R_{w}²)-, -N(R_{w}²) -CO-, -N(R_{w}²)-CO-N(R_{w}²*)-, -N(R_{w}²) -CO-O-, -O-, -S-, -SO₂- -SO₂-N(R_{w}²)-, -SO₂-O-, -CO-O-, -O-CO-, -O-CO-N(R_{w}²)-, -N(R_{w}²)- or -N(R_{w}²)-SO₂-,
R_{w}², R_{w}²* independently of one another denote hydrogen, a branched or unbranched, optionally substituted C₁-C₆-alkyl, C₂-C₆-alkenyl, C₂-C₈-alkinyl, CO-C₁-C₆-alkyl, CO-O-C₁-C₆-alkyl or SO₂-C₁-C₆-alkyl radical or an optionally substituted heteroaryl, heteroarylalkyl, arylalkyl, C₃-C₇-cycloalkyl, CO-O-alkylenearyl, CO-alkylenearyl, CO-aryl, SO₂-aryl, CO-heteroaryl or SO₂-alkylenearyl radical,
R_{E}¹⁷ denotes hydrogen, an hydroxy group, CN, halogen, a branched or unbranched, optionally substituted C₁-C₆-alkyl radical, an optionally substituted C₃-C₇-cycloalkyl, aryl, heteroaryl or arylalkyl radical, a C₂-C₆-alkinyl radical or C₂-C₆-alkenyl radical optionally substituted with C₁-C₄-alkyl or aryl, an optionally substituted C₆-C₁₂-bicycloalkyl, C₁-C₆-alkylene-C₆-C₁₂-bicycloalkyl, C₇-C₂₀-tricycloalkyl or C₁-C₆-alkylene-C₇-C₂₀-tricycloalkyl radical, or a 3-membered to 8-membered, saturated or unsaturated heterocycle substituted with up to three identical or different radicals, which heterocycle may contain up to three different or identical heteroatoms O, N, S, wherein two radicals may together form an annellated, saturated, unsaturated or aromatic carbocycle or heterocycle that may contain up to three different or identical heteroatoms O, N, S, and the cycle may optionally be substituted or a further, optionally substituted, saturated, unsaturated or aromatic cycle may be condensed thereon, or the radical R_{E}¹⁷ forms together with R_{W}² or R_{W}²* a saturated or unsaturated C₃-C₇-heterocycle, which may optionally contain up to two further heteroatoms selected from the group O, S or N,
R_{E}¹¹, R_{E}¹¹* independently of one another denote hydrogen, a branched or unbranched, optionally substituted C₁-C₆-alkyl, C₁-C₆-alkoxyalkyl, C₂-C₆-alkenyl, C₂-C₁₂-alkinyl, CO-C₁-C₆-alkyl, CO-O-C₁-C₆-alkyl, CO-NH-C₁-C₆-alkoxyalkyl, CO-NH-C₁-C₆-alkyl or SO₂-C₁-C₆-alkyl radical or an optionally substituted heteroaryl, arylalkyl, C₃-C₇-cycloalkyl, CO-O-alkylenearyl, CO-NH-alkylenearyl, CO-alkylenearyl, CO-aryl, CO-NH-aryl, SO₂-aryl, CO-heteroaryl, SO₂-alkylenearyl, SO₂-heteroaryl or SO₂-alkyleneheteroaryl radical,
R_{E}¹² denotes hydrogen, a branched or unbranched, optionally substituted C₁-C₆-alkyl, C₂-C₆-alkenyl, C₂-C₈-alkinyl, an optionally substituted C₃-C₇-cycloalkyl, heteroaryl, arylalkyl or heteroalkyl radical or a radical CO-R_{E}¹⁶, COOR_{E}¹⁶ or SO₂-R_{E}¹⁶,
R_{E}¹³, R_{E}¹⁴ independently of one another denote hydrogen, an hydroxy group, a branched or unbranched, optionally substituted C₁-C₆-alkyl, C₁-C₄-alkoxy, C₂-C₆-alkenyl, C₂-C₆-alkinyl or alkylenecycloalkyl radical or an optionally substituted C₃-C₇-cycloalkyl, aryl, arylalkyl, heteroaryl or heteroarylalkyl radical,
R_{E}¹⁵ denotes hydrogen, a branched or unbranched, optionally substituted C₁-C₆-alkyl, C₂-C₆-alkenyl, C₂-C₆-alkinyl or alkylenecycloalkyl radical or an optionally substituted C₃-C₇-cycloalkyl, aryl, arylalkyl, heteroaryl or heteroarylalkyl radical,
R_{E}¹⁶ denotes hydrogen, an hydroxy group, a branched or unbranched, optionally substituted C₁-C₆-alkyl, C₂-C₆-alkenyl, C₂-C₆-alkinyl or C₁-C₅-alkylene-C₁-C₄-alkoxy radical, or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-C₃-C₇-heterocycloalkyl, C₁-C₄-alkylene-C₃-C₇-heterocycloalkenyl or heteroarylalkyl radical,
with the proviso that, in the case where
Y_{E} or Z_{E} denote CO and a radical X_{E} or Q_{E} or an aromatic or heteroaromatic radical from the structural element A is directly bonded to Y_{E} or Z_{E}, a direct atomic bonding of Y_{E} or Z_{E} to the structural element G' is excluded,
A denotes a structural element selected from the group:
a 4-membered to 8-membered monocyclic saturated, unsaturated or aromatic hydrocarbon, which may contain up to 4 heteroatoms selected from the group O, N or S,
wherein in each case independently of one another the optionally contained ring nitrogen or the carbons may be substituted, with the proviso that at least one heteroatom selected from the group O, N or S is contained in the structural element A,
or
a 9-membered to 14-membered polycyclic, saturated, unsaturated or aromatic hydrocarbon, which may contain up to 6 heteroatoms selected from the group N, O or S,
wherein in each case independently of one another the optionally contained ring nitrogen or the carbons may be substituted, with the proviso that at least one heteroatom selected from the group O, N or S is contained in the structural element A,
a radical 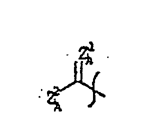 wherein
Z_{A}¹ denotes oxygen, sulfur or optionally substituted nitrogen, and
Z_{A}² denotes optionally substituted nitrogen, oxygen or sulfur,
or a radical 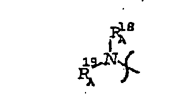 wherein
R_{A}¹⁸, R_{A}¹⁹ independently of one another denote hydrogen, a branched or unbranched, optionally substituted C₁-C₈-alkyl, C₂-C₆-alkenyl, C₂-C₆-alkinyl, C₁-C₅-alkylene-C₁-C₄-alkoxy, mono- and bis- alkylaminoalkylene or acylaminoalkylene radical, or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkyleneheterocycloalkyl, C₁-C₄-alkyleneheterocycloalkenyl or heteroarylalkyl radical, or a radical -SO₂-R_{G}¹¹, -CO-OR_{G}¹¹, -CO-NR_{G}¹¹R_{G}¹¹* or -CO-R_{G}¹¹,
as well as the physiologically compatible salts, prodrugs and the enantiomer-pure or diastereomer-pure and tautomeric forms.

2. Compound according to claim 1, **characterised in that** as structural element A there is used a structural element selected from the group of structural elements of the formulae I_{A}¹ to I_{A}¹⁸ 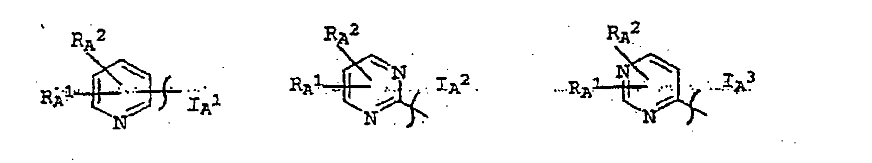 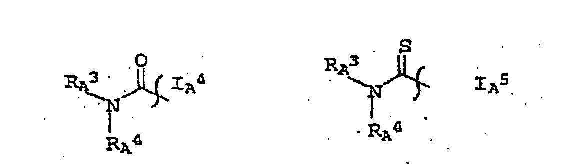 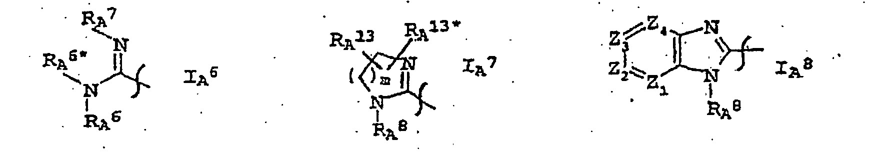 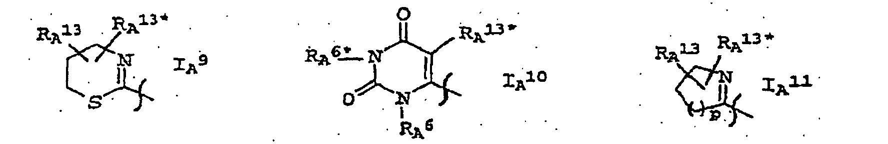 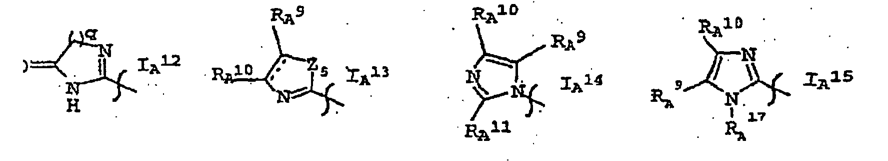 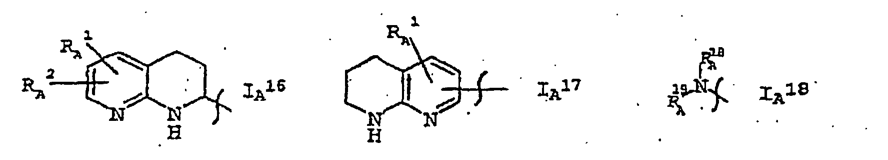 wherein
m, p, q independently of one another denote 1, 2 or 3,
R_{A}¹, R_{A}² independently of one another denote hydrogen, CN, halogen, a branched or unbranched, optionally substituted C₁-C₆-alkyl or CO-C₁-C₆-alkyl radical or an optionally substituted aryl, arylalkyl, heteroaryl, heteroarylalkyl or C₃-C₇-cycloalkyl radical or a radical CO-O-R_{A}¹⁴, O-R_{A}¹⁴, S-R_{A}¹⁴, NR_{A}¹⁵R_{A}¹⁶, CO-NR_{A}¹⁵R_{A}¹⁶ or SO₂NR_{A}¹⁵R_{A}¹⁶ or both radicals R_{A}¹ and R_{A}² together form an annellated, optionally substituted, 5-membered or 6-membered, unsaturated or aromatic carbocycle or heterocycle, which may contain up to three heteroatoms selected from the group O, N or S,
R_{A}¹³, R_{A}¹³*, independently of one another denote hydrogen, CN, halogen, a branched or unbranched, optionally substituted C₁-C₆-alkyl radical or an optionally substituted aryl, arylalkyl, heteroaryl, C₃-C₇-cycloalkyl radical or a radical CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ or CO-NR_{A}¹⁵R_{A}¹⁶,
wherein
R_{A}¹⁴ denotes hydrogen, a branched or unbranched, optionally substituted C₁-C₆-alkyl, alkylene-C₁-C₄-alkoxy, C₂-C₆-alkenyl, C₂-C₆-alkinyl or C₁-C₆-alkylene-C₃-C₇-cycloalkyl radical or an optionally substituted C₃-C₇-cycloalkyl, aryl, arylalkyl, heteroaryl or heteroarylalkyl radical,
R_{A}¹⁵, R_{A}¹⁶ independently of one another denote hydrogen, a branched or unbranched, optionally substituted C₁-C₆-alkyl, CO-C₁-C₆-alkyl, SO₂-C₁-C₆-alkyl, COO-C₁-C₆-alkyl, CO-NH-C₁-C₆-alkyl, arylalkyl, COO-alkylenearyl, SO₂-alkylenearyl, CO-NH-alkylenearyl, CO-NH-alkyleneheteroaryl or heteroarylalkyl radical or an optionally substituted C₃-C₇-cycloalkyl, aryl, CO-aryl, CO-NH-aryl, SO₂-aryl, heteroaryl, CO-NH-heteroaryl, or CO-heteroaryl radical,
R_{A}³, R_{A}⁴ independently of one another denote hydrogen, -(CH₂)ₙ-(X_{A})ⱼ-R_{A}¹², or both radicals together form a 3-membered to 8-membered, saturated, unsaturated or aromatic N-heterocycle which may in addition contain two further, identical or different heteroatoms O, N or S, wherein the cycle may optionally be substituted or a further, optionally substituted, saturated, unsaturated or aromatic cycle may be condensed onto this cycle,
wherein
n is 0, 1, 2 or 3,
j is 0 or 1,
X_{A} denotes -CO-, -CO-N(R_{X}¹)-, -N(R_{X}¹)-CO-, -N(R_{X}¹)-CO-N(R_{X}¹*)-, N(R_{X}¹)-CO-O-, -O-, -S-, -SO₂-, -SO₂-N(R_{X}¹)-, -SO₂-O-, -CO-O-, -O-CO-, -O-CO-N(R_{X}¹)-, -N(R_{X}¹)- or -N(R_{X}¹)-SO₂-,
R_{A}¹² denotes hydrogen, a branched or unbranched, optionally substituted C₁-C₆-alkyl radical, a C₂-C₆-alkinyl or C₂-C₆-alkenyl radical optionally substituted with C₁-C₄-alkyl or aryl, or a 3-membered to 6-membered, saturated or unsaturated heterocycle substituted with up to three identical or different radicals, which heterocycle may contain up to three different or identical heteroatoms O, N, S, a C₃-C₇-cycloalkyl, aryl or heteroaryl radical, wherein two radicals may together form an annellated, saturated, unsaturated or aromatic carbocycle or heterocycle that may contain up to three different or identical heteroatoms O, N, S, and the cycle may optionally be substituted or a further, optionally substituted, saturated, unsaturated or aromatic cycle may be condensed onto this cycle, or the radical R_{A}¹² forms together with R_{X}¹ or R_{X}¹* a saturated or unsaturated C₃-C₇-heterocycle, which may optionally contain up to two further heteroatoms selected from the group O, S or N,
R_{X}¹, R_{X}^{1*} independently of one another denote hydrogen, a branched or unbranched, optionally substituted C₁-C₆-alkyl, C₁-C₆-alkoxyalkyl, C₂-C₆-alkenyl, C₂-C₁₂-alkinyl, CO-C₁-C₆-alkyl, CO-O-C₁-C₆-alkyl or SO₂-C₁-C₆-alkyl radical or an optionally substituted C₃-C₇-cycloalkyl, aryl, arylalkyl, CO-O-alkylenearyl, CO-alkylenearyl, CO-aryl, SO₂-aryl, heteroaryl, CO-heteroaryl or SO₂-alkylenearyl radical,
R_{A}⁶, R_{A}⁶* denote hydrogen, a branched or unbranched, optionally substituted C₁-C₄-alkyl, -CO-O-C₁-C₄-alkyl, arylalkyl, -CO-O-alkylenearyl, -CO-O-allyl, -CO-C₁-C₄-alkyl, -CO-alkylenearyl, C₃-C₇-cycloalkyl or -CO-allyl radical, or in the structural element I_{A}⁷ both radicals R_{A}⁶ and R_{A}⁶* together form an optionally substituted, saturated, unsaturated or aromatic heterocycle that may additionally contain on the ring nitrogen up to two further different or identical heteroatoms O, N, S,
R_{A}⁷ denotes hydrogen, -OH, -CN, -CONH₂, a branched or unbranched, optionally substituted C₁-C₄-alkyl, C₁-C₄-alkoxy, C₃-C₇-cycloalkyl or -O-CO-C₁-C₄-alkyl radical, or an optionally substituted arylalkyl, -O-alkylenearyl, -O-CO-aryl, O-CO-alkylenearyl or -O-CO-allyl radical, or both radicals R_{A}⁶ and R_{A}⁷ together form an optionally substituted, unsaturated or aromatic heterocycle that may additionally contain on the ring nitrogen up to two further different or identical heteroatoms O, N, S,
R_{A}⁸ denotes hydrogen, a branched or unbranched, optionally substituted C₁-C₄-alkyl, CO-C₁-C₄-alkyl, SO₂-C₁-C₄-alkyl or CO-O-C₁-C₄-alkyl radical or an optionally substituted aryl, CO-aryl, SO₂-aryl, CO-O-aryl, CO-alkylenearyl, SO₂-alkylenearyl, CO-O-alkylenearyl or alkylenearyl radical,
R_{A}⁹, R_{A}¹⁰ independently of one another denote hydrogen, -CN, halogen, a branched or unbranched, optionally substituted C₁-C₆-alkyl radical or an optionally substituted aryl, arylalkyl, heteroaryl, C₃-C₇-cycloalkyl radical or a radical CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ or CO-NR_{A}¹⁵R_{A}¹⁶, or both radicals R_{A}⁹ and R_{A}¹⁰ together in the structural element I_{A}¹⁴ form a 5-membered to 7-membered saturated, unsaturated or aromatic carbocycle or heterocycle that may contain up to three different or identical heteroatoms O, N, S and may optionally be substituted with up to three identical or different radicals,
R_{A}¹¹ denotes hydrogen, -CN, halogen, a branched or unbranched, optionally substituted C₁-C₆-alkyl radical or an optionally substituted aryl, arylalkyl, heteroaryl, C₃-C₇-cycloalkyl radical or a radical CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ or CO-NR_{A}¹⁵R_{A}¹⁶,
R_{A}¹⁷ denotes hydrogen, or in the structural element I_{A}¹⁵ both radicals R_{A}⁹ and R_{A}¹⁷ together form a 5-membered to 7-membered saturated, unsaturated or aromatic heterocycle, which may additionally contain on the ring nitrogen up to three different or identical heteroatoms O, N, S and may optionally be substituted with up to three identical or different radicals,
R_{A}¹⁸, R_{A}¹⁹ independently of one another denote hydrogen, a branched or unbranched, optionally substituted C₁-C₈-alkyl, C₂-C₆-alkenyl, C₂-C₆-alkinyl, C₁-C₅-alkylene-C₁-C₄-alkoxy, mono- and bis-alklyaminoalkylene or acylaminoalkylene radical or an optionally substitute aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkyleneheterocycloalkyl, C₁-C₄-alkyleneheterocycloalkenyl or heteroarylalkyl radical, or a radical -SO₂-R_{G}¹¹, -CO-OR_{G}¹¹, -CO-NR_{G}¹¹R_{G}¹¹* or -CO-R_{G}¹¹ independent of R_{G}¹¹,
Z¹, Z², Z³, Z⁴ independently of one another denote nitrogen, C-H, C-halogen or a branched or unbranched, optionally substituted C-C₁-C₄-alkyl or C-C₁-C₄-alkoxy radical,
Z⁵ denotes NR_{A}⁸, oxygen or sulfur.

3. Compound according to claim 1 or 2, **characterised in that** as structural element E' there is used a structural elemenc of the formula I_{E1E2}
-E₂-E₁- I_{E1E2}
and E¹ and E² have the following meanings:
E¹ is a partial structural element of the formula I_{E1}
-(Y_{E})ₖ₁-(CR_{E}¹R_{E}²)_{c}-(Q_{E})ₖ₂-(CR_{E}³R_{E}⁴)_{d}- I_{E1}
and
E² is a partial structural element of the formula I_{E2}
- (NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}⁻¹⁰)ₕ- (NR_{E}¹¹*)ₖ₆- I_{E2},
wherein
c, d, f, g, h independently of one another denote 0, 1 or 2,
k1, k2, k3, k4, k5, k6 independently of one another denote 0 or 1,
X_{E}, Q_{E} independently of one another denote an optionally substituted 4-membered to 11-membered monocyclic or polycyclic, aliphatic or aromatic hydrocarbon which may contain up to 6 double bonds and up to 6 identical or different heteroatoms selected from the group N, O or S, wherein the ring carbons and/or the ring nitrogens may optionally be substituted,
Y_{E}, Z_{E} independently of one another denote CO, CO-NR_{E}¹², NR_{E}¹²-CO, sulfur, SO, SO₂, SO₂-NR_{E}¹², NR_{E}¹²-SO₂, CS, CS-NR_{E}¹², NR_{E}¹²-CS, CS-O, O-CS, CO-O, O-CO, oxygen, ethinylene, CR_{E}¹³-O-CR_{E}¹⁴, C(=CR_{E}¹³R_{E}¹⁴), CR_{E}¹³=CR_{E}¹⁴, -CR_{E}¹³ (OR_{E}¹⁵) -CHR_{E}¹⁴- or -CHR-_{E}¹³-CR_{E}¹⁴ (OR_{E}¹⁵)-,
R_{E}¹, R_{E}², R_{E}³, R_{E}⁴, R_{E}⁵, R_{E}⁶, R_{E}⁷, R_{E}⁸, R_{E}⁹, R_{E}¹⁰ independently of one another denote hydrogen, halogen, an hydroxy group, a branched or unbranched, optionally substituted C₁-C₆-alkyl, C₂-C₆-alkenyl, C₂-C₆-alkinyl or alkylenecycloalkyl radical, a radical -(CH₂)ₓ-(W_{E})_{Z}-R_{E}¹⁷, an optionally substituted C₃-C₇-cycloalkyl, aryl, arylalkyl, heteroaryl or heteroarylalkyl radical, or independently of one another in each case two radicals R_{E}¹ and R_{E}² or R_{E}³ and R_{E}⁴ or R_{E}⁵ and R_{E}⁶ or R_{E}⁷ and R_{E}⁸ or R_{E}⁹ and R_{E}¹⁰ together form a 3-membered to 7-membered, optionally substituted, saturated or unsaturated carbocycle or heterocycle, which may contain up to 3 heteroatoms from the group O, N or S,
x is 0, 1, 2, 3 or 4,
z is 0 or 1,
W_{E} denotes -CO-, -CO-N(R_{W}²)-, -N(R_{W}²)-CO-, -N(R_{W}²)-CO-N(R_{W}²*)-, -N(R_{W}²)-CO-O-, -O-, -S-, -SO₂- -SO₂-N(R_{W}²)-, -SO₂-O-, -CO-O-, -O-CO-, -O-CO-N(R_{W}²)-, -N(R_{W}²)- or -N(R_{W}²) -SO₂-,
R_{W}², R_{W}²* independently of one another denote hydrogen, a branched or unbranched, optionally substituted C₁-C₆-alkyl, C₂-C₆-alkenyl, C₂-C₈-alkinyl, CO-C₁-C₆-alkyl, CO-O-C₁-C₆-alkyl or SO₂-C₁-C₆-alkyl radical or an optionally substituted heteroaryl, heteroarylalkyl, arylalkyl, C₃-C₇-cycloalkyl, CO-O-alkylenearyl, CO-alkylenearyl, CO-aryl, SO₂-aryl, CO-heteroaryl or SO₂-alkylenearyl radical,
R_{E}¹⁷ denotes hydrogen, an hydroxy group, CN, halogen, a branched or unbranched, optionally substituted C₁-C₆-alkyl radical, an optionally substituted C₃-C₇-cycloalkyl, aryl, heteroaryl or arylalkyl radical, a C₂-C₆-alkinyl or C₂-C₆-alkenyl radical optionally substituted with C₁-C₄-alkyl or aryl, an optionally substituted C₆-C₁₂-bicycloalkyl, C₁-C₆-alkylene-C₆-C₁₂-bicycloalkyl, C₇-C₂₀-tricycloalkyl or C₁-C₆-alkylene-C₇-C₂₀-tricycloalkyl radical, or a 3-membered to 8-membered, saturated or unsaturated heterocycle substituted with up to three identical or different radicals, which heterocycle may contain up to three different or identical heteroatoms O, N, S, wherein two radicals may together form an annellated, saturated, unsaturated or aromatic carbocycle or heterocycle that may contain up to three different or identical heteroatoms O, N, S, and the cycle may optionally be substituted or a further, optionally substituted, saturated, unsaturated or aromatic cycle may be condensed thereon, or the radical R_{E}¹⁷ forms together with R_{W}² or R_{W}²* a saturated or unsaturated C₃-C₇-heterocycle, which may optionally contain up to two further heteroatoms selected from the group O, S or N,
R_{E}¹¹, R_{E}¹¹* independently of one another denote hydrogen, a branched or unbranched, optionally substituted C₁-C₆-alkyl, C₁-C₆-alkoxyalkyl, C₂-C₆-alkenyl, C₂-C₁₂-alkinyl, CO-C₁-C₆-alkyl, CO-O-C₁-C₆-alkyl, CO-NH-C₁-C₆-alkoxyalkyl, CO-NH-C₁-C₆-alkyl or SO₂-C₁-C₆-alkyl radical or an optionally substituted heteroaryl, arylalkyl, C₃-C₇-cycloalkyl, CO-O-alkylenearyl, CO-NH-alkylenearyl, CO-alkylenearyl, CO-aryl, CO-NH-aryl, SO₂-aryl, CO-heteroaryl, SO₂-alkylenearyl, SO₂-heteroaryl or SO₂-alkyleneheteroaryl radical,
R_{E}¹² denotes hydrogen, a branched or unbranched, optionally substituted C₁-C₆-alkyl, C₂-C₆-alkenyl, C₂-C₆-alkinyl, an optionally substituted C₃-C₇-cycloalkyl, heteroaryl, arylalkyl or heteroarylalkyl radical or a radical CO-R_{E}¹⁶, COOR_{E}¹⁶ or SO₂-R_{E}¹⁶,
R_{E}¹³, R_{E}¹⁴ independently of one another denote hydrogen, an hydroxy group, a branched or unbranched, optionally substituted C₁-C₆-alkyl, C₁-C₄-alkoxy, C₂-C₆-alkenyl, C₂-C₆-alkinyl or alkylenecycloalkyl radical or an optionally substituted C₃-C₇-cycloalkyl, aryl, arylalkyl, heteroaryl or heteroarylalkyl radical,
R_{E}¹⁵ denotes hydrogen, a branched or unbranched, optionally substituted C₁-C₆-alkyl, C₂-C₆-alkenyl, C₂-C₆-alkinyl or alkylenecycloalkyl radical or an optionally substituted C₃-C₇-cycloalkyl, aryl, arylalkyl, heteroaryl or heteroarylalkyl radical,
R_{E}¹⁶ denotes hydrogen, an hydroxy group, a branched or unbranched, optionally substituted C₁-C₆-alkyl, C₂-C₆-alkenyl, C₂-C₆-alkinyl or C₁-C₅-alkylene-C₁-C₄-alkoxy radical, or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-C₃-C₇-heterocycloalkyl, C₁-C₄-alkylene-C₃-C₇-heterocycloalkenyl or heteroarylalkyl radical,
with the proviso that, in the case where
Y_{E} = CO,
k1 and k5 = 1 and
h and k6 = 0
the sum of the indices c, k2 and d must be different from 0,
and in the case where an aromatic or heteroaromatic radical from the structural element A is directly bonded to Y_{E} or Z_{E}, a direct atomic bonding of Y_{E} or Z_{E} to the structural element G is excluded.

4. Compound of the formula I
B-G-L I
wherein B, G and L have the following meanings:
L is a structural unit of the formula I_{L}
-U-T I_{L}
wherein
T denotes a group COOH, a radical that can be hydrolysed to COOH or a radical that is bioisoteric to COOH and
-U- denotes -(X_{L})ₐ-(CR_{L}¹R_{L}²)_{b}-, -CR_{L}¹=CR_{L}²-, ethinylene or -CR_{L}¹-, wherein
a is 0 or 1,
b is 0, 1 or 2,
X_{L} denotes CR_{L}³R_{L}⁴, NR_{L}⁵, oxygen or sulfur,
R_{L}¹, R_{L}², R_{L}³, R_{L}⁴ independently of one another denote hydrogen, -T, -OH, -NR_{L}⁶R_{L}⁷, -CO-NH₂, a halogen radical, a branched or unbranched, optionally substituted C₁-C₆-alkyl, C₂-C₆-alkenyl, C₂-C₆-alkinyl, C₃-C₇-cycloalkyl, -CO-NH(C₁-C₆-alkyl), -CO-N(C₁-C₆-alkyl)₂ or C₁-C₄-alkoxy radical, an optionally substituted radical C₁-C₂-alkylene-T, C₂-alkenylene-T or C₂-alkinylene-T, an optionally substituted aryl or arylalkyl radical, or in each case independently of one another two radicals R_{L}¹ and R_{L}² or R_{L}³ and R_{L}⁴ or optionally R_{L}¹ and R_{L}³ together form an optionally substituted 3-membered to 7-membered saturated or unsaturated carbocycle or heterocycle, which may contain up to 3 different or identical heteroatoms O, N, S
R_{L}⁵, R_{L}⁶, R_{L}⁷ independently of one another denote hydrogen, a branched or unbranched, optionally substituted C₁-C₆-alkyl, C₃-C₇-cycloalkyl, CO-O-C₁-C₆-alkyl, SO₂-C₁-C₆-alkyl or CO-C₁-C₆-alkyl radical or an optionally substituted CO-O-alkylenearyl, SO₂-aryl, CO-aryl, SO₂-alkylenearyl or CO-alkylenearyl radical,
G denotes a structural element of the formula I_{G} 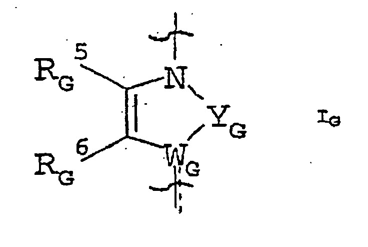
wherein
the structural element B is bound via the ring nitrogen and the structural element L is bound via W_{G} to the structural element G,
Y_{G} denotes CO, CS, C=NR_{G}² or CR_{G}³R_{G}⁴,
R_{G}² denotes hydrogen, an hydroxy group, a branched or unbranched, optionally substituted C₁-C₆-alkyl, C₁-C₄-alkoxy, C₃-C₇-cycloalkyl or -O-C₃-C₇-cycloalkyl radical or an optionally substituted aryl, -O-aryl, arylalkyl or -O-alkylenearyl radical,
R_{G}³, R_{G}⁴, independently of one another denote hydrogen or a branched or unbranched, optionally substituted C₁-C₆-alkyl, C₂-C₆-alkenyl, C₂-C₆-alkinyl or C₁-C₄-alkoxy radical or both radicals R_{G}³ and R_{G}⁴ together form a cyclic acetal -O-CH₂-CH₂-O- or -O-CH₂-O- or both radicals R_{G}³ and R_{G}⁴ together form an optionally substituted C₃-C₇-cycloalkyl radical,
R_{G}⁵ and R_{G}⁶ independently of one another denote hydrogen, an hydroxy group, a branched or unbranched, optionally substituted C₁-C₆-alkyl or C₁-C₄-alkoxy radical, an optionally substituted aryl or arylalkyl radical or
both radicals R_{G}⁵ and R_{G}⁶ together form an optionally substituted, annellated,
unsaturated or aromatic 3-membered to 10-membered carbocycle or heterocycle, which may contain up to three different or identical heteroatoms O, N, S,
W_{G} denotes a structural element selected from the group of structural elements of the formulae I_{WG}¹ to I_{WG}⁴, 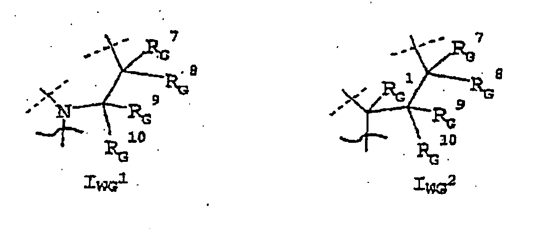 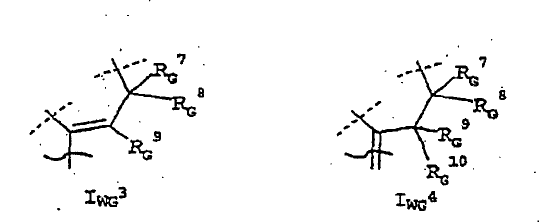
R_{G}¹ denotes hydrogen, halogen, an hydroxy group or a branched or unbranched, optionally substituted C₁-C₆-alkyl or C₁-C₄-alkoxy radical,
R_{G}⁷, R_{G}⁸, R_{G}⁹, R_{G}¹⁰ independently of one another denote hydrogen, a hydroxy group, -CN, halogen, a branched or unbranched, optionally substituted C₁-C₆-alkyl, C₂-C₆-alkenyl, C₂-C₆-alkinyl, C₁-C₄-alkylen-C₃-C₇-cycloalkyl, C₁-C₄-alkylene-C₃-C₇-heterocycloalkyl or C₁-C₄-alkylene-C₃-C₇-heterocycloalkenyl radical, a branched or unbranched, optionally substituted radical C₁-C₄-alkylene-OR_{G}¹¹, C₁-C₄-alkylene-CO-OR_{G}¹¹, C₁-C₄-alkylene-O-CO-R_{G}¹¹, C₁-C₄-alkylene-CO-R_{G}¹¹, C₁-C₄-alkylene-SO₂-NR_{G}¹²R_{G}¹³, C₁-C₄-alkylene-CO-NR_{G}¹²R_{G}¹³, C₁-C₄-alkylene-O-CO-NR_{G}¹²R_{G}¹³, C₁-C₄-alkylene-NR_{G}¹²R_{G}¹³, C₁-C₄-alkylene-SR_{G}¹¹, C₁-C₄-alkylene-SO-R_{G}¹¹, a radical -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G}¹²R_{G}¹³, -SO₂-NR_{G}¹²R_{G}¹³, -CO-NR_{G}¹²R_{G}¹³, -NR_{G}¹²R_{G}¹³ or CO-R_{G}¹¹, an optionally substituted C₃-C₇-cycloalkyl, C₃-C₇-heterocycloalkyl, C₃-C₇-heterocycloalkenyl, aryl, heteroaryl, arylalkyl or heteroarylalkyl radical, or in each case independently of one another two radicals R_{G}⁷ and R_{G}⁹ or R_{G}⁸ and R_{G}¹⁰ or R_{G}⁷ and R_{G}⁸ or R_{G}⁹ and R_{G}¹⁰ together form an optionally substituted, saturated or unsaturated, non-aromatic, 3-membered to 7-membered carbocycle or heterocycle which may contain up to three heteroatoms selected from the group O, N, S and up to two double bonds,
R_{G}¹¹ denotes hydrogen, a branched or unbranched, optionally substituted C₁-C₈-alkyl, C₂-C₆-alkenyl, C₂-C₆-alkinyl, C₁-C₅-alkylene-C₁-C₄-alkoxy, mono- and bis-alkylaminoalkylene or acylaminoalkylene radical or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-heterocycloalkyl, C₁-C₄-alkylene-heterocycloalkenyl or heteroarylalkyl radical,
R_{G,}¹², R_{G,}¹³ independently of one another denote hydrogen, a branched or unbranched, optionally substituted C₁-C₈-alkyl, C₂-C₆-alkenyl C₂-C₆-alkinyl, C₁-C₅-alkylene-C₁-C₄-alkoxy, mono- and bis-alkylaminoalkylene or acylaminoalkylene radical or an optionally substituted aryl, heterocycloalkyl, heterocycloalkenyl, heteroaryl, C₃-C₇-cycloalkyl, C₁-C₄-alkylene-C₃-C₇-cycloalkyl, arylalkyl, C₁-C₄-alkylene-heterocycloalkyl, C₁-C₄-alkylene-heterocycloalkenyl or heteroaryl radical, or a radical -SO₂-R_{G}¹¹, -CO-OR_{G}¹¹, -CO-NR_{G}¹¹R_{G}¹¹* or -CO-R_{G}¹¹, and
R_{G}¹¹* denotes a radical R_{G}¹¹ independent of R_{G}¹¹
B is a structural element containing at least one atom that, being an hydrogen acceptor, may form hydrogen bonds under physiological conditions, wherein at least one hydrogen acceptor atom along the shortest possible path along the structural element skeleton is at a distance of 5 to 14 atomic bonds to the structural element G,
as well as the physiological compatible salts, prodrugs and the enantiomer-pure or diastereomer-pure and tautomeric forms, for use as a medicament.

5. Use of a compound of the formula I as claimed in claim 4, for the production of a medicament for treating conditions in which the interaction between αᵥβ₃-integrin and its natural ligands is increased or reduced, namely arteriosclerosis, rheumatoid arthritis, re-stenosis after vascular damage or stent implants, angioplasty, acute kidney failure, angiogenesis-associated microangiopathies, diabetic angiopathies, blood platelet-mediated vascular blockage, arterial thrombosis, congestive heart failure, myocardial infarction, stroke, cancer, osteoporosis, hypertension, psoriasis or viral, parasitic or bacterial diseases, inflammations, cicatrisation, hyperparathyroidism, Paget's disease, malign hypercalcemia or metastatic osteolytic lesions.

6. Medicament preparation containing at least one compound as claimed in claim 4, optionally medicament auxiliary substances and at least one further compound selected from the group comprising blood platelet adhesion, activation or aggregation inhibitors, anticoagulants that prevent thrombin activity or formation, antagonists of blood platelet-activating compounds or selectin antagonists.

7. Use of the medicament preparation according to claim 6 for the production of a medicament for treating blood platelet-induced vascular blockage or thrombosis.

8. Medicament preparation containing at least one compound as claimed in claim 4, optionally medicament auxiliary substances and at least one further compound selected from the group comprising inhibitors of blood platelet activation or aggregation, serine protease inhibitors, fibrinogen-lowering compounds, selectin antagonists, antagonists of ICAM-1 or VCAM-1, leucocyte adhesion inhibitors, inhibitors of vascular wall transmigration, fibrinolysis-modulating compounds, inhibitors of complement factors, endothelin receptor antagonists, tyrosine kinase inhibitors, antioxidants or interleukin 8 antagonists.

9. Use of the medicament preparation according to claim 8 for the production of a medicament for treating myocardial infarction or stroke.

10. Medicament preparation containing at least one compound as claimed in claim 4, optionally medicament auxiliary substances and at least one further compound selected from the group comprising endothelin antagonists, ACE inhibitors, angiotensin receptor antagonists, endopeptidase inhibitors, beta-blockers, calcium channel antagonists, phosphodiesterase inhibitors or caspase inhibitors.

11. Use of the medicament preparation according to claim 10 for the production of a medicament for treating congestive heart failure.

12. Medicament preparation containing at least one compound as claimed in claim 4, optionally medicament auxiliary substances and at least one further compound selected from the group comprising thrombin inhibitors, inhibitors of factor Xa, inhibitors of the coagulation pathway leading to thrombin formation, inhibitors of blood platelet adhesion, activation or aggregation, endothelin receptor antagonists, nitrogen oxide synthase inhibitors, CD44 antagonists, selectin antagonists, MCP-1 antagonists, inhibitors of signal transduction in proliferating cells, antagonists of the cellular response mediated by EGF, PDGF, VEGF or bFGF, or antioxidants.

13. Use of the medicament preparation according to claim 12 for the production of a medicament for treating re-stenosis after vascular damage or stent implants.

14. Medicament preparation containing at least one compound as claimed in claim 4, optionally medicament auxiliary substances and at least one further compound selected from the group comprising antagonists of the cellular response mediated by EGF, PDGF, VEGF or bFGF, heparin or low molecular weight heparins or further GAGs, inhibitors of MMPs, selectin antagonists, endothelin antagonists, ACE inhibitors, angiotensin receptor antagonists, glycosilation inhibitors or AGE formation inhibitors or AGE breakers and antagonists of their receptors.

15. Use of the medicament preparation according to claim 14 for the production of a medicament for treating diabetic angiopathies.

16. Medicament preparation containing at least one compound as claimed in claim 4, optionally medicament auxiliary substances and at least one further compound selected from the group comprising fat-lowering compounds, selectin antagonists, antagonists of ICAM-1 or VCAM-1, heparin or low molecular weight heparins or further GAGs, inhibitors of MMPs, endothelin antagonists, apolipoprotein A1 antagonists, cholesterol antagonists, HMG CoA reductase inhibitors, ACAT inhibitors, ACE inhibitors, angiotensin receptor antagonists, tyrosine kinase inhibitors, protein kinase C inhibitors, calcium channel antagonists, LDL receptor function stimulants, antioxidants, LCAT mimetics, or free radical collectors.

17. Use of the medicament preparation according to claim 16 for the production of a medicament for treating arteriosclerosis.

18. Medicament preparation containing at least one compound as claimed in claim 4, optionally medicament auxiliary substances and at least one further compound selected from the group comprising cytostatic or antineoplastic compounds, proliferation-inhibiting compounds, or heparin or low molecular weight heparins or further GAGs.

19. Use of the medicament preparation according to claim 18 for the production of a medicament for treating cancer.

20. Medicament preparation containing at least one compound as claimed in claim 4, optionally medicament auxiliary substances and at least one further compound selected from the group comprising compounds for anti-resorptive therapy, compounds for hormone replacement therapy, human growth hormone recombinants, bisphosphonates, compounds for calcitonin therapy, calcitonin stimulants, calcium channel agonists, bone formation stimulants, interleukin-6 antagonists or Src tyrosine kinase inhibitors.

21. Use of the medicament preparation according to claim 20 for the production of a medicament for treating osteoporosis.

22. Medicament preparation containing at least one compound as claimed in claim 4, optionally medicament auxiliary substances and at least one further compound selected from the group comprising TNF antagonists, antagonists of VLA-4 or VCAM-1, antagonists of LFA-1, Mac-1 or ICAMs, complement inhibitors, immunosuppressants, interleukin-1, interleukin-5 or interleukin-8 antagonists, or dihydrofolate reductase inhibitors.

23. Use of the medicament preparation according to claim 22 for the production of a medicament for treating rheumatoid arthritis.

24. Medicament preparation containing at least one compound as claimed in claim 4, optionally medicament auxiliary substances and at least one further compound selected from the group comprising collagenase, PDGF antagonists or MMPs.

25. Use of the medicament preparation according to claim 24 for the production of a medicament for improving cicatrisation.

## Revendications

1. Composés de formule I'
A-E'-G'-L I'
où A, E', G' et L ont la signification suivante :
L représente un élément structural de formule I_{L}
-U-T I_{L}
où
T représente un groupe COOH, un groupement hydrolysable en COOH ou un groupement bioisostère de COOH et
-U- représente -(X_{L})ₐ-(CR_{L}¹R_{L}²)_{b}-, -CR_{L}¹=CR_{L}²-, éthynylène ou =CR_{L}¹-,
où
a représente 0 ou 1,
b représente 0, 1 ou 2,
X_{L} représente CR_{L}³R_{L}⁴, NR_{L}⁵, l'oxygène ou le soufre,
R_{L}¹, R_{L}², R_{L}³, R_{L}⁴ représentent indépendamment les uns des autres l'hydrogène, -T, -OH, -NR_{L}⁶R_{L}⁷, -CO-NH₂, un groupement halogène, un groupement ramifié ou non ramifié, éventuellement substitué, C₁-C₆-alkyle, C₂-C₆-alcényle, C₂-C₆-alcynyle, C₃-C₇-cycloalkyle, -CO-NH(C₁-C₆-alkyle), -CO-N(C₁-C₆-alkyle)₂ ou C₁-C₄-alcoxy, un groupement C₁-C₂-alkylène-T, C₂-alcénylène-T ou C₂-alcynylène-T éventuellement substitué, un groupement aryle ou arylalkyle éventuellement substitué ou bien, à chaque fois indépendamment l'un de l'autre, deux groupements R_{L}¹ et R_{L}² ou R_{L}³ et R_{L}⁴ ou éventuellement R_{L}¹ et R_{L}³ représentent ensemble un carbocycle ou un hétérocycle saturé ou insaturé à 3 à 7 chaînons, éventuellement substitué, qui peut contenir jusqu'à trois hétéroatomes O, N, S différents ou identiques,
R_{L}⁵, R_{L}⁶, R_{L}⁷ représentent indépendamment les uns des autres l'hydrogène, un groupement ramifié ou non ramifié, éventuellement substitué, C₁-C₆-alkyle, C₃-C₇-cycloalkyle, CO-O-C₁-C₆-alkyle, SO₂-C₁-C₆-alkyle ou CO-C₁-C₆-alkyle ou un groupement CO-O-alkylène-aryle, SO₂-aryle, CO-aryle, SO₂-alkylène-aryle ou CO-alkylène-aryle éventuellement substitué,
G' représente un élément structural de formule I_{G} 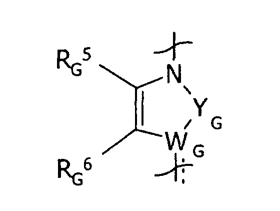
où
l'élément structural E' est lié à l'élément structural G par le biais de l'azote cyclique et l'élément structural L est lié à l'élément structural G' par le biais de W_{G},
Y_{G} représente CO, CS, C=NR_{G}² ou CR_{G}³R_{G}⁴,
R_{G}² représente l'hydrogène, un groupe hydroxyle, un groupement ramifié ou non ramifié, éventuellement substitué, C₁-C₆-alkyle, C₁-C₄-alcoxy, C₃-C₇-cycloalkyle ou -O-C₃-C₇-cycloalkyle ou un groupement aryle, -O-aryle, arylalkyle ou -O-alkylène-aryle éventuellement substitué,
R_{G}³, R_{G}⁴ représentent indépendamment l'un de l'autre l'hydrogène ou un groupement ramifié ou non ramifié, éventuellement substitué, C₁-C₆-alkyle, C₂-C₆-alcényle, C₂-C₆-alcynyle ou C₁-C₄-alcoxy ou bien les deux groupements R_{G}³ et R_{G}⁴ représentent ensemble un acétal cyclique -O-CH₂-CH₂-O- ou -O-CH₂-O- ou bien les deux groupements R_{G}³ et R_{G}⁴ représentent ensemble un groupement C₃-C₇-cycloalkyle éventuellement substitué,
R_{G}⁵, R_{G}⁶ représentent indépendamment l'un de l'autre l'hydrogène, un groupe hydroxyle, un groupement ramifié ou non ramifié, éventuellement substitué, C₁-C₆-alkyle ou C₁-C₄-alcoxy un groupement aryle ou arylalkyle éventuellement substitué ou bien les deux groupements R_{G}⁵ et R_{G}⁶ représentent ensemble un carbocycle ou hétérocycle à 3 à 10 chaînons, insaturé ou aromatique, condensé, éventuellement substitué, qui peut contenir jusqu'à trois hétéroatomes O, N, S différents ou identiques,
W_{G} représente un élément structural choisi dans le groupe des éléments structuraux des formules I_{WG}¹ à I_{WG}⁴, 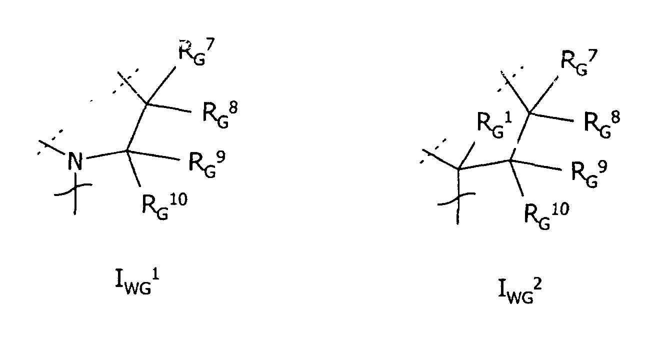 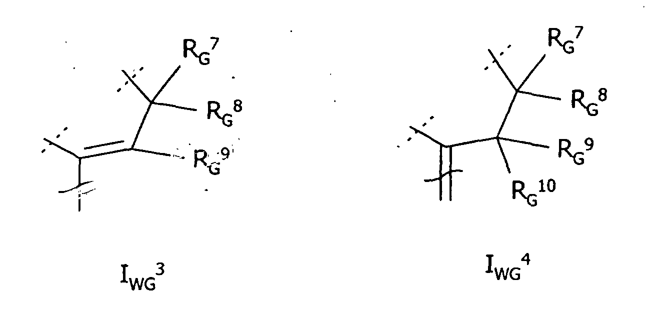
R_{G}¹ représente l'hydrogène, un halogène, un groupe hydroxyle ou un groupement ramifié ou non ramifié, éventuellement substitué, C₁-C₆-alkyle ou C₁-C₄-alcoxy
R_{G}⁷, R_{G}⁸, R_{G}⁹, R_{G}¹⁰ représentent indépendamment les uns des autres l'hydrogène, un groupe hydroxyle, -CN, un halogène, un groupement ramifié ou non ramifié, éventuellement substitué, C₁-C₆-alkyle, C₂-C₆-alcényle, C₂-C₆-alcynyle, C₁-C₄-alkylène-C₃-C₇-cycloalkyle, C₁-C₄-alkylène-C₃-C₇-hétérocycloalkyle ou C₁-C₄-alkylène-C₃-C₇-hétérocycloalcényle, un groupement C₁-C₄-alkylène-OR_{G}¹¹, C₁-C₄-alkylène-CO-OR_{G}¹¹, C₁-C₄-alkylène-O-CO-R_{G}¹¹, C₁-C₄-alkylène-CO-R_{G}¹¹, C₁-C₄-alkylène-SO₂-NR_{G'}¹²R_{G'}¹³, C₁-C₄-alkylène-CO-NR_{G'}¹²R_{G'}¹³, C₁-C₄-alkylène-O-CO-NR_{G'}¹²R_{G'}¹³, C₁-C₄-alkylène-NR_{G'}¹²R_{G'}¹³ ou C₁-C₄-alkylène-SR_{G}¹¹, C₁-C₄-alkylène-SO-R_{G}¹¹, un groupement -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G'}¹²R_{G'}¹³, -SO₂-NR_{G'}¹²R_{G'}¹³, -CO-NR_{G'}¹²R_{G'}¹³, -NR_{G'}¹²R_{G'}¹³ ou CO-R_{G}¹¹, un groupement C₃-C₇-cycloalkyle, C₃-C₇-hétérocycloalkyle, C₃-C₇-hétérocycloalcényle, aryle, hétaryle, arylalkyle ou hétarylalkyle, éventuellement substitué ou bien, dans chaque cas indépendamment l'un de l'autre, deux groupements R_{G}⁷ et R_{G}⁹ ou R_{G}⁸ et R_{G}¹⁰ ou R_{G}⁷ et R_{G}⁸ ou R_{G}⁹ et R_{G}¹⁰ représentent ensemble un carbocycle ou hétérocycle à 3 à 7 chaînons, non aromatique, saturé ou insaturé, éventuellement substitué, qui peut contenir jusqu'à trois hétéroatomes choisis dans le groupe O, N, S et jusqu'à deux doubles liaisons,
R_{G}¹¹ représente l'hydrogène, un groupement ramifié ou non ramifié, éventuellement substitué C₁-C₈-alkyle, C₂-C₆-alcényle, C₂-C₆-alcynyle, C₁-C₅-alkylène-C₁-C₄-alcoxy, mono- et bis-alkylaminoalkylène ou acylaminoalkylène ou un groupement aryle, hétérocycloalkyle, hétéroacycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocyclo-alcényle ou hétarylalkyle éventuellement substitué,
R_{G'}¹², R_{G'}¹³ représentent indépendamment l'un de l'autre l'hydrogène, un groupement ramifié ou non ramifié, éventuellement substitué, C₁-C₈-alkyle, C₂-C₆-alcényle, C₂-C₆-alcynyle, C₁-C₅-alkylène-C₁-C₄-alcoxy, mono- et bis-alkylaminoalkylène ou acylaminoalkylène ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocycloalcényle ou hétarylalkyle éventuellement substitué, ou un groupement -SO₂-R_{G}¹¹, -CO-OR_{G}¹¹, -CO-NR_{G}¹¹R_{G}¹¹* ou -CO-R_{G}¹⁴ et
R_{G}¹¹* représente un groupement R_{G}¹¹ indépendant de R_{G}¹¹,
R_{G'}¹⁴ représente l'hydrogène, un groupement ramifié ou non ramifié, éventuellement substitué, C₁-C₈-alkyle, C₂-C₆-alcényle, C₂-C₆-alcynyle ou C₁-C₅-alkylène-C₁-C₄-alcoxy ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocycloalcényle ou hétarylalkyle, éventuellement substitué,
E' représente un élément structural composé de deux à quatre éléments structuraux partiels choisis dans le groupe E¹ et E², où la succession de la liaison des éléments structuraux partiels est quelconque et E¹ et E² ont la signification suivante :
E¹ représente un élément structural partiel de formule I_{E1}
-(Y_{E})ₖ₁-(CR_{E}¹R_{E}²)_{c}-(Q_{E})ₖ₂-(CR_{E}³R_{E}⁴)_{d}- I_{E1}
et
E² représente un élément structural partiel de formule I_{E2}
-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2},
où
c, d, f, g, h représentent indépendamment les uns des autres 0, 1 ou 2,
k1, k2, k3, k4, k5, k6 représentent indépendamment les uns des autres 0 ou 1,
X_{E}, Q_{E} représentent indépendamment l'un de l'autre un hydrocarbure aliphatique ou aromatique, mono- ou polycyclique, à 4 à 11 chaînons, éventuellement substitué, qui peut contenir jusqu'à 6 doubles liaisons et jusqu'à 6 hétéroatomes identiques ou différents choisis dans le groupe N, O ou S, où les carbones cycliques et/ou les azotes cycliques peuvent éventuellement être substitués,
Y_{E}, Z_{E} représentent indépendamment l'un de l'autre CO, CO-NR_{E}¹², NR_{E}¹²-CO, le soufre, SO, SO₂, SO₂-NR_{E}¹², NR_{E}¹²-SO₂, CS, CS-NR_{E}¹², NR_{E}¹²-CS, CS-O, O-CS, CO-O, O-CO, l'oxygène, éthynylène, CR_{E}¹³-O-CR_{E}¹⁴, C(=CR_{E}¹³R_{E}¹⁴), CR_{E}¹³=CR_{E}¹⁴, -CR_{E}¹³(OR_{E}¹⁵)-CHR_{E}¹⁴- ou -CHR-_{E}¹³-CR_{E}¹⁴(OR_{E}¹⁵)-,
R_{E}¹, R_{E}², R_{E}³, R_{E}⁴, R_{E}⁵, R_{E}⁶, R_{E}⁷, R_{E}⁸, R_{E}⁹, R_{E}¹⁰ représentent indépendamment les uns des autres l'hydrogène, un halogène, un groupe hydroxyle, un groupement ramifié ou non ramifié, éventuellement substitué, C₁-C₆-alkyle, C₂-C₆-alcényle, C₂-C₆-alcynyle ou alkylène-cycloalkyle, un groupement -(CH₂)ₓ(W_{E})_{Z}-R_{E}¹⁷, un groupement C₃-C₇-cycloalkyle, aryle, arylalkyle, hétaryle ou hétarylalkyle éventuellement substitué ou bien, indépendamment l'un de l'autre, dans chaque cas deux groupements R_{E}¹ et R_{E}² ou R_{E}³ et R_{E}⁴ ou R_{E}⁵ et R_{E}⁶ ou R_{E}⁷ et R_{E}⁸ ou R_{E}⁹ et R_{E}¹⁰ représentent ensemble un carbo- ou hétérocycle saturé ou insaturé, éventuellement substitué, à 3 à 7 chaînons, qui peut contenir jusqu'à trois hétéroatomes du groupe O, N ou S,
x représente 0, 1, 2, 3 ou 4,
z représente 0 ou 1,
W_{E} représente -CO-, -CO-N(R_{W}²)-, -N(R_{W}²)-CO-, -N(R_{W}²)-CO-N(R_{W}²*)-, -N(R_{W}²)-CO-O-, -O-, -S-, -SO₂-, -SO₂-N(R_{W}²)-, -SO₂-O-, -CO-O-, -O-CO-, -O-CO-N(R_{W}²)-, -N(R_{W}²)- ou -N(R_{W}²)-SO₂-,
R_{W}², R_{W}²* représentent indépendamment l'un de l'autre l'hydrogène, un groupement ramifié ou non ramifié, éventuellement substitué, C₁-C₆-alkyle, C₂-C₆-alcényle, C₂-C₈-alcynyle, CO-C₁-C₆-alkyle, CO-O-C₁-C₆-alkyle ou SO₂-C₁-C₆-alkyle ou un groupement hétaryle, hétarylalkyle, arylalkyle, C₃-C₇-cycloalkyle, CO-O-alkylène-aryle, CO-alkylène-aryle, CO-aryle, SO₂-aryle, CO-hétaryle ou SO₂-alkylène-aryle éventuellement substitué,
R_{E}¹⁷ représente l'hydrogène, un groupe hydroxyle, CN, un halogène, un groupement C₁-C₆-alkyle ramifié ou non ramifié, éventuellement substitué, un groupement C₃-C₇-cycloalkyle, aryle, hétéroaryle ou arylalkyle éventuellement substitué, un groupement C₂-C₆-alcynyle ou C₂-C₆-alcényle éventuellement substitué par C₁-C₄-alkyle ou aryle, un groupement C₆-C₁₂-bicycloalkyle, C₁-C₆-alkylène-C₆-C₁₂-bicycloalkyle, C₇-C₂₀-tricycloalkyle ou C₁-C₆-alkylène-C₇-C₂₀-tricycloalkyle éventuellement substitué, ou un hétérocycle saturé ou insaturé, à 3 à 8 chaînons, substitué par jusqu'à trois groupements identiques ou différents, qui peut contenir jusqu'à trois hétéroatomes O, N, S différents ou identiques, où deux groupements peuvent représenter ensemble un carbocycle ou hétérocycle saturé, insaturé ou aromatique condensé, qui peut contenir jusqu'à trois hétéroatomes O, N, S différents ou identiques, et le cycle peut éventuellement être substitué ou bien un autre cycle saturé, insaturé ou aromatique éventuellement substitué peut être condensé à ce cycle, ou bien le groupement R_{E}¹⁷ forme avec R_{W}² ou R_{W}²* un C₃-C₇-hétérocycle saturé ou insaturé qui peut éventuellement contenir jusqu'à deux autres hétéroatomes choisis dans le groupe O, S ou N,
R_{E}¹¹, R_{E}¹¹* représentent indépendamment l'un de l'autre l'hydrogène, un groupement ramifié ou non ramifié, éventuellement substitué C₁-C₆-alkyle, C₁-C₆-alcoxyalkyle, C₂-C₆-alcényle, C₂-C₁₂-alcynyle, CO-C₁-C₆-alkyle, CO-O-C₁-C₆-alkyle, CO-NH-C₁-C₆-alcoxyalkyle, CO-NH-C₁-C₆-alkyle ou SO₂-C₁-C₆-alkyle ou un groupement hétaryle, arylalkyle, C₃-C₇-cycloalkyle, CO-O-alkylène-aryle, CO-NH-alkylène-aryle, CO-alkylène-aryle, CO-aryle, CO-NH-aryle, SO₂-aryle, CO-hétaryle, SO₂-alkylène-aryle, SO₂-hétaryle ou SO₂-alkylène-hétaryle éventuellement substitué,
R_{E}¹² représente l'hydrogène, un groupement ramifié ou non ramifié, éventuellement substitué, C₁-C₆-alkyle, C₂-C₆-alcényle, C₂-C₈-alcynyle, un groupement C₃-C₇-cycloalkyle, hétaryle, arylalkyle ou hétarylalkyle éventuellement substitué ou un groupement CO-R_{E}¹⁶, COOR_{E}¹⁶ ou SO₂-R_{E}¹⁶,
R_{E}¹³, R_{E}¹⁴ représentent indépendamment l'un de l'autre l'hydrogène, un groupe hydroxyle, un groupement ramifié ou non ramifié, éventuellement substitué, C₁-C₆-alkyle, C₁-C₄-alcoxy, C₂-C₆-alcényle, C₂-C₆-alcynyle ou alkylène-cycloalkyle ou un groupement C₃-C₇-cycloalkyle, aryle, arylalkyle, hétaryle ou hétarylalkyle éventuellement substitué,
R_{E}¹⁵ représente l'hydrogène, un groupement ramifié ou non ramifié, éventuellement substitué, C₁-C₆-alkyle, C₂-C₆-alcényle, C₂-C₆-alcynyle ou alkylène-cycloalkyle ou un groupement C₃-C₇-cycloalkyle, aryle, arylalkyle, hétaryle ou hétarylalkyle éventuellement substitué,
R_{E}¹⁶ représente l'hydrogène, un groupe hydroxyle, un groupement ramifié ou non ramifié, éventuellement substitué, C₁-C₆-alkyle, C₂-C₆-alcényle, C₂-C₆-alcynyle ou C₁-C₅-alkylène-C₁-C₄-alcoxy ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-C₃-C₇-hétérocycloalkyle, C₁-C₄-alkylène-C₃-C₇-hétérocycloalcényle ou hétarylalkyle éventuellement substitué,
avec la condition que, pour le cas où
Y_{E} ou Z_{E} représente CO et un groupement X_{E} ou Q_{E} ou un groupement aromatique ou hétéroaromatique de l'élément structural A est lié directement à Y_{E} ou Z_{E}, une liaison atomique directe de Y_{E} ou Z_{E} à l'élément structural G' est exclue,
A représente un élément structural choisi dans le groupe : un hydrocarbure monocyclique à 4 à 8 chaînons, saturé, insaturé ou aromatique, qui peut contenir jusqu'à 4 hétéroatomes choisis dans le groupe O, N ou S, où dans chaque cas, indépendamment les uns des autres, l'azote cyclique éventuellement contenu ou les carbones peuvent être substitués,
avec la condition qu'au moins un hétéroatome choisi dans le groupe O, N ou S est contenu dans l'élément structural A, ou bien
un hydrocarbure polycyclique à 9 à 14 chaînons, saturé, insaturé ou aromatique, qui peut contenir jusqu'à 6 hétéroatomes choisis dans le groupe N, O ou S, où dans chaque cas indépendamment les uns des autres, l'azote cyclique éventuellement contenu ou les carbones peuvent être substitués,
avec la condition qu'au moins un hétéroatome choisi dans le groupe O, N ou S est contenu dans l'élément structural A, un groupement 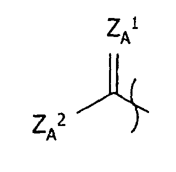
où
Z_{A}¹ représente l'oxygène, le soufre ou l'azote éventuellement substitué et
Z_{A}² représente l'azote éventuellement substitué, l'oxygène ou le soufre,
ou un groupement 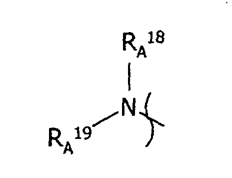 où
R_{A}¹⁸, R_{A}¹⁹ représentent indépendamment l'un de l'autre l'hydrogène, un groupement ramifié ou non ramifié, éventuellement substitué, C₁-C₈-alkyle, C₂-C₆-alcényle, C₂-C₆-alcynyle, C₁-C₅-alkylène-C₁-C₄-alcoxy, mono- et bis-alkylaminoalkylène ou acylaminoalkylène ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocycloalcényle ou hétarylalkyle éventuellement substitué, ou un groupement -SO₂-R_{G}¹¹, -CO-OR_{G}¹¹, -CO-NR_{G}¹¹R_{G}¹¹* ou -CO-R_{G}¹¹
ainsi que les sels physiologiquement acceptables, les promédicaments et les formes énantiomériquement pures ou diastéréoisomériquement pures et tautomères.

2. Composé selon la revendication 1, **caractérisé en ce que** l'on utilise comme élément structural A un élément structural choisi dans le groupe des éléments structuraux des formules I_{A}¹ à I_{A}¹⁸, 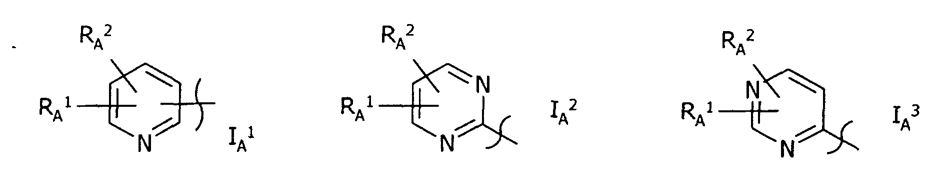 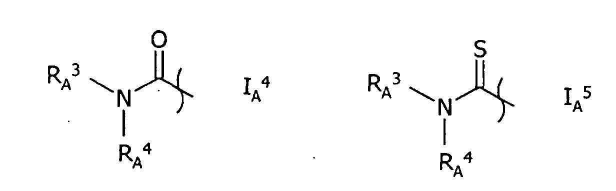 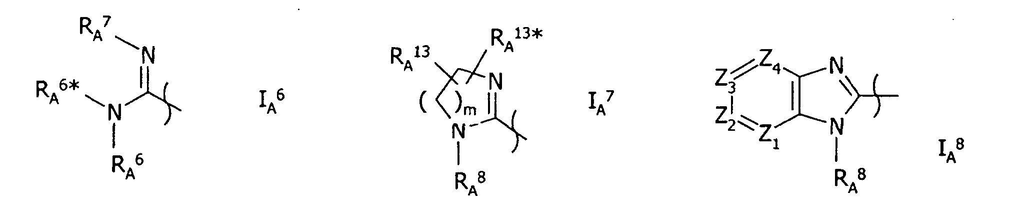 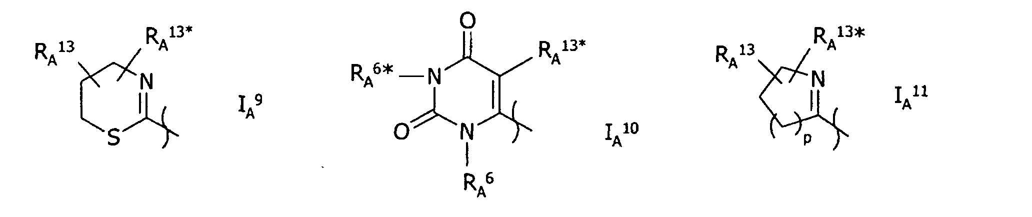 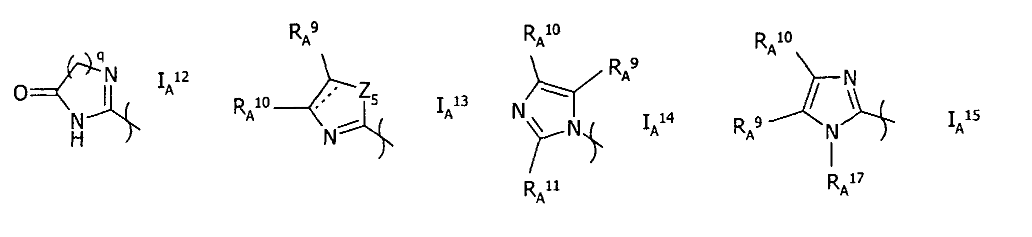 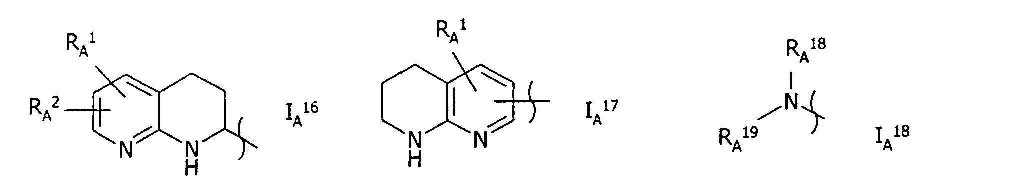 où
m, p, q représentent indépendamment les uns des autres 1, 2 ou 3,
R_{A}¹, R_{A}² représentent indépendamment l'un de l'autre l'hydrogène, CN, un halogène, un groupement ramifié ou non ramifié, éventuellement substitué, C₁-C₆-alkyle ou CO-C₁-C₆-alkyle ou un groupement aryle, arylalkyle, hétaryle, hétarylalkyle ou C₃-C₇-cycloalkyle éventuellement substitué ou un groupement CO-O-R_{A}¹⁴, Q-R_{A}¹⁴, S-R_{A}¹⁴, NR_{A}¹⁵R_{A}¹⁶, CO-NR_{A}¹⁵R_{A}¹⁶ ou SO₂NR_{A}¹⁵R_{A}¹⁶ ou bien les deux groupements R_{A}¹ et R_{A}² représentent ensemble un carbocycle ou hétérocycle à 5 ou 6 chaînons, insaturé ou aromatique, éventuellement substitué, condensé, qui peut contenir jusqu'à 3 hétéroatomes choisis dans le groupe O, N ou S,
R_{A}¹³, R_{A}¹³* représentent indépendamment l'un de l'autre l'hydrogène, CN, un halogène, un groupement C₁-C₆-alkyle ramifié ou non ramifié, éventuellement substitué, ou un groupement aryle, arylalkyle, hétaryle, C₃-C₇-cycloalkyle éventuellement substitué ou un groupement CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ ou CO-NR_{A}¹⁵R_{A}¹⁶,
où
R_{A}¹⁴ représente l'hydrogène, un groupement ramifié ou non ramifié, éventuellement substitué, C₁-C₆-alkyle, alkylène-C₁-C₄-alcoxy, C₂-C₆-alcényle, C₂-C₆-alcynyle ou C₁-C₆-alkylène-C₃-C₇-cycloalkyle ou un groupement C₃-C₇-cycloalkyle, aryle, arylalkyle, hétaryle ou hétarylalkyle éventuellement substitué,
R_{A}¹⁵, R_{A}¹⁶ représentent indépendamment l'un de l'autre l'hydrogène, un groupement ramifié ou non ramifié, éventuellement substitué, C₁-C₆-alkyle, CO-C₁-C₆-alkyle, SO₂-C₁-C₆-alkyle, COO-C₁-C₆-alkyle, CO-NH-C₁-C₆-alkyle, arylalkyle, COO-alkylène-aryle, SO₂-alkylène-aryle, CO-NH-alkylène-aryle, CO-NH-alkylène-hétaryle ou hétarylalkyle ou un groupement C₃-C₇-cycloalkyle, aryle, CO-aryle, CO-NH-aryle, SO₂-aryle, hétaryle, CO-NH-hétaryle ou CO-hétaryle éventuellement substitué,
R_{A}³, R_{A}⁴ représentent indépendamment l'un de l'autre l'hydrogène, -(CH₂)ₙ-(X_{A})ⱼ-R_{A}¹², ou bien les deux groupements représentent ensemble un N-hétérocycle à 3 à 8 chaînons saturé, insaturé ou aromatique, qui peut contenir en outre deux autres hétéroatomes O, N et S identiques ou différents, où le cycle peut éventuellement être substitué ou bien un autre cycle saturé, insaturé ou aromatique éventuellement. substitué peut être condensé à ce cycle,
où
n représente 0, 1, 2 ou 3,
j représente 0 ou 1,
X_{A} représente -CO-, -CO-N(Rₓ¹)-, -N(Rₓ¹)-CO-, -N(Rₓ¹)-CO-N(Rₓ¹*)-, -N(Rₓ¹)-CO-O-, -O-, -S-, -SO₂-, -SO₂-N(Rₓ¹)-, -SO₂-O-, -CO-O-, -O-CO-, -O-CO-N(Rₓ¹)-, -N(Rₓ¹)- ou -N(Rₓ¹)-SO₂-,
R_{A}¹² représente l'hydrogène, un groupement C₁-C₆-alkyle ramifié ou non ramifié, éventuellement substitué, un groupement C₂-C₆-alcynyle ou C₂-C₆-alcényle éventuellement substitué par C₁-C₄-alkyle ou aryle ou un hétérocycle à 3 à 6 chaînons, saturé ou insaturé, substitué par jusqu'à trois groupements identiques ou différents, qui peut contenir jusqu'à trois hétéroatomes O, N, S différents ou identiques, un groupement C₃-C₇-cycloalkyle, aryle ou hétéroaryle, où deux groupements peuvent former ensemble un carbocycle ou hétérocycle saturé, insaturé ou aromatique condensé, qui peut contenir jusqu'à trois hétéroatomes O, N, S différents ou identiques, et le cycle peut éventuellement être substitué ou bien un autre cycle saturé, insaturé ou aromatique éventuellement substitué peut être condensé à ce cycle, ou bien le groupement R_{A}¹² forme avec Rₓ¹ ou Rₓ¹* un C₃-C₇-hétérocycle saturé ou insaturé qui peut contenir éventuellement jusqu'à deux autres hétéroatomes choisis dans le groupe O, S ou N,
Rₓ¹, Rₓ¹* représentent indépendamment l'un de l'autre l'hydrogène, un groupement ramifié ou non ramifié, éventuellement substitué, C₁-C₆-alkyle, C₁-C₆-alcoxyalkyle, C₂-C₆-alcényle, C₂-C₁₂-alcynyle, CO-C₁-C₆-alkyle, CO-O-C₁-C₆-alkyle ou SO₂-C₁-C₆-alkyle ou un groupement C₃-C₇-cycloalkyle, aryle, arylalkyle, CO-O-alkylène-aryle, CO-alkylène-aryle, CO-aryle, SO₂-aryle, hétaryle, CO-hétaryle ou SO₂-alkylène-aryle éventuellement substitué,
R_{A}⁶, R_{A}⁶* représentent l'hydrogène, un groupement ramifié ou non ramifié, éventuellement substitué, C₁-C₄-alkyle, -CO-O-C₁-C₄-alkyle, arylalkyle, -CO-O-alkylène-aryle, -CO-O-allyle, -CO-C₁-C₄-alkyle, -CO-alkylène-aryle, C₃-C₇-cycloalkyle ou -CO-allyle ou bien dans l'élément structural I_{A}⁷, les deux groupements R_{A}⁶ et R_{A}⁶* représentent ensemble un hétérocycle saturé, insaturé ou aromatique éventuellement substitué qui peut contenir en plus de l'azote cyclique jusqu'à deux autres hétéroatomes O, N, S différents ou identiques,
R_{A}⁷ représente l'hydrogène, -OH, -CN, -CONH₂, un groupement ramifié ou non ramifié, éventuellement substitué, C₁-C₄-alkyle, C₁-C₄-alcoxy, C₃-C₇-cycloalkyle ou -O-CO-C₁-C₄-alkyle ou un groupement arylalkyle, -O-alkylène-aryle, -O-CO-aryle, -O-CO-alkylène-aryle ou -O-CO-allyle éventuellement substitué, ou bien les deux groupements R_{A}⁶ et R_{A}⁷ représentent ensemble un hétérocycle insaturé ou aromatique éventuellement substitué qui peut contenir en plus de l'azote cyclique jusqu'à deux autres hétéroatomes O, N, S différents ou identiques,
R_{A}⁸ représente l'hydrogène, un groupement ramifié ou non ramifié, éventuellement substitué, C₁-C₄-alkyle, CO-C₁-C₄-alkyle, SO₂-C₁-C₄-alkyle ou CO-O-C₁-C₄-alkyle ou un groupement aryle, CO-aryle, SO₂-aryle, CO-O-aryle, CO-alkylène-aryle, SO₂-alkylène-aryle, CO-O-alkylène-aryle ou alkylène-aryle éventuellement substitué,
R_{A}⁹, R_{A}¹⁰ représentent indépendamment l'un de l'autre l'hydrogène, -CN, un halogène, un groupement C₁-C₆-alkyle ramifié ou non ramifié, éventuellement substitué, ou un groupement aryle, arylalkyle, hétaryle, C₃-C₇-cycloalkyle éventuellement substitué ou un groupement CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ ou CO-NR_{A}¹⁵R_{A}¹⁶, ou bien les deux groupements R_{A}⁹ et R_{A}¹⁰ représentent ensemble dans l'élément structural I_{A}¹⁴ un carbocycle ou hétérocycle saturé, insaturé ou aromatique à 5 à 7 chaînons, qui peut contenir jusqu'à 3 hétéroatomes O, N, S différents ou identiques et qui peut éventuellement être substitué par jusqu'à trois groupements identiques ou différents,
R_{A}¹¹ représente l'hydrogène, -CN, un halogène, un groupement C₁-C₆-alkyle ramifié ou non ramifié, éventuellement substitué ou un groupement aryle, arylalkyle, hétaryle, C₃-C₇-cycloalkyle éventuellement substitué ou un groupement CO-O-R_{A}¹⁴, O-R_{A}¹⁴, S-R_{A}¹⁴, NR_{A}¹⁵R_{A}¹⁶, SO₂-NR_{A}¹⁵R_{A}¹⁶ ou CO-NR_{A}¹⁵R_{A}¹⁶,
R_{A}¹⁷ représente l'hydrogène ou bien, dans l'élément structural I_{A}¹⁵, les deux groupements R_{A}⁹ et R_{A}¹⁷ représentent ensemble un hétérocycle à 5 à 7 chaînons saturé, insaturé ou aromatique qui peut contenir en plus de l'azote cyclique jusqu'à trois hétéroatomes O, N, S différents ou identiques et qui peut éventuellement être substitué par jusqu'à trois groupements identiques ou différents,
R_{A}¹⁸, R_{A}¹⁹ représentent indépendamment l'un de l'autre l'hydrogène, un groupement ramifié ou non ramifié, éventuellement substitué C₁-C₈-alkyle, C₂-C₆-alcényle, C₂-C₆-alcynyle, C₁-C₅-alkylène-C₁-C₄-alcoxy, mono- et bis-alkylaminoalkylène ou acylaminoalkylène ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocyclo-alcényle ou hétarylalkyle éventuellement substitué ou un groupement -SO₂-R_{G}¹¹, -CO-OR_{G}¹¹, -CO-NR_{G}¹¹R_{G}¹¹* ou -CO-R_{G}¹¹ indépendant de R_{G}¹¹
Z¹, Z², Z³, Z⁴ représentent indépendamment les uns des autres l'azote, C-H, C-halogène ou un groupement ramifié ou non ramifié, éventuellement substitué,C-C₁-C₄-alkyle ou C-C₁-C₄-alcoxy
Z⁵ représente NR_{A}⁸, l'oxygène ou le soufre.

3. Composé selon la revendication 1 ou 2, **caractérisé en ce que** l'on utilise comme élément structural E' un élément structural de formule I_{E1E2}
-E₂-E₁- I_{E1E2}
et E¹ et E² ont la signification suivante :
E¹ représente un élément structural partiel de formule I_{E1}
-(Y_{E})ₖ₁-(CR_{E}¹R_{E}²)_{c}-(Q_{E})ₖ₂-(CR_{E}³R_{E}⁴)_{d}- I_{E1}
et
E² représente un élément structural partiel de formule I_{E2}
-(NR_{E}¹¹)ₖ₃-(CR_{E}⁵R_{E}⁶)_{f}-(Z_{E})ₖ₄-(CR_{E}⁷R_{E}⁸)_{g}-(X_{E})ₖ₅-(CR_{E}⁹R_{E}¹⁰)ₕ-(NR_{E}¹¹*)ₖ₆- I_{E2},
où
c, d, f, g, h représentent indépendamment les uns des autres 0, 1 ou 2,
k1, k2, k3, k4, k5, k6 représentent indépendamment les uns des autres 0 ou 1,
X_{E}, Q_{E} représentent indépendamment l'un de l'autre un hydrocarbure aliphatique ou aromatique, mono- ou polycyclique, à 4 à 11 chaînons, éventuellement substitué, qui peut contenir jusqu'à 6 doubles liaisons et jusqu'à 6 hétéroatomes identiques ou différents choisis dans le groupe N, O ou S, où les carbones cycliques et/ou les azotes cycliques peuvent éventuellement être substitués,
Y_{E}, Z_{E} représentent indépendamment l'un de l'autre CO, CO-NR_{E}¹², NR_{E}¹²-CO, le soufre, SO, SO₂, SO₂-NR_{E}¹², NR_{E}¹²-SO₂, CS, CS-NR_{E}¹², NR_{E}¹²-CS, CS-O, O-CS, CO-O, O-CO, l'oxygène, l'éthynylène, CR_{E}¹³-O-CR_{E}¹⁴, C(=CR_{E}¹³R_{E}¹⁴), CR_{E}¹³=CR_{E}¹⁴, -CR_{E}¹³(OR_{E}¹⁵)-CHR_{E}¹⁴- ou -CHR-_{E}¹³-CR_{E}¹⁴(OR_{E}¹⁵)-,
R_{E}¹, R_{E}², R_{E}³, R_{E}⁴, R_{E}⁵, R_{E}⁶, R_{E}⁷, R_{E}⁸, R_{E}⁹, R_{E}¹⁰ représentent indépendamment les uns des autres l'hydrogène, un halogène, un groupe hydroxyle, un groupement ramifié ou non ramifié, éventuellement substitué, C₁-C₆-alkyle, C₂-C₆-alcényle, C₂-C₆-alcynyle ou alkylène-cycloalkyle un groupement -(CH₂)ₓ (W_{E})_{Z}-R_{E}¹⁷, un groupement C₃-C₇-cycloalkyle, aryle, arylalkyle, hétaryle ou hétarylalkyle éventuellement substitué ou bien, indépendamment l'un de l'autre, dans chaque cas deux groupements R_{E}¹ et R_{E}² ou R_{E}³ et R_{E}⁴ ou R_{E}⁵ et R_{E}⁶ ou R_{E}⁷ et R_{E}⁸ ou R_{E}⁹ et R_{E}¹⁰ représentent ensemble un carbo- ou hétérocycle saturé ou insaturé, éventuellement substitué, à 3 à 7 chaînons, qui peut contenir jusqu'à trois hétéroatomes du groupe O, N ou S,
x représente 0, 1, 2, 3 ou 4,
z représente 0 ou 1,
W_{E} représente -CO-, -CO-N(R_{W}²)-, -N(R_{W}²)-CO-, -N(R_{W}²)-CO-N(R_{W}²*)-, -N(R_{W}²)-CO-O-, -O-, -S-, -SO₂-, -SO₂-N(R_{W}²)-, -SO₂-O-, -CO-O-, -O-CO-, -O-CO-N(R_{W}²)-, -N(R_{W}²)- ou -N(R_{W}²)-SO₂-,
R_{W}², R_{W}²* représentent indépendamment l'un de l'autre l'hydrogène, un groupement ramifié ou non ramifié, éventuellement substitué, C₁-C₆-alkyle, C₂-C₆-alcényle, C₂-C₈-alcynyle, CO-C₁-C₆-alkyle, CO-O-C₁-C₆-alkyle ou SO₂-C₁-C₆-alkyle ou un groupement hétaryle, hétarylalkyle, arylalkyle, C₃-C₇-cycloalkyle, CO-O-alkylène-aryle, CO-alkylène-aryle, CO-aryle, SO₂-aryle, CO-hétaryle ou SO₂-alkylène-aryle éventuellement substitué,
R_{E}¹⁷ représente l'hydrogène, un groupe hydroxyle, CN, un halogène, un groupement C₁-C₆-alkyle ramifié ou non ramifié, éventuellement substitué, un groupement C₃-C₇-cycloalkyle, aryle, hétéroaryle ou arylalkyle éventuellement substitué, un groupement C₂-C₆-alcynyle ou C₂-C₆-alcényle éventuellement substitué par C₁-C₄-alkyle ou aryle, un groupement C₆-C₁₂-bicycloalkyle, C₁-C₆-alkylène-C₆-C₁₂-bicycloalkyle, C₇-C₂₀-tricycloalkyle ou C₁-C₆-alkylène-C₇-C₂₀-tricycloalkyle éventuellement substitué, ou un hétérocycle saturé ou insaturé, à 3 à 8 chaînons, substitué par jusqu'à trois groupements identiques ou différents, qui peut contenir jusqu'à trois hétéroatomes O, N, S différents ou identiques, où deux groupements peuvent représenter ensemble un carbocycle ou hétérocycle saturé, insaturé ou aromatique condensé, qui peut contenir jusqu'à trois hétéroatomes O, N, S différents ou identiques, et le cycle peut éventuellement être substitué ou bien un autre cycle saturé, insaturé ou aromatique éventuellement substitué peut être condensé à ce cycle, ou bien le groupement R_{E}¹⁷ forme avec R_{W}² ou R_{W}²* un C₃-C₇-hétérocycle saturé ou insaturé qui peut éventuellement contenir jusqu'à deux autres hétéroatomes choisis dans le groupe O, S ou N,
R_{E}¹¹, R_{E}¹¹* représentent indépendamment l'un de l'autre l'hydrogène, un groupement ramifié ou non ramifié, éventuellement substitué, C₁-C₆-alkyle, C₁-C₆-alcoxyalkyle, C₂-C₆-alcényle, C₂-C₁₂-alcynyle, CO-C₁-C₆-alkyle, CO-O-C₁-C₆-alkyle, CO-NH-C₁-C₆-alcoxyalkyle, CO-NH-C₁-C₆-alkyle ou SO₂-C₁-C₆-alkyle ou un groupement hétaryle, arylalkyle, C₃-C₇-cycloalkyle, CO-O-alkylène-aryle, CO-NH-alkylène-aryle, CO-alkylène-aryle, CO-aryle, CO-NH-aryle, SO₂-aryle, CO-hétaryle, SO₂-alkylène-aryle, SO₂-hétaryle ou SO₂-alkylène-hétaryle éventuellement substitué,
R_{E}¹² représente l'hydrogène, un groupement ramifié ou non ramifié, éventuellement substitué, C₁-C₆-alkyle, C₂-C₆-alcényle, C₂-C₈-alcynyle, un groupement C₃-C₇-cycloalkyle, hétaryle, arylalkyle ou hétarylalkyle éventuellement substitué ou un groupement CO-R_{E}¹⁶, COOR_{E}¹⁶ ou SO₂-R_{E}¹⁶,
R_{E}¹³, R_{E}¹⁴ représentent indépendamment l'un de l'autre l'hydrogène, un groupe hydroxyle, un groupement ramifié ou non ramifié, éventuellement substitué, C₁-C₆-alkyle, C₁-C₄-alcoxy, C₂-C₆-alcényle, C₂-C₆-alcynyle ou alkylène-cycloalkyle ou un groupement C₃-C₇-cycloalkyle, aryle, arylalkyle, hétaryle ou hétarylalkyle éventuellement substitué,
R_{E}¹⁵ représente l'hydrogène, un groupement ramifié ou non ramifié, éventuellement substitué C₁-C₆-alkyle, C₂-C₆-alcényle, C₂-C₆-alcynyle ou alkylène-cycloalkyle ou un groupement C₃-C₇-cycloalkyle, aryle, arylalkyle, hétaryle ou hétarylalkyle éventuellement substitué,
R_{E}¹⁶ représente l'hydrogène, un groupe hydroxyle, un groupement ramifié ou non ramifié, éventuellement substitué, C₁-C₆-alkyle, C₂-C₆-alcényle, C₂-C₆-alcynyle ou C₁-C₅-alkylène-C₁-C₄-alcoxy ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-C₃-C₇-hétérocycloalkyle, C₁-C₄-alkylène-C₃-C₇-hétérocycloalcényle ou hétarylalkyle éventuellement substitué,
avec la condition que, pour le cas où
Y_{E} = CO,
k1 et k5 = 1 et
h et k6 = 0,
la somme des indices c, k2 et d doit être différente de 0
et pour le cas où un groupement aromatique ou hétéroaromatique de l'élément structural A est lié directement à Y_{E} ou Z_{E}, une liaison atomique directe de Y_{E} ou Z_{E} à l'élément structural G est exclue.

4. Composé de formule I
B-G-L I
où B, G et L ont la signification suivante :
L représente un élément structural de formule I_{L}
-U-T I_{L}
où
T représente un groupe COOH, un groupement hydrolysable en COOH ou un groupement bioisostère de COOH et
-U- représente -(X_{L})ₐ-(CR_{L}¹R_{L}²)_{b}-, -CR_{L}¹=CR_{L}²-, éthynylène ou =CR_{L}¹-,
où
a représente 0 ou 1,
b représente 0, 1 ou 2,
X_{L} représente CR_{L}³R_{L}⁴, NR_{L}⁵, l'oxygène ou le soufre,
R_{L}¹, R_{L}², R_{L}³, R_{L}⁴ représentent indépendamment les uns des autres l'hydrogène, -T, -OH, -NR_{L}⁶R_{L}⁷, -CO-NH₂, un groupement halogène, un groupement ramifié ou non ramifié, éventuellement substitué, C₁-C₆-alkyle, C₂-C₆-alcényle, C₂-C₆-alcynyle, C₃-C₇-cycloalkyle, -CO-NH(C₁-C₆-alkyle), -CO-N(C₁-C₆-alkyle)₂ ou C₁-C₄-alcoxy, un groupement C₁-C₂-alkylène-T, C₂-alcénylène-T ou C₂-alcynylène-T éventuellement substitué, un groupement aryle ou arylalkyle éventuellement substitué ou bien, à chaque fois indépendamment l'un de l'autre, deux groupements R_{L}¹ et R_{L}² ou R_{L}³ et R_{L}⁴ ou éventuellement R_{L}¹ et R_{L}³ représentent ensemble un carbocycle ou un hétérocycle saturé ou insaturé à 3 à 7 chaînons, éventuellement substitué, qui peut contenir jusqu'à trois hétéroatomes O, N, S différents ou identiques,
R_{L}⁵, R_{L}⁶, R_{L}⁷ représentent indépendamment les uns des autres l'hydrogène, un groupement ramifié ou non ramifié, éventuellement substitué, C₁-C₆-alkyle, C₃-C₇-cycloalkyle, CO-O-C₁-C₆-alkyle, SO₂-C₁-C₆-alkyle ou CO-C₁-C₆-alkyle ou un groupement CO-O-alkylène-aryle, SO₂-aryle, CO-aryle, SO₂-alkylène-aryle ou CO-alkylène-aryle éventuellement substitué,
G représente un élément structural de formule I_{G} 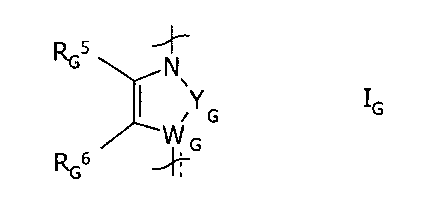
où
l'élément structural B est lié à l'élément structural G par le biais de l'azote cyclique et l'élément structural L est lié à l'élément structural G par le biais de W_{G},
Y_{G} représente CO, CS, C=NR_{G}² ou CR_{G}³R_{G}⁴,
R_{G}² représente l'hydrogène, un groupe hydroxyle, un groupement ramifié ou non ramifié, éventuellement substitué, C₁-C₆-alkyle, C₁-C₄-alcoxy, C₃-C₇-cycloalkyle ou -O-C₃-C₇-cycloalkyle ou un groupement aryle, -O-aryle, arylalkyle ou -O-alkylène-aryle éventuellement substitué,
R_{G}³, R_{G}⁴ représentent indépendamment l'un de l'autre l'hydrogène ou un groupement ramifié ou non ramifié, éventuellement substitué, C₁-C₆-alkyle, C₂-C₆-alcényle, C₂-C₆-alcynyle ou C₁-C₄-alcoxy ou bien les deux groupements R_{G}³ et R_{G}⁴ représentent ensemble un acétal cyclique -O-CH₂-CH₂-O- ou -O-CH₂-O- ou bien les deux groupements R_{G}³ et R_{G}⁴ représentent ensemble un groupement C₃-C₇-cycloalkyle éventuellement substitué,
R_{G}⁵, R_{G}⁶ représentent indépendamment l'un de l'autre l'hydrogène, un groupe hydroxyle, un groupement ramifié ou non ramifié, éventuellement substitué, C₁-C₆-alkyle ou C₁-C₄-alcoxy, un groupement aryle ou arylalkyle éventuellement substitué ou bien les deux groupements R_{G}⁵ et R_{G}⁶ représentent ensemble un carbocycle ou hétérocycle à 3 à 10 chaînons, insaturé ou aromatique, condensé, éventuellement substitué, qui peut contenir jusqu'à trois hétéroatomes O, N, S différents ou identiques,
W_{G} représente un élément structural choisi dans le groupe des éléments structuraux des formules I_{WG}¹ à I_{WG}⁴, 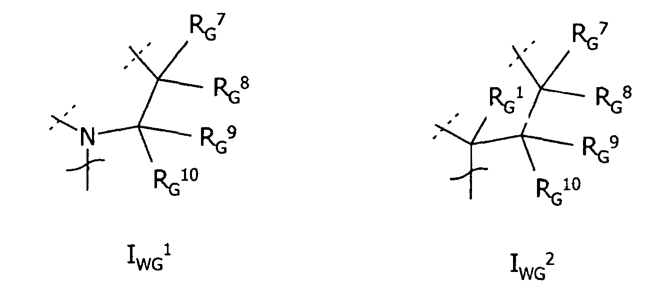 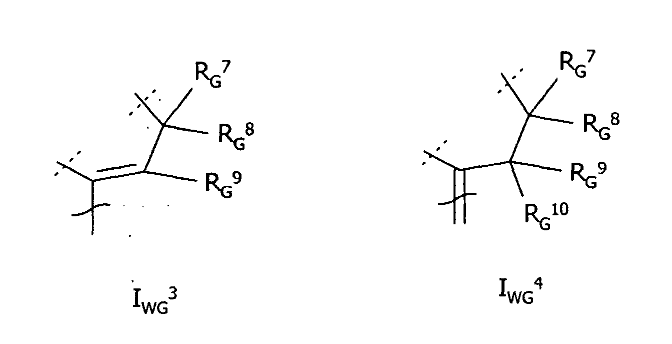
R_{G}¹ représente l'hydrogène, un halogène, un groupe hydroxyle ou un groupement ramifié ou non ramifié, éventuellement substitué, C₁-C₆-alkyle ou C₁-C₄-alcoxy
R_{G}⁷, R_{G}⁸, R_{G}⁹, R_{G}¹⁰ représentent indépendamment les uns des autres l'hydrogène, un groupe hydroxyle, -CN, un halogène, un groupement ramifié ou non ramifié, éventuellement substitué, C₁-C₆-alkyle, C₂-C₆-alcényle, C₂-C₆-alcynyle, C₁-C₄-alkylène-C₃-C₇-cycloalkyle, C₁-C₄-alkylène-C₃-C₇-hétérocycloalkyle ou C₁-C₄-alkylène-C₃-C₇-hétérocycloalcényle, un groupement C₁-C₄-alkylène-OR_{G}¹¹, C₁-C₄-alkylène-CO-OR_{G}¹¹, C₁-C₄-alkylène-O-CO-R_{G}¹¹, C₁-C₄-alkylène-CO-R_{G}¹¹, C₁-C₄-alkylène-SO₂-NR_{G}¹²R_{G}¹³, C₁-C₄-alkylène-CO-NR_{G}¹²R_{G}¹³, C₁-C₄-alkylène-O-CO-NR_{G}¹²R_{G}¹³, C₁-C₄-alkylène-NR_{G}¹²R_{G}¹³ ou C₁-C₄-alkylène-SR_{G}¹¹, C₁-C₄-alkylène-SO-R_{G}¹¹, un groupement -S-R_{G}¹¹, -O-R_{G}¹¹, -SO-R_{G}¹¹, -SO₂-R_{G}¹¹, -CO-OR_{G}¹¹, -O-CO-R_{G}¹¹, -O-CO-NR_{G}¹²R_{G}¹³, -SO₂-NR_{G}¹²R_{G}¹³, -CO-NR_{G}¹²R_{G}¹³, -NR_{G}¹²R_{G}¹³ ou CO-R_{G}¹¹, un groupement C₃-C₇-cycloalkyle, C₃-C₇-hétérocycloalkyle, C₃-C₇-hétérocycloalcényle, aryle, hétaryle, arylalkyle ou hétarylalkyle, éventuellement substitué ou bien, dans chaque cas indépendamment l'un de l'autre, deux groupements R_{G}⁷ et R_{G}⁹ ou R_{G}⁸ et R_{G}¹⁰ ou R_{G}⁷ et R_{G}⁸ ou R_{G}⁹ et R_{G}¹⁰ représentent ensemble un carbocycle ou hétérocycle à 3 à 7 chaînons, non aromatique, saturé ou insaturé, éventuellement substitué, qui peut contenir jusqu'à trois hétéroatomes choisis dans le groupe O, N, S et jusqu'à deux doubles liaisons,
R_{G}¹¹ représente l'hydrogène, un groupement ramifié ou non ramifié, éventuellement substitué, C₁-C₈-alkyle, C₂-C₆-alcényle, C₂-C₆-alcynyle, C₁-C₅-alkylène-C₁-C₄-alcoxy, mono- et bis-alkylaminoalkylène ou acylaminoalkylène ou un groupement aryle, hétérocycloalkyle, hétéroacycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocyclo-alcényle ou hétarylalkyle éventuellement substitué,
R_{G}¹², R_{G}¹³ représentent indépendamment l'un de l'autre l'hydrogène, un groupement ramifié ou non ramifié, éventuellement substitué, C₁-C₈-alkyle, C₂-C₆-alcényle, C₂-C₆-alcynyle, C₁-C₅-alkylène-C₁-C₄-alcoxy, mono- et bis-alkylaminoalkylène ou acylaminoalkylène ou un groupement aryle, hétérocycloalkyle, hétérocycloalcényle, hétaryle, C₃-C₇-cycloalkyle, C₁-C₄-alkylène-C₃-C₇-cycloalkyle, arylalkyle, C₁-C₄-alkylène-hétérocycloalkyle, C₁-C₄-alkylène-hétérocyclo-alcényle ou hétarylalkyle éventuellement substitué, ou un groupement -SO₂-R_{G}¹¹, -CO-OR_{G}¹¹, -CO-NR_{G}¹¹R_{G}¹¹* ou ―CO-R_{G}¹¹ et
R_{G}¹¹* représente un groupement R_{G}¹¹ indépendamment de R_{G}¹¹,
B représente un élément structural contenant au moins un atome qui, dans les conditions physiologiques, peut former des ponts hydrogène comme accepteur d'hydrogène, où au moins un atome accepteur d'hydrogène présente sur le chemin le plus court possible le long du squelette de l'élément structural une distance de 5 à 14 liaisons atomiques avec l'élément structural G,
ainsi que les sels physiologiquement acceptables, les promédicaments et les formes énantiomériquement pures ou diastéréoisomériquement pures et tautomères, destinés à être utilisés comme médicament.

5. Utilisation d'un composé de formule I telle que représentée dans la revendication 4 pour la production d'un médicament pour le traitement de maladies dans lesquelles l'interaction entre l'αᵥβ₃-intégrine et son ligand naturel est augmentée ou abaissée, à savoir l'athérosclérose, la polyarthrite rhumatoïde, la resténose après une lésion vasculaire ou une implantation d'extenseur, l'angioplastie, l'insuffisance rénale aiguë, les microangiopathies associées à une angiogenèse, les angiopathies diabétiques, l'obturation vasculaire à médiation par les plaquettes sanguines, la thrombose artérielle, l'insuffisance cardiaque congestive, l'infarctus du myocarde, l'apoplexie, le cancer, l'ostéoporose, l'hypertension sanguine, le psoriasis ou les maladies virales, parasitaires ou bactériennes, les inflammations, la cicatrisation des plaies, l'hyperparathyroïdie, la maladie de Paget, l'hypercalcémie maligne ou les lésions ostéolytiques métastatiques.

6. Préparation médicamenteuse contenant au moins un composé tel que représenté dans la revendication 4, éventuellement des adjuvants de médicaments et au moins un autre composé choisi dans le groupe des inhibiteurs de l'adhésion, de l'activation ou de l'agrégation des plaquettes sanguines, des anticoagulants qui empêchent l'activité de la thrombine ou la formation de la thrombine, les antagonistes des composés activant les plaquettes sanguines ou des antagonistes de sélectine.

7. Utilisation de la préparation médicamenteuse selon la revendication 6 pour la production d'un médicament pour le traitement de l'obturation vasculaire à médiation par les plaquettes sanguines ou de la thrombose.

8. Préparation médicamenteuse contenant au moins un composé tel que représenté dans la revendication 4, éventuellement des adjuvants de médicaments et au moins un autre composé choisi dans le groupe des inhibiteurs de l'activation ou de l'agrégation des plaquettes sanguines, des inhibiteurs de sérine-protéases, des composés abaissant le fibrinogène, des antagonistes de sélectine, des antagonistes de ICAM-1 ou VCAM-1, des inhibiteurs de l'adhésion des leucocytes, des inhibiteurs de la diapédèse dans les parois vasculaires, des composés modulant la fibrinolyse, des inhibiteurs de facteurs du complément, des antagonistes de récepteurs d'endothéline, des inhibiteurs de thyrosine-kinase, des antioxydants ou des antagonistes d'interleukine 8.

9. Utilisation de la préparation médicamenteuse selon la revendication 8 pour la production d'un médicament pour le traitement de l'infarctus du myocarde ou de l'apoplexie.

10. Préparation médicamenteuse contenant au moins un composé tel que représenté dans la revendication 4, éventuellement des adjuvants de médicaments et au moins un autre composé choisi dans le groupe des antagonistes d'endothéline, des inhibiteurs d'ACE, des antagonistes de récepteurs d'angiotensine, des inhibiteurs d'endopeptidase, des bêta-bloquants, des antagonistes de canal calcique, des inhibiteurs de phosphodiestérase ou des inhibiteurs de caspase.

11. Utilisation de la préparation médicamenteuse selon la revendication 10 pour la production d'un médicament pour le traitement de l'insuffisance cardiaque congestive.

12. Préparation médicamenteuse contenant au moins un composé tel que représenté dans la revendication 4, éventuellement des adjuvants de médicaments et au moins un autre composé choisi dans le groupe des inhibiteurs de thrombine, des inhibiteurs du facteur Xa, des inhibiteurs de la voie de coagulation qui conduit à la formation de thrombine, des inhibiteurs de l'adhésion, de l'activation ou de l'agrégation des plaquettes sanguines, des antagonistes de récepteurs d'endothéline, des inhibiteurs d'oxyde d'azote synthase, des antagonistes de CD44, des antagonistes de sélectine, des antagonistes de MCP-1, des inhibiteurs de la transduction de signaux dans les cellules qui prolifèrent, des antagonistes de la réponse cellulaire à médiation par EGF, PDGF, VEGF ou bFGF ou des antioxydants.

13. Utilisation de la préparation médicamenteuse selon la revendication 12 pour la production d'un médicament pour le traitement de la resténose après une lésion vasculaire ou une implantation d'extenseur.

14. Préparation médicamenteuse contenant au moins un composé tel que représenté dans la revendication 4, éventuellement des adjuvants de médicaments et au moins un autre composé choisi dans le groupe des antagonistes de la réponse cellulaire à médiation par EGF, PDGF, VEGF ou bFGF, de l'héparine ou des héparines de faible masse moléculaire ou d'autres GAG, des inhibiteurs de MMP, des antagonistes de sélectine, des antagonistes d'endothéline, des inhibiteurs d'ACE, des antagonistes de récepteurs d'angiotensine, des inhibiteurs de glycosylation ou des inhibiteurs de formation d'AGE ou des AGE-breaker et des antagonistes de leurs récepteurs.

15. Utilisation de la préparation médicamenteuse selon la revendication 14 pour la production d'un médicament pour le traitement des angiopathies diabétiques.

16. Préparation médicamenteuse contenant au moins un composé tel que représenté dans la revendication 4, éventuellement des adjuvants de médicaments et au moins un autre composé choisi dans le groupe des composés abaissant les graisses, des antagonistes de sélectine, des antagonistes de ICAM-1 ou VCAM-1, de l'héparine ou des héparines de faible masse moléculaire ou d'autres GAG, des inhibiteurs de MMP, des antagonistes d'endothéline, des antagonistes d'apolipoprotéine A1, des antagonistes du cholestérol, des inhibiteurs de HMG CoA réductase, des inhibiteurs d'ACAT, des inhibiteurs d'ACE, des antagonistes de récepteurs d'angiotensine, des inhibiteurs de tyrosinekinase, des inhibiteurs de protéinekinase C, des antagonistes de canal calcique, des stimulants de la fonction des récepteurs de LDL, des antioxydants, des mimétiques de LCAT ou des piégeurs de radicaux libres.

17. Utilisation de la préparation médicamenteuse selon la revendication 16 pour la production d'un médicament pour le traitement de l'athérosclérose.

18. Préparation médicamenteuse contenant au moins un composé tel que représenté dans la revendication 4, éventuellement des adjuvants de médicaments et au moins un autre composé choisi dans le groupe des composés cytostatiques ou antinéoplasiques, des composés qui inhibent la prolifération ou de l'héparine ou des héparines de faible masse moléculaire ou d'autres GAG.

19. Utilisation de la préparation médicamenteuse selon la revendication 18 pour la production d'un médicament pour le traitement du cancer.

20. Préparation médicamenteuse contenant au moins un composé tel que représenté dans la revendication 4, éventuellement des adjuvants de médicaments et au moins un autre composé choisi dans le groupe des composés pour la thérapie anti-résorptive, des composés pour la thérapie d'échange hormonal, de l'hormone de croissance humaine recombinée, des bisphosphonates, des composés pour la thérapie à la calcitonine, des stimulants de la calcitonine, des antagonistes de canal calcique, des stimulants de la formation osseuse, des antagonistes de l'interleukine-6 ou des inhibiteurs de Src tyrosinekinase.

21. Utilisation de la préparation médicamenteuse selon la revendication 20 pour la production d'un médicament pour le traitement de l'ostéoporose.

22. Préparation médicamenteuse contenant au moins un composé tel que représenté dans la revendication 4, éventuellement des adjuvants de médicaments et au moins un autre composé choisi dans le groupe des antagonistes de TNF, des antagonistes de VLA-4 ou VCAM-1, des antagonistes de LFA-1, Mac-1 ou des ICAM, des inhibiteurs du complément, des immunosuppressifs, des antagonistes d'interleukine-1, -5 ou -8 ou des inhibiteurs de dihydrofolate réductase.

23. Utilisation de la préparation médicamenteuse selon la revendication 22 pour la production d'un médicament pour le traitement de la polyarthrite rhumatoïde.

24. Préparation médicamenteuse contenant au moins un composé tel que représenté dans la revendication 4, éventuellement des adjuvants de médicaments et au moins un autre composé choisi dans le groupe de la collagénase, des antagonistes de PDGF ou des MMP.

25. Utilisation de la préparation médicamenteuse selon la revendication 24 pour la production d'un médicament pour améliorer la cicatrisation des plaies.
